# EUROPEAN PATENT SPECIFICATION

(11) **EP 3 189 142 B1**
(45) Date of publication and mention of the grant of the patent: **15.07.2020**
(21) Application number: 15757508.5
(22) Date of filing: 03.09.2015
(51) Int. Cl.: C12N 15/113, A61K 31/7088, A61K 31/712, A61K 31/7125

(54) **ANTISENSE OLIGONUCLEOTIDES FOR THE TREATMENT OF LEBER CONGENITAL AMAUROSIS**
ANTISENSE-OLIGONUKLEOTIDE ZUR BEHANDLUNG VON LEBERSCHER KONGENITALER AMAUROSE
OLIGONUCLÉOTIDES ANTISENS POUR LE TRAITEMENT DE L'AMAUROSE CONGÉNITALE DE LEBER

(30) Priority: 05.09.2014 US 201414479229
(43) Date of publication of application: 12.07.2017
(73) Proprietor: Stichting Katholieke Universiteit, 6525 GA Nijmegen (NL)
(72) Inventor: COLLIN, Robert Wilhelmus Johanna, NL-5913 VP Venlo (NL); CREMERS, Franciscus Peter Maria, NL-6581 DW Malden (NL); DEN HOLLANDER, Antonia Ingrid, NL-6562 DD Groesbeek (NL)
(74) Representative: Vossius & Partner Patentanwälte Rechtsanwälte mbB
(86) International application number: PCT/EP2015/070172
(87) International publication number: WO 2016/034680

(56) References cited:
- WO-A1-2012/168435
- WO-A1-2013/036105
- ROB WJ COLLIN ET AL.: "Antisense Oligonucleotide (AON)-based Therapy for Leber Congenital Amaurosis Caused by a Frequent Mutation in CEP290", MOLECULAR THERAPY - NUCLEIC ACIDS, vol. 1, no. 3, 1 March 2012 (2012-03-01), page e14, XP055088070, DOI: 10.1038/mtna.2012.3
- GARANTO A. ET AL.: "AAV-Mediated Antisense Oligonucleotide-Based Therapy for CEP290-Associated LCA", MOLECULAR THERAPY, vol. 22, no. Suppl. 1, May 2014 (2014-05), pages S46-S47, XP002750374, 17TH ANNUAL MEETING OF THE AMERICAN-SOCIETY-OF-GENE-AND-CELL-THERAPY (ASGCT); WASHINGTON, DC, USA; MAY 21 -24, 2014
- XAVIER GERARD ET AL.: "AON-mediated Exon Skipping Restores Ciliation in Fibroblasts Harboring the Common Leber Congenital Amaurosis CEP290 Mutation", MOLECULAR THERAPY - NUCLEIC ACIDS, vol. 1, no. 6, 1 June 2012 (2012-06-01), page e29, XP55088066, ISSN: 2162-2531, DOI: 10.1038/mtna.2012.21 -& Xavier Gerard ET AL.: "Supplementary Materials and Methods", , 26 June 2012 (2012-06-26), XP055225774, Retrieved from the Internet: URL:http://www.nature.com/mtna/journal/v1/ n6/extref/mtna201221x6.doc [retrieved on 2015-11-04] -& Xavier Gerard et al.: "Supplementary information", , 26 June 2012 (2012-06-26), XP002750375, Retrieved from the Internet: URL:http://www.nature.com/mtna/journal/v1/ n6/suppinfo/mtna201221s1.html?url=/mtna/jo urnal/v1/n6/full/mtna201221a.html [retrieved on 2015-11-04]
- XAVIER GÉRARD ET AL.: "Intravitreal Injection of Splice-switching Oligonucleotides to Manipulate Splicing in Retinal Cells", MOLECULAR THERAPY-NUCLEIC ACIDS, vol. 4, no. 9, 1 September 2015 (2015-09-01), page e250, XP055225550, DOI: 10.1038/mtna.2015.24 -& Xavier Gérard ET AL.: "Supplementary Material", , 1 September 2015 (2015-09-01), XP055225780, Retrieved from the Internet: URL:http://www.nature.com/mtna/journal/v4/ n9/extref/mtna201524x1.doc [retrieved on 2015-11-04]

## Description

### BACKGROUND

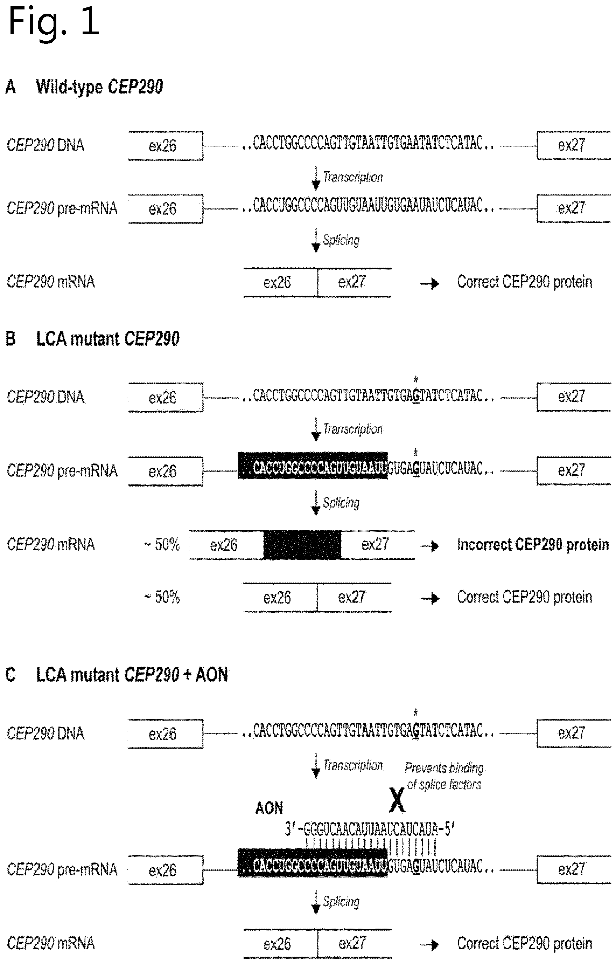

Leber congenital amaurosis (LCA) is the most severe form of inherited retinal dystrophy, with an onset of disease symptoms in the first years of life (Leber, T., 1869) and an estimated prevalence of approximately 1 in 50,000 worldwide (Koenekoop et al, 2007; Stone, 2007). Genetically, LCA is a heterogeneous disease, with fifteen genes identified to date in which mutations are causative for LCA (den Hollander et al, 2008; Estrada-Cuzcano et al, 2011). The most frequently mutated LCA gene is *CEP290,* accounting for ∼15% of all cases (Stone, 2007; den Hollander, 2008; den Hollander, 2006; Perrault et al, 2007). Severe mutations in *CEP290* have been reported to cause a spectrum of systemic diseases that, besides retinal dystrophy, are characterized by brain defects, kidney malformations, polydactyly and/or obesity (Baal et al, 2007; den Hollander et al, 2008; Helou et al, 2007; Valente et al, 2006). There is no clear-cut genotype-phenotype correlation between the combination of *CEP290* mutations and the associated phenotypes, but patients with LCA and early-onset retinal dystrophy very often carry hypomorphic alleles (Stone, 2007; den Hollander et al, 2006; Perrault et al, 2007; Coppieters et al, 2010; Liitink et al 2010). The by far most frequently occurring hypomorphic *CEP290* mutation, especially in European countries and in the US, is a change in intron 26 of *CEP290* (c.2991+1655A>G) (Stone, 2007; den Hollander et al, 2006; Perrault et al, 2007; Liitink et al, 2010). This mutation creates a cryptic splice donor site in intron 26 which results in the inclusion of an aberrant exon of 128 bp in the mutant *CEP290* mRNA, and inserts a premature stop codon (p.C998X) (see figure 1A and 1B). Besides the mutant *CEP290* mRNA, also the wild-type transcript that lacks the aberrant exon is still produced, explaining the hypomorphic nature of this mutation (Estrada-Cuzcano et al, 2011).
LCA, and other retinal dystrophies, for long have been considered incurable diseases. However, the first phase I/II clinical trials using gene augmentation therapy have lead to promising results in a selected group of adult LCA/RP patients with mutations in the *RPE65* gene (Bainbridge et al, 2008; Cideciyan et al, 2008; Hauswirth et al, 2008; Maguire et al, 2008). Unilateral subretinal injections of adeno-associated viruses particles carrying constructs encoding the wild-type *RPE65* cDNA were shown to be safe and moderately effective in some patients, without causing any adverse effects. In a follow-up study using adults and children, visual improvements were more sustained, especially in the children who all gained ambulatory vision (Maguire et al, 2009). Together, these studies have shown the potential to treat LCA, and thereby enormously boosted the development of therapeutic strategies for other genetic subtypes of retinal dystrophies (den Hollander et al, 2010). However, due to the tremendous variety in gene size, and technical limitations of the vehicles that are used to deliver therapeutic constructs, gene augmentation therapy may not be applicable to all genes. The *RPE65* cDNA is for instance only 1.6 kb, whereas the *CEP290* cDNA amounts to about 7.4kb, thereby exceeding the cargo size of many available vectors, including the presently used adeno-associated vectors (AAV). In addition, using gene replacement therapy, it is hard to control the expression levels of the therapeutic gene which for some genes need to be tightly regulated. It is therefore an objective of the present invention to provide a convenient therapeutic strategy for the prevention, treatment or delay of Leber congenital amaurosis as caused by an intronic mutation in *CEP290.*

### SUMMARY

The invention relates to the embodiments as defined in the claims. In particular the invention relates to a viral vector expressing an exon skipping antisense oligonucleotide that binds to and/or is complementary to a polynucleotide with the nucleotide sequence as shown in SEQ ID NO: 17, wherein said oligonucleotide consists of a sequence selected from the group consisting of: SEQ ID NO: 20, SEQ ID NO: 21, SEQ ID NO: 22 and SEQ ID NO: 23, when placed under conditions conducive to expression of the exon skipping antisense oligonucleotide. The invention also relates to a pharmaceutical composition comprising the viral vectors of the invention and a pharmaceutically acceptable excipient, wherein the viral vector is an AAV vector. The invention further relates to such viral vectors for the treatment of a *CEP290* related diseas or condition requiring modulating splicing of *CEP290,* as well as *in vitro* methods for modulating splicing of *CEP290* in a cell, said method comprising contacting said cell with the viral vectors of the invention.

### DETAILED DESCRIPTION

Surprisingly, it has now been demonstrated that specific antisense oligonucleotides (AONs) are able to block the aberrant splicing of *CEP290* that is caused by the intronic LCA mutation.

Accordingly, disclosed herein is an exon skipping molecule that binds to and/or is complementary to a polynucleotide with the nucleotide sequence as shown in SEQ ID NO: 6, preferably SEQ ID NO: 17, more preferably SEQ ID NO: 8, even more preferably SEQ ID NO: 7, or a part thereof.

As used herein, the terms "*modulating splicing*" and "*exon skipping*" are synonymous. In respect of *CEP290,* "*modulating splicing*" or "*exon skipping*" are to be construed as the exclusion of the aberrant 128 nucleotide exon (SEQ ID NO: 4) from the *CEP290* mRNA (see figure 1A and 1B). The term *exon skipping* is herein defined as the induction within a cell of a mature mRNA that does not contain a particular exon that would be present in the mature mRNA without exon skipping. Exon skipping is achieved by providing a cell expressing the pre-mRNA of said mature mRNA with a molecule capable of interfering with sequences such as, for example, the (cryptic) splice donor or (cryptic) splice acceptor sequence required for allowing the enzymatic process of splicing, or with a molecule that is capable of interfering with an exon inclusion signal required for recognition of a stretch of nucleotides as an exon to be included in the mature mRNA; such molecules are herein referred to as exon skipping molecules The term *pre-mRNA* refers to a non-processed or partly processed precursor mRNA that is synthesized from a DNA template in the nucleus of a cell by transcription.

The term "*antisense oligonucleotide*" is understood to refer to a nucleotide sequence which is substantially complementary to a target nucleotide sequence in a pre-mRNA molecule, hrRNA (heterogenous nuclear RNA) or mRNA molecule. The degree of complementarity (or substantial complementarity) of the antisense sequence is preferably such that a molecule comprising the antisense sequence can form a stable hybrid with the target nucleotide sequence in the RNA molecule under physiological conditions.

The terms "*antisense oligonucleotide*" and "*oligonucleotide*" are used interchangeably herein and are understood to refer to an oligonucleotide comprising an antisense sequence.

An exon skipping molecule as defined herein can be a compound molecule that binds and/or is complementary to the specified sequence, or a protein such as an RNA-binding protein or a non-natural zinc-finger protein that has been modified to be able to bind to the indicated nucleotide sequence on a RNA molecule. Methods for screening compound molecules that bind specific nucleotide sequences are, for example, disclosed in PCT/NL01/00697 and US Patent 6,875,736. Methods for designing RNA-binding Zinc-finger proteins that bind specific nucleotide sequences are disclosed by Friesen and Darby, Nature Structural Biology 5: 543- 546 (1998). Binding to one of the specified SEQ ID NO: 6, 7, 8 or 17 sequence, preferably in the context of the aberrant 128 nucleotide *CEP290* exon (SEQ ID NO: 4) may be assessed via techniques known to the skilled person. A preferred technique is gel mobility shift assay as described in EP 1 619 249. In a preferred embodiment, an exon skipping molecule is said to bind to one of the specified sequences as soon as a binding of said molecule to a labeled sequence SEQ ID NO: 6, 7, 8 or 17 is detectable in a gel mobility shift assay.

An exon skipping molecule as described herein is preferably a nucleic acid molecule, preferably an oligonucleotide. Preferably, an exon skipping molecule is a nucleic acid molecule, preferably an oligonucleotide, which is complementary or substantially complementary to a nucleotide sequence as shown in SEQ ID NO: 6, preferably SEQ ID NO: 17, more preferably SEQ ID NO: 8, even more preferably SEQ ID NO: 7, or a part thereof as later defined herein.

The term "*substantially complementary*" used herein indicates that some mismatches in the antisense sequence are allowed as long as the functionality, i.e. inducing skipping of the aberrant 128 nucleotide *CEP290* exon (SEQ ID NO: 4), is still acceptable. Preferably, the complementarity is from 90% to 100%. In general this allows for 1 or 2 mismatch(es) in an oligonucleotide of 20 nucleotides or 1, 2, 3 or 4 mismatches in an oligonucleotide of 40 nucleotides, or 1, 2, 3, 4, 5 or 6 mismatches in an oligonucleotide of 60 nucleotides, etc.

Also disclosed herein is a method for designing an exon skipping molecule, preferably an oligonucleotide able to induce skipping of the aberrant 128 nucleotide *CEP290* exon (SEQ ID NO: 4). First, said oligonucleotide is selected to bind to one of SEQ ID NO: 6, 7, 8 or 17, or a part thereof as defined later herein. Subsequently, in a preferred method at least one of the following aspects has to be taken into account for designing, improving said exon skipping molecule any further:
- The exon skipping molecule preferably does not contain a CpG or a stretch of CpG,
- The exon skipping molecule has acceptable RNA binding kinetics and/or thermodynamic properties.

The presence of a CpG or a stretch of CpG in an oligonucleotide is usually associated with an increased immunogenicity of said oligonucleotide (Dorn and Kippenberger, 2008). This increased immunogenicity is undesired since it may induce damage of the tissue to be treated, i.e. the eye. Immunogenicity may be assessed in an animal model by assessing the presence of CD4+ and/or CD8+ cells and/or inflammatory mononucleocyte infiltration. Immunogenicity may also be assessed in blood of an animal or of a human being treated with an oligonucleotide by detecting the presence of a neutralizing antibody and/or an antibody recognizing said oligonucleotide using a standard immunoassay known to the skilled person.

An increase in immunogenicity may be assessed by detecting the presence or an increasing amount of a neutralizing antibody or an antibody recognizing said oligonucleotide using a standard immunoassay.

Also provided are methods for designing an oligonucleotide with acceptable RNA binding kinetics and/or thermodynamic properties. The RNA binding kinetics and/or thermodynamic properties are at least in part determined by the melting temperature of an oligonucleotide (Tm; calculated with the oligonucleotide properties calculator (www.unc.edu/∼cail/biotool/oligo/index.html) for single stranded RNA using the basic Tm and the nearest neighbor model), and/or the free energy of the AON-target exon complex (using RNA structure version 4.5). If a Tm is too high, the oligonucleotide is expected to be less specific. An acceptable Tm and free energy depend on the sequence of the oligonucleotide. Therefore, it is difficult to give preferred ranges for each of these parameters. An acceptable Tm may be ranged between 35 and 70°C and an acceptable free energy may be ranged between 15 and 45 kcal/mol.

The skilled person may therefore first choose an oligonucleotide as a potential therapeutic compound as binding and/or being complementary to SEQ ID NO: 6, 7, 8 or 17, or a part thereof as defined later herein. The skilled person may check that said oligonucleotide is able to bind to said sequences as earlier defined herein. Optionally in a second step, he may further optimize said oligonucleotide by checking for the absence of CpG and/or by optimizing its Tm and/or free energy of the AON-target complex. He may try to design an oligonucleotide wherein preferably no CpG and/or wherein a more acceptable Tm and/or free energy are obtained by choosing a distinct sequence of *CEP290* (including SEQ ID NO: 6, 7, 8 or 17) to which the oligonucleotide is complementary. Alternatively, if an oligonucleotide complementary to a given stretch within SEQ ID NO: 6, 7, 8 or 17, comprises a CpG, and/or does not have an acceptable Tm and/or free energy, the skilled person may improve any of these parameters by decreasing the length of the oligonucleotide, and/or by choosing a distinct stretch within any of SEQ ID NO: 6, 7, 8 or 17 to which the oligonucleotide is complementary and/or by altering the chemistry of the oligonucleotide.

At any step of the method, the oligonucleotide is preferably an oligonucleotide able to exhibit an acceptable level of functional activity. A functional activity of said oligonucleotide is preferably to induce the skipping of the aberrant 128 nucleotide *CEP290* exon (SEQ ID NO: 4) to a certain extent, to provide an individual with a functional *CEP290* protein and/or mRNA and/or at least in part decreasing the production of an aberrant *CEP290* protein and/or mRNA. In a preferred embodiment, an oligonucleotide is said to induce skipping of the aberrant 128 nucleotide *CEP290* exon (SEQ ID NO: 4), when the aberrant 128 nucleotide *CEP290* exon (SEQ ID NO: 4) skipping percentage as measured by real-time quantitative RT-PCR analysis (is at least 30%, or at least 35%, or at least 40%, or at least 45%, or at least 50%, or at least 55%, or at least 60%, or at least 65%, or at least 70%, or at least 75%, or at least 80%, or at least 85%, or at least 90%, or at least 95%, or 100%.

Preferably, a nucleic acid molecule as described herein, preferably an oligonucleotide, which comprises a sequence that is complementary or substantially complementary to a nucleotide sequence as shown in SEQ ID NO: 6, preferably SEQ ID NO: 17, more preferably SEQ ID NO: 8, even more preferably SEQ ID NO: 7, or part thereof of *CEP290* is such that the (substantially) complementary part is at least 50% of the length of the oligonucleotide , more preferably at least 60%, even more preferably at least 70%, even more preferably at least 80%, even more preferably at least 90% or even more preferably at least 95%, or even more preferably 98% or even more preferably at least 99%, or even more preferably 100%. Preferably, the oligonucleotide comprises or consists of a sequence that is complementary to part of SEQ ID NO: 6, 7, 8 or 17. As an example, an oligonucleotide may comprise a sequence that is complementary to part of SEQ ID NO: 6, 7, 8 or 17 and additional flanking sequences. More preferably, the length of said complementary part of said oligonucleotide is of at least 8, 9, 10, 11, 12, 13, 14, 15, 16, 17 , 18, 19, 20, 21, 22, 23, 24, 25, 26, 27 , 28 , 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 67, 68, 69, 70, 75, 80, 85, 90, 95, 100, 110, 115, 120, 125, 130, 135, 140, 141, 142 or 143 nucleotides. Additional flanking sequences may be used to modify the binding of a protein to the oligonucleotide, or to modify a thermodynamic property of the oligonucleotide, more preferably to modify target RNA binding affinity.

It is thus not absolutely required that all the bases in the region of complementarity are capable of pairing with bases in the opposing strand. For instance, when designing the oligonucleotide one may want to incorporate for instance a residue that does not base pair with the base on the complementary strand. Mismatches may, to some extent, be allowed, if under the circumstances in the cell, the stretch of nucleotides is sufficiently capable of hybridizing to the complementary part. In this context, "*sufficiently*" preferably means that using a gel mobility shift assay as described in example 1 of EP1619249, binding of an oligonucleotide is detectable. Optionally, said oligonucleotide may further be tested by transfection into retina cells of patients. Skipping of a targeted exon may be assessed by RT-PCR (as described in EP1619249). The complementary regions are preferably designed such that, when combined, they are specific for the exon in the pre-mRNA. Such specificity may be created with various lengths of complementary regions as this depends on the actual sequences in other (pre-)mRNA molecules in the system. The risk that the oligonucleotide also will be able to hybridize to one or more other pre-mRNA molecules decreases with increasing size of the oligonucleotide. It is clear that oligonucleotides comprising mismatches in the region of complementarity but that retain the capacity to hybridize and/or bind to the targeted region(s) in the pre-mRNA, can be used in the methods described herein. However, preferably at least the complementary parts do not comprise such mismatches as these typically have a higher efficiency and a higher specificity, than oligonucleotides having such mismatches in one or more complementary regions. It is thought, that higher hybridization strengths, (i.e. increasing number of interactions with the opposing strand) are favorable in increasing the efficiency of the process of interfering with the splicing machinery of the system. Preferably, the complementarity is from 90% to 100%. In general this allows for 1 or 2 mismatch(es) in an oligonucleotide of 20 nucleotides or 1, 2, 3 or 4 mismatches in an oligonucleotide of 40 nucleotides, or 1, 2, 3, 4, 5 or 6 mismatches in an oligonucleotide of 60 nucleotides, etc.

An exon skipping molecule is preferably a nucleic acid molecule or nucleotide-based molecule, preferably an (antisense) oligonucleotide, which is complementary to a sequence selected from SEQ ID NO: 6, 7, 8 and 17.

A preferred exon skipping molecule is a nucleic acid molecule comprising an antisense oligonucleotide which antisense oligonucleotide has a length from about 8 to about 143 nucleotides, more preferred from about 8 to 60, more preferred from about 10 to 50 nucleotides, more preferred from about 10 to about 40 nucleotides, more preferred from about 12 to about 30 nucleotides, more preferred from about 14 to about 28 nucleotides, nucleotides, most preferred about 20 nucleotides, such as 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 44 nucleotide or 45 nucleotides.

A preferred exon skipping molecule is an antisense oligonucleotide comprising or consisting of from 8 to 143 nucleotides, more preferred from about 10 to 50 nucleotides, more preferred from about 10 to 40 nucleotides, more preferred from 12 to 30 nucleotides, more preferred from 14 to 20 nucleotides, or preferably comprises or consists of 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 67, 68, 69, 70, 75, 80, 85, 90, 95, 100, 110, 115, 120, 125, 130, 135, 140, 141, 142 or 143 nucleotides.

When an exon skipping molecule comprises or consists of an antisense oligonucleotide that binds to or is complementary to at least the part of SEQ ID NO: 6 that comprises the c.2991+1655A>G mutation, said exon skipping molecule preferably comprises an "C" nucleotide on the position complementary to the mutated "G" nucleotide in SEQ ID NO: 6.

Also disclosed herein is an exon skipping molecule comprising or preferably consisting of an antisense oligonucleotide selected from the group consisting of: SEQ ID NO: 10, SEQ ID NO: 11, SEQ ID NO: 12, SEQ ID NO: 20, SEQ ID NO: 21, SEQ ID NO: 22, SEQ ID NO: 23 and SEQ ID NO: 24.

A more preferred exon skipping molecule disclosed herein comprises or preferably consists of the antisense oligonucleotide SEQ ID NO: 10. It was found that this molecule is very efficient in modulating splicing of the aberrant 128 nucleotide *CEP290* exon. This preferred exon skipping molecule comprising SEQ ID NO: 10 preferably comprises from 16 to 143 nucleotides, more preferred from 16 to 40 nucleotides, more preferred from 16 to 30 nucleotides, more preferred from 16 to 20 nucleotides, or preferably comprises or consists of 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 11. It was found that this molecule is very efficient in modulating splicing of the aberrant 128 nucleotide *CEP290* exon. This preferred exon skipping molecule comprising SEQ ID NO: 11 preferably comprises from 17 to 143 nucleotides, more preferred from 17 to 40 nucleotides, more preferred from 17 to 30 nucleotides, more preferred from 17 to 20 nucleotides, or preferably comprises or consists of 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 12. It was found that this molecule is very efficient in modulating splicing of the aberrant 128 nucleotide *CEP290* exon. This preferred exon skipping molecule comprising SEQ ID NO: 12 preferably comprises from 18 to 143 nucleotides, more preferred from 18 to 40 nucleotides, more preferred from 18 to 30 nucleotides, more preferred from 18 to 20 nucleotides, or preferably comprises or consists of 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 20. It was found that this molecule is very efficient in modulating splicing of the aberrant 128 nucleotide *CEP290* exon. This preferred exon skipping molecule comprising SEQ ID NO: 20 preferably comprises from 44 to 143 nucleotides, more preferably from 44 to 60 nucleotides, more preferably from 44 to 50 nucleotides, preferably comprises or consists of 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 21. It was found that this molecule is very efficient in modulating splicing of the aberrant 128 nucleotide *CEP290* exon. This preferred exon skipping molecule comprising SEQ ID NO: 21 preferably comprises from 8 to 143 nucleotides, more preferably from 45 to 60 nucleotides, more preferably from 45 to 50 nucleotides, or preferably comprises or consists of 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 22. It was found that this molecule is very efficient in modulating splicing of the aberrant 128 nucleotide *CEP290* exon. This preferred exon skipping molecule comprising SEQ ID NO: 22 preferably comprises from 21 to 143 nucleotides, more preferably from 21 to 40 nucleotides, more preferably from 21 to 30 nucleotides, more preferably from 21 to 25 nucleotides, or preferably comprises or consists of 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 23. It was found that this molecule is very efficient in modulating splicing of the aberrant 128 nucleotide *CEP290* exon. This preferred exon skipping molecule comprising SEQ ID NO: 23 preferably comprises from 44 to 143 nucleotides, more preferably from 44 to 60 nucleotides, more preferably from 44 to 50 nucleotides, or preferably comprises or consists of 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

Another more preferred exon skipping molecule comprises or preferably consists of the antisense oligonucleotide SEQ ID NO: 24. It was found that this molecule is very efficient in modulating splicing of the aberrant 128 nucleotide *CEP290* exon. This preferred exon skipping molecule comprising SEQ ID NO: 24 preferably comprises from 23 to 143 nucleotides, more preferably from 23 to 40 nucleotides, more preferably from 23 to 30 nucleotides, more preferably from 23 to 25 nucleotides, or preferably comprises or consists of 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 65, 66, 67, 68, 69, 70, 75, 80, 85, 90, 95, 100, 110, 115, 120, 125, 130, 135, 140, 141, 142 or 143 nucleotides.

An exon skipping molecule as described herein may contain one of more RNA residues (consequently a "t" residue will be a "u" residue as RNA counterpart), or one or more DNA residues, and/or one or more nucleotide analogues or equivalents, as will be further detailed herein below.

It is preferred that an exon skipping molecule as disclosed herein comprises one or more residues that are modified to increase nuclease resistance, and/or to increase the affinity of the antisense oligonucleotide for the target sequence. Therefore, the antisense nucleotide sequence preferably comprises at least one nucleotide analogue or equivalent, wherein a nucleotide analogue or equivalent is defined as a residue having a modified base, and/or a modified backbone, and/or a non-natural internucleoside linkage, or a combination of these modifications.

The nucleotide analogue or equivalent preferably comprises a modified backbone. Examples of such backbones are provided by morpholino backbones, carbamate backbones, siloxane backbones, sulfide, sulfoxide and sulfone backbones, formacetyl and thioformacetyl backbones, methyleneformacetyl backbones, riboacetyl backbones, alkene containing backbones, sulfamate, sulfonate and sulfonamide backbones, methyleneimino and methylenehydrazino backbones, and amide backbones. Phosphorodiamidate morpholino oligomers are modified backbone oligonucleotides that have previously been investigated as antisense agents. Morpholino oligonucleotides have an uncharged backbone in which the deoxyribose sugar of DNA is replaced by a six membered ring and the phosphodiester linkage is replaced by a phosphorodiamidate linkage. Morpholino oligonucleotides are resistant to enzymatic degradation and appear to function as antisense agents by arresting translation or interfering with pre-mRNA splicing rather than by activating RNase H. Morpholino oligonucleotides have been successfully delivered to tissue culture cells by methods that physically disrupt the cell membrane, and one study comparing several of these methods found that scrape loading was the most efficient method of delivery; however, because the morpholino backbone is uncharged, cationic lipids are not effective mediators of morpholino oligonucleotide uptake in cells. A recent report demonstrated triplex formation by a morpholino oligonucleotide and, because of the non-ionic backbone, these studies showed that the morpholino oligonucleotide was capable of triplex formation in the absence of magnesium.

It is further preferred that the linkage between the residues in a backbone do not include a phosphorus atom, such as a linkage that is formed by short chain alkyl or cycloalkyl internucleoside linkages, mixed heteroatom and alkyl or cycloalkyl internucleoside linkages, or one or more short chain heteroatomic or heterocyclic internucleoside linkages.

A preferred nucleotide analogue or equivalent comprises a Peptide Nucleic Acid (PNA), having a modified polyamide backbone (Nielsen, et al. (1991) Science 254, 1497-1500). PNA-based molecules are true mimics of DNA molecules in terms of base-pair recognition. The backbone of the PNA is composed of N-(2-aminoethyl)-glycine units linked by peptide bonds, wherein the nucleobases are linked to the backbone by methylene carbonyl bonds. An alternative backbone comprises a one-carbon extended pyrrolidine PNA monomer (Govindaraju and Kumar (2005) Chem. Commun, 495-497). Since the backbone of a PNA molecule contains no charged phosphate groups, PNA-RNA hybrids are usually more stable than RNA-RNA or RNA-DNA hybrids, respectively (Egholm et al (1993) Nature 365, 566-568).

A further preferred backbone comprises a morpholino nucleotide analog or equivalent, in which the ribose or deoxyribose sugar is replaced by a 6-membered morpholino ring. A most preferred nucleotide analog or equivalent comprises a phosphorodiamidate morpholino oligomer (PMO), in which the ribose or deoxyribose sugar is replaced by a 6-membered morpholino ring, and the anionic phosphodiester linkage between adjacent morpholino rings is replaced by a non-ionic phosphorodiamidate linkage.

Further, a nucleotide analogue or equivalent may comprise a substitution of one of the non-bridging oxygens in the phosphodiester linkage. This modification slightly destabilizes base-pairing but adds significant resistance to nuclease degradation. A preferred nucleotide analogue or equivalent comprises phosphorothioate, chiral phosphorothioate, phosphorodithioate, phosphotriester, aminoalkylphosphotriester, H-phosphonate, methyl and other alkyl phosphonate including 3'-alkylene phosphonate, 5'-alkylene phosphonate and chiral phosphonate, phosphinate, phosphoramidate including 3'-amino phosphoramidate and aminoalkylphosphoramidate, thionophosphoramidate, thionoalkylphosphonate, thionoalkylphosphotriester, selenophosphate or boranophosphate.

A further preferred nucleotide analogue or equivalent comprises one or more sugar moieties that are mono- or disubstituted at the 2', 3' and/or 5' position such as a - OH; -F; substituted or unsubstituted, linear or branched lower (C1-C10) alkyl, alkenyl, alkynyl, alkaryl, allyl, or aralkyl, that may be interrupted by one or more heteroatoms; O-, S-, or N-alkyl; O-, S-, or N-alkenyl; O-, S-or N-alkynyl; O-, S-, or N-allyl; O-alkyl-O-alkyl, -methoxy, -aminopropoxy; methoxyethoxy; -dimethylaminooxyethoxy; and - dimethylaminoethoxyethoxy. The sugar moiety can be a pyranose or derivative thereof, or a deoxypyranose or derivative thereof, preferably ribose or derivative thereof, or deoxyribose or derivative of. A preferred derivatized sugar moiety comprises a Locked Nucleic Acid (LNA), in which the 2'-carbon atom is linked to the 3' or 4' carbon atom of the sugar ring thereby forming a bicyclic sugar moiety. A preferred LNA comprises 2'-O,4'-C-ethylene-bridged nucleic acid (Morita et al. 2001. Nucleic Acid Res Supplement No. 1: 241-242). These substitutions render the nucleotide analogue or equivalent RNase H and nuclease resistant and increase the affinity for the target RNA.

A nucleotide analogue or equivalent may also comprise one or more base modifications or substitutions. Modified bases comprise synthetic and natural bases such as inosine, xanthine, hypoxanthine and other -aza, deaza, -hydroxy, -halo, -thio, thiol, -alkyl, -alkenyl, -alkynyl, thioalkyl derivatives of pyrimidine and purine bases that are or will be known in the art.

It is understood by a skilled person that it is not necessary for all positions in an antisense oligonucleotide to be modified uniformly. In addition, more than one of the aforementioned analogues or equivalents may be incorporated in a single antisense oligonucleotide or even at a single position within an antisense oligonucleotide. The oligonucleotide as described herein may have at least two different types of analogues or equivalents.

A preferred exon skipping molecule comprises a 2'-O alkyl phosphorothioate antisense oligonucleotide, such as 2'-O-methyl modified ribose (RNA), 2'-O-ethyl modified ribose, 2'-O-propyl modified ribose, and/or substituted derivatives of these modifications such as halogenated derivatives.

An effective antisense oligonucleotide comprises a 2'-O-methyl ribose with a phosphorothioate backbone.

It will also be understood by a skilled person that different antisense oligonucleotides can be combined for efficiently skipping of the aberrant 128 nucleotide exon of *CEP290.* It is preferred that a combination of at least two antisense oligonucleotides are used in a method disclosed herein, such as two different antisense oligonucleotides, three different antisense oligonucleotides, four different antisense oligonucleotides, or five different antisense oligonucleotides.

An antisense oligonucleotide can be linked to a moiety that enhances uptake of the antisense oligonucleotide in cells, preferably retina cells. Examples of such moieties are cholesterols, carbohydrates, vitamins, biotin, lipids, phospholipids, cell-penetrating peptides including but not limited to antennapedia, TAT, transportan and positively charged amino acids such as oligoarginine, poly-arginine, oligolysine or polylysine, antigen-binding domains such as provided by an antibody, a Fab fragment of an antibody, or a single chain antigen binding domain such as a cameloid single domain antigen-binding domain.

An exon skipping molecule as described herein may be indirectly administrated using suitable means known in the art. When the exon skipping molecule is an oligonucleotide, it may for example be provided to an individual or a cell, tissue or organ of said individual in the form of an expression vector wherein the expression vector encodes a transcript comprising said oligonucleotide. The expression vector is preferably introduced into a cell, tissue, organ or individual via a gene delivery vehicle. In a preferred embodiment, there is provided a viral-based expression vector comprising an expression cassette or a transcription cassette that drives expression or transcription of an exon skipping molecule as identified herein. Accordingly, the present invention provides a viral vector expressing an exon skipping molecule when placed under conditions conducive to expression of the exon skipping molecule, specifically wherein the antisense exon skipping molecule is an antisense oligonucleotide that binds to and/or is complementary to a polynucleotide with the nucleotide sequence as shown in SEQ ID NO:17, wherein said oligonucleotide consists of a sequence selected from the group consisting of: SEQ ID NO:20, SEQ ID NO:21, SEQ ID NO:22 and SEQ ID NO:23. A cell can be provided with an exon skipping molecule capable of interfering with essential sequences that result in highly efficient skipping of the aberrant 128 nucleotide *CEP290* exon by plasmid-derived antisense oligonucleotide expression or viral expression provided by adenovirus- or adeno-associated virus-based vectors. Expression may be driven by a polymerase III promoter, such as a U1, a U6, or a U7 RNA promoter. A preferred delivery vehicle is a viral vector such as an adeno-associated virus vector (AAV), or a retroviral vector such as a lentivirus vector and the like. Also, plasmids, artificial chromosomes, plasmids usable for targeted homologous recombination and integration in the human genome of cells may be suitably applied for delivery of an oligonucleotide as defined herein. Preferred are those vectors wherein transcription is driven from PolIII promoters, and/or wherein transcripts are in the form fusions with U1 or U7 transcripts, which yield good results for delivering small transcripts. It is within the skill of the artisan to design suitable transcripts. Preferred are PolIII driven transcripts. Preferably, in the form of a fusion transcript with an Ulor U7 transcript. Such fusions may be generated as described (Gorman L et al, 1998 or Suter D et al, 1999).

The exon skipping molecule as described herein, preferably an antisense oligonucleotide, may be delivered as such. However, the exon skipping molecule may also be encoded by the viral vector. Typically, this is in the form of an RNA transcript that comprises the sequence of an oligonucleotide in a part of the transcript.

One preferred antisense oligonucleotide expression system is an adenovirus associated virus (AAV)-based vector. Single chain and double chain AAV-based vectors have been developed that can be used for prolonged expression of small antisense nucleotide sequences for highly efficient skipping of the aberrant 128 nucleotide *CEP290* exon.

A preferred AAV-based vector for instance comprises an expression cassette that is driven by a polymerase III-promoter (Pol III). A preferred Pol III promoter is, for example, a U1, a U6, or a U7 RNA promoter.

Therefore, a viral-based vector, comprising a Pol III-promoter driven expression cassette for expression of an antisense oligonucleotide for inducing skipping of aberrant 128 nucleotide *CEP290* exon is also disclosed herein.

An AAV vector is a recombinant AAV vector and refers to an AAV vector comprising part of an AAV genome comprising an encoded exon skipping molecule encapsidated in a protein shell of capsid protein derived from an AAV serotype as depicted elsewhere herein. Part of an AAV genome may contain the inverted terminal repeats (ITR) derived from an adeno-associated virus serotype, such as AAV1, AAV2, AAV3, AAV4, AAV5, AAV8, AAV9 and others. Protein shell comprised of capsid protein may be derived from an AAV serotype such as AAV1, 2, 3, 4, 5, 8, 9 and others. A protein shell may also be named a capsid protein shell. AAV vector may have one or preferably all wild type AAV genes deleted, but may still comprise functional ITR nucleic acid sequences. Functional ITR sequences are necessary for the replication, rescue and packaging of AAV virions. The ITR sequences may be wild type sequences or may have at least 80%, 85%, 90%, 95, or 100% sequence identity with wild type sequences or may be altered by for example in insertion, mutation, deletion or substitution of nucleotides, as long as they remain functional. In this context, functionality refers to the ability to direct packaging of the genome into the capsid shell and then allow for expression in the host cell to be infected or target cell. A capsid protein shell may be of a different serotype than the AAV vector genome ITR. An AAV vector may thus be composed of a capsid protein shell, *i.e.* the icosahedral capsid, which comprises capsid proteins (VP1, VP2, and/or VP3) of one AAV serotype, e.g. AAV serotype 2, whereas the ITRs sequences contained in that AAV5 vector may be any of the AAV serotypes described above, including an AAV2 vector. An "AAV2 vector" thus comprises a capsid protein shell of AAV serotype 2, while e.g. an "AAV5 vector" comprises a capsid protein shell of AAV serotype 5, whereby either may encapsidate any AAV vector genome ITR .

Preferably, a recombinant AAV vector comprises a capsid protein shell of AAV serotype 2, 5, 8 or AAV serotype 9 wherein the AAV genome or ITRs present in said AAV vector are derived from AAV serotype 2, 5, 8 or AAV serotype 9; such AAV vector is referred to as an AAV2/2, AAV 2/5, AAV2/8, AAV2/9, AAV5/2, AAV5/5, AAV5/8, AAV 5/9, AAV8/2, AAV 8/5, AAV8/8, AAV8/9, AAV9/2, AAV9/5, AAV9/8, or an AAV9/9 vector.

More preferably, a recombinant AAV vector according to the present invention comprises a capsid protein shell of AAV serotype 2 and the AAV genome or ITRs present in said vector are derived from AAV serotype 5; such vector is referred to as an AAV 2/5 vector.

More preferably, a recombinant AAV vector according to the present invention comprises a capsid protein shell of AAV serotype 2 and the AAV genome or ITRs present in said vector are derived from AAV serotype 8; such vector is referred to as an AAV 2/8 vector.

More preferably, a recombinant AAV vector according to the present invention comprises a capsid protein shell of AAV serotype 2 and the AAV genome or ITRs present in said vector are derived from AAV serotype 9; such vector is referred to as an AAV 2/9 vector.

More preferably, a recombinant AAV vector according to the present invention comprises a capsid protein shell of AAV serotype 2 and the AAV genome or ITRs present in said vector are derived from AAV serotype 2; such vector is referred to as an AAV 2/2 vector.

A nucleic acid molecule encoding an exon skipping molecule as described herein represented by a nucleic acid sequence of choice is preferably inserted between the AAV genome or ITR sequences as identified above, for example an expression construct comprising an expression regulatory element operably linked to a coding sequence and a 3' termination sequence.

"AAV helper functions" generally refers to the corresponding AAV functions required for AAV replication and packaging supplied to the AAV vector *in trans.* AAV helper functions complement the AAV functions which are missing in the AAV vector, but they lack AAV ITRs (which are provided by the AAV vector genome). AAV helper functions include the two major ORFs of AAV, namely the *rep* coding region and the *cap* coding region or functional substantially identical sequences thereof. Rep and Cap regions are well known in the art, see *e.g.* Chiorini et al. (1999, J. of Virology, Vol 73(2): 1309-1319) or US 5,139,941. The AAV helper functions can be supplied on a AAV helper construct, which may be a plasmid. Introduction of the helper construct into the host cell can occur *e.g.* by transformation, transfection, or transduction prior to or concurrently with the introduction of the AAV genome present in the AAV vector as identified herein. The AAV helper constructs as disclosed herein may thus be chosen such that they produce the desired combination of serotypes for the AAV vector's capsid protein shell on the one hand and for the AAV genome present in said AAV vector replication and packaging on the other hand.

"AAV helper virus" provides additional functions required for AAV replication and packaging. Suitable AAV helper viruses include adenoviruses, herpes simplex viruses (such as HSV types 1 and 2) and vaccinia viruses. The additional functions provided by the helper virus can also be introduced into the host cell via vectors, as described in US 6,531,456.

Preferably, an AAV genome as present in a recombinant AAV vector disclosed herein does not comprise any nucleotide sequences encoding viral proteins, such as the *rep* (replication) or *cap* (capsid) genes of AAV. An AAV genome may further comprise a marker or reporter gene, such as a gene for example encoding an antibiotic resistance gene, a fluorescent protein (*e.g. gfp*) or a gene encoding a chemically, enzymatically or otherwise detectable and/or selectable product (*e.g. lacZ, aph, etc.*) known in the art.

Preferably, an AAV vector as described hereinis constructed and produced according to the methods in Example 2 herein.

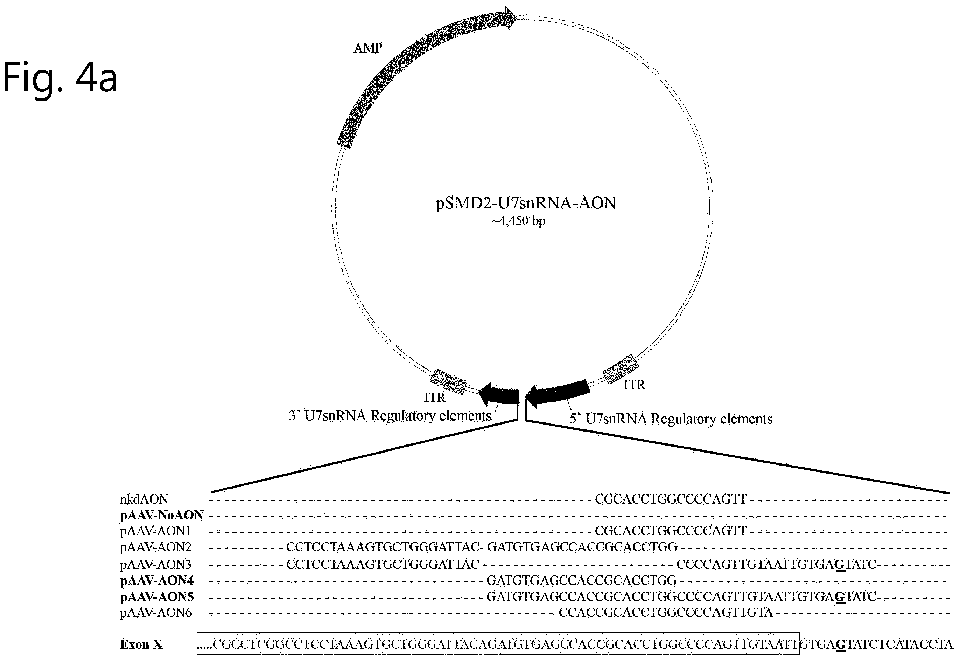

A preferred AAV vector is an AAV vector, preferably an AAV2/5, AAV2/8, AAV2/9 or AAV2/2 vector, expressing an exon skipping molecule as disclosed herein comprising an antisense oligonucleotide, wherein said antisense oligonucleotide comprises or consists of a sequence selected from the group consisting of: SEQ ID NO: 10, SEQ ID NO: 11, SEQ ID NO: 12, SEQ ID NO: 20, SEQ ID NO: 21, SEQ ID NO: 22, SEQ ID NO: 23, and SEQ ID NO: 24. More preferably, said antisense oligonucleotide comprises or consists of a sequence selected from the group consisting of: SEQ ID NO: 20, SEQ ID NO: 21, SEQ ID NO: 22, SEQ ID NO: 23, and SEQ ID NO: 24. Even more preferably, said antisense oligonucleotide comprises or consists of a sequence selected from the group consisting of: SEQ ID NO: 22, and SEQ ID NO: 23.
A further preferred AAV vector is an AAV vector, preferably an AAV2/5, AAV2/8, AAV2/9 or AAV2/2 vector, expressing an exon skipping molecule as disclosed herein comprising an antisense oligonucleotide, wherein said antisense oligonucleotide consists of a sequence selected from the group consisting of: SEQ ID NO: 10, SEQ ID NO: 11, SEQ ID NO: 12, SEQ ID NO: 20, SEQ ID NO: 21, SEQ ID NO: 22, SEQ ID NO: 23, and SEQ ID NO: 24. More preferably, said antisense oligonucleotide consists of a sequence selected from the group consisting of: SEQ ID NO: 20, SEQ ID NO: 21, SEQ ID NO: 22, SEQ ID NO: 23, and SEQ ID NO: 24. Even more preferably, said antisense oligonucleotide consists of a sequence selected from the group consisting of: SEQ ID NO: 22, and SEQ ID NO: 23.
A further preferred AAV vector is an AAV vector, preferably an AAV2/5, AAV2/8, AAV2/9 or AAV2/2 vector, expressing an exon skipping molecule as disclosed herein selected from the group consisting of: SEQ ID NO: 10, SEQ ID NO: 11, SEQ ID NO: 12, SEQ ID NO: 20, SEQ ID NO: 21, SEQ ID NO: 22, SEQ ID NO: 23, and SEQ ID NO: 24. More preferably, said exon skipping molecule consists of a sequence selected from the group consisting of: SEQ ID NO: 20, SEQ ID NO: 21, SEQ ID NO: 22, SEQ ID NO: 23, and SEQ ID NO: 24. Even more preferably, said exon skipping molecule consists of a sequence selected from the group consisting of: SEQ ID NO: 22, and SEQ ID NO: 23.
A more preferred AAV vector, preferably an AAV2/5, AAV2/8, AAV2/9 or AAV2/2 vector, is a virion corresponding to one of pAAV-AON1, pAAV-AON2, pAAV-AON3, pAAV-AON4, pAAV-AON5, and pAAV-AON6 as depicted in Figure 4a.
A further more preferred AAV vector, preferably an AAV2/5, AAV2/8, AAV2/9 or AAV2/2 vector, is a virion corresponding to one of pAAV-AON4 and pAAV-AON5, as depicted in Figure 4a.

An exon skipping molecule according to the invention can be delivered as is to an individual, a cell, tissue or organ of said individual. When administering an exon skipping molecule according to the invention, it is preferred that the molecule is dissolved in a solution that is compatible with the delivery method. Retina cells can be provided with a plasmid for antisense oligonucleotide expression by providing the plasmid in an aqueous solution. Alternatively, a plasmid can be provided by transfection using known transfection agentia. For intravenous, subcutaneous, intramuscular, intrathecal and/or intraventricular administration it is preferred that the solution is a physiological salt solution. Particularly preferred is the use of an excipient or transfection agentia that will aid in delivery of each of the constituents as defined herein to a cell and/or into a cell, preferably a retina cell. Preferred are excipients or transfection agentia capable of forming complexes, nanoparticles, micelles, vesicles and/or liposomes that deliver each constituent as defined herein, complexed or trapped in a vesicle or liposome through a cell membrane. Many of these excipients are known in the art. Suitable excipients or transfection agentia comprise polyethylenimine (PEI; ExGen500 (MBI Fermentas)), LipofectAMINE™ 2000 (Invitrogen) or derivatives thereof, or similar cationic polymers, including polypropyleneimine or polyethylenimine copolymers (PECs) and derivatives, synthetic amphiphils (SAINT-18), lipofectinTM, DOTAP and/or viral capsid proteins that are capable of self assembly into particles that can deliver each constitutent as defined herein to a cell, preferably a retina cell. Such excipients have been shown to efficiently deliver an oligonucleotide such as antisense nucleic acids to a wide variety of cultured cells, including retina cells. Their high transfection potential is combined with an excepted low to moderate toxicity in terms of overall cell survival. The ease of structural modification can be used to allow further modifications and the analysis of their further (in vivo) nucleic acid transfer characteristics and toxicity.

Lipofectin represents an example of a liposomal transfection agent. It consists of two lipid components, a cationic lipid N-[1-(2,3 dioleoyloxy)propyl]-N,N,N-trimethylammonium chloride (DOTMA) (cp. DOTAP which is the methylsulfate salt) and a neutral lipid dioleoylphosphatidylethanolamine (DOPE). The neutral component mediates the intracellular release. Another group of delivery systems are polymeric nanoparticles.

Polycations such like diethylaminoethylaminoethyl (DEAE)-dextran, which are well known as DNA transfection reagent can be combined with butylcyanoacrylate (PBCA) and hexylcyanoacrylate (PHCA) to formulate cationic nanoparticles that can deliver each constituent as defined herein, preferably an oligonucleotide, across cell membranes into cells.

In addition to these common nanoparticle materials, the cationic peptide protamine offers an alternative approach to formulate an oligonucleotide with colloids. This colloidal nanoparticle system can form so called proticles, which can be prepared by a simple self-assembly process to package and mediate intracellular release of an oligonucleotide. The skilled person may select and adapt any of the above or other commercially available alternative excipients and delivery systems to package and deliver an exon skipping molecule for use as disclosed herein to deliver it for the prevention, treatment or delay of a *CEP290* related disease or condition. "*Prevention*, *treatment or delay of a CEP290 related disease or condition"* is herein preferably defined as preventing, halting, ceasing the progression of, or reversing partial or complete visual impairment or blindness that is caused by a genetic defect in the *CEP290* gene.

In addition, an exon skipping molecule as described herein could be covalently or non-covalently linked to a targeting ligand specifically designed to facilitate the uptake into the cell, cytoplasm and/or its nucleus. Such ligand could comprise (i) a compound (including but not limited to peptide(-like) structures) recognising cell, tissue or organ specific elements facilitating cellular uptake and/or (ii) a chemical compound able to facilitate the uptake in to cells and/or the intracellular release of an oligonucleotide from vesicles, e.g. endosomes or lysosomes.

Therefore, it is preferred that an exon skipping molecule as disclosed herein is formulated in a composition or a medicament or a composition, which is provided with at least an excipient and/or a targeting ligand for delivery and/or a delivery device thereof to a cell and/or enhancing its intracellular delivery.

It is to be understood that if a composition comprises an additional constituent such as an adjunct compound as later defined herein, each constituent of the composition may not be formulated in one single combination or composition or preparation. Depending on their identity, the skilled person will know which type of formulation is the most appropriate for each constituent as defined herein. It is preferred that a composition or a preparation is in the form of a kit comprising an exon skipping molecule as described herein and a further adjunct compound as later defined herein.

If required, an exon skipping molecule or a vector, preferably a viral vector, expressing an exon skipping molecule can be incorporated into a pharmaceutically active mixture by adding a pharmaceutically acceptable carrier.

Accordingly, also disclosed herein is a composition, preferably a pharmaceutical composition, comprising an exon skipping molecule as described herein, or a viral vector as described herein and a pharmaceutically acceptable excipient. Such composition may comprise a single exon skipping molecule , but may also comprise multiple, distinct exon skipping molecules . Such a pharmaceutical composition may comprise any pharmaceutically acceptable excipient, including a carrier, filler, preservative, adjuvant, solubilizer and/or diluent. Such pharmaceutically acceptable carrier, filler, preservative, adjuvant, solubilizer and/or diluent may for instance be found in Remington, 2000. Each feature of said composition has earlier been defined herein.

If multiple distinct exon skipping molecules are used, concentration or dose defined herein may refer to the total concentration or dose of all oligonucleotides used or the concentration or dose of each exon skipping molecule used or added. Therefore, a composition is disclosed wherein each or the total amount of exon skipping molecules used is dosed in an amount ranged from 0.1 and 20 mg/kg, preferably from 0.5 and 20 mg/kg.

A preferred exon skipping molecule described herein, is for the treatment of a *CEP290* related disease or condition of an individual. As used herein, the term "*treatment*" is understood to include the prevention and/or delay of the *CEP290* related disease or condition. An individual, which may be treated using an exon skipping molecule as described herein may already have been diagnosed as having a *CEP290* related disease or condition. Alternatively, an individual which may be treated using an exon skipping molecule as described herein may not have yet been diagnosed as having a *CEP290* related disease or condition but may be an individual having an increased risk of developing a *CEP290* related disease or condition in the future given his or her genetic background. A preferred individual is a human being. A preferred *CEP290* related disease or condition is Leber congenital amaurosis.

Accordingly, the present invention further provides a viral vector expressing an exon skipping molecule according to the invention, or a pharmaceutical composition comprising such a viral vector, for treating a *CEP290* related disease or condition requiring modulating splicing of *CEP290.* A preferred *CEP290* related disease or condition is Leber congenital amaurosis. Each feature of said use has earlier been defined herein.

The invention further provides the use of a viral vector expressing an exon skipping molecule according to the invention, or a pharmaceutical composition comprising such a viral vector for the treatment of a *CEP290* related disease or condition requiring modulating splicing of *CEP290.* A preferred*CEP290* related disease or condition is Leber congenital amaurosis.The present invention further provides the use of a viral vector expressing an exon skipping molecule according to the invention, or a pharmaceutical composition comprising such a viral vector, for the preparation of a medicament for treating a *CEP290* related disease or condition requiring modulating splicing of *CEP290.* A preferred *CEP290* related disease or condition is Leber congenital amaurosis. Therefore in a further aspect, there is provided the use of an exon skipping molecule, viral vector or composition as defined herein for the preparation of a medicament, for the preparation of a medicament for treating a condition requiring modulating splicing of *CEP290* and for the preparation of a medicament for the prevention, treatment or delay of a *CEP290* related disease or condition. A preferred *CEP290* related disease or condition is Leber congenital amaurosis. Each feature of said use has earlier been defined herein. An exon skipping molecule as described herein, or a viral vector as described herein, or a composition as described herein for the preparation of a medicament as described herein is preferably administered systemically or intraocularly, preferably intravitreally or subretinally.

A treatment in a use or in a method disclosed herein is at least one week, at least one month, at least several months, at least one year, at least 2, 3, 4, 5, 6 years or more. Each exon skipping molecule or exon skipping oligonucleotide or equivalent thereof as defined herein for use as described herein may be suitable for direct administration to a cell, tissue and/or an organ in vivo of individuals already affected or at risk of developing *CEP290* related disease or condition, and may be administered directly *in vivo*, *ex vivo* or *in vitro.* The frequency of administration of an oligonucleotide, composition, compound or adjunct compound described herein may depend on several parameters such as the age of the patient, the mutation of the patient, the number of exon skipping molecules (i.e. dose), the formulation of said molecule. The frequency may be ranged between at least once in two weeks, or three weeks or four weeks or five weeks or a longer time period.

Dose ranges of an exon skipping molecule, preferably an oligonucleotide are preferably designed on the basis of rising dose studies in clinical trials (in vivo use) for which rigorous protocol requirements exist. An exon skipping molecule or an oligonucleotide as defined herein may be used at a dose which is ranged from 0.1 and 20 mg/kg, preferably from 0.5 and 20 mg/kg.

It is preferred that a concentration of an oligonucleotide as defined herein, which is ranged from 0.1 nM and 1 µM is used. Preferably, this range is for *in vitro* use in a cellular model such as retina cells or retinal tissue. More preferably, the concentration used is ranged from 1 to 400 nM, even more preferably from 10 to 200 nM, even more preferably from 50 to 100nm. If several oligonucleotides are used, this concentration or dose may refer to the total concentration or dose of oligonucleotides or the concentration or dose of each oligonucleotide added.

It is preferred that a viral vector, preferably an AAV vector as described earlier herein, as delivery vehicle for a molecule as described herein, is administered in a dose ranging from 1x10⁹ - 1x10¹⁷ virusparticles per injection, more preferably from 1x10¹⁰ - 1x10¹⁴, and most preferably 1x10¹⁰ - 1x10¹² virusparticles per injection.

The ranges of concentration or dose of oligonucleotide(s) as given above are preferred concentrations or doses for *in vitro* or *ex vivo* uses. The skilled person will understand that depending on the oligonucleotide(s) used, the target cell to be treated, the gene target and its expression levels, the medium used and the transfection and incubation conditions, the concentration or dose of oligonucleotide(s) used may further vary and may need to be optimized any further.

An exon skipping molecule as described herein, or a viral vector as described herein, or a composition as described herein for the herein disclosed uses may be suitable for administration to a cell, tissue and/or an organ *in vivo* of individuals already affected or at risk of developing a *CEP290* related disease or condition, and may be administered *in vivo*, *ex vivo* or *in vitro.* Said exon skipping molecule, or a viral vector, or a composition may be directly or indirectly administrated to a cell, tissue and/or an organ in vivo of an individual already affected by or at risk of developing a *CEP290* related disease or condition, and may be administered directly or indirectly *in vivo*, *ex vivo* or *in vitro.* As Leber congenital amaurosis has a pronounced phenotype in retina cells, it is preferred that said cells are retina cells, it is further preferred that said tissue is the retina and/or it is further preferred that said organ comprises or consists of the eye. Accordingly, an exon skipping molecule , or a viral vector , or a composition for use is preferably administered systemically or intraocularly, preferably intravitreally or subretinally.

Further disclosed is a method for modulating splicing of *CEP290* in a cell comprising contacting the cell, preferably a retina cell, with an exon skipping molecule as described herein, or a viral vector as described herein, or a composition as described herein. The features of this aspect are preferably those defined earlier herein. Contacting the cell with an exon skipping molecule , or a viral vector, or a composition may be performed by any method known by the person skilled in the art. Use of the methods for delivery of exon skipping molecules, viral vectors and compositions described herein is included. Contacting may be directly or indirectly and may be *in vivo*, *ex vivo* or *in vitro.*

Still further disclosed is a method for the treatment of a *CEP290* related disease or condition requiring modulating splicing of *CEP290* of an individual in need thereof, said method comprising contacting a cell, preferably a retina cell, of said individual with an exon skipping molecule as described herein, or a viral vector as described herein, or a composition as described herein. The features of this aspect are preferably those defined earlier herein. Contacting the cell, preferably a retina cell with an exon skipping molecule , or a viral vector , or a composition may be performed by any method known by the person skilled in the art. Use of the methods for delivery of molecules, viral vectors and compositions described herein is included. Contacting may be directly or indirectly and may be *in vivo*, *ex vivo* or *in vitro.* A preferred *CEP290* related disease or condition is Leber congenital amaurosis. Accordingly, an exon skipping molecule , or a viral vector , or a composition in a method of treatment as described herein is preferably administered systemically or intraocularly, preferably intravitreally or subretinally.

Unless otherwise indicated each embodiment as described herein may be combined with another embodiment as described herein.

As can be observed in the experimental section herein, at the RNA level, addition of various AONs targeting the aberrant *CEP290* exon indeed resulted in a conversion of aberrantly spliced *CEP290* mRNA to correctly spliced *CEP290* mRNA. This conversion will coincide with an increased synthesis of the wild-type CEP290 protein.

In fibroblasts (that can be derived from skin cells), CEP290 is abundantly expressed. Therefore, it is to be expected that addition of AONs to cultured fibroblasts from LCA patients will result in an increased amount of wild-type CEP290 protein that is detectable on Western blot, and as such will demonstrate that AON-based therapy will not only redirect normal splicing of *CEP290* mRNA but will also result in restoring CEP290 protein function. This experiment is presently ongoing.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The word "about" or "approximately" when used in association with a numerical value (e.g. about 10) preferably means that the value may be the given value (of 10) more or less 0.1% of the value.

The sequence information as provided herein should not be so narrowly construed as to require inclusion of erroneously identified bases. The skilled person is capable of identifying such erroneously identified bases and knows how to correct for such errors. In case of sequence errors, the sequence of the polypeptide obtainable by expression of the gene present in SEQ ID NO: 1 containing the nucleic acid sequence coding for the polypeptide should prevail.

### FIGURE LEGENDS

**Figure 1** ***CEP290* splicing and AON function**
   **A)** Normal *CEP290* mRNA splicing of exons 26 and 27, resulting in wild-type *CEP290* protein.
   **B)** The most frequent LCA-causing mutation is an A-to-G transition (underlined and indicated with an asterisk) in intron 26 of *CEP290.* This mutation creates a splice donor site, which results in the inclusion of an aberrant exon to ∼50% of the *CEP290* mRNA and subsequent premature termination of the *CEP290* protein.
   **C)** Upon binding of sequence-specific AONs, factors involved in splicing will not recognize the aberrant splice donor site in intron 26, resulting in redirection of normal *CEP290* splicing and synthesis of a correct *CEP290* protein.

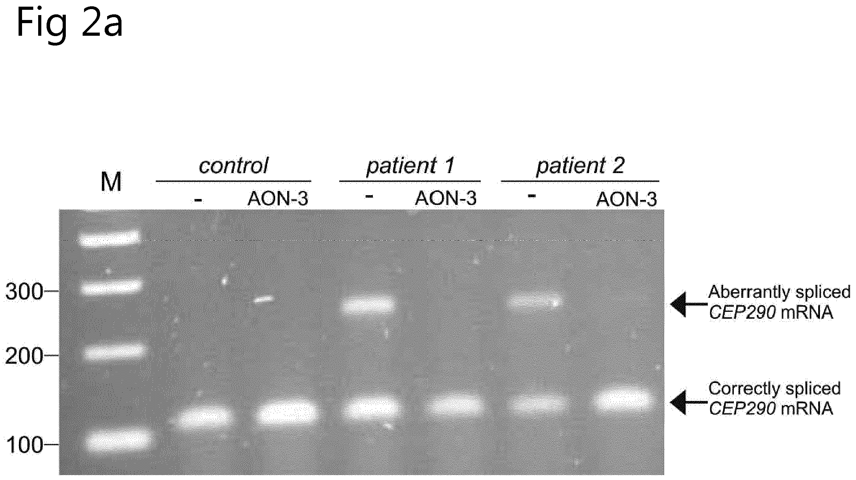

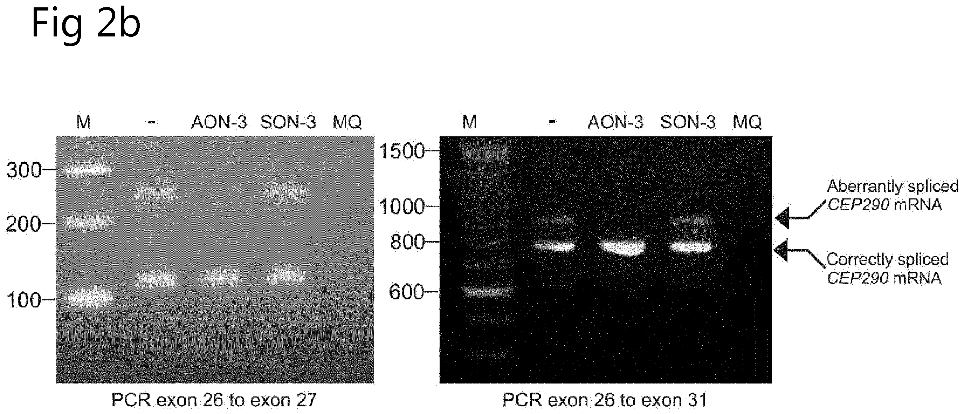

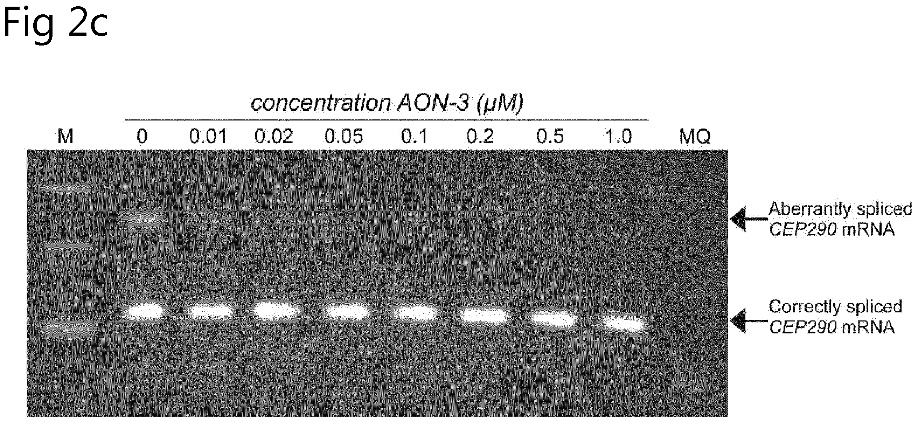

**Figure 2** **AON-based rescue of aberrant *CEP290* splicing**
   A) RT-PCR analysis of *CEP290* mRNA isolated from lymphoblastoid cells of one control individuals and two individuals affected with LCA, that were cultured in the absence or presence of a selected AON (AON-3) direct against the aberrant *CEP290* exonin a final concentration of 1.0 µM. The upper band represents the aberrant *CEP290* splice product, whereas the lower band represents the wild-type *CEP290* splice product. M: 100-bp marker. MQ: negative water control.
   B) Specificity of AON-based rescue. Similar to A), cells were transfected with AON-3, or a sense oligonucleotide directed to the same target site (SON-3). Left panel: RT-PCR reaction using primers located in exon 26 and exon 27. Right panel: RT-PCR reaction using primers located in exon 26 and exon 31.
   C) Dose-dependent rescue of *CEP290* mRNA splicing. Similar to A), cells were transfected with different concentrations of the selected AON, ranging from 0.01 to 1.0 µM.

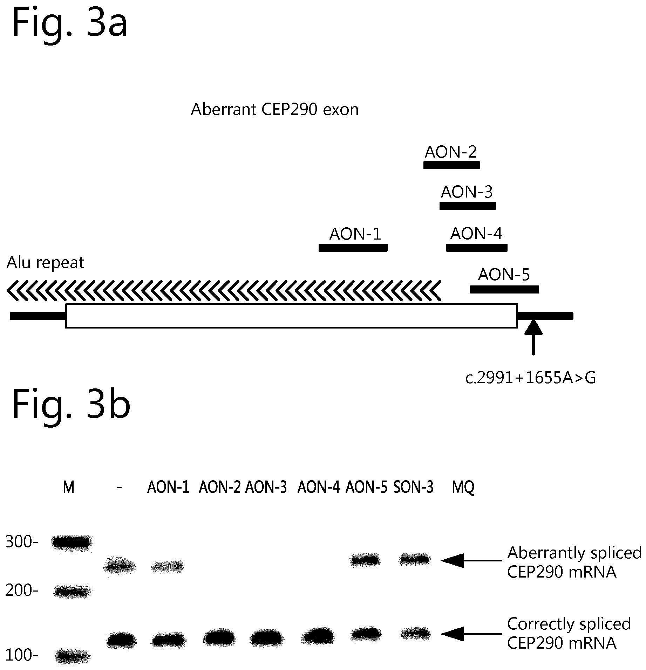

**Figure 3** **Sequence specificity in AON-based rescue of aberrant *CEP290* splicing**
   **A)** Overview of the aberrant *CEP290* exon, and the relative positions of the AONs that were selected. The 5'-end of the aberrant exon is part of an Alu repeat.
   **B)** RT-PCR analysis of *CEP290* mRNA isolated from lymphoblastoid cells of an LCA patient that were cultured in the absence or presence of different AONs direct against the aberrant *CEP290* exon (AON-1 to -5), or one sense oligonucleotide (SON-3). The
   AONs and SON were transfected in a final concentration of 0.1 µM. The upper band represents the aberrant *CEP290* splice product, whereas the lower band represents the wild-type *CEP290* splice product. M: 100-bp marker.

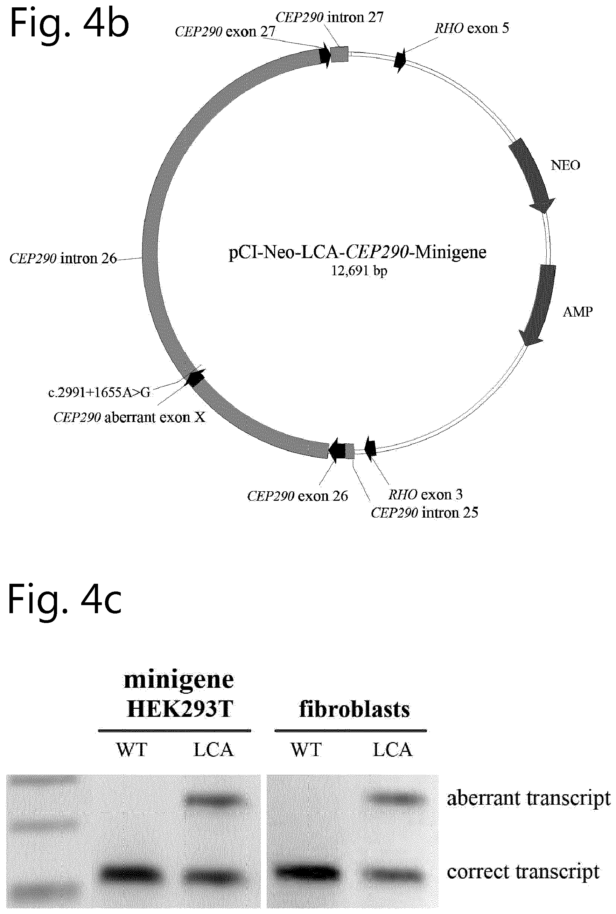

**Figure 4****. Generated constructs and assessment of minigenes**
   **A)** Upper panel: graphical representation of the pSMD2 constructs containing the modified U7snRNA gene and inserted AON sequences. Lower panel: exact sequences of the AONs used in the different constructs, aligned with the sequence of the cryptic exon ("-" is used for aligment purposes, it represents neither a gap nor a nucleotide residue. The intronic c.2991+1655A>G mutation is indicated in bold and underlined.
   **B)** Schematic drawing of the LCA minigene construct, containing the genomic DNA sequence of *CEP290* from intron 25 to intron 27, including the c.2991+1655A>G mutation in intron 26. This sequence is flanked by exon 3 and 5 of the *RHO* gene.
   **C)** RT-PCR analysis of *CEP290* on HEK293T cells transfected with the WT or LCA minigene, in comparison with that in fibroblast cells from healthy controls or patients with *CEP290*-associated LCA.

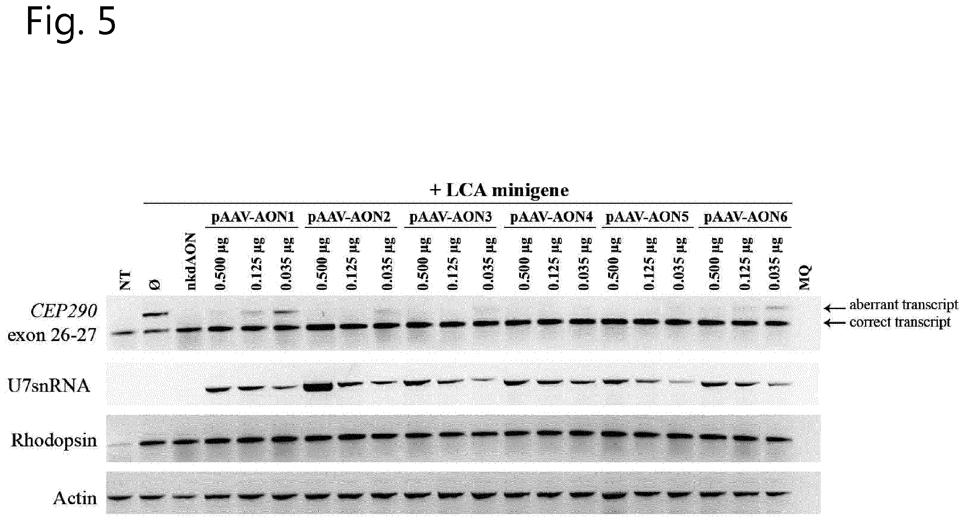

**Figure 5****. Splice correction efficacy of AON-containing vectors**
   HEK293T cells were co-transfected with the LCA minigene and three different concentrations of the six different pAAV-AON constructs (0.5, 0.125 or 0.035 µg of plasmid DNA). RT-PCR analysis from exon 26 to exon 27 of *CEP290* revealed the aberrant transcript that contains the cryptic exon X. pAAV-AON4 and pAAV-AON5 were most effective. Naked AON (nkdAON) was used as a positive control. U7snRNA and RHO amplification were used as a transfection control for the pAAV-AON and the minigene constructs, respectively. Actin was used as a loading control.

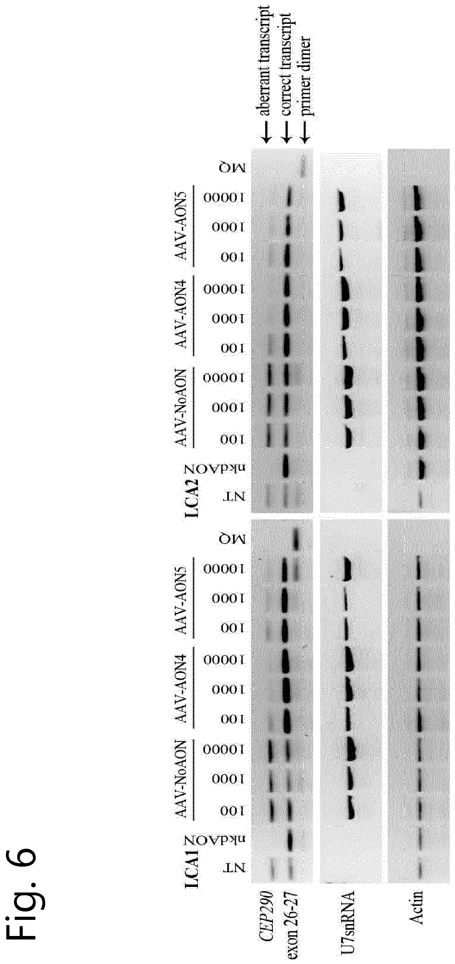

**Figure 6****. Splice correction efficacy of AON-containing AAVs**
   RT-PCR analysis on two different patient cell lines transduced with AAV-NoAON, AAV-AON4 or AAV-AON5, at three different MOIs (100; 1,000 and 10,000). Amplification from exon 26 to exon 27 of *CEP290* revealed the presence of the aberrant transcript in the non-treated (NT) as well as the AAV-NoAON-transduced cells, while it was strongly decreased or completely absent in the AAV-AON4 and AAV-AON5-treated cells. Transfection of the naked AON (nkd AON) sderved as a positive control. U7snRNA amplification was used as a measure for the transduction efficacy, and actin as a loading control.

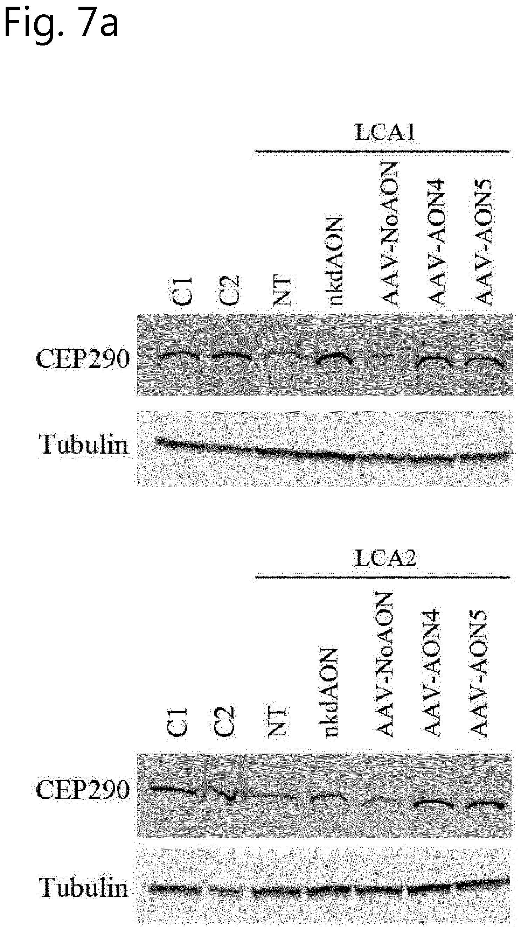

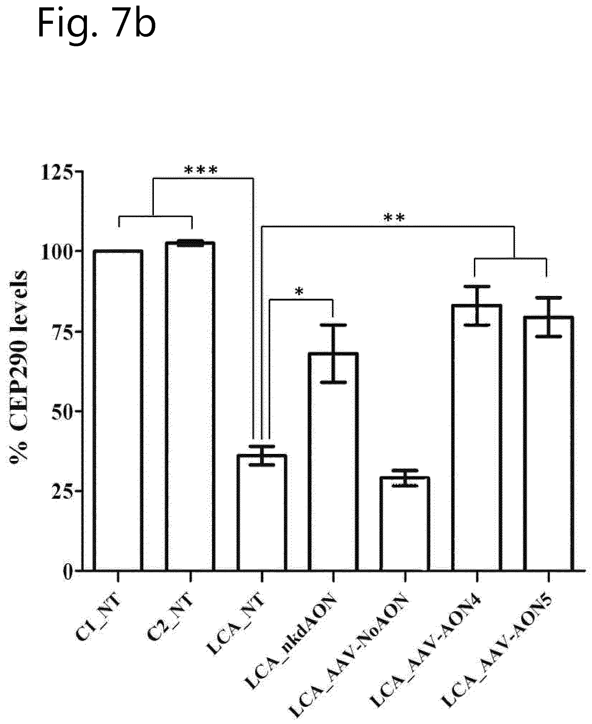

**Figure 7****. Assessment of CEP290 protein levels upon transduction of AON-containing AAVs**
   **A)** Immunodetection of CEP290 protein levels in treated and non-treated (NT) LCA fibroblast cells in comparison to the control fibroblast cells (C1 and C2). Tubulin detection was used for normalization.
   **B)** Quantification of CEP290 protein levels shown in panel A. Values were normalized against tubulin. C1 was set up as a 100% and all samples were referred to this value. Naked AON (nkdAON) and AAV-AON4 and AAV-AON5 significantly increased the CEP290 protein levels. T-test was performed: * p-value < 0.05; ** p-value < 0.01 and *** p-value <0.001.

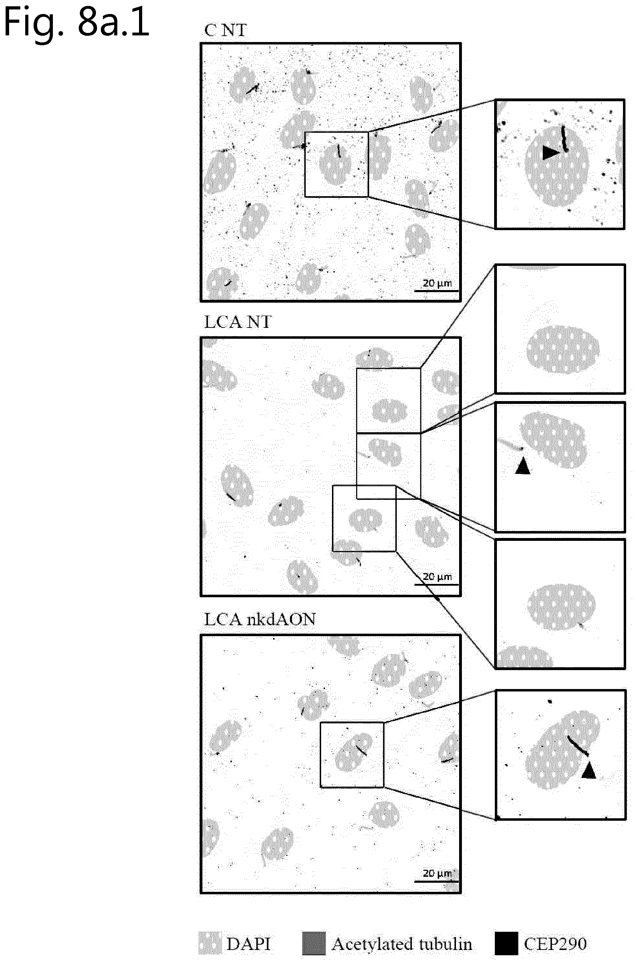

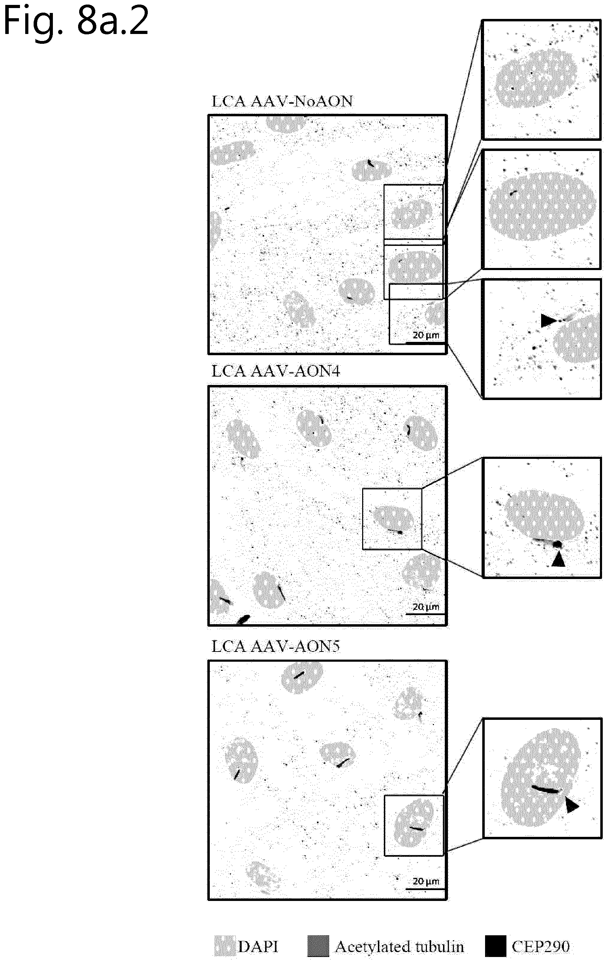

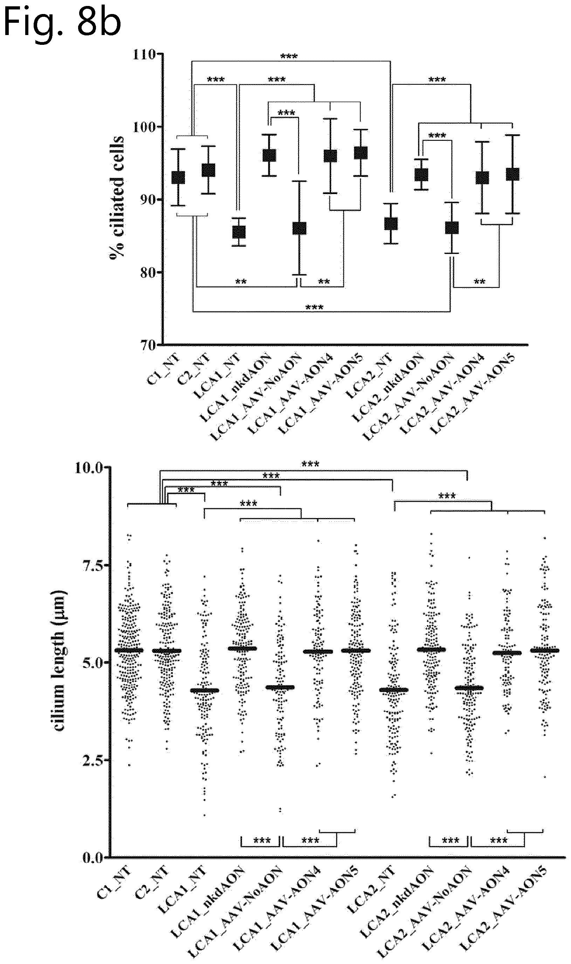

**Figure 8****. Immunocytochemical analysis of cilium integrity**
   **A1 and 8A2)** Immunocytochemistry in control (C) and patient (LCA) fibroblast cell lines. CEP290 (in black) localizes to the basal body of the cilia (as indicated by the head arrows). The cilium axoneme is stained with acetylated tubulin (in dark grey) whereas the nuclei are stained with DAPI (dotted grey).
   **B)** Quantification of the percentage of ciliated cells and the length of the cilium in treated and untreated LCA cells compared to control (C1 and C2) cells. A minimum of 150 ciliated cells were measured for each condition and a Mann-Whitney test was used for statistic analysis. ** p-value ≤ 0.01 and *** p-value ≤ 0.001.

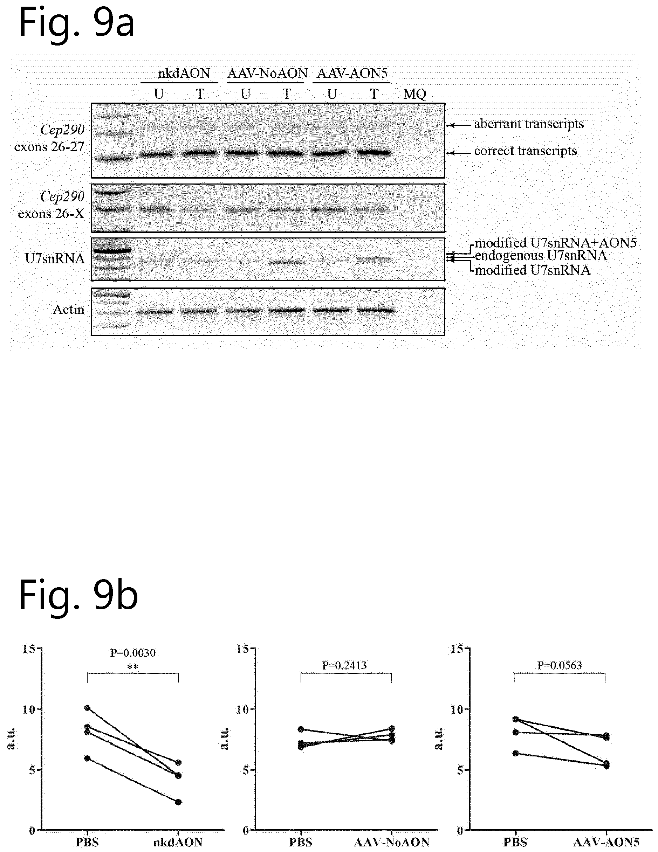

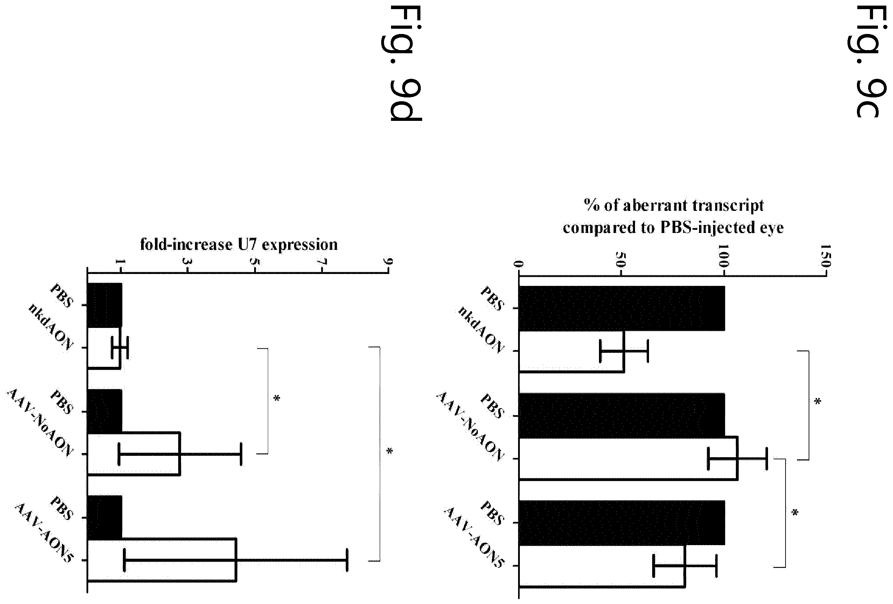

**Figure 9****. In vivo correction of aberrantly spliced CEP290**
   **A)** Representative gel electrophoresis of the PCR reactions amplifying exon 26 to 27, exon 26 to cryptic exon X, U7snRNA and actin (to normalize) of one of each replicate. MQ is the negative control of the PCR. U stands for untreated and refers to PBS-injected retinas, while T means treated and shows the effect on the AON or AAV-AON-injected retinas. **B)** Schematic representation of the decrease of aberrant exon X in each replicate. Bands were semi-quantified with ImageJ and normalized against actin. The Y axis indicated the arbitrary units (a.u.). **C)** Percentage of decrease of aberrant exon X. PBS-injected eyes were considered as a reference and placed at 100%.
   **D)** Fold-increase of U7snRNA detection. PBS-injected retinas were taken as a reference and set at 1.0. In all graphs, * p-value ≤ 0.05 and ** p-value ≤ 0.01.

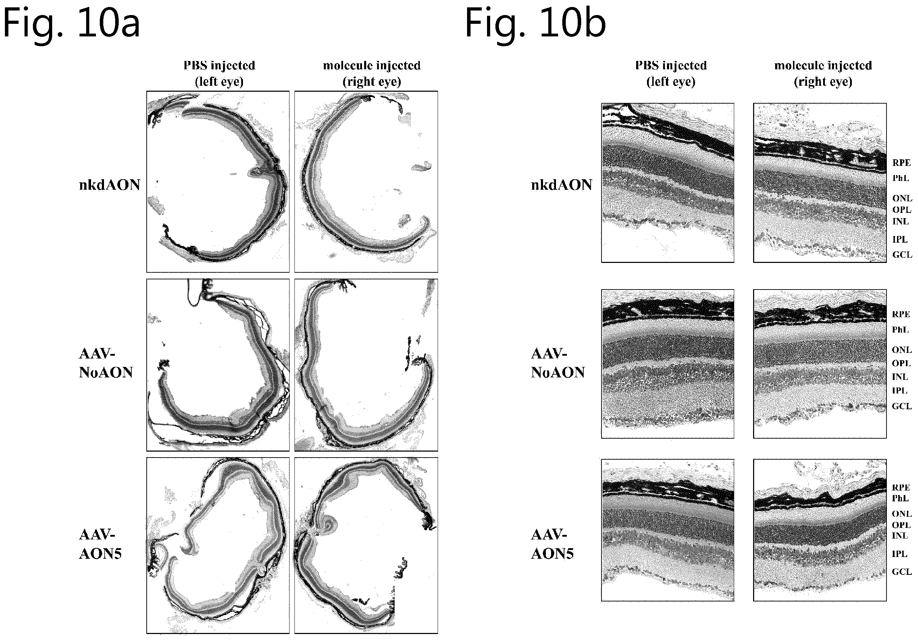

**Figure 10****. Assessment of the structure of the retina after treatment**
   Seven micrometer cryosections stained with toluidine blue. A) 50X magnification images covering the complete retina. B) 400X magnification images. RPE: Retinal Pigment Epithelium; PhL: Photoreceptor Layer; ONL: Outer Nuclear Layer; OPL: Outer Plexiform layer; INL: Inner Nuclear Later; IPL: Inner Plexiform Layer and GCL: Ganglion Cell Layer.

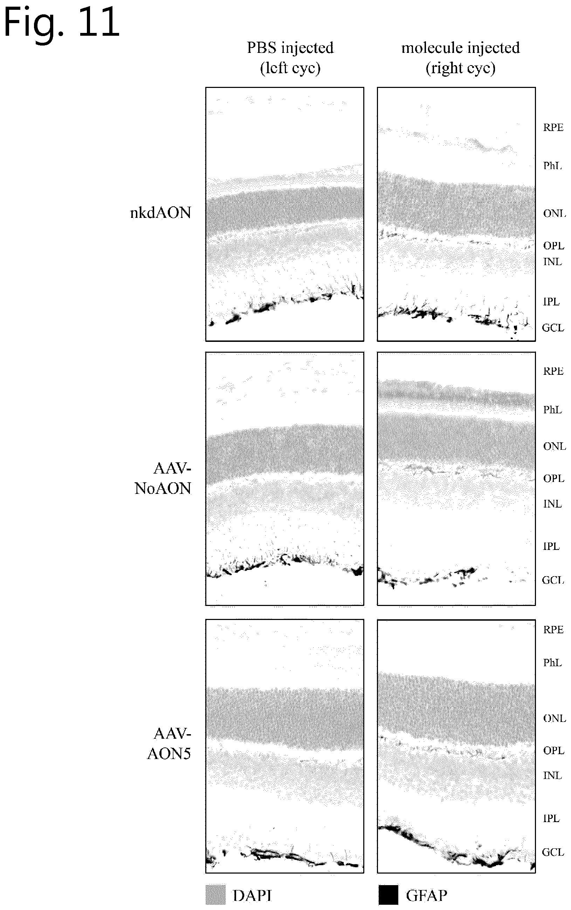

**Figure 11****. GFAP immunostaining**
   Immunostaining of seven µm cryosections from mice treated with PBS, naked AON, AAV-NoAON or AAV-AON5. DAPI (darker grey) stains the nuclei while GFAP (black) is an indicator of gliosis and structural stress in the retina. No differences were observed between PBS injected retinas and molecule injected retinas. RPE: Retinal Pigment Epithelium; PhL: Photoreceptor Layer; ONL: Outer Nuclear Layer; OPL: Outer Plexiform layer; INL: Inner Nuclear Later; IPL: Inner Plexiform Layer and GCL: Ganglion Cell Layer.

### SEQUENCES

All sequences herein are depicted from 5' → 3'

**Table 1. Sequences as set forth in the Sequence Listing**

| **SEQ ID NO:** | **SEQ type** | **Description** |
|---|---|---|
| 1 | Genomic DNA | *CEP290* |
| 2 | cDNA | *CEP290* |
| 3 | PRT | CEP290 protein |
| 4 | DNA | 128 nucleotide aberrant *CEP290* exon |
| 5 | PRT | CEP290 aberrant protein |
| 6 | Polynucleotide | 143 nucleotide motif |
| 7 | Polynucleotide | 42 nucleotide motif |
| 8 | Polynucleotide | 24 nucleotide motif |
| 9 | AON-1 | taatcccagcactttaggag |
| 10 | AON-2 | gggccaggtgcggtgg |
| 11 | AON-3 | aactggggccaggtgcg |
| 12 | AON-4 | tacaactggggccaggtg |
| 13 | AON-5 | actcacaattacaactgggg |
| 14 | SON-3 | cgcacctggccccagtt |
| 15 | PCR primer | tgctaagtacagggacatcttgc |
| 16 | PCR primer | agactccacttgttcttttaaggag |
| 17 | Polynucleotide | 69 nucleotide motif |
| 18 | Nkd AON | aactggggccaggtgcg |
| 19 | (AAV) AON1 | aactggggccaggtgcg |
| 20 | (AAV) AON2 | ccatggtgcggtggctcacatcgtaatcccagcactttaggagg |
| 21 | (AAV) AON3 | gatactcacaattacaactgggggtaatcccagcactttaggagg |
| 22 | (AAV) AON4 | ccaggtgcggtggctcacatc |
| 23 | (AAV) AON5 | gatactcacaattacaactggggccaggtgcggtggctcacatc |
| 24 | (AAV) AON6 | gatactcacaattacaactgggg |
| 25 | (pAAV) AON1 | cgcacctggccccagtt |
| 26 | (pAAV) AON2 | gcctcctaaagtgctgggattacgatgtgagccaccgcacctgg |
| 27 | (pAAV) AON3 | cctcctaaagtgctgggattacccccagttgtaattgtgaatatc |
| 28 | (pAAV) AON4 | gatgtgagccaccgcacctgg |
| 29 | (pAAV) AON5 | gatgtgagccaccgcacctggccccagttgtaattgtgaatatc |
| 30 | (pAAV) AON6 | ccccagttgtaattgtgaatatc |
| 31 | CEP 290ex26 | RT-PCR forward primer |
| 32 | CEP 290ex27 | RT-PCR reverse primer |
| 33 | U7snRNA | RT-PCR forward primer |
| 34 | U7snRNA | RT-PCR reverse primer |
| 35 | Rhodopsin | RT-PCR forward primer |
| 36 | Rhodopsin | RT-PCR reverse primer |
| 37 | Actin | RT-PCR forward primer |
| 38 | Actin | RT-PCR reverse primer |
| 39 | (pAAV) AON1 | PCR forward primer |
| 40 | (pAAV) AON1 | PCR reverse primer |
| 41 | (pAAV) AON2 | PCR forward primer |
| 42 | (pAAV) AON2 | PCR reverse primer |
| 43 | (pAAV) AON3 | PCR forward primer |
| 44 | (pAAV) AON3 | PCR reverse primer |
| 45 | (pAAV) AON4 | PCR forward primer |
| 46 | (pAAV) AON4 | PCR reverse primer |
| 47 | (pAAV) AON5 | PCR forward primer |
| 48 | (pAAV) AON5 | PCR reverse primer |
| 49 | (pAAV) AON6 | PCR forward primer |
| 50 | (pAAV) AON6 | PCR reverse primer |

Unless stated otherwise, the methods described herein will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

### EXAMPLES

### EXAMPLE 1: naked AON's

### MATERIALS and METHODS

### Design antisense oligonucleotides

The 128-bp sequence of the aberrant *CEP290* exon that is included into the mutant *CEP290* mRNA was analyzed for the presence of exonic splice enhancer motifs using the ESE finder 3.0 program (http://rulai.cshl.edu/cgi-bin/tools/ESE3/esefinder.cgi?process=home). RNA antisense oligonucleotides were purchased from Eurogentec, and designed with a Tₘ of 58°C, and modified with a 2'-O-methyl group at the sugar chain and a phosphothiorate backbone, and dissolved in phosphate buffered saline.

### Cell culture

Human B-lymphoblasts cells of LCA patients homozygously carrying the intronic mutation in *CEP290* were immortalized by transformation with the Eppstein-Barr virus, as described previously.(Wall FE, 1995). Cells were cultured in RPMI1640 medium (Gibco) containing 10% (v/v) fetal calf serum (Sigma), 1% 10 U/µl penicillin and 10 µg/µl streptomycin (Gibco), and 1% GlutaMAX (Gibco), at a density of 0.5x10⁶ cells/ml. Cells were passaged twice a week.

### Transfection of AONs

A day before transfection, 1.0x10⁶ cells were seeded in each well of a 6-wells plate, in a total volume of 2 ml complete medium. Transfection mixtures were prepared by combining 2.5 µl AON in a desired concentration, or distilled water, 5 µl transfection reagent (ExGen in vitro 500, Fermentas) and 92.5 µl 150 mM NaCl, and incubated at room temperature for 10 minutes, before addition to the cells. Six hours after transfection, 8 ml of low-serum medium (complete medium with only 1% fetal calf serum) was added. Forty-eight hours after transfection, cells were collected and washed with 1x PBS, before directly proceeding to RNA isolation.

### RNA isolation and RT-PCR

Total RNA was isolated from transfected lymphoblastoid cells using the Nucleospin RNA II isolation kit (Machery Nagel), according to manufacturer's protocol. Subsequently, 1 µg of total RNA was used for cDNA synthesis using the iScript cDNA synthesis kit (Bio-Rad). Five percent of the cDNA was used for each PCR reaction. Part of the *CEP290* cDNA was amplified under standard PCR conditions supplemented with 5% Q-solution (Qiagen), and using forward primer tgctaagtacagggacatcttgc (SEQ ID NO: 15) and reverse primer agactccacttgttcttttaaggag (SEQ ID NO: 16) that are located in exon 26 and exon 27 of the human *CEP290* gene, respectively. PCR products were resolved on a 1.5% agarose gel. Bands presumably representing correctly and aberrantly spliced *CEP290* were excised from the gel, purified using Nucleospin Extract II isolation kit and sequenced from both strands with the ABI PRISM Big Dye Terminator Cycle Sequencing V2.0 Ready Reaction kit and the ABI PRISM 3730 DNA analyzer (Applied Biosystems).

### INTRODUCTION

Here, we describe the use of AONs to redirect normal splicing of *CEP290* in patient-derived lymphoblast cells, and show a sequence-specific and dose-dependent decrease in levels of aberrantly spliced *CEP290,* thereby revealing the potential of AON-based therapy to treat *CEP290*-associated LCA.

### RESULTS

The intronic *CEP290* mutation (c.2991+1655A>G) creates a cryptic splice donor site that results in the inclusion of an aberrant exon into the *CEP290* mRNA (Figure 1A and -B). Addition of AONs directed against the aberrant exon would prevent the insertion of this exon by preventing the binding of factors that are essential for splicing such as the U1- and U2snRNP complexes, and serine-arginine rich proteins, thereby restoring normal *CEP290* splicing and protein synthesis (Figure 1C). AONs can target splice sites as well as exonic sequences, although in the particular case of the Duchenne muscular dystrophy *DMD* gene, AONs targeting exonic regions tend to outperform those that target the splice sites (Aartsma-Rus et al, 2010). In addition, previous studies have suggested a positive correlation between the capability of AONs to induce exon skipping and the presence of predicted SC35 splice factor binding sites in the target sequence (Aartsma-Rus et al, 2008). To design an AON with high exon-skipping potential, the aberrant *CEP290* exon (128 nucleotides exonic sequence plus 15 nucleotides of intronic sequence on each side) was scrutinized for exonic splice enhancer binding motifs, using the ESE finder 3.0 program (Smith et al, 2006). At the 3'-end of the aberrant exon, two SC35-binding motifs were predicted (data not shown). Hence, the first AON was designed such that it encompassed these two motifs (designated AON-3, SEQ ID NO: 11), and being complementary to the *CEP290* mRNA.

To determine whether AON-3 has exon-skipping potential *in vitro,* immortalized lympoblastoid cells of two unrelated individuals with LCA homozygously carrying the intronic *CEP290* founder mutation c.2991+1655A>G, as well as one control individual were cultured in the absence or presence of 1 µM AON-3. As expected, in the control individual, only a band representing correctly spliced *CEP290* was observed, whereas in both affected individuals two products were present, one representing correctly spliced, and one representing aberrantly spliced *CEP290* mRNA. Upon addition of AON-3, a strong decrease in aberrantly spliced *CEP290* was noted, in both individuals with LCA (Figure 2A). Next, the specificity of AON-3 was assessed by transfecting a sense oligonucleotide directed to the same target site (SON-3, SEQ ID NO: 14). RT-PCR analysis showed that in the cells transfected with SON-3, both the aberrantly spliced and the correctly spliced *CEP290* mRNA molecules are still present (Figure 2B, left panel), demonstrating the specificity of the antisense sequence. Using an additional pair of primers that amplifies larger products, similar results were obtained (Figure 2B, right panel). Interestingly, the decrease in aberrantly spliced *CEP290* appears to coincide with an increased intensity of the product representing correctly spliced *CEP290* mRNA. These data indicate that the aberrant product is not degraded, but that the AON transfection truly induces exon skipping, resulting in the synthesis of more correctly spliced wild-type *CEP290* mRNA. To determine the effective dose of AON-3, cells were transfected with various concentrations of AON-3, ranging from 0.01 to 1.0 µM. Even at the lowest concentration of 0.01 µM, a marked reduction in aberrantly spliced *CEP290* was observed. The maximum amount of exon skipping was observed at 0.05 or 0.1 µM of AON, indicating that these concentrations are sufficient to convert almost all aberrantly spliced *CEP290* (Figure 2C).

The effectiveness of AONs in splice modulation is thought to merely depend on the accessibility of the target mRNA molecule, and hence may differ tremendously between neighboring sequences. To determine whether this sequence specificity also applies for *CEP290,* several AONs were designed that target the aberrant *CEP290* exon (Table 1). This exon consists of 128 base pairs, the majority of which are part of an Alu repeat, one of the most frequent repetitive elements in the human genome (Schmidt et al, 1982), covering the entire 5'-end of the aberrant exon (Figure 3A). Hence, the majority of AONs were designed to be complementary to the 3'-end of the aberrant exon or the splice donor site (Figure 3A). In total, five AONs were transfected at a final concentration of 0.1 µM, which was shown to be optimal for AON-3. Interestingly, besides AON-3, also AON-2 (SEQ ID NO: 10) and AON-4 (SEQ ID NO: 12) resulted in high levels of exon skipping. In contrast, AON-1 (SEQ ID NO: 9) that targets the Alu repeat region, and AON-5 (SEQ ID NO: 13) that is directed against the splice donor site, hardly showed any exon skipping potential (Figure 3B). These data demonstrate the sequence specificity in AON-based exon skipping of *CEP290* and highlight a small region of the aberrant *CEP290* exon as a potential therapeutic target.

### DISCUSSION

In this study, we explored the therapeutic potential of AONs to correct a splice defect caused by an intronic mutation in *CEP290.* In immortalized lymphoblast cells of LCA patients homozygously carrying the intronic *CEP290* mutation c.2991+1655A>G, transfection of some but not all AONs resulted in skipping of the aberrant exon, thereby almost fully restoring normal *CEP290* splicing.

AONs have been the focus of therapeutic research for over a decade, for the treatment of a variety of genetic diseases (Hammond et al, 2011). These strategies include the use of AONs to block the recognition of aberrant splice sites, to alter the ratio between two naturally occurring splice isoforms, to induce skipping of exons that contain protein-truncating mutations, or to induce the skipping of exons in order to restore the reading-frame of a gene that is disrupted by a genomic deletion, allowing the synthesis of a (partially) functional protein (Hammond et al, 2011). The latter approach is already being applied in phase I/II clinical trials for the treatment of patients with Duchenne muscular dystrophy, with promising results (Kinali et al, 2009; van Deutekom et al, 2007).

The intronic *CEP290* mutation is an ideal target for AON-based therapy, since this mutation results in the inclusion of an aberrant exon in the *CEP290* mRNA which is normally not transcribed. Inducing skipping of this aberrant exon by AONs fully restores the normal *CEP290* mRNA, allowing normal levels of *CEP290* protein to be synthesized. A second major advantage is that although this AON-approach is a mutation-specific therapeutic strategy, the intronic *CEP290* mutation is by far the most frequent LCA-causing mutation.⁴ Based on the estimated prevalence of LCA (1:50,000), and the observed frequency of the intronic *CEP290* mutation in Northern-Europe (26%) (Coppieters et al, 2010) and the U.S. (10%) (Stone, 2007), at least one thousand and, depending on the frequency of the mutation in other populations, perhaps many more individuals worldwide have LCA due to this mutation. Finally, although the LCA phenotype associated with *CEP290* mutations is severe, it appears that the photoreceptor integrity, especially in the macula, as well as the anatomical structure of the visual connections to the brain, are relatively intact in LCA patients with *CEP290* mutations, which would allow a window of opportunity for therapeutic intervention (Cideciyan et al, 2007).

The study described here provides a proof-of-principle of AON-based therapy for *CEP290*-associated LCA *in vitro,* using immortalized patient lymphoblast cells. In order to determine the true therapeutic potential of this method for treating LCA, additional studies are needed that include the development of therapeutic vectors, and assessment of efficacy and safety in animal models. Although naked AONs, or conjugated to cell-penetrating peptides, can be delivered to the retina by intraocular injections, the limited stability of the AONs would require multiple injections in each individual. In contrast, by using viral vectors, a single subretinal injection would suffice to allow a long-term expression of the therapeutic construct. Previously, others have used recombinant adeno-associated viral (rAAV) vectors carrying U1- or modified U7snRNA constructs to efficiently deliver AON sequences, in the *mdx* mouse model for DMD, or in DMD patient myoblasts, respectively (Geib et al, 2009; Goyenhalle et al, 2004). In line with this, AONs targeting the aberrant exon of *CEP290* could be cloned within such constructs, and delivered to the retina by subretinal injections of rAAV-5 or -8 serotypes that efficiently transduce photoreceptor cells where the endogenous *CEP290* gene is expressed (Alloca et al, 2007; Lebherz et al, 2008). Using rAAV-2 vectors, no long-lasting immune response was evoked upon subretinal injections of these vectors in patients with *RPE65* mutations (Simonella et al, 2009), and also for rAAV-5 and rAAV-8, immune responses appear to be absent or limited, at least in animal models (Li et al, 2009; Vandenberghe et al, 2011). One final safety aspect concerns the specificity of the sequence that is used to block the splicing of the aberrant *CEP290* exon. As stated before, the majority of this exon is part of an Alu repeat, and AONs directed against this repeat will likely bind at multiple sites in the human genome, increasing the chance to induce off-target effects. The AONs that were shown to be effective in this study do not fully target the Alu repeat sequence, but are also not completely unique in the human genome. However, when blasting against the EST database, no exact hits are found, indicating that at the level of expressed genes, these sequences are unlikely to induce off-target effects and deregulate normal splicing of other genes. To further study the efficacy and safety of AON-based therapy for *CEP290*-associated LCA *in vivo*, we are currently generating a transgenic knock-in mouse model that carries part of the human *CEP290* gene (exon 26 to exon 27, with and without the intronic mutation) which is exchanged with its mouse counterpart. Compared to gene augmentation therapy, AON-based therapy has a number of advantages. First, in gene augmentation therapy, a ubiquitous or tissue-specific promoter is used to drive expression of the wild-type cDNA encoding the protein that is mutated in a certain patient. For instance in one clinical trial for *RPE65* gene therapy, the chicken beta-actin promoter was used (Maguire et al, 2008). Using these but also fragments of the endogenous promoters, it is difficult to control the levels of expression of the therapeutic gene. In some cases, like for the RPE65 protein that has an enzymatic function, expression levels beyond those of the endogenous gene might not be harmful to the retina. For other genes however, including those that encode structural proteins like *CEP290,* tightly-regulated expression levels might be crucial for cell survival, and overexpression of the therapeutic protein might exert toxic effects. Using AONs, the therapeutic intervention occurs at the pre-mRNA level, and hence does not interfere with the endogenous expression levels of the target gene. A second issue is the use of the viral vector. Of a variety of different recombinant viral vectors, rAAVs are considered to be most suitable for treating retinal dystrophies, because of their relatively high transduction efficiency of retinal cells, and their limited immunogenicity. The major drawback of rAAVs however is their limited cargo size of 4.8 kb. Again, for some genes like *RPE65,* this is not a problem. For many other retinal genes however, like *CEP290* (with an open reading frame of 7.4 kb), but also *ABCA4* and *USH2A*, the size of their full-length cDNAs exceeds the cargo size of the currently available pool of rAAVs. One way to overcome this problem is to express cDNAs that express only partial proteins with residual activity, as has been suggested for *CEP290* by expressing the N-terminal region of *CEP290* in a zebrafish model (Baye et al, 2011). Other viral vectors, like lentivirus or adenoviruses have a higher cargo capacity that rAAVs (∼8 kb), but are less efficient in transducing retinal cells, and adenoviruses have a higher immunogenic potential (den Hollander et al, 2010). For AON-based therapy, the size limitations of AAV are not a problem, since the small size of the AONs and the accompanying constructs easily fit within the available AAVs.

In conclusion, this study shows that administration of AONs to cultured patient cells almost fully corrects a splice defect that is caused by a frequent intronic mutation in *CEP290* that causes LCA. These data warrant further research to determine the therapeutic potential of AON-based therapy for *CEP290*-associated LCA, in order to delay or cease the progression of this devastating blinding disease.

### REFERENCE LIST Description and Example 1

1. Leber, T. (1869). Uber Retinitis Pigmentosa und angeborene Amaurose. von Graefe's Archives Ophthalmology 15, 1-25.
2. Koenekoop, R.K., Lopez, I., den Hollander, A.I., Allikmets, R., and Cremers, F.P. (2007). Genetic testing for retinal dystrophies and dysfunctions: benefits, dilemmas and solutions. Clin Experiment Ophthalmol 35, 473-485.
3. Stone, E.M. (2007). Leber congenital amaurosis - a model for efficient genetic testing of heterogeneous disorders: LXIV Edward Jackson Memorial Lecture. Am J Ophthalmol 144, 791-811.
4. den Hollander, A.I., Roepman, R., Koenekoop, R.K., and Cremers, F.P.M. (2008). Leber congenital amaurosis: genes, proteins and disease mechanisms. Prog Retin Eye Res 27, 391-419.
5. Estrada-Cuzcano, A., Koenekoop, R.K., Coppieters, F., Kohl, S., Lopez, I., Collin, R.W.J., De Baere, E.B., Roeleveld, D., Marek, J., Bernd, A. et al (2011). IQCB1 mutations in patients with leber congenital amaurosis. Invest Ophthalmol Vis Sci 52, 834-839.
6. den Hollander, A.I., Koenekoop, R.K., Yzer, S., Lopez, I., Arends, M.L., Voesenek, K.E., Zonneveld, M.N., Strom, T.M., Meitinger, T., Brunner, H.G. et al (2006). Mutations in the CEP290 (NPHP6) gene are a frequent cause of Leber congenital amaurosis. Am J Hum Genet 79, 556-561.
7. Perrault, I., Delphin, N., Hanein, S., Gerber, S., Dufier, J.L., Roche, O., foort-Dhellemmes, S., Dollfus, H., Fazzi, E., Munnich, A. et al (2007). Spectrum of NPHP6/CEP290 mutations in Leber congenital amaurosis and delineation of the associated phenotype. Hum Mutat 28, 416.
8. Baala, L., Audollent, S., Martinovic, J., Ozilou, C., Babron, M.C., Sivanandamoorthy, S., Saunier, S., Salomon, R., Gonzales, M., Rattenberry, E. et al (2007). Pleiotropic effects of CEP290 (NPHP6) mutations extend to Meckel syndrome. Am J Hum Genet 81, 170-179.
9. Frank, V., den Hollander, A.I., Bruchle, N.O., Zonneveld, M.N., Nurnberg, G., Becker, C., Du, B.G., Kendziorra, H., Roosing, S., Senderek, J. et al (2008). Mutations of the CEP290 gene encoding a centrosomal protein cause Meckel-Gruber syndrome. Hum Mutat 29, 45-52.
10. Helou, J., Otto, E.A., Attanasio, M., Allen, S.J., Parisi, M.A., Glass, I., Utsch, B., Hashmi, S., Fazzi, E., Omran, H. et al (2007). Mutation analysis of NPHP6/CEP290 in patients with Joubert syndrome and Senior-Loken syndrome. J Med Genet 44, 657-663.
11. Valente, E.M., Silhavy, J.L., Brancati, F., Barrano, G., Krishnaswami, S.R., Castori, M., Lancaster, M.A., Boltshauser, E., Boccone, L., Al-Gazali, L. et al (2006). Mutations in CEP290, which encodes a centrosomal protein, cause pleiotropic forms of Joubert syndrome. Nat Genet 38, 623-625.
12. Coppieters, F., Casteels, I., Meire, F., De Jaegere S., Hooghe, S., van Regemorter N., Van Esch H., Matuleviciene, A., Nunes, L., Meersschaut, V. et al (2010). Genetic screening of LCA in Belgium: predominance of CEP290 and identification of potential modifier alleles in AHI1 of CEP290-related phenotypes. Hum Mutat 31, E1709-E1766.
13. Littink, K.W., Pott, J.W., Collin, R.W.J., Kroes, H.Y., Verheij, J.B., Blokland, E.A., de Castro Miro M., Hoyng, C.B., Klaver, C.C., Koenekoop, R.K. et al (2010). A novel nonsense mutation in CEP290 induces exon skipping and leads to a relatively mild retinal phenotype. Invest Ophthalmol Vis Sci 51, 3646-3652.
14. Bainbridge, J.W., Smith, A.J., Barker, S.S., Robbie, S., Henderson, R., Balaggan, K., Viswanathan, A., Holder, G.E., Stockman, A., Tyler, N. et al (2008). Effect of gene therapy on visual function in Leber's congenital amaurosis. N Engl J Med 358, 2231-2239.
15. Cideciyan, A.V., Aleman, T.S., Boye, S.L., Schwartz, S.B., Kaushal, S., Roman, A.J., Pang, J.J., Sumaroka, A., Windsor, E.A., Wilson, J.M. et al (2008). Human gene therapy for RPE65 isomerase deficiency activates the retinoid cycle of vision but with slow rod kinetics. Proc Natl Acad Sci U S A 105, 15112-15117.
16. Hauswirth, W., Aleman, T.S., Kaushal, S., Cideciyan, A.V., Schwartz, S.B., Wang, L., Conlon, T., Boye, S.L., Flotte, T.R., Byrne, B. et al (2008). Phase I Trial of Leber Congenital Amaurosis due to Estrada- Mutations by Ocular Subretinal Injection of Adeno-Associated Virus Gene Vector: Short-Term Results. Hum Gene Ther
17. Maguire, A.M., Simonelli, F., Pierce, E.A., Pugh, E.N., Jr., Mingozzi, F., Bennicelli, J., Banfi, S., Marshall, K.A., Testa, F., Surace, E.M. et al (2008). Safety and efficacy of gene transfer for Leber's congenital amaurosis. N Engl J Med 358, 2240-2248.
18. Maguire, A.M., High, K.A., Auricchio, A., Wright, J.F., Pierce, E.A., Testa, F., Mingozzi, F., Bennicelli, J.L., Ying, G.S., Rossi, S. et al (2009). Age-dependent effects of RPE65 gene therapy for Leber's congenital amaurosis: a phase 1 dose-escalation trial. Lancet 374, 1597-1605.
19. den Hollander, A.I., Black, A., Bennett, J., and Cremers, F.P.M. (2010). Lighting a candle in the dark: advances in genetics and gene therapy of recessive retinal dystrophies. J Clin Invest 120, 3042-3053.
20. Aartsma-Rus, A., Houlleberghs, H., van Deutekom, J.C., van Ommen, G.J., and 't Hoen, P.A. (2010). Exonic sequences provide better targets for antisense oligonucleotides than splice site sequences in the modulation of Duchenne muscular dystrophy splicing. Oligonucleotides 20, 69-77.
21. Aartsma-Rus, A., van, V.L., Hirschi, M., Janson, A.A., Heemskerk, H., de Winter, C.L., de, K.S., van Deutekom, J.C., 't Hoen, P.A., and van Ommen, G.J. (2008). Guidelines for Antisense Oligonucleotide Design and Insight Into Splice-modulating Mechanisms. Mol Ther
22. Smith, P.J., Zhang, C., Wang, J., Chew, S.L., Zhang, M.Q., and Krainer, A.R. (2006). An increased specificity score matrix for the prediction of SF2/ASF-specific exonic splicing enhancers. Hum Mol Genet 15, 2490-2508.
23. Schmid, C.W. and Jelinek, W.R. (1982). The Alu family of dispersed repetitive sequences. Science 216, 1065-1070.
24. Hammond, S.M. and Wood, M.J. (2011). Genetic therapies for RNA mis-splicing diseases. Trends Genet 27, 196-205.
25. Kinali, M., rechavala-Gomeza, V., Feng, L., Cirak, S., Hunt, D., Adkin, C., Guglieri, M., Ashton, E., Abbs, S., Nihoyannopoulos, P. et al (2009). Local restoration of dystrophin expression with the morpholino oligomer AVI-4658 in Duchenne muscular dystrophy: a single-blind, placebo-controlled, dose-escalation, proof-of-concept study. Lancet Neurol 8, 918-928.
26. van Deutekom, J.C., Janson, A.A., Ginjaar, I.B., Frankhuizen, W.S., Aartsma-Rus, A., Bremmer-Bout, M., Den Dunnen, J.T., Koop, K., van der Kooi, A.J., Goemans, N.M. et al (2007). Local dystrophin restoration with antisense oligonucleotide PRO051. N Engl J Med 357, 2677-2686.
27. Coppieters, F., Lefever, S., Leroy, B.P., and De, B.E. (2010). CEP290, a gene with many faces: mutation overview and presentation of CEP290base. Hum Mutat 31, 1097-1108.
28. Cideciyan, A.V., Aleman, T.S., Jacobson, S.G., Khanna, H., Sumaroka, A., Aguirre, G.K., Schwartz, S.B., Windsor, E.A., He, S., Chang, B. et al (2007). Centrosomal-ciliary gene CEP290/NPHP6 mutations result in blindness with unexpected sparing of photoreceptors and visual brain: implications for therapy of Leber congenital amaurosis. Hum Mutat 28, 1074-1083.
29. Geib, T. and Hertel, K.J. (2009). Restoration of full-length SMN promoted by adenoviral vectors expressing RNA antisense oligonucleotides embedded in U7 snRNAs. PLoS One 4, e8204.
30. Goyenvalle, A., Vulin, A., Fougerousse, F., Leturcq, F., Kaplan, J.C., Garcia, L., and Danos, O. (2004). Rescue of dystrophic muscle through U7 snRNA-mediated exon skipping. Science 306, 1796-1799.
31. Allocca, M., Mussolino, C., Garcia-Hoyos, M., Sanges, D., Iodice, C., Petrillo, M., Vandenberghe, L.H., Wilson, J.M., Marigo, V., Surace, E.M. et al (2007). Novel adeno-associated virus serotypes efficiently transduce murine photoreceptors. J Virol 81, 11372-11380.
32. Lebherz, C., Maguire, A., Tang, W., Bennett, J., and Wilson, J.M. (2008). Novel AAV serotypes for improved ocular gene transfer. J Gene Med 10, 375-382.
33. Simonelli, F., Maguire, A.M., Testa, F., Pierce, E.A., Mingozzi, F., Bennicelli, J.L., Rossi, S., Marshall, K., Banfi, S., Surace, E.M. et al (2009). Gene Therapy for Leber's Congenital Amaurosis is Safe and Effective Through 1.5 Years After Vector Administration. Mol Ther
34. Li, W., Kong, F., Li, X., Dai, X., Liu, X., Zheng, Q., Wu, R., Zhou, X., Lu, F., Chang, B. et al (2009). Gene therapy following subretinal AAV5 vector delivery is not affected by a previous intravitreal AAV5 vector administration in the partner eye. Mol Vis 15, 267-275.
35. Vandenberghe, L.H., Bell, P., Maguire, A.M., Cearley, C.N., Xiao, R., Calcedo, R., Wang, L., Castle, M.J., Maguire, A.C., Grant, R. et al (2011). Dosage Thresholds for AAV2 and AAV8 Photoreceptor Gene Therapy in Monkey. Sci Transl Med 3, 88ra54.
36. Baye, L.M., Patrinostro, X., Swaminathan, S., Beck, J.S., Zhang, Y., Stone, E.M., Sheffield, V.C., and Slusarski, D.C. (2011). The N-terminal region of centrosomal protein 290 (CEP290) restores vision in a zebrafish model of human blindness. Hum Mol Genet 20, 1467-1477.
37. Dorn and Kippenberger, Curr Opin Mol Ther 2008 10(1) 10-20
38. Nielsen, et al. (1991) Science 254, 1497-1500
39. Govindaraju and Kumar (2005) Chem. Commun, 495-497
40. Egholm et al (1993) Nature 365, 566-568
41. Morita et al. 2001. Nucleic Acid Res Supplement No. 1: 241-242
42. Gorman L, et al, Stable alteration of pre-mRNA splicing patterns by modified U7 small nuclear RNAs. Proc Natl Acad Sci U S A 1998;95(9):4929-34
43. Suter D, et al, Double-target antisense U7 snRNAs promote efficient skipping of an aberrant exon in three human beta-thalassemic mutations. Hum Mol Genet 1999;8(13):2415-23
44. Remington: The Science and Practice of Pharmacy, 20th Edition. Baltimore, MD: Lippincott Williams & Wilkins, 2000

### EXAMPLE 2: AON's delivered by Adeno Associated Viral vectors.

### ABSTRACT

Leber congenital amaurosis (LCA) is a genetically heterogeneous disorder characterized by severe visual impairment starting in the first year of life. The most frequent genetic cause of LCA, present in up to 15% of all LCA cases in some Western populations, is an intronic mutation in *CEP290* (c.2991+1655A>G) that creates a cryptic slice donor site and results in the insertion of an aberrant exon into *CEP290* mRNA. In example 1, we have shown that antisense oligonucleotides (AONs) effectively restore normal *CEP290* splicing in patient-derived lymphoblastoid cells. Given the safety and efficacy of adeno-associated viruses (AAVs) used in ongoing clinical trials for other genetic subtypes of retinal dystrophy, we here aimed to explore the therapeutic potential of AAV-based delivery of AONs. Transduction of patient-derived fibroblast cells with effective AONs cloned into a modified U7snRNA construct and packaged into AAV2/2 fully restored normal *CEP290* pre-mRNA splicing and significantly increased CEP290 protein levels. Moreover, a ciliary phenotype present in these fibroblasts was completely rescued upon transduction of AON-containing AAVs. Together, our data show that AAVs are an excellent therapeutic vector for the delivery of AONs to restore splice defects, and highlight the tremendous potential of AONs for the treatment of *CEP290*-associated LCA.

### INTRODUCTION

Leber congenital amaurosis (LCA; OMIM 204000) is a group of early-onset, rare and severe inherited retinal dystrophies (IRDs) with a prevalence of ∼1:50,000 in the European and North-American populations (*1*, *2*). The clinical characteristics of LCA are severe and early loss of vision, amaurotic pupils, sensory nystagmus and the absence of electrical signals on electroretinogram (ERG) (*3*). LCA shows a high genetic heterogeneity and to date 20 different genes have been associated with LCA (RetNet: https://sph.uth.edu/retnet), mainly segregating in an autosomal recessive manner. The most frequently mutated LCA gene is *CEP290* (centrosomal protein 290 kDa) (*3*-*5*), a gene that encompasses 54 exons and encodes a 2479 amino acid protein (*6*) localized in the centrosome and in the basal body of cilia (*7*). Of all *CEP290* mutations that cause non-syndromic LCA, a deep-intronic variant (c.2991+1655A>G) is by far the most recurrent one, accounting for up to 15% of LCA cases in many Western countries (*2*, *5, 8, 9*). This mutation creates a cryptic splice donor site, resulting in the insertion of an aberrant exon with a premature stop codon into ∼50% of all *CEP290* transcripts (5).

For many years, retinal dystrophies (RDs) have been considered incurable diseases. However, in the last decade major progress has been accomplished, mainly in the field of gene therapy. Phase I/II clinical trials using gene augmentation therapy have shown to be safe and moderately effective in LCA and early-onset RD patients with mutations in *RPE65* (*10-13*), and in choroideremia patients with a mutation in *CHM (14).* In these studies, the wild-type cDNA of *RPE65* and *CHM*, respectively, was packaged in replication-defective recombinant adeno-associated viruses (AAVs) and delivered to the retina by a single surgical procedure. The restricted cargo size of AAVs (∼5 kb) however has so far hampered a fast and broad implementation of AAV-based gene augmentation therapy for the many other genetic subtypes of IRD, since the cDNA size of many genes that cause IRD, including that of *CEP290,* way exceed the cargo limit of AAVs.

One alternative strategy to treat *CEP290*-associated LCA utilizes antisense oligonucleotides (AONs), small RNA molecules that complementary bind to its target mRNA and subsequently can interfere with pre-mRNA splicing. AONs have already been shown to be effective therapeutic molecules in several inherited disorders *in vitro* (*15*) and are currently being used in clinical trials for Duchenne's muscular dystrophy *(16, 17*), several cancer types (*18*, *19*), familial hypercholesterolemia (*20*), viral infections or neovascular disorders (*21*) amongst others. The intronic *CEP290* mutation is an ideal target for AON-based therapy, as skipping the cryptic exon that is inserted to the *CEP290* mRNA as a result of the c.2991+1655A>G change, would fully restore normal *CEP290* splicing and restore wild-type CEP290 protein levels. Recently, we and others have shown that transfection of naked AON molecules indeed restores normal *CEP290* splicing in cultured cells of LCA patients homozygously carrying the intronic *CEP290* mutation (*22*, *23*)*.* In future trials however, administrating AONs to the retina of patients with *CEP290*-associated LCA would require multiple injections of naked AONs, as the stability of these molecules depends among others on chemical modifications, target cell or tissue, and ranges from hours to months (*21, 24*). An alternative way of delivering AONs would be to use AAVs, as a single subretinal injection of AAV could give rise to a persistent expression, potentially life long, as shown in dog and primate models (*25, 26*). Hence, we here investigated the therapeutic efficacy of AONs that are cloned into AAVs, and provide evidence that AAV-based delivery of AONs is an excellent approach to treat *CEP290*-associated LCA.

### MATERIAL AND METHODS

### Study design

The objective of this work is to assess the efficacy of AAV-mediated AON-based therapy for *CEP290*-associated LCA. Fibroblast cell lines derived from two unrelated LCA patients homozygously carrying the c.2991+1655A>G mutation, and from two age- and gender matched healthy individuals were used. Outcome measures include the correction of aberrant *CEP290* splicing via RT-PCR, the assessment of CEP290 protein levels via Western blot analysis, and a qualitative and quantitative characterization of cilium structure via immunofluorescence microscopy. All experiments were performed simultaneously in all cell lines.

### Ethics statement

Our research was conducted according to the tenets of the Declaration of Helsinki. The procedures for obtaining human skin biopsies to establish primary fibroblasts cell lines were approved by the Ethical Committee of the Radboud University Medical Centre (*Commissie Mensgebonden Onderzoek* Arnhem-Nijmegen). Written informed consent was gathered from all participating individuals by signing the Declaration of Permission for the Use of Body Material (*Toestemmingsverklaring gebruik lichaamsmateriaal*) of the Radboud University Medical Centre. All procedures were carried out in the Netherlands.

### Construct design

AON sequences were cloned into a modified U7snRNA gene in the pSMD2 shuttle vector that contains the inverted terminal repeat (ITR) sequences for AAV production, via a two-step PCR approach as described elsewhere (27). This yielded six different AON-containing vectors and one control vector (Figure 4A), coined pAAV-AON1 to pAAV-AON6.

Previously, we cloned a ∼6.4 kb fragment of the *CEP290* gene that contained part of intron 25, exon 26, intron 26 (either with or without the intronic mutation), exon 27 and part of intron 27 (*28*), and inserted into the pCI-Neo plasmid flanked by the exon 3 and 5 of the *Rhodopsin* gene (*29*). These constructs are referred to as WT and LCA minigene constructs.

### Cell culture and transfection

Fibroblast cell lines derived from skin biopsies of individuals with CEP290-associated LCA or healthy controls were cultured in DMEM, supplemented with 20% fetal bovine serum (FBS), 1% penicillin-streptomycin and 1% of sodium pyruvate at 37 °C and 5% CO₂. HEK293T cells were cultured in DMEM supplemented with 10% FBS, 1% penicillin-streptomycin and 1% sodium pyruvate at 37 °C and 5% CO₂.

In order to validate the effectiveness of the AON-containing vectors, serial dilutions were carried out by co-transfecting 1 µg of the LCA minigene construct, together with various amounts of each pAAV-AON vector (0.5 µg; 0.125 µg and 0.035 µg) in human embryonic kidney (HEK293T) cells. Co-transfections were performed by combining the two plasmids with FuGene (Promega, Madison, WI) reagent (1:3 ratio) following manufacturer's protocol. Naked AON (0.1 µmol/l) was used as a positive control. Cells were harvested for transcriptional analysis 48 h post-transfection.

### AON-containing AAV generation

The two most effective AONs were selected for AAV2/2 production. Plasmid DNA was purified by the Megaprep kit (Qiagen, Venlo, the Netherlands). The U7snRNA-AON constructs were packaged into AAV by transfection of three plasmids (AAV pSMD2 plasmid containing the U7snRNA-AON, AAV package plasmid encoding AAV Rep and Cap proteins from serotype 2 and adenovirus helper plasmid) in HEK 293 cells. Three days after transfection, cells and culture medium were collected and enzymatically treated with Benzonase and a high salt solution. The cell debris was removed by high speed centrifugation and regular filtration. The supernatant went through a tangential flow filter which concentrated the viral solution. Recombinant AAV vector particles were isolated and extracted by running the concentrated supernatant through the iodixanol density gradient. The purified supernatant was then further concentrated by running through an Amicon filter with a 100,000 molecular weight cut off. The pure AAVs were then tittered by real-time PCR and the purity was verified by SDS page gel electrophoresis. For assessing which AAV serotype most effectively transduces fibroblast cells, an existing panel of AAVs containing an expression cassette of GFP under control of the cytomegalovirus (CMV) promoter was used.

### AAV transduction in fibroblasts

Fibroblast cell lines were transfected or transduced as follow. For transfection, cells were seeded in the corresponding plate according to the amount of cells needed and, transfected with 0.1 µmol of naked AON using FuGene HD (Promega, Madison, WI) as described before. For transduction, cells were transduced with AAV at different multiplicities of infection (MOIs) (100; 1,000 and 10,000) and medium was replaced after 24 h. Cells were harvested 96 h later for transcriptional analysis. For protein and immunocytochemistry studies cells were transduced with a MOI of 10,000 or transfected with 0.1 µmol of naked AON. For protein analysis 1,800,000 cells were seeded in 10 cm dishes and harvested 96 h following transfection/transduction. For immunocytochemistry, fibroblast cells were seeded on coverslips in a 12-well plate and 48 h following transfection/transduction, medium was replaced for a low FBS (0.2%) containing medium for another 48 h.

### RNA isolation and RT-PCR analysis

Fibroblast cells were used for RNA isolation (Nucleospin RNA II, Düren, Germany) following the manufacturer's protocol. Five hundred nanograms of RNA were used for cDNA synthesis by using the iScript cDNA Synthesis kit (Bio-Rad, Hercules, CA) at a final volume of 20 µl and then diluted 3.5 times by adding 50 µl of RNAse-free H₂O. All the PCR reaction mixtures (25 µl) contained 10 µM of each primer pair, 2 µM of dNTPs, 1.5 mM MgCl₂, 10% Q-solution (Qiagen, Venlo, Netherlands), 1 U of *Taq* polymerase (Roche, Penzberg, Germany) and 5 µl of diluted cDNA. PCR conditions were 94 °C for 2 min, followed by 35 cycles of 20 s at 94 °C, 30 s at 58 °C and 30 s at 72 °C, with a final extension step of 2 min at 72 °C. Amplicons were analyzed by agarose electrophoresis. Actin expression was used to compare and normalize samples. For co-transfection of the minigene with the AON vectors, Rhodopsin and U7 primers were used to assess the transfection efficiency. All oligonucleotide sequences are listed in Supplementary Table 1.

### Western blot analysis

Fibroblast cells were homogenized in 100 µl of RIPA buffer (50 mM Tris pH 7.5, 1 mM EDTA, 150 mM NaCl, 0.5% Na-Deoxycholate, 1% NP40 plus protease inhibitors). Total protein was quantified using the BCA kit (Thermo Fisher Scientific, Waltham, MA). For CEP290 detection, ∼75 µg of total protein lysate supplemented with sample buffer was loaded onto a NuPage 3-8% tris-acetate gel (Life technologies, Carlsbad, CA). The electrophoresis was carried out for 4 h at 150 V. For normalization, ∼25 µg of the same protein lysates were loaded onto a NuPage 4-12% bis-acrylamide tris-glycine gel (Life technologies, Carlsbad, CA) for detection of α-tubulin and run for 2 h at 150 V. All lysates were boiled for 5 min at 98 °C prior to loading. Proteins were transferred to a PVDF membrane (GE Healthcare, Little Chalfont, UK) overnight at 25 V and 4 °C. Blots were blocked in 5% non-fat milk in PBS for 6 h at 4 °C, incubated overnight at 4 °C with rabbit anti-CEP290 (dilution 1:750, Novus Biological, Littleton, CO) or mouse anti-α-tubulin (dilution 1:2,000, Abcam, Cambridge, UK) in 0.5% non-fat milk in PBS solution, washed in PBST (4 x 5 min), incubated with the appropriate secondary antibody for 1 h at room temperature (RT), washed in PBST (4 x 5 min) and developed using the Odissey Imaging System (Li-Cor Biosciences, Lincoln, NE). Semi-quantification was performed using Image J software (*30*)*.*

### Immunofluorescence analysis

Cells were grown on coverslips in 12 well plates and transfected with 0.1 µmol of naked AON or transduced with AAVs (MOI of 10,000). After 48 h of serum starvation, cells were rinsed with 1X PBS (137 mM NaCl, 2.7 mM KCl, 1.5 mM KH₂PO₄, and 8 mM Na₂HPO₄, pH 7.4), fixed in 2% paraformaldehyde for 20 min, permeabilized in PBS with 0,1% Triton X for 5 min and blocked for 30 min with 2% BSA in PBS at RT. Primary antibody was diluted in blocking solution and incubated for 90 min at RT. Subsequently, slides were washed 5 min in PBS three times, incubated with 1:500 dilution of the corresponding Alexa Fluor-conjugated antibody (Molecular Probes, Eugene, OR) for 45 min. Finally, slides were washed in PBS 3 x 5 min and mounted in Vectashield with DAPI (Vector laboratories, Burlingame, CA). Primary antibodies dilutions were 1:1,000 for mouse anti-acetylated tubulin (Sigma-Aldrich, St. Louis, MO) and 1:300 for rabbit anti-CEP290 (Novus Biological, Littleton, CO). Pictures were taken at 40x with Axio Imager (Zeiss, Oberkochen, Germany) microscope. For each condition, at least 150 ciliated cells were counted and their cilia were measured by using Image J software (*30*).

### Statistical analysis

In order to study the differences between treated and untreated cells we applied the two-tailed Student's T and Mann-Whitney tests. P-values smaller than 0.05 were considered significant as indicated in the figures. Statistical analysis was performed for the quantification of the CEP290 protein levels, as well as the ciliation and cilium length measurements.

### RESULTS

In this study, we aimed to explore the therapeutic efficacy of AAV-based delivery of antisense oligonucleotides, to correct a splice defect resulting from a recurrent intronic mutation in *CEP290.* Previously, we showed that delivery of naked AONs restores normal *CEP290* splicing in lymphoblastoid cells from LCA patients homozygously carrying the intronic *CEP290* mutation (*22*). To determine the consequences of splice correction at the protein and cellular level, fibroblast cell lines were generated from LCA patients with the intronic *CEP290* mutation, as these fibroblasts, in contrast to lymphoblastoid cells, endogenously express the CEP290 protein and develop cilia when cultured under serum starved conditions. Similar to what was observed in lymphoblasts (*22*), transfection of naked AONs to the patient's fibroblast cells completely restored normal *CEP290* splicing, with a minimal effective concentration of 0.05 µmol/l (data not shown). In order to assess the efficacy of AAV-mediated AON delivery, the naked AON was used as a positive control in all experiments, at a final concentration of 0.1 µmol/l.

In order to deliver AONs in an adeno-associated viral context, a modified U7snRNA construct was used. This allows the synthesis of the RNA molecules and an effective delivery of AONs to the right nuclear compartment for splicing intervention (*27*). Different AON sequences (or combinations thereof) were cloned into the pSMD2 vector that contains the modified U7snRNA as well as inverted terminal repeat (ITR) sequences (*27*) needed for AAV generation (Figure 4A), yielding six different constructs with AONs (pAAV-AON1 to pAAV-AON6), as well as a construct without any AON that served as a negative control (pAAV-NoAON). However, upon transfection of these constructs into the patient's fibroblasts, no decrease in the amount of aberrantly spliced *CEP290* transcript was observed, due to the very low transfection efficiency in this cell type (data not shown). In order to assess the potential therapeutic efficacy of the generated constructs, two *CEP290* minigene constructs were generated that contained ∼6.4 kb of the human *CEP290* gene, including exon 26, the complete intron 26 (with and without the intronic mutation), and exon 27, flanked by two exons of the *RHO* gene (Figure 4B). Transfection of these constructs in human embryonic kidney (HEK293T) cells revealed that the proportion of aberrantly vs. correctly spliced *CEP290* transcripts was comparable to that observed in LCA fibroblast cells (Figure 4C), hence mimicking the molecular consequences of the intronic *CEP290* mutation in these cells. Subsequent co-transfection of the *CEP290* minigene construct with the six different pAAV-AON constructs into HEK293T cells revealed that all constructs were able to redirect normal *CEP290* splicing (Figure 5). To identify the most potent vectors, decreasing quantities of pAAV-AON constructs were transfected, revealing pAAV-AON4 and pAAV-AON5 as the most effective ones, as these were still able to fully restore *CEP290* splicing at the lowest concentration tested (Figure 5). Thus, pAAV-AON4 and pAAV-AON5 were selected for the generation of AAVs, together with the pAAV-NoAON construct.
The AAV capsid serotype determines the capability to infect certain cells or tissues. To determine which serotype was most suitable for our experiments, we transduced 6 different AAV serotypes carrying the cDNA of the GFP under control of a CMV promoter. AAV2/2 showed the highest transduction efficiency, already at an MOI of 100, in both control and LCA patient fibroblast cells (data not shown). Higher MOIs determined that AAV2/9 was also able to infect LCA patient fibroblast cells (data not shown), but AAV2/2 was selected for the generation of the AON-containing AAVs.

Following the generation of the three AAVs (i.e. AAV-NoAON, AAV-AON4 and AAV-AON5), the fibroblast cell lines of two unrelated LCA patients homozygously carrying the intronic *CEP290* mutation (LCA1 and LCA2) were transduced with these AAVs, at three different multiplicities of infection (MOI), or transfected with the naked AON that served as a positive control. RT-PCR analysis revealed that transduction of the two AON-containing AAVs almost completely restored normal *CEP290* splicing, with the highest efficacy observed at an MOI of 10,000 (Figure 6). In contrast, AAV-NoAON showed the same pattern as the untransduced cells, indicating that the rescue of aberrant *CEP290* splicing was caused by the actual AON sequences. Transduction of two control fibroblast cell lines (C1 and C2) with the different AAVs did not show any difference in the levels of expression of *CEP290* mRNA (data not shown).

Next, we assessed whether restoring normal *CEP290* splicing resulted in an increase of wild-type CEP290 protein levels. Patient and control fibroblast cells were transduced with the three AAVs at an MOI of 10,000, or transfected with the naked AONs. Protein lysates were subjected to Western blot analysis, using α-tubulin as a loading control. Whereas in the untreated fibroblast cells from the LCA patients, CEP290 protein levels were markedly reduced, cells transduced with AAV-AON4 and AAV-AON5 showed a significant increase in CEP290 protein levels, almost reaching those observed in healthy controls (Figure 7A and 7B). Again, no differences were observed between the untransduced cells and those transduced with AAV-NoAON (Figure 7A and 7B). In addition, we studied the effect of AAV transduction in control fibroblasts, and no change in CEP290 protein levels was observed for any of the conditions tested (data not shown).

CEP290 localizes in the basal body of the cilium and is thought to play an important role in cilium development and/or ciliary transport (*7*, *31*)*.* When cultured under serum starving conditions, fibroblast cells develop cilia (32). When comparing the appearance of cilia in control fibroblasts vs. fibroblasts of LCA patients with the intronic *CEP290* mutation, a clear and statistically significant ciliary phenotype was observed, i.e. a reduced number of ciliated cells, and a shorter average length of the cilium (Figure 8). Remarkably, the fibroblast cells of the LCA patients displayed a high heterogeneity, showing three different appearances: no cilium, a short cilium or a normal cilium (Figure 8A1 and 8A2). Only the cells with a normal cilium showed a positive signal for CEP290 staining in the basal body, indicating that the cilium length is directly correlated to a proper expression and localization of the CEP290 protein. In addition, this variability suggests that the 1:1 ratio of the aberrant and normal transcripts is an average of a population of cells, and may differ amongst individual cells. To assess whether the ciliary phenotype could be restored by AONs, fibroblast cells were again transfected (0.1 µmol/l) or transduced (MOI of 10,000) with AONs or AAV-AONs and subjected to serum starvation 48 hours following transfection/transduction. Our results showed that the ciliary phenotype was completely rescued after 96 hour treatment (Figure 8A1 and 8A2), i.e. the number of ciliated cells as well as the average length of the cilium returned to the values observed in control fibroblasts (Figure 8B). Notable, the rescue of the ciliary phenotype was accompanied by a marked increase of CEP290 staining at the base of the cilium, as is apparent from the immunocytochemistry images presented in Figure 8A1 and 8A2. No differences in ciliation and cilium length were observed following AON treatment in control cell lines (data not shown).

### DISCUSSION

Mutations in *CEP290* are the most common genetic cause of LCA in the Caucasian population, accounting for up to 20% of the cases (*3*). Intruigingly, an intronic founder mutation in *CEP290* (c.2991+1655A>G) on itself explains already 15% of all LCA cases in several Western countries, including the US, France, Belgium and The Netherlands (*2*, *5*, *8, 9*), and shows a somewhat lower prevalence in other European countries (*33*). Therefore, *CEP290,* and in particular the intronic mutation, has emerged as an attractive target for developing genetic therapies. In this study, we show that AAV-based delivery of antisense oligonucleotides effectively rescues the cellular phenotype associated with the common intronic *CEP290* mutation, highlighting the enormous potential of this therapeutic approach.

For several genetic subtypes of IRD, preclinical therapeutic intervention studies are ongoing, with encouraging results in many of these studies (*34, 35*)*.* In addition, clinical trials employing viral-based gene augmentation therapy have been initiated for five different genetic subtypes of IRD, i.e. caused by mutations in *ABCA4, CHM, MERTK, MYO7A* and *RPE65.* Subretinal delivery of AAVs carrying wild-type *RPE65* cDNA to the retinal pigment epithelium showed, besides some moderate improvement in visual function (add the same four references as in introduction) a high safety profile, with no risk of insertional mutagenesis, and very low or absent immune responses to the AAV, even upon readministration of the virus in the second eye (*36*). More recently, AAV-based delivery of *CHM* cDNA also turned out to be safe and moderately effective in six treated patients with choroideremia (*14*). A serious constraint of AAVs however is their limited cargo capacity, as only transgenes smaller than 5 kb can be efficiently packaged in these vectors (*34*). Therefore, many large genes are not eligible for AAV-mediated delivery, as is the case for *CEP290* whose coding sequence is ∼7.5 kb long. Recently, dual AAV strategies have been developed that consist of delivering two different AAV vectors, each containing half of the cDNA with a small overlapping region, allowing the assembly of the complete transcript in the target cell following transduction. Although promising results have been obtained in reconstituting full-length cDNAs of *ABCA4* and *MYO7A* in mutant mouse models for Stargardt's disease and Usher syndrome type 1B, respectively (*37*, *38*), not much success has been achieved so far for *CEP290* (Renee C. Ryals, personal communication). Alternatively, lentiviral vectors can carry cargos up to 10 kb (*34*), which would be sufficient for packaging the full-length *CEP290* cDNA. A disadvantage of lentiviruses however is that they integrate into the genome of the host cell, running the risk of insertional mutagenesis. In addition, another problem associated with gene augmentation is the fact that expression levels cannot be regulated, therefore increasing the risk of toxicity upon reaching expression levels exceeding those of the endogenous protein. Recently, it was shown that transduction of lentiviruses containing wild-type *CEP290* cDNA under control of a CMV promoter could rescue a ciliary phenotype in patient-derived fibroblast cells, although in the same study, toxic effects were observed upon transduction of these lentiviruses in other cell types (*39*). Together, these studies suggest that there are many challenges associated with developing gene augmentation therapy for *CEP290*-associated LCA. In contrast, antisense oligonucleotide therapy does not have these limitations, as these small AONs easily fit into AAVs, and since the endogenous mRNA is the therapeutic target, the maximum expression levels of the wild-type protein will never exceed that of the endogenous protein.

In this study, we employed patient-derived fibroblast cells as a model system to assess the efficacy of AAV-based AON delivery, since they endogenously express the CEP290 protein and develop cilia under serum starvation conditions. Although these cells are hard to transfect with plasmid DNA, transduction of AAVs was more efficient. When testing a panel of different AAV serotypes, AAV2/2 appeared to show the highest tropism for fibroblasts, although AAV2/5 and AAV2/9 also showed affinity for these cells and both AAV2/5 and AAV2/9 have a higher tropism for photoreceptor cells than AAV2/2 (*40, 41*)*.* Nevertheless, in order to have the optimal vector for the studies in our fibroblast model, AAV2/2 was selected for the generation of the AON-containing vectors. In our therapeutic construct, effective AON sequences were subcloned into a modified U7snRNA gene, a strategy that has previously been shown to be effective in redirecting splicing of the *DMD* gene (*27*). It remains to be determined whether the same construct is also effective in redirecting splicing in the context of a photoreceptor cell, ultimately the target cell for therapeutic intervention, but in fibroblast cells this molecular approach appears to be a very effective one.

Despite the suitability of the fibroblast cells as a preclinical model to assess the therapeutic efficacy of AON-based therapy, ideally one would like to study its potential in the context of a living animal, or at least in the context of a photoreceptor cell. We generated a humanized transgenic knock-in mouse model, where part of the mouse *Cep290* genomic DNA was replaced by its orthologous human counterpart, i.e. exon 26, intron 26 (including the LCA-causing mutation) and exon 27, allowing us to assess the therapeutic efficacy of AON therapy *in vivo*, by delivering AONs either as naked molecules or in AAVs. Unfortunately however, despite a correct genetic engineering of the transgenic mouse model, hardly any aberrant splicing of *Cep290* mRNA nor a concomitant retinal phenotype was observed in these mice (28), rendering this model inappropriate for further studies. An alternative model that can be used to assess the potential of AON therapy is the induced pluripotent stem cell (iPSC)-derived photoreceptor cells. The discovery that four basic transcription factors can transform fully differentiated adult cells into cells with pluripotent capacity (42) has revolutionized the field of stem cell biology, and has resulted in establishing procedures to successfully differentiate such cells to human photoreceptor-like cells in a culture dish (*43-48*). Starting with for instance a fibroblast cell line of an IRD patient, it is possible to generate photoreceptor-like cells in the presence of the primary IRD-causing mutation(s), allowing to study the pathophysiological mechanisms that underlie the phenotype (*43, 45*), as well to assess the efficacy of potential therapeutic strategies in a relevant molecular environment. In the case of *CEP290*-associated LCA, AONs could be administered to these cells either as naked molecules or packaged in AAVs, and the potential rescue of aberrant *CEP290* splicing, CEP290 protein levels, and a ciliary defect could be readily assessed. In addition, delivering AONs to iPSC-derived photoreceptor-like cells provides an opportunity to assess potential off-target effects of the AON via transcriptome sequencing, as these cells show a similar transcriptional profile compared to the real photoreceptors in the human eye. Of note, AONs hybridize to pre-mRNA, are selected to have unique targets and usually a single mismatch already prevents the binding to other targets and hence the ability to interfere with other pre-mRNA splicing events (15).

So far, AONs have been used in clinical trials for other ocular diseases such as CMV-induced retinitis to decrease the viral load in AIDS patients (Vitravene®), or diabetic macula edema and diabetic retinopathy to downregulate *c-Raf* expression and thereby decrease neovascularization (iCo-007) (*21*). In addition, a systemic delivery of AONs was recently shown to be successful in a humanized mouse model for Usher syndrome type 1C, characterized by a combination of hearing deficits, vestibular impairment and retinal dystrophy. Intraperitoneal injection of naked AONs targeting a cryptic splice site caused by a recurrent mutation in *Ush1C*, resulted in an increased level of correctly spliced *Ush1C* mRNA, and a strong improvement in auditory and vestibular function in these mice (*49*). A systemic delivery of AONs, whether as naked molecules or packaged in AAVs, however, would require high amounts of AON and increase the chances of evoking immune responses. The fact that the eye is an immune-privileged organ that is tightly regulated to preserve its integrity (*50*), confers the possibility to deliver the AON molecules directly to the retina, either by intravitreal or subretinal injections with little expected immunological side effects (*34*).

In terms of future therapeutic intervention in humans, there are several pros and cons to either an AAV-based administration of AONs, or delivery as a naked molecule. Due to their small size, naked AONs may be able to penetrate and reach the photoreceptor cells in the retina more easy upon intravitreal injections, compared to the subretinal delivery of currently used AAVs. Also, as naked AONs have a limited stability (ranging from weeks to months depending on the modifications added to the oligonucleotide), potential negative side effects of the AONs would also disappear after some time. Nevertheless, the use of naked AONs would require multiple, life-long injections, as *CEP290*-associated LCA manifests already in childhood. In contrast, a single administration of an effective AAV could give therapeutic benefit for many years, potentially life-long. And although with current subretinal surgery, only part of the retina can be targeted, new subclasses of AAVs are currently being developed that would allow a more easy intravitreal delivery, and a more effective targeting of the retina.
One other aspect that determines future therapeutic success, is the preservation of the retina at the time of treatment. Despite the early-onset and severe nature of the visual impairment in *CEP290*-associated LCA, the integrity of the photoreceptor layer appears to be relatively well conserved in some patients with *CEP290*-associated LCA, up to young adulthood (*51, 52*). The same is true for the connections of the visual pathway in the brain, giving hope that these patients are able to process and interpret the visual input that would become available following effective treatment (*51*). Despite this therapeutic window of opportunity, previous studies have shown a clear correlation between the age of treatment and therapeutic outcome (*13*), suggesting that LCA patients with *CEP290*-associated LCA may benefit most from early treatment.

In conclusion, we here show the therapeutic efficacy of AAV-mediated delivery of AONs to treat the most common genetic form of childhood blindness, i.e. *CEP*290-associated LCA. In fibroblast cells from LCA patients, aberrant *CEP290* splicing was corrected, CEP290 protein levels were restored, and a ciliary phenotype was completely rescued following AON administration. This unique combination of splice correction therapy and the use of safe AAV technology, provides an excellent treatment strategy that would require only a single surgical procedure. With that, AON-based therapy could be an effective way to halt the progression or even improve visual function in many severely impaired individuals worldwide.

### REFERENCE LIST Example 2

1. R. K. Koenekoop, An overview of Leber congenital amaurosis: a model to understand human retinal development. Survey of ophthalmology 49, 379-398 (2004).
2. E. M. Stone, Leber congenital amaurosis - a model for efficient genetic testing of heterogeneous disorders: LXIV Edward Jackson Memorial Lecture. American journal of ophthalmology 144, 791-811 (2007).
3. A. I. den Hollander, R. Roepman, R. K. Koenekoop, F. P. Cremers, Leber congenital amaurosis: genes, proteins and disease mechanisms. Progress in retinal and eye research 27, 391-419 (2008).
4. F. Coppieters, S. Lefever, B. P. Leroy, E. De Baere, CEP290, a gene with many faces: mutation overview and presentation of CEP290base. Human mutation 31, 1097-1108 (2010).
5. A. I. den Hollander, R. K. Koenekoop, S. Yzer, I. Lopez, M. L. Arends, K. E. Voesenek, M. N. Zonneveld, T. M. Strom, T. Meitinger, H. G. Brunner, C. B. Hoyng, L. I. van den Born, K. Rohrschneider, F. P. Cremers, Mutations in the CEP290 (NPHP6) gene are a frequent cause of Leber congenital amaurosis. American journal of human genetics 79, 556-561 (2006).
6. T. Nagase, K. Ishikawa, D. Nakajima, M. Ohira, N. Seki, N. Miyajima, A. Tanaka, H. Kotani, N. Nomura, O. Ohara, Prediction of the coding sequences of unidentified human genes. VII. The complete sequences of 100 new cDNA clones from brain which can code for large proteins in vitro. DNA research : an international journal for rapid publication of reports on genes and genomes 4, 141-150 (1997).
7. B. Craige, C. C. Tsao, D. R. Diener, Y. Hou, K. F. Lechtreck, J. L. Rosenbaum, G. B. Witman, CEP290 tethers flagellar transition zone microtubules to the membrane and regulates flagellar protein content. The Journal of cell biology 190, 927-940 (2010).
8. F. Coppieters, I. Casteels, F. Meire, S. De Jaegere, S. Hooghe, N. van Regemorter, H. Van Esch, A. Matuleviciene, L. Nunes, V. Meersschaut, S. Walraedt, L. Standaert, P. Coucke, H. Hoeben, H. Y. Kroes, J. Vande Walle, T. de Ravel, B. P. Leroy, E. De Baere, Genetic screening of LCA in Belgium: predominance of CEP290 and identification of potential modifier alleles in AHI1 of CEP290-related phenotypes. Human mutation 31, E1709-1766 (2010).
9. I. Perrault, N. Delphin, S. Hanein, S. Gerber, J. L. Dufier, O. Roche, S. Defoort-Dhellemmes, H. Dollfus, E. Fazzi, A. Munnich, J. Kaplan, J. M. Rozet, Spectrum of NPHP6/CEP290 mutations in Leber congenital amaurosis and delineation of the associated phenotype. Human mutation 28, 416 (2007).
10. W. W. Hauswirth, T. S. Aleman, S. Kaushal, A. V. Cideciyan, S. B. Schwartz, L. Wang, T. J. Conlon, S. L. Boye, T. R. Flotte, B. J. Byrne, S. G. Jacobson, Treatment of leber congenital amaurosis due to RPE65 mutations by ocular subretinal injection of adeno-associated virus gene vector: short-term results of a phase I trial. Human gene therapy 19, 979-990 (2008).
11. S. G. Jacobson, A. V. Cideciyan, R. Ratnakaram, E. Heon, S. B. Schwartz, A. J. Roman, M. C. Peden, T. S. Aleman, S. L. Boye, A. Sumaroka, T. J. Conlon, R. Calcedo, J. J. Pang, K. E. Erger, M. B. Olivares, C. L. Mullins, M. Swider, S. Kaushal, W. J. Feuer, A. Iannaccone, G. A. Fishman, E. M. Stone, B. J. Byrne, W. W. Hauswirth, Gene therapy for leber congenital amaurosis caused by RPE65 mutations: safety and efficacy in 15 children and adults followed up to 3 years. Archives of ophthalmology 130, 9-24 (2012).
12. A. M. Maguire, F. Simonelli, E. A. Pierce, E. N. Pugh, Jr., F. Mingozzi, J. Bennicelli, S. Banfi, K. A. Marshall, F. Testa, E. M. Surace, S. Rossi, A. Lyubarsky, V. R. Arruda, B. Konkle, E. Stone, J. Sun, J. Jacobs, L. Dell'Osso, R. Hertle, J. X. Ma, T. M. Redmond, X. Zhu, B. Hauck, O. Zelenaia, K. S. Shindler, M. G. Maguire, J. F. Wright, N. J. Volpe, J. W. McDonnell, A. Auricchio, K. A. High, J. Bennett, Safety and efficacy of gene transfer for Leber's congenital amaurosis. The New England journal of medicine 358, 2240-2248 (2008).
13. A. M. Maguire, K. A. High, A. Auricchio, J. F. Wright, E. A. Pierce, F. Testa, F. Mingozzi, J. L. Bennicelli, G. S. Ying, S. Rossi, A. Fulton, K. A. Marshall, S. Banfi, D. C. Chung, J. I. Morgan, B. Hauck, O. Zelenaia, X. Zhu, L. Raffini, F. Coppieters, E. De Baere, K. S. Shindler, N. J. Volpe, E. M. Surace, C. Acerra, A. Lyubarsky, T. M. Redmond, E. Stone, J. Sun, J. W. McDonnell, B. P. Leroy, F. Simonelli, J. Bennett, Age-dependent effects of RPE65 gene therapy for Leber's congenital amaurosis: a phase 1 dose-escalation trial. Lancet 374, 1597-1605 (2009).
14. R. E. MacLaren, M. Groppe, A. R. Barnard, C. L. Cottriall, T. Tolmachova, L. Seymour, K. R. Clark, M. J. During, F. P. Cremers, G. C. Black, A. J. Lotery, S. M. Downes, A. R. Webster, M. C. Seabra, Retinal gene therapy in patients with choroideremia: initial findings from a phase 1/2 clinical trial. Lancet 383, 1129-1137 (2014).
15. S. M. Hammond, M. J. Wood, Genetic therapies for RNA mis-splicing diseases. Trends in genetics : TIG 27, 196-205 (2011).
16. J. C. van Deutekom, A. A. Janson, I. B. Ginjaar, W. S. Frankhuizen, A. Aartsma-Rus, M. Bremmer-Bout, J. T. den Dunnen, K. Koop, A. J. van der Kooi, N. M. Goemans, S. J. de Kimpe, P. F. Ekhart, E. H. Venneker, G. J. Platenburg, J. J. Verschuuren, G. J. van Ommen, Local dystrophin restoration with antisense oligonucleotide PRO051. The New England journal of medicine 357, 2677-2686 (2007).
17. N. M. Goemans, M. Tulinius, J. T. van den Akker, B. E. Burm, P. F. Ekhart, N. Heuvelmans, T. Holling, A. A. Janson, G. J. Platenburg, J. A. Sipkens, J. M. Sitsen, A. Aartsma-Rus, G. J. van Ommen, G. Buyse, N. Darin, J. J. Verschuuren, G. V. Campion, S. J. de Kimpe, J. C. van Deutekom, Systemic administration of PRO051 in Duchenne's muscular dystrophy. The New England journal of medicine 364, 1513-1522 (2011).
18. H. P. Erba, H. Sayar, M. Juckett, M. Lahn, V. Andre, S. Callies, S. Schmidt, S. Kadam, J. T. Brandt, D. Van Bockstaele, M. Andreeff, Safety and pharmacokinetics of the antisense oligonucleotide (ASO) LY2181308 as a single-agent or in combination with idarubicin and cytarabine in patients with refractory or relapsed acute myeloid leukemia (AML). Investigational new drugs 31, 1023-1034 (2013).
19. A. W. Tolcher, J. Kuhn, G. Schwartz, A. Patnaik, L. A. Hammond, I. Thompson, H. Fingert, D. Bushnell, S. Malik, J. Kreisberg, E. Izbicka, L. Smetzer, E. K. Rowinsky, A Phase I pharmacokinetic and biological correlative study of oblimersen sodium (genasense, g3139), an antisense oligonucleotide to the bcl-2 mRNA, and of docetaxel in patients with hormone-refractory prostate cancer. Clinical cancer research : an official journal of the American Association for Cancer Research 10, 5048-5057 (2004).
20. C. Gebhard, G. Huard, E. A. Kritikou, J. C. Tardif, Apolipoprotein B antisense inhibition--update on mipomersen. Current pharmaceutical design 19, 3132-3142 (2013).
21. P. Hnik, D. S. Boyer, L. R. Grillone, J. G. Clement, S. P. Henry, E. A. Green, Antisense oligonucleotide therapy in diabetic retinopathy. Journal of diabetes science and technology 3, 924-930 (2009).
22. R. W. Collin, A. I. den Hollander, S. D. van der Velde-Visser, J. Bennicelli, J. Bennett, F. P. Cremers, Antisense Oligonucleotide (AON)-based Therapy for Leber Congenital Amaurosis Caused by a Frequent Mutation in CEP290. Molecular therapy. Nucleic acids 1, e14 (2012).
23. X. Gerard, I. Perrault, S. Hanein, E. Silva, K. Bigot, S. Defoort-Delhemmes, M. Rio, A. Munnich, D. Scherman, J. Kaplan, A. Kichler, J. M. Rozet, AON-mediated Exon Skipping Restores Ciliation in Fibroblasts Harboring the Common Leber Congenital Amaurosis CEP290 Mutation. Molecular therapy. Nucleic acids 1, e29 (2012).
24. N. Dias, C. A. Stein, Antisense oligonucleotides: basic concepts and mechanisms. Molecular cancer therapeutics 1, 347-355 (2002).
25. K. Stieger, J. Schroeder, N. Provost, A. Mendes-Madeira, B. Belbellaa, G. Le Meur, M. Weber, J. Y. Deschamps, B. Lorenz, P. Moullier, F. Rolling, Detection of intact rAAV particles up to 6 years after successful gene transfer in the retina of dogs and primates. Molecular therapy : the journal of the American Society of Gene Therapy 17, 516-523 (2009).
26. G. M. Acland, G. D. Aguirre, J. Bennett, T. S. Aleman, A. V. Cideciyan, J. Bennicelli, N. S. Dejneka, S. E. Pearce-Kelling, A. M. Maguire, K. Palczewski, W. W. Hauswirth, S. G. Jacobson, Long-term restoration of rod and cone vision by single dose rAAV-mediated gene transfer to the retina in a canine model of childhood blindness. Molecular therapy : the journal of the American Society of Gene Therapy 12, 1072-1082 (2005).
27. A. Goyenvalle, A. Vulin, F. Fougerousse, F. Leturcq, J. C. Kaplan, L. Garcia, O. Danos, Rescue of dystrophic muscle through U7 snRNA-mediated exon skipping. Science 306, 1796-1799 (2004).
28. A. Garanto, S. E. van Beersum, T. A. Peters, R. Roepman, F. P. Cremers, R. W. Collin, Unexpected CEP290 mRNA Splicing in a Humanized Knock-In Mouse Model for Leber Congenital Amaurosis. PloS one 8, e79369 (2013).
29. S. Shafique, S. Siddiqi, M. Schraders, J. Oostrik, H. Ayub, A. Bilal, M. Ajmal, C. Z. Seco, T. M. Strom, A. Mansoor, K. Mazhar, S. T. Shah, A. Hussain, M. Azam, H. Kremer, R. Qamar, Genetic spectrum of autosomal recessive non-syndromic hearing loss in pakistani families. PloS one 9, e100146 (2014).
30. W. S. Rasband, B. U. S. National Institutes of Health, Maryland, USA, Ed. (http://imagej.nih.gov/ij/, 1997-2012).
31. F. R. Garcia-Gonzalo, K. C. Corbit, M. S. Sirerol-Piquer, G. Ramaswami, E. A. Otto, T. R. Noriega, A. D. Seol, J. F. Robinson, C. L. Bennett, D. J. Josifova, J. M. Garcia-Verdugo, N. Katsanis, F. Hildebrandt, J. F. Reiter, A transition zone complex regulates mammalian ciliogenesis and ciliary membrane composition. Nature genetics 43, 776-784 (2011).
32. O. V. Plotnikova, E. N. Pugacheva, E. A. Golemis, Primary cilia and the cell cycle. Methods in cell biology 94, 137-160 (2009).
33. E. Vallespin, M. A. Lopez-Martinez, D. Cantalapiedra, R. Riveiro-Alvarez, J. Aguirre-Lamban, A. Avila-Fernandez, C. Villaverde, M. J. Trujillo-Tiebas, C. Ayuso, Frequency of CEP290 c.2991_1655A>G mutation in 175 Spanish families affected with Leber congenital amaurosis and early-onset retinitis pigmentosa. Molecular vision 13, 2160-2162 (2007).
34. C. L. Rowe-Rendleman, S. A. Durazo, U. B. Kompella, K. D. Rittenhouse, A. Di Polo, A. L. Weiner, H. E. Grossniklaus, M. I. Naash, A. S. Lewin, A. Horsager, H. F. Edelhauser, Drug and gene delivery to the back of the eye: from bench to bedside. Investigative ophthalmology & visual science 55, 2714-2730 (2014).
35. S. E. Boye, S. L. Boye, A. S. Lewin, W. W. Hauswirth, A comprehensive review of retinal gene therapy. Molecular therapy : the journal of the American Society of Gene Therapy 21, 509-519 (2013).
36. J. Bennett, M. Ashtari, J. Wellman, K. A. Marshall, L. L. Cyckowski, D. C. Chung, S. McCague, E. A. Pierce, Y. Chen, J. L. Bennicelli, X. Zhu, G. S. Ying, J. Sun, J. F. Wright, A. Auricchio, F. Simonelli, K. S. Shindler, F. Mingozzi, K. A. High, A. M. Maguire, AAV2 gene therapy readministration in three adults with congenital blindness. Science translational medicine 4, 120ra115 (2012).
37. P. Colella, I. Trapani, G. Cesi, A. Sommella, A. Manfredi, A. Puppo, C. Iodice, S. Rossi, F. Simonelli, M. Giunti, M. L. Bacci, A. Auricchio, Efficient gene delivery to the cone-enriched pig retina by dual AAV vectors. Gene therapy 21, 450-456 (2014).
38. V. S. Lopes, S. E. Boye, C. M. Louie, S. Boye, F. Dyka, V. Chiodo, H. Fofo, W. W. Hauswirth, D. S. Williams, Retinal gene therapy with a large MYO7A cDNA using adeno-associated virus. Gene therapy 20, 824-833 (2013).
39. E. R. Burnight, L. A. Wiley, A. V. Drack, T. A. Braun, K. R. Anfinson, E. E. Kaalberg, J. A. Halder, L. M. Affatigato, R. F. Mullins, E. M. Stone, B. A. Tucker, CEP290 gene transfer rescues Leber congenital amaurosis cellular phenotype. Gene therapy 21, 662-672 (2014).
40. E. M. Surace, A. Auricchio, Versatility of AAV vectors for retinal gene transfer. Vision research 48, 353-359 (2008).
41. L. H. Vandenberghe, P. Bell, A. M. Maguire, R. Xiao, T. B. Hopkins, R. Grant, J. Bennett, J. M. Wilson, AAV9 targets cone photoreceptors in the nonhuman primate retina. PloS one 8, e53463 (2013).
42. K. Takahashi, S. Yamanaka, Induction of pluripotent stem cells from mouse embryonic and adult fibroblast cultures by defined factors. Cell 126, 663-676 (2006).
43. B. A. Tucker, I. H. Park, S. D. Qi, H. J. Klassen, C. Jiang, J. Yao, S. Redenti, G. Q. Daley, M. J. Young, Transplantation of adult mouse iPS cell-derived photoreceptor precursors restores retinal structure and function in degenerative mice. PloS one 6, e18992 (2011).
44. D. A. Lamba, A. McUsic, R. K. Hirata, P. R. Wang, D. Russell, T. A. Reh, Generation, purification and transplantation of photoreceptors derived from human induced pluripotent stem cells. PloS one 5, e8763 (2010).
45. B. A. Tucker, R. F. Mullins, L. M. Streb, K. Anfinson, M. E. Eyestone, E. Kaalberg, M. J. Riker, A. V. Drack, T. A. Braun, E. M. Stone, Patient-specific iPSC-derived photoreceptor precursor cells as a means to investigate retinitis pigmentosa. eLife 2, e00824 (2013).
46. N. Amirpour, F. Karamali, F. Rabiee, L. Rezaei, E. Esfandiari, S. Razavi, A. Dehghani, H. Razmju, M. H. Nasr-Esfahani, H. Baharvand, Differentiation of human embryonic stem cell-derived retinal progenitors into retinal cells by Sonic hedgehog and/or retinal pigmented epithelium and transplantation into the subretinal space of sodium iodate-injected rabbits. Stem cells and development 21, 42-53 (2012).
47. Y. Seko, N. Azuma, M. Kaneda, K. Nakatani, Y. Miyagawa, Y. Noshiro, R. Kurokawa, H. Okano, A. Umezawa, Derivation of human differential photoreceptor-like cells from the iris by defined combinations of CRX, RX and NEUROD. PloS one 7, e35611 (2012).
48. N. Suzuki, J. Shimizu, K. Takai, N. Arimitsu, Y. Ueda, E. Takada, C. Hirotsu, T. Suzuki, N. Fujiwara, M. Tadokoro, Establishment of retinal progenitor cell clones by transfection with Pax6 gene of mouse induced pluripotent stem (iPS) cells. Neuroscience letters 509, 116-120 (2012).
49. J. J. Lentz, F. M. Jodelka, A. J. Hinrich, K. E. McCaffrey, H. E. Farris, M. J. Spalitta, N. G. Bazan, D. M. Duelli, F. Rigo, M. L. Hastings, Rescue of hearing and vestibular function by antisense oligonucleotides in a mouse model of human deafness. Nature medicine 19, 345-350 (2013).
50. I. Benhar, A. London, M. Schwartz, The privileged immunity of immune privileged organs: the case of the eye. Frontiers in immunology 3, 296 (2012).
51. A. V. Cideciyan, T. S. Aleman, S. G. Jacobson, H. Khanna, A. Sumaroka, G. K. Aguirre, S. B. Schwartz, E. A. Windsor, S. He, B. Chang, E. M. Stone, A. Swaroop, Centrosomal-ciliary gene CEP290/NPHP6 mutations result in blindness with unexpected sparing of photoreceptors and visual brain: implications for therapy of Leber congenital amaurosis. Human mutation 28, 1074-1083 (2007).
52. S. E. Boye, W. C. Huang, A. J. Roman, A. Sumaroka, S. L. Boye, R. C. Ryals, M. B. Olivares, Q. Ruan, B. A. Tucker, E. M. Stone, A. Swaroop, A. V. Cideciyan, W. W. Hauswirth, S. G. Jacobson, Natural history of cone disease in the murine model of Leber congenital amaurosis due to CEP290 mutation: determining the timing and expectation of therapy. PloS one 9, e92928 (2014).

### EXAMPLE 3: AON's delivered by Adeno Associated Viral vectors in vivo.

### INTRODUCTION

We aimed to determine the efficacy of AAV-AONs and that of naked AON molecules when delivered into the retina. For that purpose, we used our humanized *Cep290* mouse model (*Cep290*^{*lca*/*lca*}). This model contains the human exon 26, intron 26 with the c.2991+1655A>G mutation and exon 27. Previously, we showed that pseudo-exon-X is inserted to only a small proportion of *Cep290* transcripts, insufficient to cause a retinal phenotype (1). Fibroblast cells derived from this mouse model were transduced with AAV2/2-AON4 and -AON5, to determine which AAV-AON was most effective in a mouse molecular environment (data not shown). AON5 showed the highest efficacy as was selected for this *in vivo* study.

### MATERIALS AND METHODS

### Intraocular injections

Ten animals were used per molecule (naked molecule (nkdAON), AAV2/9-U7snRNA-NoAON (AAV-NoAON) or AAV2/9-U7snRNA-AON (AAV-AON5). Mice were anesthetized using isofluorene (also during the surgical procedure) and analgesia was injected subcutaneously. Intravitreal (for naked AONs) and subretinal (for AAVs) injections were performed on a humanized *Cep290* mouse model carrying the intronic *CEp290* mutation [1]. Three microliters of naked AON (20 µg/µl), AAV-NoAON and AAV-AON (∼3 × 10¹² GC/ml both) were injected in the right eye, whereas 3 microliters of PBS were injected in each left eye. Retinas were harvested ten days post-injection. Eight animals were used for RNA analysis, while two mice were employed to assess retinal morphology. In order to obtain sufficient RNA, two retinas were pooled per group, leading to four biological replicates for each molecule.

### Statistical analysis

In order to study the differences between treated and untreated cells we applied the two-tailed Student's T and Mann-Whitney tests. P-values smaller than 0.05 were considered significant as indicated in the figures. Statistical analysis was performed for the quantification of the CEP290 protein levels, as well as the ciliation and cilium length measurements. For the assessment of the efficacy *in vivo* a paired Student's T test was performed to determine statistical significance of the decrease in each replicate, while Student's T and Mann-Whitney tests were used to compare groups.

### RESULTS

AON5 was packaged into an AAV2/9 which presents a high tropism for photoreceptor cells *(2, 3)*. Subsequently, naked AON, AAV2/9-NoAON and AAV2/9-AON5 were delivered to the transgenic mice by intraocular injections. After 10 days, retinas were harvested and analyzed at RNA level. RT-PCR analysis from left eyes (injected with PBS) and right eyes (injected with the naked or AAV-packaged AON) revealed a statistically significant decrease of exon X in the mice treated with naked AONs (p=0.0030), an almost significant decrease for animals injected with AAV-AON5 (p=0.0563) and no differences for the animals that were administered AAV-NoAON (p=0.2413) (Figure 9A and 9B). A total of ∼50% and ∼25% reduction of exon-X-containing transcripts was observed for naked AON and AAV-AON5, respectively (Figure 9C). The amplification of U7snRNA supported the targeting of retinal cells (Figure 9D). To discard side effects of the delivery of these molecules to the retina, we performed toluidine blue staining. No morphological defects, such as photoreceptor degeneration, were detected (Figure 10). In addition, to confirm that retinal structure was not compromised, we performed GFAP immunostaining. GFAP expression is an indicator of gliosis and it is considered as a stress marker in the retina *(4)*. No differences were observed between PBS- or molecule-injected eyes (Figure 11).

### DISCUSSION

Despite the suitability of the fibroblast cells as a preclinical model to assess the therapeutic efficacy of AON-based therapy, ideally one would like to study its potential in the context of a living animal, or at least in the context of a photoreceptor cell. Previously, we generated a humanized transgenic knock-in mouse model where part of the mouse Cep290 genomic DNA was replaced by its orthologous human counterpart, i.e. exon 26, intron 26 (including the LCA-causing mutation) and exon 27, in order to assess the therapeutic efficacy of AON therapy *in vivo.* However, despite correct genetic engineering of the transgenic mouse model, only a low amount of aberrant splicing of Cep290 mRNA was observed, insufficient to compromise retinal function *(1)*. Yet, the presence of exon-X-containing transcripts did allow to study whether AON administration to the retina of these mice could redirect CEP290 splicing. The delivery of naked AONs was performed via intravitreal injections, since AONs are small molecules that can penetrate all retinal cell layers and thus reach the photoreceptor cells. In contrast, the delivery of AAVs was performed by subretinal injections, with which only a part of the retina is targeted, which increases the variability of each experiment and decreases the apparent efficacy. Indeed, we observed more variability in the decrease of exon-X-containing transcripts in mice treated with AAV-AONs compared to the naked AON molecule, where all replicates showed similar results. In addition, U7 expression levels also supported the variability observed in the delivery of AAVs into murine retina. Yet, although a decrease of only 25-30% of pseudo-exon X may seem not promising, it could well be enough to delay the progression of the disease in humans, since LCA patients carrying this mutation already present 50% of correct transcript, so an increase to ∼70% could be sufficient to halt the photoreceptor degeneration. For instance, in the Cep290^{lca/lca} mouse model, even though ∼15% of Cep290 transcripts is aberrantly spliced, no morphological or functional problems have been detected (1). Further to this, it should be noted that following subretinal AAV injections, not all photoreceptor cells are targeted and exposed to the AAV, the therapeutic effect may be 'masked' by the non-targeted retinal cells that are included in the RT-PCR analysis. Increasing the targeting efficacy (by different surgical procedures) would likely yield a higher transduction efficacy and result in a statistically significant decrease of exon-X-containing transcripts as observed with the (intravitreal) administration of naked AON molecules.

### REFERENCE LIST Example 3

1. Garanto, A., et al., Unexpected CEP290 mRNA Splicing in a Humanized Knock-In Mouse Model for Leber Congenital Amaurosis. PLoS One, 2013. 8(11): p. e79369.
2. Vandenberghe, L.H., et al., AAV9 targets cone photoreceptors in the nonhuman primate retina. PLoS One, 2013. 8(1): p. e53463.
3. Watanabe, S., et al., Tropisms of AAV for subretinal delivery to the neonatal mouse retina and its application for in vivo rescue of developmental photoreceptor disorders. PLoS One, 2013. 8(1): p. e54146.
4. Calandrella, N., et al., Carnitine reduces the lipoperoxidative damage of the membrane and apoptosis after induction of cell stress in experimental glaucoma. Cell Death Dis, 2010. 1: p. e62.

## Claims

1. A viral vector expressing an exon skipping antisense oligonucleotide that binds to and/or is complementary to a polynucleotide with the nucleotide sequence as shown in SEQ ID NO: 17, wherein said oligonucleotide consists of a sequence selected from the group consisting of: SEQ ID NO: 20, SEQ ID NO: 21, SEQ ID NO: 22 and SEQ ID NO: 23, when placed under conditions conducive to expression of the exon skipping antisense oligonucleotide.

2. A pharmaceutical composition comprising a viral vector according to claim 1 and a pharmaceutically acceptable excipient, wherein the viral vector is an AAV vector.

3. The pharmaceutical composition according to claim 2 wherein the AAV vector is an AAV2/5, AAV2/8, AAV2/9 or AAV2/2 vector, more preferably a virion corresponding to one of pAAV-AON1, pAAV-AON2, pAAV-AON3, pAAV-AON4, pAAV-AON5, as depicted in Figure 4a.

4. The viral vector according to claim 1 for treating a *CEP290* related disease or condition requiring modulating splicing of *CEP290.*

5. An *in vitro* method for modulating splicing of *CEP290* in a cell, said method comprising contacting said cell, with the viral vector according to claim 1.

## Patentansprüche

1. Viraler Vektor, der ein Antisense-Oligonucleotid exprimiert, das ein Exon überspringt, das an ein Polynucleotid bindet oder zu einem Polynucleotid komplementär ist, das die in SEQ ID NO:17 gezeigte Nucleotidsequenz hat, wobei das Oligonucleotid aus einer Sequenz besteht, die ausgewählt ist aus der Gruppe bestehend aus: SEQ ID NO:20, SEQ ID NO:21, SEQ ID NO:22 und SEQ ID NO:23, wenn er Bedingungen ausgesetzt wird, die der Expression des Antisense-Oligonucleotids, das ein Exon überspringt, förderlich sind.

2. Pharmazeutische Zusammensetzung, die einen viralen Vektor nach Anspruch 1 und einen pharmazeutisch verträglichen Exzipienten umfasst, wobei der virale Vektor ein AAV-Vektor ist.

3. Pharmazeutische Zusammensetzung nach Anspruch 2, wobei der AAV-Vektor ein AAV2/5, AAV2/8, AAV2/9 oder AAV2/2-Vektor ist, vorzugsweise ein Virion, das einem von pAAV-AON1, pAAV-AON2, pAAV-AON3, pAAV-AON4, pAAV-AON5 entspricht, wie in Figur 4a gezeigt.

4. Viraler Vektor nach Anspruch 1 zur Behandlung einer Erkrankung oder eines Zustandes, die/der mit *CEP290* in Zusammenhang steht, die/der regulierendes Splicing von *CEP290* erfordert.

5. *In vitro*-Verfahren zum Regulieren des Splicing von *CEP290* in einer Zelle, wobei das Verfahren das Inkontaktbringen der Zelle mit einem viralen Vektor nach Anspruch 1 umfasst.

## Revendications

1. Vecteur viral exprimant un oligonucléotide antisens de saut d'exon qui se lie à et/ou est complémentaire à un polynucléotide possédant la séquence de nucléotides telle que montrée dans SEQ ID NO: 17, où ledit oligonucléotide consiste en une séquence sélectionnée dans le groupe consistant en : SEQ ID NO: 20, SEQ ID NO: 21, SEQ ID NO: 22 et SEQ ID NO: 23, lorsque placé dans des conditions propices à l'expression de oligonucléotide antisens de saut d'exon.

2. Composition pharmaceutique comprenant un vecteur viral selon la revendication 1 et un excipient pharmaceutiquement acceptable, dans laquelle le vecteur viral est un vecteur AAV.

3. Composition pharmaceutique selon la revendication 2, dans laquelle le vecteur AAV est un vecteur AAV2/5, AAV2/8, AAV2/9 ou AAV2/2, de manière davantage préférée un virion correspondant à l'un de pAAV-AON1, pAAV-AON2, pAAV-AON3, pAAV-AON4, pAAV-AON5, tel que représenté sur la Figure 4a.

4. Vecteur viral selon la revendication 1 pour traiter une maladie ou une affection associée à *CEP290* nécessitant une modulation de l'épissage de *CEP290.*

5. Procédé *in vitro* pour moduler l'épissage de *CEP290* dans une cellule, ledit procédé comprenant la mise en contact de ladite cellule avec le vecteur viral selon la revendication 1.


### SEQUENCE LISTING

<110> Stichting Katholieke Universiteit
<120> Antisense oligonucleotides for the treatment of Leber congenital amaurosis
<130> P6037013PCT1
<150> US14/479,229
   <151> 2014-09-05
<160> 50
<170> Patent In version 3.5
<210> 1
   <211> 93203
   <212> DNA
   <213> Homo sapiens
<220>
   <221> 5'UTR
   <222> (1)..(909)
   <223> Intron from 318 to 882
<220>
   <221> exon
   <222> (910)..(1011)
<220>
   <221> Intron
   <222> (1012)..(1183)
<220>
   <221> exon
   <222> (1184)..(1261)
<220>
   <221> Intron
   <222> (1262)..(2652)
<220>
   <221> exon
   <222> (2653)..(2722)
<220>
   <221> Intron
   <222> (2723)..(3025)
<220>
   <221> exon
   <222> (3026)..(3072)
<220>
   <221> Intron
   <222> (3073)..(5430)
<220>
   <221> exon
   <222> (5431)..(5574)
<220>
   <221> Intron
   <222> (5575)..(10998)
<220>
   <221> exon
   <222> (10999)..(11052)
<220>
   <221> Intron
   <222> (11053)..(11651)
<220>
   <221> exon
   <222> (11652)..(11672)
<220>
   <221> Intron
   <222> (11673)..(11796)
<220>
   <221> exon
   <222> (11797)..(11949)
<220>
   <221> Intron
   <222> (11950)..(12340)
<220>
   <221> exon
   <222> (12341)..(12523)
<220>
   <221> Intron
   <222> (12524)..(13181)
<220>
   <221> exon
   <222> (13182)..(13271)
<220>
   <221> Intron
   <222> (13272)..(15778)
<220>
   <221> exon
   <222> (15779)..(15901)
<220>
   <221> Intron
   <222> (15902)..(16847)
<220>
   <221> exon
   <222> (16848)..(16971)
<220>
   <221> Intron
   <222> (16972)..(21050)
<220>
   <221> exon
   <222> (21051)..(21220)
<220>
   <221> Intron
   <222> (21221)..(21940)
<220>
   <221> exon
   <222> (21941)..(22103)
<220>
   <221> Intron
   <222> (22104)..(23473)
<220>
   <221> exon
   <222> (23474)..(23574)
<220>
   <221> Intron
   <222> (23575)..(23646)
<220>
   <221> exon
   <222> (23647)..(23734)
<220>
   <221> Intron
   <222> (23735)..(25071)
<220>
   <221> exon
   <222> (25072)..(25184)
<220>
   <221> Intron
   <222> (25185)..(27034)
<220>
   <221> exon
   <222> (27035)..(27119)
<220>
   <221> Intron
   <222> (27120)..(27654)
<220>
   <221> exon
   <222> (27655)..(27797)
<220>
   <221> Intron
   <222> (27798)..(30358)
<220>
   <221> exon
   <222> (30359)..(30523)
<220>
   <221> Intron
   <222> (30524)..(30865)
<220>
   <221> exon
   <222> (30866)..(31015)
<220>
   <221> Intron
   <222> (31016)..(33035)
<220>
   <221> exon
   <222> (33036)..(33151)
<220>
   <221> Intron
   <222> (33152)..(35118)
<220>
   <221> exon
   <222> (35119)..(35221)
<220>
   <221> Intron
   <222> (35222)..(35311)
<220>
   <221> exon
   <222> (35312)..(35542)
<220>
   <221> Intron
   <222> (35543)..(39205)
<220>
   <221> exon
   <222> (39206)..(39379)
<220>
   <221> Intron
   <222> (39380)..(45217)
   <223> Aberrant exon included in mutant CEP290 mRNA position 40902-41209 Mutated nucleotide A>G in LCA patients at position 41034
<220>
   <221> exon
   <222> (45218)..(45329)
<220>
   <221> Intron
   <222> (45330)..(48241)
<220>
   <221> exon
   <222> (48242)..(48447)
<220>
   <221> Intron
   <222> (48448)..(49384)
<220>
   <221> exon
   <222> (49385)..(49536)
<220>
   <221> Intron
   <222> (49537)..(51377)
<220>
   <221> exon
   <222> (51378)..(51489)
<220>
   <221> Intron
   <222> (51490)..(52729)
<220>
   <221> exon
   <222> (52730)..(53185)
<220>
   <221> Intron
   <222> (53186)..(54272)
<220>
   <221> exon
   <222> (54273)..(54437)
<220>
   <221> Intron
   <222> (54438)..(55718)
<220>
   <221> exon
   <222> (55719)..(55826)
<220>
   <221> Intron
   <222> (55827)..(56043)
<220>
   <221> exon
   <222> (56044)..(56178)
<220>
   <221> Intron
   <222> (56179)..(57364)
<220>
   <221> exon
   <222> (57365)..(57631)
<220>
   <221> Intron
   <222> (57632)..(58262)
<220>
   <221> exon
   <222> (58263)..(58370)
<220>
   <221> Intron
   <222> (58371)..(58986)
<220>
   <221> exon
   <222> (58987)..(59186)
<220>
   <221> Intron
   <222> (59187)..(61821)
<220>
   <221> exon
   <222> (61822)..(62035)
<220>
   <221> Intron
   <222> (62036)..(62987)
<220>
   <221> exon
   <222> (62988)..(63125)
<220>
   <221> Intron
   <222> (63126)..(64298)
<220>
   <221> exon
   <222> (64299)..(64520)
<220>
   <221> Intron
   <222> (64521)..(64872)
<220>
   <221> exon
   <222> (64873)..(64995)
<220>
   <221> Intron
   <222> (64996)..(70290)
<220>
   <221> exon
   <222> (70291)..(70436)
<220>
   <221> Intron
   <222> (70437)..(70767)
<220>
   <221> exon
   <222> (70768)..(70923)
<220>
   <221> Intron
   <222> (70924)..(73571)
<220>
   <221> exon
   <222> (73572)..(73695)
<220>
   <221> Intron
   <222> (73696)..(78101)
<220>
   <221> exon
   <222> (78102)..(78236)
<220>
   <221> Intron
   <222> (78237)..(79438)
<220>
   <221> exon
   <222> (79439)..(79525)
<220>
   <221> Intron
   <222> (79526)..(81222)
<220>
   <221> exon
   <222> (81223)..(81387)
<220>
   <221> Intron
   <222> (81388)..(82196)
<220>
   <221> exon
   <222> (82197)..(82319)
<220>
   <221> Intron
   <222> (82320)..(83196)
<220>
   <221> exon
   <222> (83197)..(83369)
<220>
   <221> Intron
   <222> (83370)..(86499)
<220>
   <221> exon
   <222> (86500)..(86641)
<220>
   <221> Intron
   <222> (86642)..(87803)
<220>
   <221> exon
   <222> (87804)..(87877)
<220>
   <221> Intron
   <222> (87878)..(88470)
<220>
   <221> exon
   <222> (88471)..(88565)
<220>
   <221> Intron
   <222> (88566)..(91783)
<220>
   <221> exon
   <222> (91784)..(91863)
<220>
   <221> Intron
   <222> (91864)..(92802)
<220>
   <221> exon
   <222> (92803)..(93033)
<220>
   <221> 3'UTR
   <222> (93034)..(93203)
<400> 1
   atttgaagtc ctcgttccac gccttctcat catcctgaac accgagctct gggactccgg     60
   cggagaatct aaacgtaaag catcacccac ggtcgtgaac tgtaggctct cctggcatcc    120
   gggatcttat tctggccttg gcggagttgg ggatggtgtc gcctagcagc cgctgccgct    180
   ttggcttgct cgggaccatt tggctggacc cagagtccgc gtggaaccgc gatagggatc    240
   tgtcagggcc cgcggccggg tccagcttgg tggttgcggt agtgagaggc ctccgctggt    300
   tgccaggctt ggtctaggtg ggtggatcct tgtaagcagg attagcgagt cactccacgc    360
   tcaggttctt tagcctgagg gcccgtgtgc cacagcatag ctaccccgcc cttccagcct    420
   cgggtcccta atactgcctt gcttcggttc cagtttccgc cgcacaactt cactcattcc    480
   aaatgttaat ttctgcgttt tttttcagcc ccaattctgt ttctccaaat cagggatgat    540
   tgtcggcctt ccacagaccc tcgcgcttgc caggattagg gtgttcgcgc gcattgtggg    600
   taggggtgtg gaggaaggga tccagaaatc ttaagtatta acttagatta gtgttagcaa    660
   ggaagccgtc acattttatt tagccgggac actctgacag tttgtgccga ctgctatttt    720
   tgatcaaggc tattttgccc acttgtctat tttgtggccc aattgtctgt tttgctaaca    780
   tcagaaagtt ataatgaaat aatctgcaaa aaatgtaagg tgctagaaaa ccaataatac    840
   tgtgtacctt gaaaatgcta atatacacct gttttgttac agaggtggag cacagtgaaa    900
   gaattcaag atg cca cct aat ata aac tgg aaa gaa ata atg aaa gtt gac    951
             Met Pro Pro Asn Ile Asn Trp Lys Glu Ile Met Lys Val Asp
             1               5                   10
   cca gat gac ctg ccc cgt caa gaa gaa ctg gca gat aat tta ttg att      999
   Pro Asp Asp Leu Pro Arg Gln Glu Glu Leu Ala Asp Asn Leu Leu Ile
   15                  20                  25                  30
   tcc tta tcc aag gtgcttaatt ggtcaataat aatagatata tacattaact        1051
   Ser Leu Ser Lys
   tatgattaat ttattaataa aatatgaatt tatttttttc agggacaact ataattgtca   1111
   caatctggaa gtgttcttat attttgcttg aaggttataa aatataaaac agttgctttt   1171
   ctgtttactt ag gtg gaa gta aat gag cta aaa agt gaa aag caa gaa aat   1222
                 Val Glu Val Asn Glu Leu Lys Ser Glu Lys Gln Glu Asn
                 35                  40                  45
   gtg ata cac ctt ttc aga att act cag tca cta atg aag gtttgtatgt      1271
   Val Ile His Leu Phe Arg Ile Thr Gln Ser Leu Met Lys
           50                  55                  60
   agtaggtttt aactataggt ttggctatta gtggaactat aaaaatctgt tcttatataa   1331
   ggtaatcttt gtgaaaatac ctggtaatat ctacatcacc actaaaaaat gcaatatatt   1391
   taaatgtgaa ttaagtattt tagtgtataa aacattgcta gtttctactt aaagtttcta   1451
   aaagggtgtg taggggaaat agaatgagta tgttgaaaag taacataagg aaatatatct   1511
   tgaggtccaa atgacaaatg cagacaatga ctgctatagg gatttgttaa gaggggaaat   1571
   gatttaagag atgtcagaag acttcacaaa ggatcaatac tgaggagtag tgttagataa   1631
   gtggaaggca atgcagtggt aagatagtaa gggaattcta gagctgttgg ttaccataaa   1691
   taaatactga gaacaggaaa tatgtttatt ctttatattt gaggaaacaa ggtgcagcaa   1751
   gtttgtagca gactgtagag aaaacaaatc ttgggtaagt actttgagat aggttgttga   1811
   gggccttaaa ggtgtatttt atgctatcag caattgagaa ggcagtaaag gttttcgaaa   1871
   cacaattgat aggtacaaaa atacacctta agaaggcaaa actgagtata ttatgtagga   1931
   caaactgaag gaaattggag ctttgtagac atcacattat agcggagttt aaacctgaaa   1991
   ttatggatta gaataatagc aattggaaca gaaaaaaagt agtggaaaga cattacaaag   2051
   ggagatgttg cattactgga tataagactt gaggacttga ggtaaaaagg agaatcaaaa   2111
   atgtttcatg ctattaaaaa tctagaaatt gtagtcttaa gtaagaaaat tgcctggcat   2171
   ggtggctcac gtctgtaatc ccagcacttt gggaggccaa ggcaggagga ttgcttgagc   2231
   ctgggagttc aagactagcc tggataatat agtgagtcct tgcctgtacg aaaaaatttg   2291
   ccgagcatga tggcacacca agcatgatgg cacgccaagc atgatggcat gcacctgtag   2351
   tcccagctac tcaggagact gagatgggaa gattgcttga gcccaggagg caggaggttg   2411
   cagtgagctg agattgtgcc actgcactcc agcctgggtg acaaagtgag gccctatctc   2471
   aaaagcaaaa aaaacaaaaa caaaaaccaa aaactattta ttcagcaaat atttactgaa   2531
   cgtctccatg tgccagccat tgctggcact aaggatcata acaaataaaa cagaattttt   2591
   attttcagtg cttacattcc agtataaagg catattgaaa taaccttttt ttaatgttta   2651
   g atg aaa gct caa gaa gtg gag ctg gct ttg gaa gaa gta gaa aaa gct   2700
     Met Lys Ala Gln Glu Val Glu Leu Ala Leu Glu Glu Val Glu Lys Ala
                   65                  70                  75
   gga gaa gaa caa gca aaa ttt g gtaagcacct tggaaaaagt ttattatggt      2752
   Gly Glu Glu Gln Ala Lys Phe
               80
   attaaataat gaattccatt tgttcattaa actgtagaaa attaaattat attctataaa   2812
   atatatatat tcagtttatt tttaatatat aacatttaat aataaatatt tctagactcc   2872
   tattttatgg atctgccata taatactttt tgttacctta taatcatgat ggactctttt   2932
   aaaagaatta attttgttat tgaaatttat ttaaaagttt gttttgtggt aactaatcaa   2992
   ttaaaacgtt tttctttttt tttaaaaaaa tag aa  aat caa tta aaa act aaa    3045
                                        Glu Asn Gln Leu Lys Thr Lys
                                            85                  90
   gta atg aaa ctg gaa aat gaa ctg gag gtatgtcttt ttgtattccc           3092
   Val Met Lys Leu Glu Asn Glu Leu Glu
                   95
   taggatgtaa ttgtcattaa ttttattttg aattgttttc aaattttaaa attattgttg   3152
   gctggaaaaa ttataaggat gattgtaatc atggttattt gtttattctg tatatgttct   3212
   acatgcctat tatgtgcctt atatagtact aaggactgag catatggttg tgaacaaaat   3272
   aagaagttaa ctgctggatg gagcttatag tcttgggaaa tatacagaaa gattactagt   3332
   aactgaggtg gagggtgggt ggggatttga ggaatagtga cgaaagggtg ttatagaagt   3392
   aatttttgac aaagctgaag gctaaaatat gaatgtattg ttgaagaaca aaatacattg   3452
   agattcctga gaaggtagga atgtgataca aatggatcag cctttgaaag gaggaatacc   3512
   cttttccttt gtgttaggag aggaggatga gtggatgagc gtgggaagag tggatgtgta   3572
   tagaggcttt tatgtttgta ggcataatgc ttggaagttg aggggttggt gatgacatct   3632
   tctgttaaaa agagtgggaa atggtgtggt cacattttaa ggaaattagg taaaatttga   3692
   aatatattgg agacaggact ggagagttgg ggatctggag tcagacagat ttgagttcta   3752
   gtcctgattc ttctactcgt taactctctg aacttggatg acctattgtt tttgattgta   3812
   tatccagctc ctgggaaaat gccaagcact ttcaataaat actaaatgaa ttatggagtt   3872
   ggatcagttc tgtgttagtg tttagctagg tagctgctgt agaatagaag ggtagcacag   3932
   ttgaagatat tggtaggaaa gtggttgaag tgatgattat gaagtcttaa ctgaatagat   3992
   aaaatcaaga ttggggttgg gtgggcagaa gggtagggat atggagggag aagatgaggg   4052
   gttagagtgt cctgtgaggt cgaaggacag gcatagtggg aataattgaa agaatgttct   4112
   ggttggacaa ggatctgatg tgggtgtggg agtgagagac tatagtgaat tcaagaaaaa   4172
   aatagactag aacaaaagtt atgtggagat tgcttagtgg gcatttgata gacatctgtg   4232
   ggccacatgc ttaaattccc agtgcatttt gcggagttac tggaaggttg gtggcttgtt   4292
   tctaccatga gtaggtaaag atggagagca ggatattttg tgagaaagca gctgaagttt   4352
   ctataggatg atggaggaat gataggaatg atcacctgaa gttgcagggt ggggtaaacc   4412
   tagaagcacc aacaccttct tctgaccctc atgtatttgg aatctgaaag aatgagcacc   4472
   ttccaattga aagagttcca agggcattag tatactaaag gatccaaatt gcagctaagc   4532
   caaggagatg gaaaggagga ttcagtaaag aatctgagga tgtgaaatat taatttatct   4592
   tggaagagaa ttttagagag cacaatggaa tgctttttgg aggagagaaa gagtaagaac   4652
   aatttggtta aggtagagga ataacagaac tataaggtga agaaatgaat gtgagacaca 4712
   ttagatgacc aaatgatttg atgttcttgg ccatgacctg aattaacaag actgtgaggt 4772
   aaaatggatt taatcggcta caaatcttaa gataaccaaa acctgagctg tttaatatgg 4832
   tagcactagc actaaccact tgtagctatt tatatttaca ttggttaaaa ttaaaatgaa 4892
   aaatttagtt cttcagttgc actagccaca cttcaaatgc ccgaacatag ctacatgtag 4952
   cgagtggcta ttgaactgga cagcactgac agcatgtcca ttatgctaga aagtcctatg 5012
   ggacagcact ggtctaaaca gtgcatggta tgagagaaag ggcaggttaa ggcactcagc 5072
   ttcactgact ggggtggaga ttctgatggt ttgtactcag gttccagatc cctgaggctc 5132
   aggaaccttt gcagtttagt ctggttacct gtggcccagt ggttacaaca gaatgattaa 5192
   cagtcaattc tttgcatctc tgggtggctc aggaaaaatt taaggagtta ttagctgtga 5252
   actaacctta agtaagttaa attaaaaaaa aaaaagttct taagctaata tgattttaaa 5312
   tatctgcact gaagtataat gcaaatttaa attcagcata attatttgct tgttgttgac 5372
   tcatttgaac ctcaaaatat aatgggatta atttatactt tgggtttatt actttaag 5430
   atg gct cag cag tct gca ggt gga cga gat act cgg ttt tta cgt aat     5478
   Met Ala Gln Gln Ser Ala Gly Gly Arg Asp Thr Arg Phe Leu Arg Asn
   100                 105                 110                 115
   gaa att tgc caa ctt gaa aaa caa tta gaa caa aaa gat aga gaa ttg     5526
   Glu Ile Cys Gln Leu Glu Lys Gln Leu Glu Gln Lys Asp Arg Glu Leu
                   120                 125                 130
   gag gac atg gaa aag gag ttg gag aaa gag aag aaa gtt aat gag caa     5574
   Glu Asp Met Glu Lys Glu Leu Glu Lys Glu Lys Lys Val Asn Glu Gln
               135                 140                 145
   gtaaagcact ttttttttcc atgaatcttc actgttcaag ttacctggct ttttattatt 5634
   attggtaaca atatcaattt ttatattgta tgttatattt gaaaaatgat gtacacttat 5694
   ctctaaggtt ttatatcact gttcattttg tcatcaccaa ttttaaaata taatggtact 5754
   tctagtgaat atgacttgaa gattaattct ttatatttgg aagtacattt ttctcaggac 5814
   atcaaacttg ttacctaaaa ttaatgcttt tgtctggaag attggtatca agtaactaat 5874
   agattttcat aaagaagtga tctttctagt gccatagttt attttgggta aaagttatat 5934
   ttgttcattt caatgtattt atatgattag tagattcgca aatgaatctt tcgatatatt 5994
   caataatggt taattaaata tcttgttttt ggttgtacct tattttatgt gagatatata 6054
   tatatatgta tagtttttga aaagttgtgt tcatgtcagc agtttataaa tcacatattt 6114
   aaaataacat ttttaatgca tagtttttat tacctcgtta ttccttgtta taaactaata 6174
   attcttgcag tgttcacttg aatttagttt taggaaaaaa gttttttgca gatcaacttg 6234
   tatttcctgg aagaaaattt cctattttac ctcagcttcc tatttaatgt attatttatt 6294
   tatttactta acatttattt gttttttatt tcacctgaac tgttagtaaa cttagtaaaa 6354
   tttggtgcct acatgtggta actgtcctgt cccttatact cagaaacgtt ttccaccttt 6414
   gtgtccttta ggtcattgtt gtgttatatt ccatttattt tattttgtcc attgttctct 6474
   cagaaattga gggtcataca ttttaagaaa acaatgatat gctatttaag agaatgtatc 6534
   ataaattgat ttgtaaggaa aagtatcccc attcttcatg tatgtatttt actctaaaat 6594
   gttgaagaat catatagaag ttagctatga aaacaatgtg gtagagaaag tatggatcga 6654
   tgccacttaa atgttaggaa gaagctctta gagcattatc tgtttagcta actgcaaaac 6714
   atagcagaca tgtggatttt ttaatagtca tcaaggatct aacttataat atacactggt 6774
   agaattgctt agggggatgt ctgtggtttt ctggactttt gttcttctat atagacctgt 6834
   atcagttgac ttatcattca taccacacac ccttagctaa tcagaactac cttgtccatt 6894
   tatatcttag actattgtct tttttcatag tcacacacag agaaaacttg aatatatggc 6954
   ctgtgttcct ttttggctgc tcaattcctt gagatgaaat atgggtatgg gttgctttgg 7014
   caattacttc tttgccgtta accagtcatt cagttttatt gagtctttac agcataccag 7074
   aggctgctag ttactagtga tatagtgggc aactatgttc tggttctcaa gaatattcat 7134
   agtcaataat aagcataaca tagtgataat atgatactta gggagataca taaggtcata 7194
   ttctggcata ctctggagag agataccgta atcagccttg aggtgcagga tgtgatctgt 7254
   aaactgagac ctgaagtata gttagactgg taagaggaat gaggatatat atggtggtta 7314
   ataaaagaac attctgggta gaagatatag catttgctaa gacctagagg taagagatgt 7374
   tatggagtat ttaggaaact acagttattc attttgactg aaatataagt gaaaatagct 7434
   ttcatagagt ccttactatg tgccaggcac ttcatatgca ttaattcatt attgcttatt 7494
   tgatacttgt catatgagat agttgtcatt tctgccatga tacagatgaa gaaatggaga 7554
   cacagaaaga gtaattgccc atggttgcac agcttataaa tggtaaaggt aggatttgaa 7614
   aacagtctta ctcaagagtc tgtgctatct tgccttccca gttttatttt ttatgatcct 7674
   ctggagagat aagcaagggc cagttcctaa tgaatttggt tcttttcctg aaaggagcca 7734
   gtgaagagtt ttgagcacag gatatcatga tcagatctat actttaaaag tttactgtac 7794
   tttgtagaga gtggattgaa aagggccaag actagtaagg aaacatttgt gttaattcag 7854
   ggaagtgcta atgatggcat ttgcctgaga aagacaagtg tgagagaagt agatgtaatt 7914
   ggatgtggtg aatgtaattg gttgttggag gagagggagg atggagagtc tgcctaattt 7974
   tgtgggttgg gccactaaat aggtagatag tgccattcat taaggaggaa cacaagagga 8034
   atttggaaag cttgagatta tttcagtttt gtagatgttg agtttgaggt tcttctgggc 8094
   atattcaaaa agggtatctg tggatatgga attcacaaga gaccctgtac agatgatgag 8154
   gatttatgaa tcatcaatgt agacattatt gaagccagag aagtgattgt aaggcacgtc 8214
   tctgagaaat gtctaataaa gcaatgaaat aggaagagtg cttcaaggaa aagctcaaga 8274
   aaggagaaac agagtgtgat gtttgagaag acaagggaaa aaaacattaa tagcattaaa 8334
   tgctttagca ttaagttctt ggcttctctt cttgtaaaaa tttcccaatt cagaacacag 8394
   tgggattatt aactttcaat tgataataat aatgataggc aaacttctaa aatttgtatt 8454
   gtagtttgca ttttattata aactttcttt aaatttttat tttgaaaaat gtcatatctt 8514
   cataaagatt gtaagaaaca cactgttggt gttaatgtaa attagttcaa ccattgtggg 8574
   agacagtgtg gcaattcctc gaagatctag aagcagaaat accacttgac ccagcaatcc 8634
   cattactggg tatataccca aaagaatata aatcattttc ttataaagat acttgcacac 8694
   atatgttcat tgcagcacta ttcacaatag caaagacatg gaatcaaccc aaatgctcat 8754
   caatgataga ctggataatg aaaatgtgga acatatacat catagaatac tatgcagcca 8814
   tcaaaagaga atgagaggtc aagcgtggtg actcatgcct acagtcccag cactttggga 8874
   ggccgaggca ggcagatcac ttgaggtcag gagttcaaga ccagcctggc cagtatggtg 8934
   aaaccccatc tctacaaaaa caaaacaaaa caaacaaaaa ttaactggtc atggtactgt 8994
   atgcctgcag tcccagctac ttgggaggct gaggcaggag aatgacttga acccagaagg 9054
   cagaggttgc agtgagctga gatcgcacca ctggactcta gccttagcaa caaaactaga 9114
   gtttgtctca aaaaaaaaaa aaaaaaaaaa ccggaacaag atcatgtcct ttgcagggac 9174
   atgggatgga ggtggaagcc attatcctca gcaaactcac acaggaacag aaaaccaaac 9234
   actgcatgtt ctcacttata agtgggagct gaacaatgag aacacatgga cacatggtgg 9294
   ggaacaacac acactgggac ccgtcaaggg gtcggggtgg gagaacatca ggaagaatag 9354
   ctaatggatg ctgggcttaa tatctaggtt atgggttgat ctgtgcagca agccaccatt 9414
   gtacacattt acctaagtaa caaacctgca catcttacac atgtacccca gaacttaaaa 9474
   gttgatggga aaaagaaaaa caataaccac ccacataccc ttcatataga ttcaccagtt 9534
   cttaatgttg tgccaacttt gctttatctt tttgtcagta tttttacaca cacatgtatt 9594
   tctctgtctc ttgtttgttc aatcacattt tttgctgagt catttaagag ctaattgcag 9654
   atatgatact ttgcacttaa atatttcagc ttgtctgttt gaaaaagaaa gatgttctcc 9714
   tacaatgaac acaatataat tgtcatgctc aggaatttta atattgattc aacaccatta 9774
   tctagtccat aatgagattt cttctaatgg cccaataata tccttcagtc tccccacctc 9834
   caatatccaa agttctgtca aggatcacat actacatttg gttctttatt atagactttt 9894
   taaatatcgt tgtataccat tgtgattcta tcgtctcctt taataaagag gagaaccaga 9954
   aaaatgaaag gtcataagag gaatgaggtt tggagaatag gtgaaaaaag gcatcataat 10014
   gtttataata atgtttgcct gttcagagaa acaagaatca cagataaagt cacttatatg 10074
   tagataagag aatgctgtat tactttttgc tattctattc actgatcatt tttctaagaa  10134
   ctctgtatgc ttcttgttta actcttatgt cagcatgtat gagaaaactg agttaaagag  10194
   atgttaagta actcattcat gctttactag aaattggttg atgagggaca taaacctagg  10254
   ccggtgtgat tttagattgc ttcttttaac cattgtgttg tattgcctta tatttctaag  10314
   taatttatgt tcactgagag caaataatag tctagctatg acttagaaaa gtaaaataaa  10374
   gatgttgggc agaaaaccat tttattaggg gtttttttgg aggagcagat taatttgttt  10434
   ctgtattctt tggttagttt gtgtgtgtgt tctttttaat tctttaaaat gaaactgttt  10494
   aatccttaaa tccttaagtt ttgaaaattt tggcctatta tttatgtgtt aggttgatat  10554
   taaatcctta atagctttaa cattttctac tttgttagag aggatttaaa atttaagtag  10614
   ataagctgaa tatctggctt tatattaaat tactgctgat ggccaggcac agtggctcat  10674
   gtctgaaatc ctagcacttt gggaggttga ggcagatgga tcacttgagg ccaggagttc  10734
   aagaccagcc tggctaacac agtgaaaccc cgtctctact aaaaatacaa aaattagcca  10794
   gttatggtaa tgcatgccag taattccagc tactcggtag gctgaggtgg gagaattgct  10854
   tgaaccggga ggcagaggtt gcagtgagcc gagatcgcac cactgtactc cagcctaggc  10914
   gacaaagact ttgtctcaaa aaaaaaaaaa attactgctg aattttatct tcttcttatt  10974
   tatttttttt ttttactatt ttag ttg gct ctt cga aat gag gag gca gaa     11025
                               Leu Ala Leu Arg Asn Glu Glu Ala Glu
                                           150                 155
   aat gaa aac agc aaa tta aga aga gag gtaaaaaatt ttagtagttg          11072
   Asn Glu Asn Ser Lys Leu Arg Arg Glu
               160                 165
   tggtggttca acaaaggtac ttattaaaat aagtacctaa gtttacataa atttatattt  11132
   taaccaggac tggagtcttc taagtaactg atgttttcag actgatttta tggtatgact  11192
   ttgtctcagg gaaatagaaa acaaagcaaa atgtgaggcc attaagtatt acattcatct  11252
   caggtctatg cgggtaaatc tttttttgtt gttttataag ccattctttg ctagttttct  11312
   aattgaatag atgactggat ttctattctt atttctctta cccagaatcc tttaaaattt  11372
   tttgttactt gtggaatctt ataaattctg attatcattt ggttctactg agccaaataa  11432
   tgtttgtaca ttgtttattc tgatagaagt tcttaagttt ctaacataat tgaaatatta  11492
   tttgttttgg tagataatta gtattctttc tttggttatt caagataata tgcatcattt  11552
   tcccaaaatt tttttgtttt ctttagtttc tgattattat ttttaattat gtattacctt  11612
   tctcatttct aattaccgtt ttcctgtcct tttctgtag aac aaa cgt cta aag     11666
                                             Asn Lys Arg Leu Lys
                                                             170
   aaa aag gtgaggcttt aagtgtggtg aaatcttggg aatttaaaat atgttgtgag     11722
   Lys Lys
   agcactattt agaggatatg attttgttat tctgaatagt tttgtaattg aatgttgtgt 11782
   ttggttacct tcag aat gaa caa ctt tgt cag gat att att gac tac cag 11832
   Asn Glu Gln Leu Cys Gln Asp Ile Ile Asp Tyr Gln
   175 180
   aaa caa ata gat tca cag aaa gaa aca ctt tta tca aga aga ggg gaa 11880
   Lys Gln Ile Asp Ser Gln Lys Glu Thr Leu Leu Ser Arg Arg Gly Glu
   185 190 195 200
   gac agt gac tac cga tca cag ttg tct aaa aaa aac tat gag ctt atc 11928
   Asp Ser Asp Tyr Arg Ser Gln Leu Ser Lys Lys Asn Tyr Glu Leu Ile
   205 210 215
   caa tat ctt gat gaa att cag gtaaaatggc tagaagtcaa ttcagagcaa 11979
   Gln Tyr Leu Asp Glu Ile Gln
   220
   tggttcctaa aaactttaat ttcattacaa tgtaaatata atatttagcc ctacatgtaa 12039
   attccctggt ataaatctgt cactatgtac ttgtaaaatg tgaaataaat tacatctttg 12099
   aagttgcaac tttttagcca tttttatatt tgcctgtctt ggtcattaag aacaattgag 12159
   gtccttatgt actattttct tgattcaatt tgatttaatt ggtcaatgcc aattagtaaa 12219
   ggtctataaa gaattctctt tttttctaga ggacacttat ggctgcgttt aattttaatt 12279
   tggtttaaat ttcagttttt ttaaaattac tttttaatta tagtgtcttt aactttttta 12339
   g act tta aca gaa gct aat gag aaa att gaa gtt cag aat caa gaa atg 12388
   Thr Leu Thr Glu Ala Asn Glu Lys Ile Glu Val Gln Asn Gln Glu Met
   225 230 235
   aga aaa aat tta gaa gag tct gta cag gaa atg gag aag atg act gat 12436
   Arg Lys Asn Leu Glu Glu Ser Val Gln Glu Met Glu Lys Met Thr Asp
   240 245 250 255
   gaa tat aat aga atg aaa gct att gtg cat cag aca gat aat gta ata 12484
   Glu Tyr Asn Arg Met Lys Ala Ile Val His Gln Thr Asp Asn Val Ile
   260 265 270
   gat cag tta aaa aaa gaa aac gat cat tat caa ctt caa gtaagaatta 12533
   Asp Gln Leu Lys Lys Glu Asn Asp His Tyr Gln Leu Gln
   275 280
   cttttagaat aacttattta ttcagacttc atattatctc attactattt atttgacact 12593
   agaaagtact ttttctagga tgtgaatttt tgtctgtctt tttaatagtg taatatcttg 12653
   tcatgttggt atatttgtcc atatgtgttt ctccaatcac ctcacaaaca ctaatttttg 12713
   caatttagga tatataaatg atacttgaat gaatgtgtag atagcagtca ttatggggtt 12773
   ttctataaaa gactactgaa aatcctgtgg atcataacat ttcattttat cttaaaataa 12833
   atacattata aatgtattag aaaccaatac attgttcagt atttatgtgg attaaatttg 12893
   tttaaaaggt agaataatgt ttaaaaataa aattttctag taatgaaaga taattatgca 12953
   attataagat gcagaaacta ttaaatgtca cctataattc caggatgact tcaatgataa 13013
   atacacatat gtaatgtaat gtatccgtat gtatgtgtat ataagtatga atacgtatgt  13073
   gtgtgtatgt agatatattt atatatataa tgtatatgta aatatgcaca ggtgtaaata  13133
   tatgttacat cagtttgcaa caactcttga aataactttg tcttttag gtg cag gag   13190
                                                        Val Gln Glu
                                                        285
   ctt aca gat ctt ctg aaa tca aaa aat gaa gaa gat gat cca att atg    13238
   Leu Thr Asp Leu Leu Lys Ser Lys Asn Glu Glu Asp Asp Pro Ile Met
           290                 295                 300
   gta gct gtc aat gca aaa gta gaa gaa tgg aag gtattttttt tcaattgaca  13291
   Val Ala Val Asn Ala Lys Val Glu Glu Trp Lys
       305                 310
   taataacttt ttctttttgt attttagatt taaattttag tcttattttt ctttaaatgt  13351
   cttatactgg tttataacac gtttattagg gtttttaaac ataagtttat tttatttatt  13411
   ggttagaaaa gctctagaac tgtccttttt gatctctagc taatttgtta ttgaatgacc  13471
   tctttcacat caatgagttt aactttaaac tttttgatag aagtctaact ccaaaatata  13531
   tttggcatct aaaatatata attcgaaata taatttaaat ttttttactt aactcatagt  13591
   taccttatat acattagtta aatagttgca ggtttaattt tagtttttct aactaaatgt  13651
   caggttcatc agtgggaatg ggaataagca aagggatcag aataacttgg gaagcctttt  13711
   caaaatacac ttttcttcct caccaccact ctccaacctt aaccaaattg tcaggcctta  13771
   ccatattaga agctgggatt atgatggttg tatacttgaa aaacatcaga gattattctg  13831
   aatgaataat tctaatttta aaaactatca cttctagagt cattgctttc tagtatggtt  13891
   cacataaatc ttgtgggcag tttggaactg gttagcatct agggagctca gataacctat  13951
   attttaaaca aaagcattag caatggaaat aaggcctata gaatcagtca tgtctccata  14011
   aactttatat aaagggccag acagtgaata ttttagacca cctggtctct gctataacta  14071
   aactctgctt atagcatgaa agcagccatt gacaatacgt aaatgagtga gcaaggtggt  14131
   tttccggtaa aattttattt acaaaagcag atgggaggcc agatttgacc tttgggccat  14191
   agtctaccaa cccctggaaa aaacagttgt ctttaccaga ttgaatgttg gcagggtaaa  14251
   tggtgacatg ttatatgtat tctgtacttt gttttgactt aataccattt cataattatt  14311
   ttatatcagt acgtatagta ttgctgttct ttttaaaggc tatgtaattt ttctttttat  14371
   acaggtgtta atttgataat ttgtgaagtt tatgaagttt ccaattttgg ggttgtaaac  14431
   tgttttaatg aatatcctta tatatgttat tttgcaaatg tacaagtata tctgtggaat  14491
   aaattgctgc aagtgttgta attgtcatgt atgttgcaaa tacattctaa cagtttgtca  14551
   ctttttttgc tttatggcat tttttgctgt gaaatatttc tttttatgct tagttaaatt  14611
   tattattttt taatgacttt tgacatttgt tataatgaga aaggcttctg agtataaact  14671
   tgttttctca tcttttctcc taatatcttg ttttgttttt gtttttgttt ttgtttttga  14731
   gacagagtct cactcagttg cttaggctgg agtgcaatgg tacaatctca gctcactgca  14791
   aatgccacct cctgggttca ggtggttctt gtgcctcagc ctcctgagta gctgggatta  14851
   caggcatgtg ccgccatgcg cagctaattt ttgtagtttt agtagacatg gggtcacact  14911
   gtgttggcca ggctggtctt gaacccctgg cctcaagtga tcctcctgcc tgggcctccc  14971
   aaagtgctgg aattacaggt gtgactctgc ctggcctttt tttacattta aatcttcgaa  15031
   acatataatt cattttgatg taaggagtat catgtggatt caacagagct actctgttgt  15091
   ccaaacatct tttattgatt atttcatctt ttattgaatt gattgatcta ttttctagca  15151
   gtgtatactt gttttaattt gtgtatgttt taatatctaa aaacgttatt atttttctgc  15211
   ttttagactt ctttatgaat atttttaatg tgaattatag aactggcttg tccagttctt  15271
   aaaaaatatc ttgtggattt ttattgggta tgtgttaaag ttataaattg ttttatagat  15331
   tgatttagga taaacctttt tatgttattt ggtccttcta gctaaagaac acaagatacc  15391
   ttttctttca ttcattcaag atattttatg cctcttggtt gcattttaat gcatacttca  15451
   taaagatcaa ttgtataaaa cttttcacag ttgtatggaa gtacttcttg tttataaatg  15511
   agttttgaaa ggttgaaata tttttaaaga ttgaattata aaaaaagaaa attcggtata  15571
   tattttaaaa tcattttcta tttgaatttc aggttgtata tacaaaagga acagagatta  15631
   tgccagtagt tgctcatact ttctcatttc aaataatttt tattttctgt atcataaatc  15691
   tactaacggt gtttattatt tatgataatg aagaatgttt tattaacttt ccttttgcat  15751
   aacagattct attgtgttta tttctag cta att ttg tct tct aaa gat gat gaa  15805
                                 Leu Ile Leu Ser Ser Lys Asp Asp Glu
                                 315                 320
   att att gag tat cag caa atg tta cat aac cta agg gag aaa ctt aag     15853
   Ile Ile Glu Tyr Gln Gln Met Leu His Asn Leu Arg Glu Lys Leu Lys
       325                 330                 335
   aat gct cag ctt gat gct gat aaa agt aat gtt atg gct cta cag cag     15901
   Asn Ala Gln Leu Asp Ala Asp Lys Ser Asn Val Met Ala Leu Gln Gln
   340                 345                 350                 355
   gtaaaatctt aacagaattt tgtttatcaa ccagttttat tacagttgga actctgaacg  15961
   atgtctttta tttattatat catcagtgcc tagtgtagcg gctggtacta ccaagtgtat  16021
   aataatgtct tttgaaattt cttctaccac ctggtcccaa taaaaaatta gaattaagtt  16081
   tagatcacgg attagactta gaactagagt tactgtgttt atttttctat gtttatgtgg  16141
   atagtacaca cattgttttg gttagaaatt atttaacaag aaatgattaa aaacttttag  16201
   aaatttaaaa taattttata ctcttttaag gtttatttta ctgtatctta gtcctaacat  16261
   accctataca atgtgaaata agctaaaagc atggttataa tttgactgtg ctacctattt  16321
   tatttttagt gaaaataacc caaataaaag gaagtaatac ttttattatt tgtgctgtag  16381
   ttatagtcca caagtaagaa gatgatttga aaagtgtatg ctgaataaga acaattacag  16441
   gggacaacat tttttaataa agtacgaaag gggaaaaagc taagttgaat aaaagagaaa  16501
   gcacagagca aaacagaaac atacaaaatg gtaaaaaggt ggaattgaat ggaggatgag  16561
   gaaagtaaca tataaggaag tatagaagcc ataaacatta gggagttctg gaaatcctat  16621
   tttccagagt gttagccatt atatccatct ttcagtattg gagtaacagc agtgtaccta  16681
   tcattgtgta ttacagttga agtgtacaaa atggtaaaag gcatacttgt acccacaaga  16741
   aaatatgttc tacagtcttg ttgaaaaaaa tcagacgtac ttttttcctt acctttttag  16801
   gttaatattc atgaagggat atatattgtt ttaaaatatt ttatag ggt ata cag     16856
                                                       Gly Ile Gln
   gaa cga gac agt caa att aag atg ctc acc gaa caa gta gaa caa tat    16904
   Glu Arg Asp Ser Gln Ile Lys Met Leu Thr Glu Gln Val Glu Gln Tyr
       360                 365                 370
   aca aaa gaa atg gaa aag aat act tgt att att gaa gat ttg aaa aat    16952
   Thr Lys Glu Met Glu Lys Asn Thr Cys Ile Ile Glu Asp Leu Lys Asn
   375                 380                 385                 390
   gag ctc caa aga aac aaa g gtatttttat aaatatatag ttattttata         17001
   Glu Leu Gln Arg Asn Lys
                   395
   tacaattatg tttttaacga ctttattttt attaaaataa aatgtcaagt caatattgag  17061
   ttttctccat ttgaatttta tattttcaaa aaattgtaca agatatttat tattatactt  17121
   atattactag tgcttacatt tgtaaatgat ggatgcattt tctattattt ttctcctctg  17181
   gtgaaaatta cattaacgtt tattaccagg tcactggtat gaaagaaatg aaaaattgtg  17241
   atacaattat ttttatttaa ctttttataa ttaacaaaga atggaagata ataaaatttt  17301
   gaccagtgta acagcattgc agatagtttt cagaggtaat ttcacattaa tcttacccaa  17361
   attaatgttt catcatattc tccttaccct gagccatatt acctttttta acacatcaaa  17421
   ttctatgaat ataagttctt acaatatctg tgttgttata tttccatagc actacatact  17481
   atagttatgc cagggcacac tagtgcgaac tgttcatggg aaattcatgg acatgtttat  17541
   tataattggt gactatgtat atatgtatac actacattta tacacacgcg catggaatca  17601
   ctatttcttc ttcatgtcat atatatatac atatatacac atatatatac atgtcatatg  17661
   tgtgtgtgta tatatatata tttgtatata tgacatgaag aagaaatagt gattccgtgc  17721
   acatatgtgt gtgtaagtgt agtgatgtgt ttgcaggtac ggttgtaatt tcaaaaatga  17781
   agcaaaagcc ttgctcagga gataattgaa ccaatactta aaggaagtaa aggagtgaaa  17841
   catgcagatg gctctaagca gtgggaataa gttcaaaggc agtaaagcag gagtgtacca  17901
   atcatgtctg agaacaacaa agaagtcttt ttggctggag tagagtcagc aagtgaggca  17961
   gtgataagac cagagaggta aacagaggcc atatcatatg gggccttata gttcattgtg  18021
   cagacttggc ttttaagtga gaagggacac cggggaaagt ttctgaagat agaaatgata 18081
   taatttgact taggctgtgt ttgcagtaga ctgtaggagt ggtaaataag aatcagggag 18141
   acctgttaga agactattgc aataatctgg agaaaagtga tggtggtttg gggcatggtg 18201
   gtagcagtgg agttactgga tgcagcagtt ctggatgtat tttgaaagtg ataaaaatgg 18261
   aatttgctaa cagatcagat gtaggatgtg agagagagag aactcttggt ctgaaccaaa 18321
   agttttggtc atggtggggt tgtgggaaga gcaggttgag agataatcag gtacttaatt 18381
   ttagacatgt taggtttgag atgcttatta gacattcaag tgaaggtgtt aagtaggcac 18441
   ttgtatataa aagtttaagg tttaggacaa caatctaggc taaagatatg tttggtaact 18501
   gtctctgtaa aagtaattga aataatgagg ctggctaaga tcaccaaggg agtaaatgta 18561
   ggttaagaag aaaaatctaa agagcttcta ctttagcagc tggggagata aaaaggagct 18621
   accaaaggag actgaaaagg aaagcccaga gagctaggag gaaaagcagg agtatggaga 18681
   gccctgaaaa ccacatgagg aatgtaacca aggaagaaga aacaactgct ttcagagctg 18741
   tgttcattgc tgctgatagg tcaagatgat cactaaaagt tgactattgg acttagcaat 18801
   ggtcattttt ggttcaagag aaaatgggta gagaggaaat gtaataaaga aatataggaa 18861
   cccttttcca ggactgtttc tataaagaga aggagaaaac aaggtggtag cttgagggga 18921
   aagagggatt aagaaaacat ttttctcttt aagatggaag aaataactca tgattttagg 18981
   ttaataggag agctccatta aagaagaaac attaatgaat caatgaagtg gagagagaga 19041
   acttctggaa caataatatt tttaagaatg caatgggatg ggatcctagt gtgccagtga 19101
   agaggttggc cttaactagg aacacagagt tcatccataa ttgtagaaaa gaaggtagag 19161
   tgtatagata tcgatgtagg tggcttggta gacatcctgg taatgggaat ttgtggaagt 19221
   tctaaactgg ttgctgcttt tttctcagtg aacaagggag caaggttctt agctgaaggt 19281
   gaggatagga gaagatgttt cataagtttg aggagaaaga agagaagtga aagtataaaa 19341
   tggtcatctg aaagattgaa gacgtggaga atgtggtatg actgttgagt aacttcaaga 19401
   gcccacgata tatatatgta tttctattta tgtgtttatt atatttgtat cagaacactt 19461
   tgaaagtagt ttaaactgct ttaaaaggat gactaatagt atggattgtg cgtattctaa 19521
   ttactaggag aaaaagtggc aattgatctc tgctgtcaaa taaggaaaag gacttatctg 19581
   ataacacttt agtcagtccg tagttatata atccctaaag ctcacagaag gtgtgtgtac 19641
   tagactgtac tctacatctt gaacttaact tgtaaaacgt aatggctaat ggtattcttc 19701
   cttcataaga ttaggattag gtttagttat caggaacaga gagctgaaga ataatggcaa 19761
   aatcaagata gacatttatt tctcatctat gtaatggcct agaattaagc attccagggt 19821
   gttgccttca tctgccccat ccaaaatgga tggaatgcag ctttatctca tgtctgtgtc 19881
   ccaaacagca agacagagga agaggggcaa gagttaaaag catgtgctga aggataggca  19941
   ggtaaatata gtgtttattg tgtagggcca tgtggaagaa tgataggaga atagatatgt  20001
   ggatggaagg gagaatagat actgggggac aactcagcct gtgtcatgtt ccacagctta  20061
   gatgttagct ccagacagct gtgctcattt cttaaaaact tttgtgatct caaacgtact  20121
   agttttatgc ctaagtccaa tattaaatat ataacctata tattagtaaa tgcttataat  20181
   gaatgagtgt gagaatgatc tgtcaatcaa ttttggaatg atagcaatat tatgttttgg  20241
   tcttttaaca atttagtaag atattacaag taggcattta ggaagttttt agcttagttt  20301
   ggattaaatt tagctgcaag tgacagaaaa atcaagcata atacaataat ttaaacaaga  20361
   tagaaattta tttctctata atatagacaa agttgaagca actagggcag gatttgtgtg  20421
   acagatgctc aaatatcccc tatcaggaac cctgtctctt gttgctgtgc ctatctcaac  20481
   atgtggtttc taactcatgt gaagttgcca ccctcatatc catgtggatt tcagctagca  20541
   ggaaggagga aagagaagag agattactcc tttattttaa aaacattttt tttttttttt  20601
   ttgaaattca catatgaact ttgcgtttat attccattac tgacatgacc acacatagct  20661
   gcttgtgtgt aagtggaaat ttagttcttt atttcaaatg gccacgtgtc aagctaaaaa  20721
   tccatagttt tagtacagtg gacaaaaggg aggttaaata ttaggaacag ctagcagtct  20781
   gtatcacaat gatcattttt tgtaaagcag tattttgcaa ccttttaaaa tccatacccc  20841
   ttcagctaag aaggttttac tgaacttcag ttttttagta aattgtatta gtaaaaccaa  20901
   aacaaaactt tcatcttaca aatataaaat gacaacttta aaggattttt ttttaatggc  20961
   ataccacttt tcttgccacc atgttgggat cactgatttg aaggaataag tagtcaattc  21021
   aattcatgat ttttgttttt actctgtag gt  gct tca acc ctt tct caa cag    21073
                                   Gly Ala Ser Thr Leu Ser Gln Gln
                                                   400
   act cat atg aaa att cag tca acg tta gac att tta aaa gag aaa act    21121
   Thr His Met Lys Ile Gln Ser Thr Leu Asp Ile Leu Lys Glu Lys Thr
   405                 410                 415                 420
   aaa gag gct gag aga aca gct gaa ctg gct gag gct gat gct agg gaa    21169
   Lys Glu Ala Glu Arg Thr Ala Glu Leu Ala Glu Ala Asp Ala Arg Glu
               425                 430                 435
   aag gat aaa gaa tta gtt gag gct ctg aag agg tta aaa gat tat gaa    21217
   Lys Asp Lys Glu Leu Val Glu Ala Leu Lys Arg Leu Lys Asp Tyr Glu
           440                 445                 450
   tcg gtatgtattt ttatcttgtc attcaaggag cttagaatta ttcttgccat         21270
   Ser
   tcacagacta ttctgtgcta tttactgcat accatttaaa aaacattcca taagtatctt  21330
   ttgataaaga ttatcctcat taatttatac taaactattg aaacctttga gcatttactt  21390
   tttgccagaa ttgttttcaa acttttgatc acagtgattt gtccaaataa tcagttttgg  21450
   tgaagcagca ggattacttt tttttattat ctgtgttcat tgggccacca tgtagatgtg  21510
   acaccactgg ccaatttgac agaatttatg acaggaacat actgtgtcaa tacaacctgc  21570
   tctccacttt ttatactttt tcattggtta caactaattc aagcaactaa tgacttactt  21630
   attctactgg tattgctgat ttgcttttac taattctttt agtattttgg taagtgtttt  21690
   ttatatgtaa tgcatattca gagtcacttt gcctttagga tattatactg gaaagtttta  21750
   actgttgcat attacatcat tattattact ggatttggtt tataaaagca caataaaaaa  21810
   ccagtgtaat gatataaatt ataggcatat gtacattttc ctttagactt agtaaaaaaa  21870
   aaatcatgaa cttgataaat ttattcaagt aaaccatgtt atattttaaa ttaaattgga  21930
   tatttttcag gga gta tat ggt tta gaa gat gct gtc gtt gaa ata aag     21979
              Gly Val Tyr Gly Leu Glu Asp Ala Val Val Glu Ile Lys
                  455                 460                 465
   aat tgt aaa aac caa att aaa ata aga gat cga gag att gaa ata tta    22027
   Asn Cys Lys Asn Gln Ile Lys Ile Arg Asp Arg Glu Ile Glu Ile Leu
               470                 475                 480
   aca aag gaa atc aat aaa ctt gaa ttg aag atc agt gat ttc ctt gat    22075
   Thr Lys Glu Ile Asn Lys Leu Glu Leu Lys Ile Ser Asp Phe Leu Asp
           485                 490                 495
   gaa aat gag gca ctt aga gag cgt gtg g gtaagccatg ttttaagtta        22123
   Glu Asn Glu Ala Leu Arg Glu Arg Val
       500                 505
   catagtttgc gcaacctgat ttacaagtct ttttttttaa tttaaatttt gtttattatt  22183
   atttattaag tagtttaatg cttttttcaa atgcttttat aaaacattta atacaaataa  22243
   aagtggagct aacctgattg aagtggaatc agattttatg gggttggagt ggtgggtggg  22303
   cagggctgga acattgcttt atttggtcta gcatctcctc agtaatagct gcttgtttaa  22363
   aaagatgaaa gtttattaat accacatatc agagattaac cttttttttt cccaacaaaa  22423
   gtagggtctg tattacccat gtttgtttgc aaaatgctct tgtaacagat gagatattta  22483
   aacttcttgc tctgtgttgt gattctcctg cctctgcctc ctgagtagct gggattacag  22543
   gtgtgcacca ctatgcccgg ctaatttttg tatttttggt agagatggga tttcaccatg  22603
   ttggctaggc tggtctccaa ctcctgacct taagtgatcc acccgccttg gcctcccaaa  22663
   gtgctgggat aataggcatg agccaccgcg cctggcctgt taaaatcttt taaagatttt  22723
   taagtacttg atttttataa tttagactac ttacgtttta ctttgttcga gtattttaag  22783
   gagtaattag taatatagct tgagagttta tatatttatt ttaataaata gcctattagt  22843
   taatattact aatttgagtg ttatgatagt gcagactaag ttgctgcttt aaaatgaaaa  22903
   taaatatcta aatatcaatt tcattattgc taaatttcat ttaatgcttt cttagttaaa  22963
   aatgatcatt tgtaaaaact attatctaaa gaaaagacaa atagacaaat aagtatttta  23023
   tacagatata tatgtgtgaa aagtatctaa cttggatccg tagttgtgct aggaccccaa  23083
   attagacttc tgatcaactt ggactatcag atcacagcct tctgatcaac ttggactatc  23143
   agatcacagc caagaatctg gaagttccta aagatgactt ctggcccgtc taggtagctg  23203
   tcatagacat catattttct gtgcttaaaa agctccaaat cttggtttat aatttcattt  23263
   aggtttttgt taggatttcc attaataatt gtgataaaat tttaacttgg gttacagttt  23323
   aaatatctgg aaaattcttt cacagaaagt tacctcattc ttcagtgata ctggctaagt  23383
   gaattataac cagttgcttg atggtatatg acatttttgc agcttatttg aatgttttta  23443
   agtttttaat tatattgctt tctattgtag gc  ctt gaa cca aag aca atg att   23496
                                    Gly Leu Glu Pro Lys Thr Met Ile
                                            510                 515
   gat tta act gaa ttt aga aat agc aaa cac tta aaa cag cag cag tac   23544
   Asp Leu Thr Glu Phe Arg Asn Ser Lys His Leu Lys Gln Gln Gln Tyr
                   520                 525                 530
   aga gct gaa aac cag att ctt ttg aaa gag gcaagtgtgg tagtcagttg     23594
   Arg Ala Glu Asn Gln Ile Leu Leu Lys Glu
               535                 540
   attattttct tggctgaact atagagaaat actaataatt tatactttgc ag att gaa  23652
                                                              Ile Glu
   agt cta gag gaa gaa cga ctt gat ctg aaa aaa aaa att cgt caa atg   23700
   Ser Leu Glu Glu Glu Arg Leu Asp Leu Lys Lys Lys Ile Arg Gln Met
       545                 550                 555
   gct caa gaa aga gga aaa aga agt gca act tca g gtatactcag          23744
   Ala Gln Glu Arg Gly Lys Arg Ser Ala Thr Ser
   560                 565                 570
   ttattctaaa cctttaaaaa gaattattga taagtgagtt gtctggatat gaaattattt  23804
   gtgtcttagc tgtttttgct gttctattgt ggatctgcta caaatttaat aaatgacaat  23864
   aataacctga aggagataag tgagtgtcag tgggttcagt cctgaatctg aaatagacaa  23924
   aaacaaaaca aaacaaaata acaaaaacca agcaaacaaa aaagaaaaaa accttagaat  23984
   tatggaattt ttgaaaagtt ttatagtata gtattttaat ttctagacag caccaatatg  24044
   ttgttattaa taataataaa acttagtagt ttttatgtta atatatgtta ctcaacattt  24104
   tccctttcct taaggactat gcattgaaaa gcttttcttg taagttatta ttattattat  24164
   tattattaat atttgagatg gagtctgtct tgttctattg cccaggctgg agtgcactgg  24224
   tgcgatcttg ctcattgcaa cctccgcctc ccgggttcta gtgattcttg tgcttcagcc  24284
   tcctgagtag ttgagactac aggcgtgagc caccacgcct gacttatttt tgtattttta  24344
   gtagaaacag ggtttcacca tgttggccca ggctggtctt gaactcctga cctcaagtga  24404
   tccatccact ttggctcccc aaagtgctgg aattataggc gtgagccacc atgcctggcc  24464
   ttaaattatt cttttctaag tgaaagtaat gttttattga atataaatta acatctttct  24524
   tgggtttatt ttacttgagc taaagagaac agttggttaa gttttataat agccattgca  24584
   gtgctttttt gtaagaagac cacacagaag gactgtcttt ttcacttgcc ccaaatcccc  24644
   aagcacgtat atgagtaata gcagagtggt tctttttagc attatgattt ctataataca  24704
   tccaaaactt tctcaagaaa aaacttcatg atttattagt acaataatca gtttactcat  24764
   tactcatcat ttatatttac tttatatgtc ttttaactgg tgcttattaa gtagcacttt  24824
   aatatagaat aggcaaagaa tggtagagaa gatgaaattc aaaaattagg ttctcacatt  24884
   attaatagtt cattaaaagt gagctaaatg agaagcttgt attggctatg tagaattttg  24944
   gagggatttt ggaaacaatt attctacctt tgcattaaaa cttgattgta ggttttaaga  25004
   attaaagtgt tggaatagta ggagggttat tttaatgttt ttagtttgtt aattctctta  25064
   tatatag ga  tta acc act gag gac ctg aac cta act gaa aac att tct    25112
           Gly Leu Thr Thr Glu Asp Leu Asn Leu Thr Glu Asn Ile Ser
                           575                 580
   caa gga gat aga ata agt gaa aga aaa ttg gat tta ttg agc ctc aaa    25160
   Gln Gly Asp Arg Ile Ser Glu Arg Lys Leu Asp Leu Leu Ser Leu Lys
   585                 590                 595                 600
   aat atg agt gaa gca caa tca aag gtaatagtaa agtattgcaa agagagtaaa   25214
   Asn Met Ser Glu Ala Gln Ser Lys
                   605
   ggaaaatatt tttttttttt tttttttttg agacggagtc tcgctctgtc tcccaggctg  25274
   gagtgcagtg gcgcgatctc ggctcactgc aagctccgcc tcccgggttc atgccattct  25334
   cctgcctcag cctcccaagt agctgggact acaggcgccc gccaccacgc ccggctaatt  25394
   ttttgtattt ttagtagaga cggggtttca ccgttttagc cgggatggtc tcgatcttct  25454
   gacctcgtga tccgcccgcc tcggcctccc aaagtgctgg gattacaggc gtgagccacc  25514
   gcgcccggcc aggaaaatat ttttattgtg ttttcatttc ttcccccttt atctcattct  25574
   tgaacatcta atcttattat tgttgttaaa taagtagagg gaaatatttg cttatttaac  25634
   ctgttgattc aaagattgat taatgagaca ttatttactc tgaatacaga ttaggagttc  25694
   agataaagca gagctgctgc ataggagatc atcattcaat accccacagt cagatcagaa  25754
   tgagacagaa gagaatatga ccataggatc attatcaaga atgttatctg aaattcacca  25814
   tagtgtagaa agtggaatgc atccttttgt ccctttaact agactttctt catccatgca  25874
   agttaaagag aattcaactc cagaaactat tacaataaga gagattttta aagcaccatg  25934
   tctgcagtct tcaagaaatc tagaatcgtt agtcagcacc tttagtaggg aaagccatga  25994
   agaaataaat gacatatgcc ttttttctga tgactgtatg aagaaggtgt caagaagcca  26054
   tcaagcacta gagaagacta gttttgtaca aaaaagcaat tcatcttttc atggcttatc  26114
   aacagcttca gacataatgc agaagttatc acttaggcaa aaatctgcaa tattttgtca  26174
   acaaattcat gaaaatagag ctgacatgga taaatcacaa gtagcaacat tagaagaaga  26234
   acaggttcat tcccaagtaa agtatgctga tatcaatttg aaagaagata taataaaaag  26294
   tgaagtaccc ttacagacag agatattgaa aaataagctt aaggttaatc ttccagaccc  26354
   tgtgtctatt actgcacaat caaaattatc tcagataaat tctcttgaaa atcttataga  26414
   acagttacgg agagagctag tatttcttag atctcaggtg agtttttctc caaattatat  26474
   ttctgtggtt gttcttttat gacgtctcta acaaagttct gtaacaatta tagttagaat  26534
   atttttgttt gcactttaac atcagttata cacattgtac tttttaaaat ctaaaatgca  26594
   gtacattgat atgaactcat tgacttgtct aatttattaa atttttcttt agaatgaaat  26654
   catagcacag gaattcttga tcaaagaagc agagtgtaga aatgcagata tagagcttga  26714
   acatcacaga agccaggcag aacaggtagt gtaaaggcag aacattaaaa gagatgattg  26774
   tggtactaaa gacaaaaacc gttatatctt tttgcctctt accatggatg ttgggagagg  26834
   gagaaagtgg gattaagatc accatctgct ttactgttta gattttagtt tatttttatg  26894
   attgctgcta tgtcttcata gctcgttttt tttgttttgt tttgttatac ttaattgatc  26954
   aaacttttct taacttgaaa attatagact tgtgatattt tgttgaaaaa aatcaatttt  27014
   attctctctg cttttttcag aat gaa ttt ctt tca aga gaa cta att gaa aaa  27067
                         Asn Glu Phe Leu Ser Arg Glu Leu Ile Glu Lys
                             610                 615
   gaa aga gat tta gaa agg agt agg aca gtg ata gcc aaa ttt cag aat    27115
   Glu Arg Asp Leu Glu Arg Ser Arg Thr Val Ile Ala Lys Phe Gln Asn
   620                 625                 630                 635
   aaa t gtaagttaca attatctttt acttttctgt tcttattttt cctatactta       27169
   Lys
   aaatcatggg cctaaaaggg cgttaacaca ttctctgttt tctaatctgc tttactccta  27229
   attacctctg tactgtatat acttcagtct gtcactatcc agttgatttg ccttgctgtt  27289
   ttcattgtga gagaatgtta ctaatatgaa ttttttgtga gaatatataa ctcctttttc  27349
   ttgtgtgttc ttcaatcaaa atgaagttag aacaccaaat ttaaaatact ttaatataaa  27409
   gcatagttta agttaaggca gaagtatgcc ttatatacgt gtgtatatgc acgtgatata  27469
   aataggtctg tcatttaact caactattca cgttggattt atagttgaat ttttttgtat  27529
   gtttatttac atttggattt ttccaatgat gtctttggta tatgtgaaat atttgtcatc  27589
   tgtatagcat agtgtaaatt gtgaaaaaga tctgatcatc caatgagaaa actgtgtaat  27649
   tacag ta  aaa gaa tta gtt gaa gaa aat aag caa ctt gaa gaa ggt atg  27698
         Leu Lys Glu Leu Val Glu Glu Asn Lys Gln Leu Glu Glu Gly Met
                     640                 645                 650
   aaa gaa ata ttg caa gca att aag gaa atg cag aaa gat cct gat gtt    27746
   Lys Glu Ile Leu Gln Ala Ile Lys Glu Met Gln Lys Asp Pro Asp Val
               655                 660                 665
   aaa gga gga gaa aca tct cta att atc cct agc ctt gaa aga cta gtt    27794
   Lys Gly Gly Glu Thr Ser Leu Ile Ile Pro Ser Leu Glu Arg Leu Val
           670                 675                 680
   aat gtaagttatt tttttcatgt taatgttttt cccctatcac tttagagaga         27847
   Asn
   ttttctgctg tgtacagatc tccatagttt ctgatgagat atttttagtc atttgaatca  27907
   ttgtttccct gtatgtaaag tgtagttttt cttgagctgc tttcaatact tttcttctac  27967
   caattggata attgttatta atctgtcttc aagttcactg acattttcct ctttatctgt  28027
   gttcttttgg ttcaagggtc agcttgagac cttgaggagt tttttacacc gactttggag  28087
   ctcgtttttg ctgactcttt tcttattggg attttccttt cacttatccc atggctttgg  28147
   gctgtatcct gtggttttct agatgagaaa gatgatagat ctctgcaatt gcaccctgcc  28207
   ctatgactaa atctttaaaa atggcaaagt caatctttgc tggtcctgtc ttccgtattt  28267
   gaggggtttt ttcccaaaat ctgcttgctt ttgttcattt tctagaacat ctaggtagtt  28327
   ttttttcatt cattttttat ttatgggagt gtagatctct taggaactta tgccatcaga   28387
   agtattatga aatggcttta ttctaaatgt ttaaagattt actcattgct acaagaaaga  28447
   tttagccatc actaatattc tatatatatt taccatatag ggacttgaga atttcacagg  28507
   attcagtatc tgtatataaa cttgaataat atacacattt tagattgtta atatttaagt  28567
   atatgtcatt tatgttatct gaacatattt agcgtacatt gtcatattat ttcccaaatt  28627
   tgtgcttgat ttcaaatggg aaaaaaattc ttattattta ttgaattgtt tttttaaaaa  28687
   aatcatgatt aatcagtaat tggatacttt ttaaaataac actataattg ttaacagaga  28747
   atgagagtga tactggtatg ttaaaaactt cctgaggcaa gaaaataatt tgattcccat  28807
   tatatctttc tcatactgac tttccttctc tgattggtga ttttgttttg cctctgccac  28867
   tttgaatgtc taaaatgatt ctttatgctt tttttatgtg aacatctttt gtccgtgatg  28927
   atgcccacta ctgatactgt gtcccagatc aaacttaatt ttccaagggc agctctactt  28987
   agtgaccaaa tgaaaacaca gtgaatagcc caagaaatcc taacttctat ttatgttgac  29047
   aatctctgga ccttcctgaa gccactgttt gcatagactt catttacttt tatccgggat  29107
   tgtcattgtt ttttcagatt cataggccct atctgaaatt cacaaatcac ctagcaatac  29167
   ttctctaaga aatcttcaga atccatgaca atttagacca gacaatgctg gattatgcac  29227
   ttcagttcac tttttgttac tacaaggtat ttttcagtgc ccccaacagc tatcttaact  29287
   cattctcatt ttaccaaagt ccatgtagac acggcactat tcctcaatga gacaactaac  29347
   tagaccacct tgttgtcagt cagagtacct tcctctacct acttttatct tccttatatc  29407
   ctctttgagt tagtataagt tattactctg catgacctgc tctaatctcc ttcaggggaa  29467
   ggcttttaca aatctactac ctagagttaa accccagatc accttcctga gtaggagatt  29527
   gcatttggtt ctattcattt taccttattt ggcttctacc ttcacttttt aagacttact  29587
   ttgcctttaa cagttttttc catacagttc atctaaagtc caaatatatt tattagatgt  29647
   gtgcattgtg tgtatatact tagatatgcc actgttggag atttcgggcc agtgatgcca  29707
   ctctgataat attttaatat ttgacatatt atttttgctt actcattatt cttagataat  29767
   atcatgttat gataccttgc ctttattttt atttatgctt caactatgtg gagaggaagc  29827
   actgaaaaat tcacttaatt gaatgttgta ttgatcaatt gttcaatatt gtattccatt  29887
   cctttgcgca tgctttgaat gcaggtgcta tataatttca gagaaaaata cctcattttg  29947
   actgtacaaa aaccccatgt agggagcaga gctcacattg ttttcccctt ttagagacaa  30007
   gaaaactaag atacagagaa tttaagtcac ttgcccagct gttaagtgac tgattaaaat  30067
   ttgaaccctg gtcatcttat tcccgtctgg ttgtttttct agtctaccag tctattaaga  30127
   ttagctaggt gttttttaat tgttttaatg aagtaattac tatgcttggt aatgtaaatg  30187
   aaagttttat agattcataa ataagaattt gaattggcat actttattat catgcttggc  30247
   aatgaaaata ggaaaatgct taaatgtcca ttttatttaa agacagactg ttttttacta  30307
   tgattttact gtttttctcc acatttctaa tatataatat aaatttgcta g gct ata   30364
                                                            Ala Ile
                                                            685
   gaa tca aag aat gca gaa gga atc ttt gat gcg agt ctg cat ttg aaa    30412
   Glu Ser Lys Asn Ala Glu Gly Ile Phe Asp Ala Ser Leu His Leu Lys
               690                 695                 700
   gcc caa gtt gat cag ctt acc gga aga aat gaa gaa tta aga cag gag    30460
   Ala Gln Val Asp Gln Leu Thr Gly Arg Asn Glu Glu Leu Arg Gln Glu
           705                 710                 715
   ctc agg gaa tct cgg aaa gag gct ata aat tat tca cag cag ttg gca    30508
   Leu Arg Glu Ser Arg Lys Glu Ala Ile Asn Tyr Ser Gln Gln Leu Ala
       720                 725                 730
   aaa gct aat tta aag gtgagaattt tattaaataa aagaaaatgc taaacataag    30563
   Lys Ala Asn Leu Lys
   735
   aatgtagatt taataggaaa tttttaattt tttaaaaaga atgctttatg agaaaatgcc  30623
   ccttgaatta attctttcaa tattaagaaa ctggatttct cttataaaat tataagtgga  30683
   aaataagtgc cttataagat tgaaaagaat acaaaaattc taaatctcat acctaggcat  30743
   ttctaagcag aaactgaagt atggttgagg taaaattcct ggcagggcat tcacatatct  30803
   gtcaatttgt ctttctttgg gtgtaagagt tgtgattctc attgctggat ttttttttcc  30863
   ag ata gac cat ctt gaa aaa gaa act agt ctt tta cga caa tca gaa     30910
      Ile Asp His Leu Glu Lys Glu Thr Ser Leu Leu Arg Gln Ser Glu
      740                 745                 750
   gga tca aat gtt gtt ttt aaa gga att gac tta cct gat ggg ata gca    30958
   Gly Ser Asn Val Val Phe Lys Gly Ile Asp Leu Pro Asp Gly Ile Ala
   755                 760                 765                 770
   cca tct agt gcc agt atc att aat tct cag aat gaa tat tta ata cat    31006
   Pro Ser Ser Ala Ser Ile Ile Asn Ser Gln Asn Glu Tyr Leu Ile His
                   775                 780                 785
   ttg tta cag gtattgaaaa ttttgttaca ggtattgaaa attttacatg            31055
   Leu Leu Gln
   tgaataacaa aaatcattgg tagtatgttt ctttatgttt ttatttttat tttactttat  31115
   tttaattttt ccatcaccaa agcatgcaga tagtactttt ctcaatattt agtcttcatg  31175
   tattcctgag ttctcaaaat agtaacagtg aaatatattt tttatggatt ttgatgttag  31235
   atggattata aataaaagca atttatacca ttcattccat tcatctgcat gagcagcatg  31295
   ttcatacatc ttgttcgcac acctgtcatt catgtgaaat atatggttca caagcagaac  31355
   aacaagcagc tattataaag cagtgttaag taaatgagca cttttatttc ttgctgggtg  31415
   gaaaacaaaa gaataaagtc tgtcaaggct ttttagtgtc atgatagaat tgttcccctt  31475
   tttgcattca caagtaaaaa ctactttttt tttgagacag agcctcactc tgtcactcag  31535
   gctggagtgc agttgcgcta tcttggctca ctgcaacttc cacctcctga gtttaagtga  31595
   ttctcatgcc tcagcctcct gagtagctgg gactacaggc atgcatccct ggctaatttt  31655
   tgtatttttt tttagtagag atggtgtgtc gtcatattgg ccaggctggt ctcaaactcc  31715
   tggtctcaag tgattcgcct gccttggcct cccaaggtgc tagggttaca gacgtgagcc  31775
   actgcacaca gccataagca aaaacttcta aaccaaatta ttcttcatct ttgtcttccc  31835
   tttacgcaat aaaatgttaa tctaccacca aagaggaaag ggtactctac tatactacct  31895
   gccctgggtt tctcagtttt gctgtctata taatggtcgt tatgaatgtc ctaatgacag  31955
   atccttttca ttattttatt tgaaatttga ctatctataa catcacatac attataaata  32015
   taattacaaa tatatgttca gaatcaatga aaatatattt ttgattatat gggccactat  32075
   ttctctctgc taggtgatcc atttgtgagt atacttgagt tataattatt aagtactcat  32135
   ttttattttg gaaattacag taattcatct ttttctcaat attgggattt ttattattat  32195
   tttatgttgt ctaaggacag ccttaactac ttattagaat attgctttgt atgtgatatt  32255
   attattttta aatgtataat tttaacatta ttatttctct tatttacctg aggtatagga  32315
   acactatcag caaatattgg tagtatggca ttgtcgtatt ttttgagata aaattcatga  32375
   tttttaatct ttgtataaga aatatatcag aagtttgtag tagattagag agtaccaact  32435
   gggagtctga aaagctgtcc aaagtggcaa aacaggtact tagactctca atcctaaggc  32495
   tgtatagagc tataaacgtg gcaagacctt tggagtcaga cagacccaaa ctcaaatgtt  32555
   ggatccatgt atatggaaag cacctgacaa caagcctagc atatgtactt ggtaaaaatg  32615
   attgccaagt gtagtgttaa tgagtttttg gatattgagt aagttattta aatttcaatt 32675
   tcatctttaa aatgaaataa ttggaaagga taatttgagt gagggtatga aattatgtgt 32735
   tcataagaga gggtatgtgg ccgagtgact agaggcgagt ttataactat tctatctaat 32795
   aaaactttgt aatctggtaa tttgtgtgct aaaaataact ttacctgttg tatagtactc 32855
   tttttttatg ccttaaacta aagtgttcaa aatatcatgg aaaaatgatc tgtgttgctt 32915
   acagatttgg tgacttttaa ctttcctata atgttgtcag aatatgaatt tatactttca 32975
   aattcagcat ttattctatt gtgttttttt ttgcattctt atttctaaac cacttttcag 33035
   gaa cta gaa aat aaa gaa aaa aag tta aag aat tta gaa gat tct ctt 33083
   Glu Leu Glu Asn Lys Glu Lys Lys Leu Lys Asn Leu Glu Asp Ser Leu
   790 795 800 805
   gaa gat tac aac aga aaa ttt gct gta att cgt cat caa caa agt ttg 33131
   Glu Asp Tyr Asn Arg Lys Phe Ala Val Ile Arg His Gln Gln Ser Leu
   810 815 820
   ttg tat aaa gaa tac cta ag gtataggtat tagcaaaact ataaatataa 33181
   Leu Tyr Lys Glu Tyr Leu Ser
   825
   ttgcagtata ttcttgttaa ttgtgaaagt aacgtaagaa taatttatgt tttgttcttc 33241
   ccttcttctt cttcctttgc aattgtattt ttttttactc tggtaactac tgttaggaac 33301
   ttatttatgg agacagtgta gcttaatgat tacattaagc ctgggattat cctgcctggg 33361
   tttgagtcat ttaacgtttg ctttttgtaa gagcttgagc aagtcatctt acctatctgt 33421
   gtctcagttt ccttatctgt aagttacttt gtaagtaata cccttttcat aggattattg 33481
   taaaacgtaa atgaattatt agatgaaaat gctcggacta gtgtgtggca catatgaaca 33541
   gtttgtaaat gttagctgtt gttagcatca ttcatcatca tcacaatcat cattgttcat 33601
   atatgtttat agggaactaa catatttctc cttatttctg tcatctcatc taaatcaata 33661
   gaatgatttc cttaatagga attagaatac ctaatcaaag gtgatttaaa cactaagaat 33721
   aattattatc tgacctaacc agaaccacaa agctagttgt agggcaggtc atatttgaag 33781
   gttgttgtta tcgcctatga tggttgtaaa atagctgcat gaattcaaga aagatgatgt 33841
   gcccattgaa gaagaggagc atttttttct acatagcttt tatttttaaa taaacatttt 33901
   tttctggtga tacctggcag acattgactc cgatctcatt tgctagaatt ggatcacatg 33961
   tccaagtctg aaccattcag ttgcaaagag aatgataccg ctatactggg tttatgccaa 34021
   gaacattaca catgtttgtg gaatgctcat gtgtagacaa cagtgtctta cacaacttca 34081
   aaaaaataat ttatatataa atatgtttta aattactttt taaattcaca agaatttatg 34141
   gtatacaaca tggtgttcta tatatgtata tactatgcta tacaacatgg tgttctatat 34201
   atgtatatac tatgctatac aacatggtgt tctatatatg tatatactgt ggaatggcta 34261
   aatcaagcta cttaacatat gtattacctc gcatactttt tttttttttt ccttgagaca 34321
   gagtcttgct ctgtcaccca ggctggagtg cagtggcgct atcttggctc actgcaacct  34381
   ctgcctcctg ggtccaagtt attttcttgc ctcagcctcc caagtagctg agattacagg  34441
   catgtgccac cacgcctggc taatttttgt atttttggta aagacggagt tttgccatat  34501
   tgtccacgct agtctcaaaa ttcctagcct caagcaatct gcccaccttg gcctcccaaa  34561
   gtgctgggat tacagcatac ttcttcttat tttttttttt ttttgcacta agaacactta  34621
   aaatttactc tcttagcaat tttaaagtat ataatatact gttattaact ttggtcacta  34681
   ttttaattag acttaagatg tgtttgtatt caaattattt tgtaagcatt taacacccaa  34741
   atttgagagt ggggtcagaa tgttggaatt tgatttctag aattagtata gggtattatt  34801
   ttcctacttt ttttctgtgt tcaataaaat gtttataaga ttcagcttca attatattat  34861
   aacccattta gtggtgaatc agggaagaat gaaaataatt tgataacttt gttgccttgc  34921
   atttatttaa aaaattttta attctaggct aaaccctttt taaatgaaag tttaacttct  34981
   tgtgttttca gatactgaat agctatgata cctcttgtgt tgagaaaact ttaaatttgc  35041
   ataatctgaa gttatctttt cttataaaca ttttattagg tttacagtat tgtctttttg  35101
   ttttgttttg tttttag t gaa aag gag acc tgg aaa aca gaa tct aaa aca   35152
                        Glu Lys Glu Thr Trp Lys Thr Glu Ser Lys Thr
                            830                 835
   ata aaa gag gaa aag aga aaa ctt gag gat caa gtc caa caa gat gct    35200
   Ile Lys Glu Glu Lys Arg Lys Leu Glu Asp Gln Val Gln Gln Asp Ala
   840                 845                 850                 855
   ata aaa gta aaa gaa tat aat gtaagtaaaa catttttaac attagtatgc       35251
   Ile Lys Val Lys Glu Tyr Asn
                   860
   aatattgtac aaagtaggat agctagattc aacaagtaat atggatgtgt ctttgtgcag  35311
   aat ttg ctc aat gct ctt cag atg gat tcg gat gaa atg aaa aaa ata    35359
   Asn Leu Leu Asn Ala Leu Gln Met Asp Ser Asp Glu Met Lys Lys Ile
           865                 870                 875
   ctt gca gaa aat agt agg aaa att act gtt ttg caa gtg aat gaa aaa    35407
   Leu Ala Glu Asn Ser Arg Lys Ile Thr Val Leu Gln Val Asn Glu Lys
       880                 885                 890
   tca ctt ata agg caa tat aca acc tta gta gaa ttg gag cga caa ctt    35455
   Ser Leu Ile Arg Gln Tyr Thr Thr Leu Val Glu Leu Glu Arg Gln Leu
   895                 900                 905                 910
   aga aaa gaa aat gag aag caa aag aat gaa ttg ttg tca atg gag gct    35503
   Arg Lys Glu Asn Glu Lys Gln Lys Asn Glu Leu Leu Ser Met Glu Ala
                   915                 920                 925
   gaa gtt tgt gaa aaa att ggg tgt ttg caa aga ttt aag gtacatctga     35552
   Glu Val Cys Glu Lys Ile Gly Cys Leu Gln Arg Phe Lys
               930                 935
   ttcttatttt gctttttctg actatgaaaa atttcaaata tgcagaagat aggatggtat  35612
   caataatgct catcacctga attaatagtt aacatttatt aacattttgt cataattgct 35672
   tcttctgatt tttgtgggat gtttgaattg cagacattcc tcccctaaat atttaatgta 35732
   cccttttgaa aaaggctttt ttctttaact aaccatagta actttattat acctaacaaa 35792
   atgacagtaa ttttctaata tcgcctaata ccctgattat agtcacattt tttacatttt 35852
   ttgatcaaag aataagcatt tggatgttac atctcataaa tctttttaat atagaatccc 35912
   cttggttttc tttttctcca aaaaatgttt gaagatgtat ctaacttttg tgtgtgtgtc 35972
   attttacttg ttcctgtgtc ccttgtatta ctaaaagtta ggtcagaacc ctaagttaca 36032
   ttcaggttta aacatttttg gcaagaatac ttcataagta gtgttctata ctttatattg 36092
   catcacttca agagtatctg gttgttccat gttttgtaat tgattactct gttaaggaaa 36152
   agacaagcag accaagtatg gtggctcatg cctataattc caacattttg gaaggcccag 36212
   gcaggaaaat ttcctgagcc cagaccagcc taggcaatat agtgagactc cgtctctaca 36272
   aaaaatgttt ttttttttgt ttgtttgttt ttaattagct tggtgtagtg gcacatgctt 36332
   gtaatcccag ctacctggga cattgaggtg ggaggatcgc ttgagcccag gaagttgggg 36392
   gctgcagtga gctgtgatca tgtgccactg atctccagcc tatgtgcctg tataacagag 36452
   cgagtctctc tcttaaaaga aaaaaagaag aagaagaaga agaaaagata accatatacc 36512
   tccattatta agcaatttag ctaactggtg atattttggt accatacaaa taacaaatta 36572
   tttgtcagtc ctaatgattt tagcatctgc tgatgattgt tgcctaaccc aattattaaa 36632
   agttgcaaac atcataattt tctagttata ttatgcactt acatttatta acagacatgc 36692
   ttttgtaaaa taaatagcgt ttcctcatta gcccaggcta tttgtttatc ttgaagttta 36752
   gctcctacta caaaggcaag ataaatgctt ttctctttaa ttaccagttt tcagaataca 36812
   cacttggtgt actctgcact acctgctttt tttgtcccct ccgctttctc ttttttaagt 36872
   atcagattag actcacagat ttttaaatat tccatgtgtt ttagttggag tcatattctt 36932
   ttgtctcaac tttagccaaa gagagtcctt taaagttgac tcttatattg tcttgacaaa 36992
   aattcattag tcttttgaac gaagcctcaa agcttgactt gttttctagc ataagatgtc 37052
   ttagacttac ctacatactt catgcccata cttggaataa accatttctt taaagagccc 37112
   aggttccttt tagtggggaa ggcatttaga taccaaaaac tggccactgg gcatcattgc 37172
   tctcagagta tcattgccac tagtctctca gtagacaagt tagaaaaata tgtatatatt 37232
   taaaccatga gttcatattg ttatttccag tttaattata acattatggg gtaagtaaat 37292
   agtatcggat ttttactaag cttctttgat tttgcacttg tatttttttc ttacatagaa 37352
   aacctttatt attaacatta aaatatttgt tttatcctac aatatacata caataatttg 37412
   aaaaataata cttgaattga tattaatagt aacaacaaca gcactgctgc caaacatagt 37472
   ttaaagtttt atttcaggtc ttattttctt cagaatatat cttgctgaga atgtataggc 37532
   aaagtattct acacttactt gaaataattg tcttcatgcg gttatgttat acatttgata  37592
   tatagttagg ctcatttgtt tttcattttt tttattttag ggattttttt cctttattga  37652
   attttaatat atacaatatt tatatatgca aaatatttaa tcagagaaat cttaattctg  37712
   gtcttacgcc tttcatatta ttctgctcca ccctctgtag gtaacttatt atctttctca  37772
   tgtttccttt ttggaaacat aaacaaagac aagacaggtt acatgacatg tatacccttc  37832
   tgcacctagt tttatacctt accttgtagt ttatttttaa gcatgtaaat gttcaatgtt  37892
   catgactaaa tttggacagg atcataggaa cacagaattc aaagtgaaat taaaatgggc  37952
   ttgggttctt tactttccac tttaaaggtt gtaatgggtg atgtcaggct aataaaccta  38012
   ttttcagctt gatctaaagc ttaatactga gcatcaagaa attctttaat aaatataagt  38072
   gatatttatt cagacatgta ataaggaaat gttcatgtct tatttttgtg ttagattttt  38132
   ttagaatcta cttttgttag agttttataa atacagttag tgtttgagat agaaagagaa  38192
   aagaattagt tttcttcctc ttctacctgc tcatgaactt gatttttttc tcccaacaat  38252
   tgaagagcca agaaaaaggg agattcttaa gagatgggaa atagaatctc atctacccct  38312
   gtttccccca gaacagtgaa actgaatctt aagggtaaga tagaatagtg tgtacttaac  38372
   ttagatggag aagaaaggct gccaaaatga gatctgaagc gctattacaa atatttccat  38432
   cgttactgta cttcagaatg aattacaacc gtaagttttt ttacttcctc attcataaat  38492
   ttgattattc cttataccac ttctcagctt tcatcattct ttattgtact tttctatgta  38552
   atgtttgcct attatacagc aacttaagag aactgtaagt ttggacattt cattttggtg  38612
   ttgataatag aatatctttg aatagttcta tagttgatga gtagaaccat gaaccaagta  38672
   acttaaagtc cttgatgtta tttattacag agaactataa tagaagctct cccgctaatg  38732
   tttccatcat gtgtacaaaa agttttcttg ttattaaagc tagtccgttt aacttacaat  38792
   aagcataaat agctaagctg tgaaagttac ctgtgataat gctaattttc ccatttatta  38852
   aaaggcaagt tgttttccga tcataagaaa tttagaaaag ccatccaaag ataaattccg  38912
   agtgatatat tcctgctgtt tgttatgttt tctcaaatta attgagtttt attttacaat  38972
   gacaggagtt attaaagtat tttattttta ttatgattaa gattttcaaa gtaacatttc  39032
   ttatatgaaa gaaattatgt taatgcatgt ttttcttaca tgggaaatca tatattttaa  39092
   aaatgatttt aaaattcgtt ttactttaag ttgtattatc tttctcaaaa gtggctagtg  39152
   cttgaccaga aaaaaagaca ccagcataac tcagtgtatc tttatttaca tag gaa     39208
                                                               Glu
                                                               940
   atg gcc att ttc aag att gca gct ctc caa aaa gtt gta gat aat agt    39256
   Met Ala Ile Phe Lys Ile Ala Ala Leu Gln Lys Val Val Asp Asn Ser
                   945                 950                 955
   gtt tct ttg tct gaa cta gaa ctg gct aat aaa cag tac aat gaa ctg     39304
   Val Ser Leu Ser Glu Leu Glu Leu Ala Asn Lys Gln Tyr Asn Glu Leu
               960                 965                 970
   act gct aag tac agg gac atc ttg caa aaa gat aat atg ctt gtt caa     39352
   Thr Ala Lys Tyr Arg Asp Ile Leu Gln Lys Asp Asn Met Leu Val Gln
           975                 980                 985
   aga aca agt aac ttg gaa cac ctg gag gtaagtttgt gtgattcttg           39399
   Arg Thr Ser Asn Leu Glu His Leu Glu
       990                 995
   aaccttgtga aattagccat ttttcttcaa tatttttgtg tttgggggga tttggcagat  39459
   tttaattaaa gtttgcctgc atttatataa atttaacaga gatataatta tccatattat  39519
   tcattcagtt tagttataaa tattttgttc ccacataaca cacacacaca cacacaatat  39579
   attatctatt tatagtggct gaatgacttc tgaatgatta tctagatcat tctccttagg  39639
   tcacttgcat gatttagctg aatcaaacct cttttaacca gacatctaag agaaaaagga  39699
   gcatgaaaca ggtagaatat tgtaatcaaa ggagggaagc actcattaag tgcccatccc  39759
   tttctcttac ccctgtaccc agaacaaact attctcccat ggtccctggc ttttgttcct  39819
   tggaatggat gtagccaaca gtagctgaaa tattaagggc tcttcctgga ccatggatgc  39879
   actctgtaaa ttctcatcat tttttattgt agaataaatg tagaatttta atgtagaata  39939
   aatttattta atgtagaata aaaaataaaa aaactagagt agaatatcat aagttacaat  39999
   ctgtgaatat ggaccagacc ctttgtagtt atcttacagc cacttgaact ctataccttt  40059
   tactgaggac agaacaagct cctgatttgt tcatcttcct catcagaaat agaggcttat  40119
   ggattttgga ttattcttat ctaagatcct ttcacaggag tagaataaga tctaattcta  40179
   ttagctcaaa agcttttgct ggctcataga gacacattca gtaaatgaaa acgttgttct  40239
   gagtagcttt caggattcct actaaattat gagtcatgtt tatcaatatt atttagaagt  40299
   aatcataatc agtttgcttt ctgctgcttt tgccaaagag aggtgattat gttacttttt  40359
   atagaaaatt atgcctattt agtgtggtga taatttattt ttttccattc tccatgtcct  40419
   ctgtcctatc ctctccagca ttagaaagtc ctaggcaaga gacatcttgt ggataatgta  40479
   tcaatgagtg atgtttaacg ttatcatttt cccaaagagt atttttcatc tttcctaaag  40539
   attttttttt tttttttttg agatggagtt tcattctgtc acccaggctg agtgcagtgg  40599
   cacgatctcg gcttaacgct tactgcatcc tctgcctccc agattcaagc agttctcctg  40659
   cctcagcctc tgagtagctg ggattacagg tgtgcaccac cacaccagct aatttttttt  40719
   tttttttttt tttttttgag gcagagtctc gctctgtcac ccaggctgga gtgcagtggc  40779
   gccatcttgg ctcactgcaa gctccacctc ccgggttcag gccgttctcc tgcctcagcc  40839
   tcctgagtag ctggtaccac aggcacccac catcatgccc ggctaatttt ttgtattttt  40899
   agtagagatg gggtttcacc ttgttagcca ggatggtgtc gatctcctga actcgtgatc  40959
   cacccgcctc ggcctcctaa agtgctggga ttacagatgt gagccaccgc acctggcccc 41019
   agttgtaatt gtgaatatct catacctatc cctattggca gtgtcttagt tttatttttt 41079
   attatcttta ttgtggcagc cattattcct gtctctatct ccagtcttac atcctcctta 41139
   ctgccacaag aatgatcatt ctaaacatga atcctaccct gtgactccca tgtgactccc 41199
   cgccttaaaa actgtcaaaa gctaccggtt acctgaaggg taaaagtcaa gtcccctact 41259
   tacctcatgt catctagagc aagagatgaa ctagctgagt tttctgacca cagtgttctt 41319
   tcttatgtat gttcttttgt acgtgctctt ttctatatat agggaaccat ttctctcttc 41379
   cagttgtttt gctcagtgaa tttctattcc tgtttcaaaa cttgttcagg cattaccttt 41439
   tttttcttaa gcatactttt tttaatggaa caaagtcact cctgtctaca ctagttctgc 41499
   atcttataca taggttttgt acatagtaca tatttatatc acatcaaatt atatgtgttt 41559
   acatatctgt cttccttaat ggaatataag tcttttgata taaggaacta tttaatttgt 41619
   ttctgtgtgt tgagtatctc ctgtttggca cagagttcaa gctaatacat gagagtgatt 41679
   agtggtggag agccacagtg catgtggtgt caaatatggt gcttaggaaa ttattgttgc 41739
   tttttgagag gtaaaggttc atgagactag aggtcacgaa aatcagattt catgtgtgaa 41799
   gaatggaata gataataagg aaatacaaaa actggatggg taataaagca aaagaaaaac 41859
   ttgaaatttg atagtagaag aaaaaagaaa tagatgtaga ttgaggtaga atcaagaaga 41919
   ggattctttt tttgttgttt ttttttttga aacagagtct cactgtgttg cccaggctgg 41979
   agtgcagtgg agtgatcttg gcttactgca acctctgcct cccaggttca agcgattctt 42039
   ctgcttcagt ctcccgagta gctggaatta caggtgccca ccagcacggc cggctaattt 42099
   agtagagaca gggttttgcc atgttggccg ggctggtctc aaactttgga tctcaggtaa 42159
   tccgccagcc tcaacttccc aaagtgctgg gattacaggc atgagccact gtgcccagcc 42219
   tgtttttttt tttttaaagg agaccagtga agtttcagga ggagggaaag aaaatttaga 42279
   gttactaggg agagagtgat gaagataaga gatgaaagtg gtaataaggg aaatagcaaa 42339
   atatcagggt aggtgggaga aaaagagatt tgtaacaaac aataggatta tcctgtgaaa 42399
   aaggatgaaa ggaagaaaaa aatggataga aagatattta aaacaccctc agcctcctgt 42459
   tttccctcct gtgtattcat agtatataaa actataatta tgtactttac ttaaaaaata 42519
   tattattatt accttatcgt gcttatttaa tcatagcatg tcctcttttt agtctcatta 42579
   ccctgtttgt attattcttc ataacactta atacctgaca ttgtattata tattggctta 42639
   ttttccaggt actccactca aatataagtt ctaggatata atttatttat cactgaaatc 42699
   cattgcttag agtacctggc atgtagtaaa taggcattct gttttttcaa ataaaaaata 42759
   aaggaactta agatatatat ttatgttata tcgccagcct ttttcctcac agctctattc 42819
   tgttgtacag aattacctac tttacaattc ctgtgtttca aggggatctc aaatttaacg 42879
   tgtccacaat gaactcctga tttctgtttc tctcctagtc attcttattt caatatatgt 42939
   tcagttacct aaccagctag tcaaggcaga tactttagag ttattctgta gtcattcttt 42999
   ttccctacca tttttgtttt ccaaatgtaa tttatgtgtg tcttcttcat cctcgcagct 43059
   ctaacccttg tccaaaccag catcatcact catctggagt tccacaatgt ctttctggct 43119
   agtttccctg atttctctat tgaccccttt attctccaca gtgcagccag aatgattgtt 43179
   taaaacttcc tccttaaaat ctttaaattg ttttctttta tacgttaagt taaattccag 43239
   ttccttgtct tggcatgcca tgccctgcct ggtgtggccc ctgatggtct ctccaacttc 43299
   atgttttact actattgact cttatttttg cttactctgc ttgggtgctc cagtcctcca 43359
   aatcatttcc tgctccaatc atttcaatca ttttttcctc tcagatctta tagtattcca 43419
   aatgctttct tcctttggag catctgggtt tactaataaa tacttcgtac ctcacagttc 43479
   agcttaaata tcaattattt ggtggttaag acatccttca accgctctat ctaaatgttc 43539
   ctttctatta ttcactggct cagtactctg tttttatttt ctttctaaat gtcaactttt 43599
   ttttttttga gtcagggtct cactgttgcc caggctcgag tgcagttgca caatcatagc 43659
   tcattgcagc cttgccctcc tgggatcaag taattctccc acctcagcct ccaaaatagc 43719
   tgggattaca ggtatgcatc accatgctca gctaattttt tgtgtttttt tgtagagatg 43779
   aggtctcact ttgttgccca ggctggtctc aaactcctgg actcaagtga ttctcccacc 43839
   tcagcctccc aaagtgctgg ggttacaggt gtgagccact gcacctggtc gatactgact 43899
   tttttttttt tttgagatgg agttttgctc tgttgcccag gctagagcgc agtggtgtga 43959
   tctcagctca ctgcaacctc cacctcccag gttaaaggga ttcttctgcc tcagtctcct 44019
   gagtagctgg gattacaggc aagtgccatc atgactggct aatttttgta tttttagcac 44079
   tatgtttagt actgtgttgg ccaggcttgt ctcgaactcc tgacctcaag tgatccaccc 44139
   acctcagcct cccaaagtgc tgggattaca ggtgtgagcc accgtaatcg gccaacattg 44199
   acatttttag tagacttttt gtttgtttac ttgcttatta tctgctgcct tccacactct 44259
   ggcgaaatcc tgccacccac ccacacacac ataggcactg aatgggcaga actctgaagg 44319
   ccagaatttt atatttcttt tcactgtaaa catcatcatc tgtcactgat ggcacactag 44379
   gatgctcagc aactgtgtgc atgaaggaag taagcactag tttgtgaagg ctgcaaaact 44439
   cttgagtatt ctaagagttt tggccaaaat gaatgtacag ctttagtggc agaagctaat 44499
   actcagaaat tgaggccgta tattggataa cacaggattt ggatgattat tttaaaataa 44559
   tattttacat tgtatatatg tgtgtgtgtg tgtgtgtgtg tgtgtgtatg tgtgtgtgtg 44619
   tgtatatata tatgtatgta tgtgtattag tccgttctca tgctgctatg aagaaatacc 44679
   tgagactggg taatttataa aggaaagagg tttaattgac tcacagttcc acagagctgg 44739
   ggaggcctca gaaaacttaa cagttatggc agaaggggaa gcaaacacat ttttcttcac  44799
   atggtggccg gaattagaag aatgtgagcc gagcaaaggg gaaagcccct tataaaacca  44859
   tcagacatcg tgagaactta ctattatgag aatagcgtgg gggaaaccac ccccacgatt  44919
   caattacctc ccaccaaatc cctcccatga catatgagga ttatgggaac tatgattcaa  44979
   gatgagattt gggtagggac acagccaaac catatcagta tgtatatgta tacaagtatt  45039
   atatatatat gtatgtgttt gtatgcatac atgtattata tatggaggaa attctaattt  45099
   tgtaaaaaac tggattgtga gttttaagga gatgttatat aaagttaaga caatgtcatt  45159
   ttgtggtatt ggtctgaatt acaatgtagt ttcttagtga tatttttcct ttattcag    45217
   tgt gaa aac  atc tcc tta aaa gaa  caa gtg gag tct ata  aat aaa     45262
   Cys Glu Asn  Ile Ser Leu Lys Glu  Gln Val Glu Ser Ile  Asn Lys
           1000                 1005                 1010
   gaa ctg gag  att acc aag gaa aaa  ctt cac act att gaa  caa gcc     45307
   Glu Leu Glu  Ile Thr Lys Glu Lys  Leu His Thr Ile Glu  Gln Ala
           1015                 1020                 1025
   tgg gaa cag  gaa act aaa tta g gtaagtttta tgactctgat aatataaaat    45359
   Trp Glu Gln  Glu Thr Lys Leu
           1030
   gattaacatc taataatgaa tatttcttat ttaaagttcc ttttttatgc tagattaaaa  45419
   ggaagtattt tgactaaaaa aagaaagaac tttctgccta ataatttaac ttaggcagat  45479
   gaataatcct gtacttaacc ccaccaaagt ttagttttca gtccttaagt tagatttgtt  45539
   tctaatgaaa tcatatatgt taaaaattta tgactaagta ttagctactt tgaaccgttt  45599
   aacaattaaa actgatgata ttttattaat ggtattatga gttctttcac tgagtgcaag  45659
   ttatattagt tatatatcac ttgatatttt taaattaaaa gataccagga aacagcaaag  45719
   aaaatgtgaa aagaagttgt atttctcata gttttactac tatattactg tatatttttg  45779
   ctcctatatg cttacatatt ttatatattt taaattatta taaacatggt tttatactgt  45839
   atttagatag taatatcaaa aatattttta tggccggcgc agtggctcac acctgtaatt  45899
   ccagcacttg ggaggctgag gagagcagat cccctggggt caggagttcg agaccagcct  45959
   ggccaacatg gcaaaacccc atctctacta aaagtacaaa aattagccag gcgtggtggc  46019
   agttgcctgt aattccatct actcaggagg ctgaggcagg agaattgctt gaacctagga  46079
   gtcagaggtt gcagtgagcc aagatcatac cacagcactc cagcctaggc gataagagtg  46139
   agactccgtc tcaaaaaaaa aaaaaaattt gttttattca tcatacttat aaatacttat  46199
   acaatagcct aatgtgtttg agtgattaaa tcactagctt tttatatttt tgctattgct  46259
   tatagtgcca cagtgaacat tttcatgtat atctaacaga gatattactg tctcagaagg  46319
   tattgaaatc tttgttgctc tcattagagt tttccatatt aatttttcaa acagttatat  46379
   agtttataag attttcataa ttttatctca tatattgtgc ttcataattt tcaaataaat 46439
   ttgctgcttt cgataatgta ttttcatgta tttgtttcct agacgttaga gctattcaag 46499
   gtttttatta ctaaatagag ctgttctctt aaattggtaa tgagatactt ggtttagaga 46559
   agcctaacac tgggaaatct tacataagct acttttagaa atgtaatttt tagctcaata 46619
   agagattaaa tatgaattga cttttgtgta gtatttgcat ggaagaaggt accatttaaa 46679
   tgaagacatg agagtattac gtacaatttt agtaggttct ttttatttta tcatctttat 46739
   ttttaataaa tgctgaattc cctacagaaa ttctttaatt tttacatatc ttgatctctt 46799
   tcatatatgg atttatatca ccgaagtttt aagagtgttt ccctattccc tgttgccctt 46859
   atatctttgt ttaaaaatgt cacatcatta gctttttttc atctaggaat ttgttagtgt 46919
   tgggctgttg tgctctaccc tctctttaag aaaactccaa acccaaaaac atacaagatg 46979
   gctagtctgc ttcagccttt gtgatgtgct tttctcttct aatcagagtt tagcacaata 47039
   cagaatggag aaggactcct ttatatattg gtatttattg cagtattttt ctacatggtg 47099
   cctaaggtta cttgaatgag tctttattcc ataatgaact gatttactaa tgcttttagc 47159
   acctgttagt gatccattat tgttagttac ttgattactg cttgccacag ctattctaaa 47219
   ataatacatt ttaaagataa atacagaaca taatgaagta ctttttaaaa ctgagataga 47279
   gaccaatttt tttttcagga aatgtatatt actttgagaa aactcagtta taaaacttga 47339
   acttatgaag ctggaaaaac aggagggggc attattggta ttgtaaaagg ctgtttacaa 47399
   agtgagttgc tgcttagttc ctttaagtaa ttggctaccc taaacacatc agttttaagt 47459
   tgctgaaaag caaaacactc taccaaattt tgtttttttt ctagaccatg tttacaaagc 47519
   aaaagtatgt tttcttcccc ccccctcaaa aaatgactaa tgacactcct atgcgatgcc 47579
   tttttatggt aaattgaggc ttttagttct ctttccattt agccacagac ttttgtgtcc 47639
   aaagacaagc tgcgtaactg catatataag gttaaggcat aactactaat aaaagaatgt 47699
   aaaatatttg atattaggtc tgtacaaaga ccaaataata ctcatgatta gacaagatta 47759
   tatttggtag aatctatcca tcatatggct tcagatttta cttttcagct tggctttgtg 47819
   agactttaaa aatcaagtca ttgcacttat attcacaaag tcacattgct ttactgcatt 47879
   gcttctcata cagtttatct cctttcagta aaatgtttac ttgccatttt taaaatttct 47939
   tatatgtgac acttctacac taagtccttt atgttgttag ttccacaatt ctgtgaggaa 47999
   taggtttttt tttttaatca tttgattgat gaagaacatt aagttccaca gagattaaat 48059
   ggtacaggca tcacacaggc aggaagtaac agagctaaga ttagagtcca ggtctgatgg 48119
   aattcagaaa gctaatgtgc tttccatgga actataatgc tttctaatat acagcatcta 48179
   aaatatctga ggtaatttta atataaacag catgagattg acttaaatat tattgcatgt 48239
   ag gt aat gaa tct agc atg gat aag gca aag aaa tca ata acc aac 48285
   Gly Asn Glu Ser Ser Met  Asp Lys Ala Lys Lys  Ser Ile Thr Asn
                       1040                 1045
   agt  gac att gtt tcc att  tca aaa aaa ata act  atg ctg gaa atg     48330
   Ser  Asp Ile Val Ser Ile  Ser Lys Lys Ile Thr  Met Leu Glu Met
   1050                 1055                 1060
   aag  gaa tta aat gaa agg  cag cgg gct gaa cat  tgt caa aaa atg     48375
   Lys  Glu Leu Asn Glu Arg  Gln Arg Ala Glu His  Cys Gln Lys Met
   1065                 1070                 1075
   tat  gaa cac tta cgg act  tcg tta aag caa atg  gag gaa cgt aat     48420
   Tyr  Glu His Leu Arg Thr  Ser Leu Lys Gln Met  Glu Glu Arg Asn
   1080                 1085                 1090
   ttt  gaa ttg gaa acc aaa  ttt gct gag gtttgatatt ataagtttta        48467
   Phe  Glu Leu Glu Thr Lys  Phe Ala Glu
   1095                 1100
   tcatacaatt atagaataaa gaattagttt tggtagacat tgtattattg ttaagtggtt  48527
   tgtctggatc tctgaaatat cttattaata tagtgcctat gttttgtgta ataaataaat  48587
   aaaagattta aatctgaatt gtttaaaagg aaagcagata tttctgtaag tttttctcac  48647
   caatgttata ttattagatt taatttatga aatgttattt actaaacaat ggaattgcct  48707
   ttcaccacca tcccttcatt taacaaatat ttattcattg cctattacat gtcagaccct  48767
   gtgttgggac tggcagtata gcaagaaaca aaatagacaa taatctctac tttcagggac  48827
   tttacattct aattggtggt tttatatatt tttgatgtgg tcagaatcat taaactgtgt  48887
   ggcagtaaat atagtttgca agtatttaac aatttatgat taaacacaac tcttacagtg  48947
   tttgcttacc ttgagattta atatattttc aaagcattta tatcattttt gttttaacta  49007
   tgtcactaaa tctatatgag taagatttta ttaactcatt tggatttatt tatagatgat  49067
   acaattgaag taaaatataa tgagcagatt gcattctaag caaagtaaga atattgcaag  49127
   ttcagatatt attagataat gagttgccta ataaaaatga cttttggtgg attggaatat  49187
   aaccagagtt tccatagttt gtttctgatt ctttcatatt ttttaccctc cttcagtctg  49247
   ttcttaacac ttcacactta atataatatg tgaactaagg ccaagtaaag aggattgcag  49307
   tactttaaaa gctaaattac aaagaaaacc tcaccaaaaa ttgatgtatc tgaacatttt  49367
   ttgttacatt tccttag ctt acc  aaa atc aat ttg gat  gca cag aag gtg  49417
                      Leu Thr  Lys Ile Asn Leu Asp  Ala Gln Lys Val
                          1105                 1110
   gaa  cag atg tta aga gat  gaa tta gct gat agt  gtg agc aag gca     49462
   Glu  Gln Met Leu Arg Asp  Glu Leu Ala Asp Ser  Val Ser Lys Ala
   1115                 1120                 1125
   gta  agt gat gct gat agg  caa cgg att cta gaa  tta gag aag aat     49507
   Val  Ser Asp Ala Asp Arg  Gln Arg Ile Leu Glu  Leu Glu Lys Asn
   1130                 1135                 1140
   gaa  atg gaa cta aaa gtt  gaa gtg tca aa  gtaagtgcat ataagcattt    49556
   Glu  Met Glu Leu Lys Val  Glu Val Ser Lys
   1145 1150
   tagccatttg actagatgta tcttctttaa tttgtcttta agaaacccaa ttacaggtat 49616
   acaattctta gtagtaattg atactgattt ctttttataa gaacaggatt aagtaatatt 49676
   aagatcggtt ttaacagggt taaataataa tattgacgag aataatattg ttaaagagga 49736
   agtgacctct caagatttgc attttttaga gttcaggaat attattgcag aaaggtccag 49796
   ttcctccaca tattgatttt ttggggaagg ggtgatggag gaggaatggt tgtttattgt 49856
   atttaaactt aagtttcttc attttaataa gggagtaata gtacctcttc tacctgtttc 49916
   ataaggttgc tgtaagaata taataaaaaa ttcagatttt gatttagttt acatttatcg 49976
   ggcatctact atgtactagt cacggtgcaa ggtattaaac atatattgac ttgtacaatt 50036
   atacttaacc ttgaggttat atttttgttt tcattttaca tgaagaaata tgcccagcta 50096
   gtttagaaca caaaatatat ataaggagta aatactgcgt gctggctggg cgtggtgaca 50156
   tgtgcctgta gccccagcta ctcgggaggc agaggcagga gaatcgcttg atcccgggag 50216
   gtggaggttg cagtgagccg agatcgcgcc actgcactcc tgcctggtga cagagcgaga 50276
   ctctgtcaaa caaacaaaca aacaaagaaa aacaaaacaa aaaaaccgtg tgccagctat 50336
   atgctgtatt ttcattctct tttgtaatta ggtgatattt cagtagaaaa gtataaggag 50396
   cacttagtta atctgtcaag cataaatagt aaaaatattt tatggcctac tcataaaaat 50456
   ataaccattc ctttggagcc ttgatagttc tcttgggaat atcagttttt gacatctttt 50516
   tcactatgaa agaccctttt ttttaaaaaa attgatcctt tcttctcatg gacctctttt 50576
   gatataaact aacttataat agttcatttt aatcatattt tgttaatcat gcaactggca 50636
   atgagagcct ctcatcagta tgaggaaacc tgccttatct ataatactga actaaaatta 50696
   ttctaaccca aagcaaagaa actttacatt ttgctttgcc tgtattagct tatcacagta 50756
   ttcatgaggg aatttgaagg acttattacc attaggctat ctcttttttt ttttttttgt 50816
   aattttatta aatgcatgtt ttgtttcttt tcacattact gataacttgt agattaaaac 50876
   aaatcaaaac atgcattaat ccatctaagg atcctagaaa ttttacattt ctgtgttctt  50936
   aactgtgtga tggtcttaga taaatgtact aaatacctta tcctagcata ttccaaatta  50996
   tgacaataaa tgttttatgg aaaaaagtat gggaacagaa gttctttggc tatatacatt 51056
   tggaaaatac tatatagtaa gtatgatttg agataattat atatgataga acctctggga 51116
   gcactgaata tatgttagga atattcaaga gggaggaggg atgttgagaa tgaagttttt 51176
   tttatatagc aaacatgata acctctgatg gaattatgtt tcatgaaaca gtttaggaaa 51236
   tcctgtttta atatttcata caaagaagag atagatgctg aaaacgaatg gctttttgaa 51296
   aaagggtcta gaaattttga attttggcat ttacttagaa agtgtactta attgttcctg  51356
   aaatacctta tcatttccta g a ctg  aga gag att tct gat  att gcc aga    51405
                           Leu  Arg Glu Ile Ser Asp  Ile Ala Arg
                           1155                 1160
   aga caa  gtt gaa att ttg aat  gca caa caa caa tct  agg gac aag     51450
   Arg Gln  Val Glu Ile Leu Asn  Ala Gln Gln Gln Ser  Arg Asp Lys
       1165                1170                1175
   gaa gta  gag tcc ctc aga atg  caa ctg cta gac tat  cag gtatgtgcag  51499
   Glu Val  Glu Ser Leu Arg Met  Gln Leu Leu Asp Tyr  Gln
       1180                1185                1190
   tattggctct tctacataga atccactttt ttccctaaat ttacattaga tgttgggagt  51559
   gggatatgtt atactttttg tttgtttcga gatagggtct cattctgttg cccagggtgg  51619
   agtgcagtgg tacattcaag gctcattgca gccttcacca cctgggttca ggtgatcctc  51679
   ccacctcagc ctcttagaca gctgggacta caggcacgtg ccaccacacc taattttttt  51739
   gcattttttg tagagacagg gtttcaccat gttgcctagg ctggtcccaa actcctgggt  51799
   taaaatgatc tgcccacctt gacttcccag aatgctggga ttacaggtat gagccaccat  51859
   gctgggccat tgttacattt ttaatcaaaa gatataccaa ccagaggctg ttattcttgt  51919
   tagttggaac ctgattagaa agctctttaa tttgaaatat tgttcagtaa tccagtacag  51979
   catttaaatg cctatagatg aattatgctg ctgatcaaaa ttaggacact gagaattgta  52039
   gttagtaaat ctttaataac aatattttct cttgtattta tatgtaactt tttacatatt  52099
   cttacgttat atatgttggg aattataaaa acatacacat tgtcctgatc agtattatgt  52159
   tacttgcaat ggaggttaaa aaaaaactgt aacagtcagg catggtggct cacgcctgta  52219
   atcccagcac tctgggaggc cgaggcaggc ggatcacgag gtcaggagtt cgagaccagc  52279
   ctgaccaata tggtgaaacc ccgtccctac taaaaataca aaagttagcc aggcgtggtg  52339
   gcatgtgcct gtaatcccag ctacccagga ggctgaggca ggagaattgc ttgaacccgg  52399
   gaggtggagg ttgcagtgag ccaaaatcac gccattgcac tccagcttgg gtgacagagt  52459
   gaaactctgt ctcaaaaaaa aaaaaaaaaa acaccagtaa catacccact gttattcagt  52519
   tacatttgga ttttaagttt gtttgattct aggttttttc ttttacagtt ctttggtaat  52579
   tatttgtatt aaagcaaagt tacatttttg tagatctcat gtgccactgt gttaaaactt  52639
   tgcttagtaa attgtgaatt ttaaatctgt gataactttc actggaaaaa tttgaaactt  52699
   actacaaata tatatttttt ttaatatcag gca cag tct gat  gaa aag tcg ctc  52753
                                    Ala Gln Ser Asp  Glu Lys Ser Leu
                                                     1195
   att  gcc aag ttg cac caa  cat aat gtc tct ctt  caa ctg agt gag     52798
   Ile  Ala Lys Leu His Gln  His Asn Val Ser Leu  Gln Leu Ser Glu
   1200                1205                1210
   gct  act gct ctt ggt aag  ttg gag tca att aca  tct aaa ctg cag     52843
   Ala  Thr Ala Leu Gly Lys  Leu Glu Ser Ile Thr  Ser Lys Leu Gln
   1215                1220                1225
   aag  atg gag gcc tac aac  ttg cgc tta gag cag  aaa ctt gat gaa     52888
   Lys  Met Glu Ala Tyr Asn  Leu Arg Leu Glu Gln  Lys Leu Asp Glu
   1230                1235                 1240
   aaa  gaa cag gct ctc tat  tat gct cgt ttg gag  gga aga aac aga     52933
   Lys  Glu Gln Ala Leu Tyr  Tyr Ala Arg Leu Glu  Gly Arg Asn Arg
   1245                1250                 1255
   gca  aaa cat ctg cgc caa  aca att cag tct cta  cga cga cag ttt     52978
   Ala  Lys His Leu Arg Gln  Thr Ile Gln Ser Leu  Arg Arg Gln Phe
   1260                1265                 1270
   agt  gga gct tta ccc ttg  gca caa cag gaa aag  ttc tcc aaa aca     53023
   Ser  Gly Ala Leu Pro Leu  Ala Gln Gln Glu Lys  Phe Ser Lys Thr
   1275                1280                 1285
   atg  att caa cta caa aat  gac aaa ctt aag ata  atg caa gaa atg     53068
   Met  Ile Gln Leu Gln Asn  Asp Lys Leu Lys Ile  Met Gln Glu Met
   1290                1295                 1300
   aaa  aat tct caa caa gaa  cat aga aat atg gag  aac aaa aca ttg     53113
   Lys  Asn Ser Gln Gln Glu  His Arg Asn Met Glu  Asn Lys Thr Leu
   1305                1310                 1315
   gag  atg gaa tta aaa tta  aag ggc ctg gaa gag  tta ata agc act     53158
   Glu  Met Glu Leu Lys Leu  Lys Gly Leu Glu Glu  Leu Ile Ser Thr
   1320                1325                 1330
   tta  aag gat acc aaa gga  gcc caa aag gtaaacattt aaacttgatt        53205
   Leu  Lys Asp Thr Lys Gly  Ala Gln Lys
   1335                1340
   ttttttttta agagacagta tcttgatctg tttcccaggc tggagttcag tggtgcaaac  53265
   atagctggaa ctcctgggct caagggactc tctagcctca gcctcctgag tagttgtagc  53325
   tggcagtaca ggtgcacacc accataccta cctaattttt taaaattttt aaattttttt  53385
   gtagagacaa ggtctcactt tgtcacccag gctggccttg aactcctggc ttcaagtaat  53445
   cctcctgctt tggtctctca aaagtgctga gattacaggc atgagccact gtgcccagcc  53505
   aattttaaat tcattatctt caaaagagtt acatgataat ttcttaatat atgcctatat  53565
   gaaaaatgct taagatacaa attccaatta tgattcatta atttagattt tataacttag  53625
   cagtgttggc tatttgaatg tctattatac gtaaaaataa aattaggctt ttctaaccaa  53685
   agattttagt gggaatgttc agattgtata atagcaaaga attttaatta ctataggaaa  53745
   atttatatta attaaacact aattattata tttaaacatt gtagtagtta tcagttgatt  53805
   tctactgttc ataattatct ttgatctaca agtagtgggc ccacatttac ttttaatatg  53865
   gtttaatctt catttagaaa gaattaaatg aaaaataatt atcttgcaac tacatcctgt  53925
   tctctaggct agaaacattt aggatttctg tttttgaaag taataccaaa gttccaatga  53985
   cctgcttata gtcagtgttc aataaacgta taacaaatga aagtgaatat tagtgatgtc  54045
   cattccaaca taatttgaag atttttattg taaaatccca catatttgta gaaaagtcta  54105
   tggaaatcct aaataagatt ttgtcatgta gtttgacaaa agataacatt gtgtcttatt  54165
   ttattttaga atggccatta ctttcaatta aaatcattat catcaatgga ggaatgttat  54225
   ttgttaatat agcatttata tttgtgtata taaattgtaa atcttag gta atc  aac    54281
                                                        Val Ile  Asn
                                                            1345
   tgg cat atg aaa  ata gaa gaa ctt cgt  ctt caa gaa ctt aaa  cta     54326
   Trp His Met Lys  Ile Glu Glu Leu Arg  Leu Gln Glu Leu Lys  Leu
               1350                 1355                 1360
   aat cgg gaa tta  gtc aag gat aaa gaa  gaa ata aaa tat ttg  aat     54371
   Asn Arg Glu Leu  Val Lys Asp Lys Glu  Glu Ile Lys Tyr Leu  Asn
               1365                 1370                 1375
   aac ata att tct  gaa tat gaa cgt aca  atc agc agt ctt gaa  gaa     54416
   Asn Ile Ile Ser  Glu Tyr Glu Arg Thr  Ile Ser Ser Leu Glu  Glu
               1380                 1385                 1390
   gaa att gtg caa  cag aac aag gttttatttt atatttattt catttttttc      54467
   Glu Ile Val Gln  Gln Asn Lys
               1395
   cctaagtttt tttttttttt tttttttttt gagatggagt ctcactctgt cgcccagact  54527
   ggagtgcagt ggcgtgatct cggctcactg caagctctgc ctcccgggtt catgccattc  54587
   tcctgcctca gcctcccaag tagctgggac tacaggcacc cgccaccgtg cctggctaat  54647
   tttttgtatt tttagtagag acggggtttc accatattag ccaggatggt cttgatctcc  54707
   tgacctcatg atccgcccgc ctcggcctcc caaagtgctg ggattacagg cgtgagcccc  54767
   taagatttta aacaagaata ttgcacaaat gactatgtta tccttctaat taagtgcacc  54827
   ttccattact aattgattat ataataattt gtttttattt ttctaaacta ttctaaaaat  54887
   tcatatttat ttagctttta taacagtagt cttaatctta aaaacggcaa tacataagca  54947
   acctcatttg gtaagttaat ttttattttg atattggtta tttgactttt cacagttcca  55007
   cgtttctact ggctctcact gatagagtaa gaagtcagct tcttatagaa taaagtatat  55067
   acttcagaga cagatgaaat tcgtcaaaca tatgactgtc tcagagattg ttccccctgc  55127
   ttaaattgtt cttaccctag atacctttgg tatttacact gtcagtgcct gcaggtctta  55187
   gctcaaatgt cttaccttat cagtgtatcc ttcaccagcc acctaatata caacagtaaa  55247
   tcctactatc cagattccta aatagagatt aattaactta atttttctcc aaagtgcttg  55307
   taaccttctg acgtattaca tacttactgg tttattattg actgtctttc cttcgccaga  55367
   atgcaagttc cgtggtgaca cggacttggt tttgtttact gccatgtttg tatttcctag  55427
   aatgatgctt ggcacataat atatgtcatc aaatatcttt cgtatagctg aacggatgga  55487
   tggatggatg gatggatgga tggatagact gaaatcctta cttcacatct gcctttgtga  55547
   tcttacacaa gttacttcac ctctctgagt ttgtattttt ttccataaaa ggaaaataat  55607
   tacagtttct tcaatgtgtt gtgaggatta gataagaaaa tatatataaa atgcctgtta  55667
   tgtgcctgat gtcttcgtgt atgtgtctga cacaaattgt ccttttttta g ttt cat   55724
                                                            Phe His
                                                               1400
   gaa gaa aga caa atg  gcc tgg gat caa aga  gaa gtt gac ctg gaa      55769
   Glu Glu Arg Gln Met  Ala Trp Asp Gln Arg  Glu Val Asp Leu Glu
                   1405                 1410                 1415
   cgc caa cta gac att  ttt gac cgt cag caa  aat gaa ata cta aat      55814
   Arg Gln Leu Asp Ile  Phe Asp Arg Gln Gln  Asn Glu Ile Leu Asn
                   1420                 1425                 1430
   gcg gca caa aag gtatgaatga ttaatcttgt ttgttactct gtagcatagt        55866
   Ala Ala Gln Lys
   ctagagtgtt aactcacaga aatatttcct gtatcagatg taattttaat tgatgttata  55926
   ttgtatattt aaaatataag aggggtttaa tctatgtttt atcatacagc tgtaaaaatt  55986
   aatagttact ctcaatgctg caactgcttt tttaaaaaac atactatttc ttaatag     56043
   ttt  gaa gaa gct aca gga  tca atc cct gac cct  agt ttg ccc ctt     56088
   Phe  Glu Glu Ala Thr Gly  Ser Ile Pro Asp Pro  Ser Leu Pro Leu
   1435                 1440                 1445
   cca  aat caa ctt gag atc  gct cta agg aaa att  aag gag aac att     56133
   Pro  Asn Gln Leu Glu Ile  Ala Leu Arg Lys Ile  Lys Glu Asn Ile
   1450                 1455                 1460
   cga  ata att cta gaa aca  cgg gca act tgc aaa  tca cta gaa gag     56178
   Arg  Ile Ile Leu Glu Thr  Arg Ala Thr Cys Lys  Ser Leu Glu Glu
   1465                 1470                 1475
   gtaattagaa gaatttgcat tttgattagt gtattatttg gtatgtttgg ggggctttct  56238
   aaataatatt tctttatgag ggcaatgcat agaatgatga atctattgct aatttcacta  56298
   tttttctatt ctcctataat gtttctaata gccaataatg aacagcagat atagttaatt  56358
   tgaattcact atttaattat tagttggtac ctttcggtac actgaatatg aaaggaaata  56418
   aaaagcattt aattgtagtt ctatgagcaa tatattctct tatatgatct ctttattctt  56478
   acttttttgg ttttattttg aagtgcatgt tacataatct atgaatcaat tttcagttca  56538
   ttgcctttaa tgcatggtta aagggttgaa ggtaaattag aaattacttt ctgttttaac  56598
   ctagatcttg aatttgatta gtaggtgatc aaatctgtca tcttcattaa attattcaga  56658
   aaataatgta aactgaatgt gttttcattt tagttttcat ctaaataaac tgcaaataca  56718
   tttaaaatat acataaagaa gtttttcaag taaaactgta catttttaat catttcagga  56778
   aacgtagatt ttcttcagta attttaagat ttgtcattta tgtgaattgc cattgaatta  56838
   cttaatttaa aatactcacc ttaatcctct tgaagagtaa aaatttttct gtttttttct  56898
   ctttgtttta ataagctgcg gattttatat tcgtaattta ttgagttggg cctctaaaat  56958
   tccagttttg tacttaactg acttatagat tagtctccta atgctctgct agtcaatgga  57018
   ccaaaataaa agaaataatt tattacatat tcttcctaaa tctagtacca ccatacatgt  57078
   ataattctaa actgtaatat ctcaataaag taccttaatt aaattttatg ttcatcataa  57138
   caatgaagtt tctagcatat gtaatagtct tataaataag catgcaaata actgctgtca  57198
   attagaatta gtcagtttaa ccttattaag tatcaaatgg ctattgtaca tatgatgtga  57258
   aaaataaagt gaattttttt tggctaataa ctaatctaaa attcagatga agcattttaa  57318
   agggaaaaag atactttaat gatttattat aatttaatca ttgcag aaa  cta aaa    57373
                                                      Lys  Leu Lys
                                                      1480
   gag aaa gaa  tct gct tta agg tta  gca gaa caa aat ata  ctg tca     57418
   Glu Lys Glu  Ser Ala Leu Arg Leu  Ala Glu Gln Asn Ile  Leu Ser
           1485                1490                1495
   aga gac aaa  gta atc aat gaa ctg  agg ctt cga ttg cct  gcc act     57463
   Arg Asp Lys  Val Ile Asn Glu Leu  Arg Leu Arg Leu Pro  Ala Thr
           1500                1505                1510
   gca gaa aga  gaa aag ctc ata gct  gag cta ggc aga aaa  gag atg     57508
   Ala Glu Arg  Glu Lys Leu Ile Ala  Glu Leu Gly Arg Lys  Glu Met
           1515                1520                1525
   gaa cca aaa  tct cac cac aca ttg  aaa att gct cat caa  acc att     57553
   Glu Pro Lys  Ser His His Thr Leu  Lys Ile Ala His Gln  Thr Ile
           1530                1535                1540
   gca aac atg  caa gca agg tta aat  caa aaa gaa gaa gta  tta aag     57598
   Ala Asn Met  Gln Ala Arg Leu Asn  Gln Lys Glu Glu Val  Leu Lys
           1545                1550                1555
   aag tat caa  cgt ctt cta gaa aaa  gcc aga gag gtattttatt           57641
   Lys Tyr Gln  Arg Leu Leu Glu Lys  Ala Arg Glu
           1560                1565
   atattatgag ttatgctgtt atccattagt ttttttaagc aaatgctaaa tattatttta  57701
   ccctaaagtg gtatttcttt tcttgctttc aaatgattct atttaagaat tgttacttgc  57761
   atgtgattgg attacacctc tgtcagtaaa actggaagtt tgtgtacatg tatctttcta  57821
   ttatacactg actaaaccac gagtagctat catggtgaaa tcatatgatt ttgaaaaata  57881
   ttttaattga gtttataggt gaggattgag gcaatagggt ggaatgaaat atatcacacc  57941
   ggtaatcagt agaaatcaga tttgttagaa cttcgtgggg gaaagctaac atttaatttt  58001
   ttctagaagt aagttaaaag atgatagata catgtcattc taatgttaag aataaattat  58061
   gaactgaggc tgggcttgtc aacttgaaca ttgtctgagg ggacatgcat accagtctag  58121
   atacatacat atatggagat actgtttctt cctcatctca aaggaatttt agaagattga  58181
   agagaaaata tataaggtct tcaaaatgtg aatttgtttt aatcacaatt taagatatag  58241
   tttcgatttt ctgtaaaaca g gag caa  aga gaa att gtg aag  aaa cat gag  58292
                           Glu Gln  Arg Glu Ile Val Lys  Lys His Glu
                               1570                1575
   gaa gac  ctt cat att ctt cat  cac aga tta gaa cta  cag gct gat     58337
   Glu Asp  Leu His Ile Leu His  His Arg Leu Glu Leu  Gln Ala Asp
       1580                1585                1590
   agt tca  cta aat aaa ttc aaa  caa acg gct tgg gtaagattct          58380
   Ser Ser  Leu Asn Lys Phe Lys  Gln Thr Ala Trp
       1595                1600
   aagaactttg ttccattctt tattgatttt tgtgaccatg taaattaaaa ttcagctctc  58440
   ttcttttttg gaatggaagt tacccttttt gttgccaaaa taatcttctg aaaacatagc  58500
   tctgatcatt cttcctcctg tagctcaccg ctgttcacaa aattatattt ataattctta  58560
   gccatgtact caatctgcta tgaacctacc tgcctttctt ttcaaattct actcactgtg  58620
   agtttagcta tatctaactt ccagaattca gctcatattt gcctcttttg accattctgt  58680
   tccatatgta tgaaatgaca tgtctttcat cttttaatgt gtaaccttag catatttgag  58740
   cattacctcg ttaattcggt caacacttat tgatctcctg ctacgtgcag acattttgct  58800
   agctattgta aatacaaata ataaagtctg catttcctgt cttctttaag ccttcattgc  58860
   ctattaaatc attacatttt agattagata ttatattttg atcatttgag gaaccaaatt  58920
   aaaaatatgg aataagtatg gcattgaatt atacatgcct attgctaata tattcatatt  58980
   ttatag gat  tta atg aaa cag tct  ccc act cca gtt cct  acc aac aag  59028
          Asp  Leu Met Lys Gln Ser  Pro Thr Pro Val Pro  Thr Asn Lys
          1605                1610                1615
   cat ttt  att cgt ctg gct gag  atg gaa cag aca gta  gca gaa caa     59073
   His Phe  Ile Arg Leu Ala Glu  Met Glu Gln Thr Val  Ala Glu Gln
       1620                1625                1630
   gat gac  tct ctt tcc tca ctc  ttg gtc aaa cta aag  aaa gta tca     59118
   Asp Asp  Ser Leu Ser Ser Leu  Leu Val Lys Leu Lys  Lys Val Ser
       1635                1640                1645
   caa gat  ttg gag aga caa aga  gaa atc act gaa tta  aaa gta aaa     59163
   Gln Asp  Leu Glu Arg Gln Arg  Glu Ile Thr Glu Leu  Lys Val Lys
       1650                1655                1660
   gaa ttt  gaa aat atc aaa tta  ca  gtaagtcttc gaaatgtatt           59206
   Glu Phe  Glu Asn Ile Lys Leu  Gln
       1665                1670
   gtaaaaatag gcaaatgata agtgatataa tgaagataaa cataagtgtt tgctatgcca  59266
   ggcactgttc taagactttt aagtatattg tctcattttt atcctcagga ctgctggtta  59326
   catatgttat cattttcccc attttaaaga gaggatatgg cctcaggaat gcttaatagc  59386
   atgtctgggg gtagatggga aagccataat ttgaaactag tcagtctgac tcaaaagcca  59446
   atacaaattc ttttccagaa tctcattttt accttctttg agcctcagtt tcatcttatt  59506
   tatttatttt tatttttgag acaaggtctg gctctatttc ctaggctgga gtgcagtgac  59566
   ataatctcag ctcactgcaa ccttgacctt ccaggctcaa accatcttcc cacctcagcc  59626
   tgcagagtag ctggcactac aggcaggtgc caccacacct gggtagtttt tttgtatttt  59686
   tgtagagaca aggtttctcc atgttgccca ggctggtctt gaactcgtga gctcaagtga  59746
   tccgcccact tcggcctccc aaagtgctgg gattacaggc ctgagccatt gcacccagcc 59806
   tcatcatctt taaaatggaa ataataatac ttaccctggc cctttcaggg tggttatatg 59866
   aaggtcaaat tataccgtgt atgaaagtaa tttgaaaact gtaaaataac atacagatag 59926
   aaaacttttg attacacact tataagagtg tctgtcatat aatagagatt ctaaacattg 59986
   ttcaaccact ttatcagaac gtagatttta aactcaaaat aggtttatag ttaggtagtt 60046
   tctaatcatt ataatattat ctctatgggc ctaaatttta ttatctgaaa aaacatgaga 60106
   aaattgaact gcttgactta taattccatt tcagctctca agcccctgct agagtctttg 60166
   attctttact cacttattca aatgcctctg acagaattaa cactattttt gctttgctaa 60226
   ggagctgcca ctgttaagaa attactctct aaaagaaaga aaattggcaa cagcatatgt 60286
   gtattttcag tctcttttcc tcactctatt aaattttgta caagagatgt tatttttggt 60346
   ctagtaaatt tctgtcatgt tttggagtat aaaattactt gtgcttttgc atctaatttg 60406
   tgggtgtaga aaatcataat cttttgaaat accttatata atacattttt ttgccacagg 60466
   aaatacttga agttattgtt gtgtacctta cgtcatttta gtccaaaatt atacttgtgt 60526
   tctctgtgtg catattttga tatgtattag gagattatgg atctgtgtga tttcttaagt 60586
   aaatcctgat attttcacaa tttgatgatg actctttaaa gttagactta agttttgcca 60646
   aaagcaagaa gcctcaaaga gtaacatttg ttcatgtctt aacactatct ccctcttatt 60706
   ggtcagaatc tcagtatgga tgcagtgtcc atatgcacaa caatatatta attcagttta 60766
   acagacttaa tgctgaataa gcaataagat taattgaatt aactaaatct tttgatagta 60826
   tccacttcca tatatatagt tatagatata atgctagtga atttgaacca taaacaaatt 60886
   aataatacat gtgatttctg tgaaaattta tattagtctt ttcaatatgt caatataggg 60946
   cagtatttct caaatataga ggatcagttt ttcaccattg tccctcttgg ggacatttgg 61006
   cgatgtctgg agacattttt gattgtcatg gctcgggggt gctactggta tccagtgggt 61066
   agaatcaaaa gatgctgcta aacatcctat catgcacaag gcagccccac caccaacaaa 61126
   gaattatcca gtcaaaaatg ttactagtag tatggttagg aaactatcat atagaggaag 61186
   caatcacatt ttacaagagc cataatattt aaaatgcctt tttgttcatt ctctgtatat 61246
   ttgactagag tcacaaaata acttgataag attgttgcca aaaatattag aaactagaag 61306
   aaaaatgtgt tgttaagtct aagagtagtt aaatgaaata aagaattatt cttctttgga 61366
   tttggatgcc tgcatcaaga tttagattgt aaggatactt aggactgaac atttgctcta 61426
   tatgaaattt gtattaatca aggtatgaat tgcagcaacc actctattaa ttacatatgt 61486
   ttggccaggt gtggtggctc acacctgtaa tcccagcaat ttgggatgcc aaagcgggct 61546
   tatcacctga ggtcatgcgt tcaaactggc ctggccaaca tggtgaaacc ccatctctac 61606
   taaaaataca aaaattagct gggcctgatg gtgcacgccc gtagtcccag ctactcagga  61666
   agttgaggca aaaaaatcac ttgaatctgg gaggcagagg ttgcagtcag ccgagattgc  61726
   gctgctgcac tccagcctgg gtgacagagt gagactgggt ctcaaaaaaa ttaaaaatta  61786
   aaaaacacac acacacatat gtttatttac atcag g ctt caa gaa aac  cat gaa  61840
                                            Leu Gln Glu Asn  His Glu
                                                             1675
   gat gaa gtg  aaa aaa gta aaa gcg  gaa gta gag gat tta  aag tat     61885
   Asp Glu Val  Lys Lys Val Lys Ala  Glu Val Glu Asp Leu  Lys Tyr
           1680                 1685                 1690
   ctt ctg gac  cag tca caa aag gag  tca cag tgt tta aaa  tct gaa     61930
   Leu Leu Asp  Gln Ser Gln Lys Glu  Ser Gln Cys Leu Lys  Ser Glu
           1695                 1700                 1705
   ctt cag gct  caa aaa gaa gca aat  tca aga gct cca aca  act aca     61975
   Leu Gln Ala  Gln Lys Glu Ala Asn  Ser Arg Ala Pro Thr  Thr Thr
           1710                 1715                 1720
   atg aga aat  cta gta gaa cgg cta  aag agc caa tta gcc  ttg aag     62020
   Met Arg Asn  Leu Val Glu Arg Leu  Lys Ser Gln Leu Ala  Leu Lys
           1725                 1730                 1735
   gag aaa caa  cag aaa gtaagtaaca acagaaaatt atcaacattt aggaaaaata   62075
   Glu Lys Gln  Gln Lys
           1740
   tgtggtagat tgcttttaga gaagatttgt aaatttataa aagatggtag tataaatctc  62135
   cgtgttgtaa taaaaagtat gagctttatc ttatgctgtt aaacaaggta ttttagacaa  62195
   tgctgttttt gtgggcagat atagtccaat ttatcttttt atgttttcgt caatctgatt  62255
   tgtgaattat ctatatgaag ttaggaaaaa tcttaatgta cattacaaaa atataatata  62315
   tattacattg tattttcttt ttttctactg gaattttatg ctactgaggc tatttttaac  62375
   aaatgaacaa ttttgaacaa tttgagggat tgagggaagt atgataatga caaaaaggga  62435
   tgaaaaaagg gggtcataga gatgtttttg tgagaaggag ttggtcagtg tattctgatt  62495
   tattagggtt ttttttagtt tatctcagat ttgatctatt taaattgttt tagaagatgc  62555
   tggtgttttt ctgtgctagc tatgaaattt atgggtaaac tttaagcctt tcctagtcct  62615
   tttgttgtct acctaaattc aattaatttc atatggaagg atgtagtaag tgagtaatat  62675
   aaatatctaa aattggatgt ttgaaaacaa aacatacctg ttttttgtaa tagcttgatt  62735
   taatgctgag ttctcaaaat cattattaag attttgaact ttcacattca atgtggaaag  62795
   aattgagtgt aattacaaaa gatttatttg aaaaagttga gttgttaatt tgtgaaatat  62855
   gttccattaa actcataata ttttagaaaa atagtaggaa gtaataaagc ttgtttattt  62915
   tttatatcat atattcatat aaaatgtcag ttttccttta aaaattacat tttttttttg  62975
   gttaattttt ag gca ctt agt  cgg gca ctt tta gaa  ctc cgg gca gaa     63023
                 Ala Leu Ser  Arg Ala Leu Leu Glu  Leu Arg Ala Glu
                         1745                 1750
   atg  aca gca gct gct gaa  gaa cgt att att tct  gca act tct caa     63068
   Met  Thr Ala Ala Ala Glu  Glu Arg Ile Ile Ser  Ala Thr Ser Gln
   1755                 1760                 1765
   aaa  gag gcc cat ctc aat  gtt caa caa atc gtt  gat cga cat act     63113
   Lys  Glu Ala His Leu Asn  Val Gln Gln Ile Val  Asp Arg His Thr
   1770                 1775                 1780
   aga  gag cta aag gtgaacatca acacgtgtta atgtaacaaa atttctgata       63165
   Arg  Glu Leu Lys
   1785
   attcctattg gaagagaatt cactatgata tatagtaatt ttgttgatga atagggaatt  63225
   tataatgcac tgttggtggc tagacataga cacacacatg catttttcaa caataagtct  63285
   ctttatgata ctcatttact gattatcatc ttggggatta ggaaaggata ggccattatg  63345
   aactactgtt tctaatgaaa ttaaatttaa gaaatatttt acttaggatt ttttttaaga  63405
   ctttattatt tttttagagc aattttaggt tcacagcaaa attgagagga aggtacagag  63465
   atttcctgta tatctcctac cctgaaagtg gtacatttgt taaaattgat gaacctatat  63525
   tgatacatca taatcaccca aagtccaagt ttacctctat tttagctctt ggtattttac  63585
   actctgtgtg tttagacaaa tgtataatga tatgtatcca tcattatagt attatacagg  63645
   gtattttcac tgccctaaaa atcttctgtg cctctcttct tcattccttc ctctgcacct  63705
   caccaaaccc ctggcaacca gtgatctttt tactgtctcc atagtttcac cttttccaga  63765
   atatgttata gatggaaaca tacagtgtgt ccccatcatt ctcaccatag gacagctagg  63825
   aactcctttc tagtggcata catattgtct agtattgtaa gttacccttt tatatcttat  63885
   ctttgtaaac taggttagaa attacttcaa gtcagagatt tgttctgtac tactcttatg  63945
   cttcatagtg tttaaaacgt tgtcatatat attgttatat acttgtttgt ttaattaatt  64005
   cagccaaaat gaaacgtgca tatttgataa aattttgttt gtgggtgttt gttgaagatg  64065
   aattgcttta cactagtttt tttttttttt ctcaaagtcg acttttttcc tcaaggtaga  64125
   cttgacatga atatggaaaa atatatgtag tttgtggtta ttttttttct cttgtgtact  64185
   taaaaattca gactgaattt ttcttataat ggtatatttt ctgttttatg ttccttttat  64245
   cattgatact tcttgaagag tcatgaataa tacctttctt tttctcttat tag aca     64301
                                                              Thr
   caa  gtt gaa gat tta aat  gaa aat ctt tta aaa  ttg aaa gaa gca     64346
   Gln  Val Glu Asp Leu Asn  Glu Asn Leu Leu Lys  Leu Lys Glu Ala
   1790                 1795                 1800
   ctt  aaa aca agt aaa aac  aga gaa aac tca cta  act gat aat ttg     64391
   Leu  Lys Thr Ser Lys Asn  Arg Glu Asn Ser Leu  Thr Asp Asn Leu
   1805                 1810                 1815
   aat  gac tta aat aat gaa  ctg caa aag aaa caa  aaa gcc tat aat     64436
   Asn  Asp Leu Asn Asn Glu  Leu Gln Lys Lys Gln  Lys Ala Tyr Asn
   1820                1825                1830
   aaa  ata ctt aga gag aaa  gag gaa att gat caa  gag aat gat gaa     64481
   Lys  Ile Leu Arg Glu Lys  Glu Glu Ile Asp Gln  Glu Asn Asp Glu
   1835                1840                1845
   ctg  aaa agg caa att aaa  aga cta acc agt gga  tta cag gtaattttat  64530
   Leu  Lys Arg Gln Ile Lys  Arg Leu Thr Ser Gly  Leu Gln
   1850                1855                1860
   atttaactct gataatgtct gatttacaat atagaggtag tagtttattt ctactttatc  64590
   attttatcta tggtatttgt taaaactgac tttcaaatca ctttgattaa tgtaattaat  64650
   ttcttttgtg acttctattg tgtttatagt tctagagtag catattagta tgttgtatta  64710
   aaatgcagaa gcagctacca gattatctta tgtattaagt gtcatttaga aagtatggtc  64770
   agtgatagct tcagaaagtt gctattatat aattgaaata tttactgtct attttgtttt  64830
   acatttattt gtaaaaatat aaagttacat tttatttttt ag ggc aaa ccc  ctg    64884
                                                  Gly Lys Pro  Leu
                                                              1865
   aca gat aat aaa  caa agt cta att gaa  gaa ctc caa agg aaa  gtt     64929
   Thr Asp Asn Lys  Gln Ser Leu Ile Glu  Glu Leu Gln Arg Lys  Val
               1870                1875                1880
   aaa aaa cta gag  aac caa tta gag gga  aag gtg gag gaa gta  gac     64974
   Lys Lys Leu Glu  Asn Gln Leu Glu Gly  Lys Val Glu Glu Val  Asp
               1885                1890                1895
   cta aaa cct atg  aaa gaa aag gtatgtgaag aaacatactg acttatatgc      65025
   Leu Lys Pro Met  Lys Glu Lys
               1900
   ttaaggtagt gacagagtaa gttaaataca tagctgatta acagttaata tactgcctta  65085
   atttgatgac ctggctgtat taattctgta ttaattttga ggactataag cagtattgaa  65145
   taacgtagaa aagtctaagt ttctgttctg taggaattta gagtctactt gaggagatac  65205
   ctataatgta actcttattt ggaaattact acatcaattt cattcatctt tctgacatta  65265
   gagtacctct gaagttcctt cacaccttaa catattcaac tgtgtatcat ttctctccaa  65325
   agtaatcatt tacacaggtt ggtgcttttg acttttggga cagaaagata gacattttaa  65385
   gataccccac tttgacccaa ataggtcctt tttaatcctt caggagacta ggctgttatt  65445
   tcagatagca aagttatttg gaatatcttc agtatttgca gtaataatca gtaaccaatc  65505
   tgctcataga ttaattctgt gggagaaatt gcttaaaatt ttatagttca tagtaaactg  65565
   ttttgtaata aaaattactg attgaaataa ccccaaaaaa aactaaaatt ggctaaaatg  65625
   cgtgtaatta aatttgttat ggacaataaa ttggagataa cttgttggta acattcaaaa  65685
   tatcgaaagt gaactgggaa atgttgatgt tagcagtaat atttgccatt gaagaaaatc  65745
   agtatggagg agctatggtt aggaaaattt ttattataaa atttacccag aaaatattta  65805
   atgtctataa aataatttca atcacatgaa aatggaaaag aaaattctgt ctttaaaggc  65865
   attgaataga aaataggtaa tggaattcaa atttcttaat agagtatgct cccaaaatta 65925
   ttttctatga aaattcatta atgtcagtgt aatttattga cactatttgc gtggagtcac 65985
   aacatgcttg ctgtcagaag ctttgctggt gaaaactgta agatcaaagt gtccttaatc 66045
   ttttggattt ccatctttct aactccctaa ttggggatag gcctgatctt atccctaaat 66105
   ggggataggt tagaaactgg tatgtttgtt cctaactggt gtgtttctat accagtttct 66165
   aacctgattc ctatcagaat gttttaagag ccttgtggct ttgcctggac tcttctatgc 66225
   tacagtttat ttagtttatt tattcagttt attcctcctt aaagtgggaa taatactatc 66285
   tgtattgcca gtttctcagg attattttac ataaaatgat atgatatgcg gaagtctttt 66345
   gtaagccatc acatccatag cagtataaga tattactact aactagaaag agaaaacagg 66405
   ggtctatgcc cagtattaaa attggcattc aggaatctag tgagaatatt ttttcaggtt 66465
   cattgcttgg gcatttctaa tttatactca agaaatgctt tcatattgtt tggaaatttt 66525
   agtacccttt tctctgtaaa cagaatttgt agtctaccta tgtaacaaaa cccacccctg 66585
   tgccttgcat ttcattctcc ttagcattta ttactatctt aacatactag acatgtactt 66645
   gtcttttgtt catctttttt ttttcttttt ttattagacc ataaactttg atggcaggaa 66705
   ctttgcctat tttatttatt attgtattcc cagcacctag aacaatcgct ggcacatagt 66765
   agatgctcag tatttgttga atgaatataa atttttaaat gttataataa tattattctg 66825
   aaatctatgc atacgaagct tttggtacag aaaacatgaa aagagaacta ctgccttatc 66885
   atccagtctt cttccctctt ctcattcagt ctagaacata acctgttttg gaaaaagttc 66945
   tcaaaccata tgtttatctt gccctcaaac cataacaaca atcaatgcaa aagacttctg 67005
   tgacccccag aatatgtggg gatttctcca catcagcaag caagcagttg gttttgtagc 67065
   agacaccaac tgggtgtcgt ccaattcaat tcatcatcta cctggagata gtgtcagatc 67125
   ccacagatat cttacttcga tcaaatcaca agtccaggcc tccgtgactt ccgaagttcc 67185
   cacatcccca gcccccagct ttgggtttga ttaatttcct ggagtggctc acagaactca 67245
   gggaaacatt tacttacatt taccagttta taataaaggt tattacaaag gatacaggtt 67305
   aagagatgtg taagaagaga tatgggggaa ggggtgtgga ccttccatgc ctttctgggg 67365
   tgccaccttc ctctagaaac ctccacatgt tcagttctcc agaacctctc tgaacccagt 67425
   cctcttggtt tttagggaag cttcatgaca tcagtatttc ttctcctagg gtatggggca 67485
   ggaccccctc gtattagggt tttaagaccc acagtcagaa aggcagggga agattacagt 67545
   cctgccttag ggcaggtgaa aggaggatgg gagaaggtca gagagactct tttctgaggt 67605
   gtgctcggaa ggcctaacac actcaatatt ataactaaag atgaggacaa gggctatgag 67665
   agttataagc caggaaccat ggaaaaaagc ctatatgtaa taacaccaca atacccatgg 67725
   taccattcac gtttgttgtt tttctgtttt tcaattgttc tttcagtctt ggttccctta 67785
   atcttaattt agcaagtaat gccaggtggg ataaaattgc ccaaacccaa caaagtactg 67845
   tgtgctgcag gattatttaa tgacatacct tatgtccccc actagtattt acatttctgg 67905
   gagtacagaa aaattcttgt acatatttca gaaaaaatga aattaataac tatcaaccac 67965
   ttagtgaagt ttttactttt ttttttgaga tggagtttta ttcttgtcac ccaggctgga 68025
   gtgcaatggc gcaatctcag ctcactgcaa cctccgcctc ctgggttcaa gtgattctcc 68085
   tgcatcaacc tcccaagtag ctgggattac aggtgcctgg caccacgact ggctaatttt 68145
   tgaattttta gtaaagatgg ggtttcacca tgttggccag gctagtctca aactcctgac 68205
   ctcaggtgat ctgcccgcct tggcccccca aagtgctgga ttacaggtat gagccaccac 68265
   acccagactg aagtttttac attttttaaa gggcacttat tagctgaatt aaataaggta 68325
   aaaaattgac tagtattaga gacaagaatt ggagaatata gttctctagt attcgagaaa 68385
   gtcgttttga taggacaact aatcttagtg agaatttggc tttatttcat atttttttaa 68445
   ttttttgaga tgacgtctta ctatgttgcc ctggctggtc tttgaactct gggctcaaac 68505
   aatcttcctg cctcggcctc ccaaagtgct gagattataa gcatgagcca tctccccagg 68565
   aatttgactt taaaccatgg ttctcaaccc tttcagattc aacattccct ttaataaaaa 68625
   atataatgtt tcataatttc ccctttacta ttataattga aatgcatagt taacataaac 68685
   tctacctact tacataattt caaaaatgtc attatgaatg tcctaaatga aatatatagg 68745
   gggaacataa aaggaatatt catatttcaa catgtaaatg ctttggcatg actccattgg 68805
   aaaatataat gaactagtca tgtgcttgca ccttcattaa tgtgagttca aagctacgat 68865
   tgcagactga cacaaatgtg ttctattggc aactgatggg tcatgatggt attgccattt 68925
   gtaatttgat ttccaaaatg gtaaacaaat tgttggtgca gttctcagca aaacaatgtc 68985
   tataatctta ccttttataa gactgttgta ttcctagaaa acttagtgta tagtaaaacc 69045
   attaaaaaat tacttagtgt gaatatgtta gttggagata aattcttagc tcagaccagt 69105
   gtaagcagaa ttttttactg tattaatatc cagtagaaca tttgaaagtt gttcagtgca 69165
   tgagactatt ctgcattgga taggctttct ttggctcctt tatcatagtt ataataaacc 69225
   atgacaccta cccctgaaat gccctaattc ccttccgttt ctttttcttt tttcttttta 69285
   gcacttaaaa ctagctaact tactacaaaa tagatttaga tttatttctt gttttgttat 69345
   ctgtatcgtt tgctcccttc tccccaatct atctaaccaa ctagtataaa ctagatagta 69405
   agattcatga agatacactt ttttatctga ttttattcat ttgttctatt cctattgcct 69465
   ctagagtagt acttggcaca tggttagcac taaataagta cctgtcaaat gagtgaagta 69525
   atgtgcattg aagacttgaa ggggctctga tgctaggaaa ttgtcatggg ataatagatg 69585
   aggttggtcg tttgtacaga ggattcttgt tagaagctta ctctagtcat gattgtatta 69645
   gaatcttcat ttaaaggctc ctgaagggtg ttggcattag tcagaactgt ctcccagaat  69705
   tttatttgtc ttgtgataga ataaagcata gttagcctaa agagcagttt tcctaatagc  69765
   tcggcatgcc caaagattct aggagttata caggttgaac atctaatcca aaaatctgaa  69825
   atgctccaag atacaaaatt ttttgagcac caatatgatg ccacaagtgg aaaattctga  69885
   tgtgacctca tatgatgagt cacagtcaaa acacagtcaa aactttgttt catgtacaaa  69945
   attattaaaa aatattgtat aatactacct ccaagctatg tgtagaaggt gtatgtgaaa  70005
   cataagtgaa ttttgtgttt ggacttggga cccatcccta agatatctca ttatgtatat  70065
   gcaaatattc caaaaatatt ttttaaaaaa atccaaattc taaaacacgg ctggttccaa  70125
   gcgtttcgta agggatactc aacctgtata gcaaaatgaa catatttaca tattctctag  70185
   gaaatattag tttacaattt ttctaggcaa attataattg ataaatcata aagaaaattt  70245
   aaaataacac tggtaatttt cctacctcct tcgttattgt tacag aat gct  aaa     70299
                                                     Asn Ala  Lys
                                                         1905
   gaa gaa tta att  agg tgg gaa gaa ggt  aaa aag tgg caa gcc  aaa     70344
   Glu Glu Leu Ile  Arg Trp Glu Glu Gly  Lys Lys Trp Gln Ala  Lys
               1910                 1915                 1920
   ata gaa gga att  cga aac aag tta aaa  gag aaa gag ggg gaa  gtc     70389
   Ile Glu Gly Ile  Arg Asn Lys Leu Lys  Glu Lys Glu Gly Glu  Val
               1925                 1930                 1935
   ttt act tta aca  aag cag ttg aat act  ttg aag gat ctt ttt  gcc     70434
   Phe Thr Leu Thr  Lys Gln Leu Asn Thr  Leu Lys Asp Leu Phe  Ala
               1940                 1945                 1950
   aa  gtgagtttaa atatcattat aaaactaatt atgtgtaaaa tcctttagtg         70486
   Lys
   acctggaaat tatatagctt tatcatagtt gataatatga gaaatggtct agtttaaatg  70546
   atcatttatt atctatgatt tacttacttt ttattttctt taaaatctgt tttaaatata  70606
   ttgtaacaat tatagatgga ttttcctgtg atctcgttgt aaattagctt atgacaaata  70666
   tagggtgtta caattattgt aatttggttt ggtaatgagt atgcaattga aaagccaaac  70726
   actgaatggt atatttcatg attctatatt aaattccaca g a gcc gat aaa  gag   70780
                                                   Ala Asp Lys  Glu
                                                           1955
   aaa ctt act ttg  cag agg aaa cta aaa  aca act ggc atg act  gtt     70825
   Lys Leu Thr Leu  Gln Arg Lys Leu Lys  Thr Thr Gly Met Thr  Val
               1960                 1965                 1970
   gat cag gtt ttg  gga ata cga gct ttg  gag tca gaa aaa gaa  ttg     70870
   Asp Gln Val Leu  Gly Ile Arg Ala Leu  Glu Ser Glu Lys Glu  Leu
               1975                 1980                 1985
   gaa gaa tta aaa  aag aga aat ctt gac  tta gaa aat gat ata  ttg     70915
   Glu Glu Leu Lys  Lys Arg Asn Leu Asp  Leu Glu Asn Asp Ile  Leu
                1990                1995                2000
   tat atg ag  gtaagctatt atgtggaaat gtgccaccca ttgtaatgaa         70963
   Tyr Met Arg
   aaactggttg acccctagaa attgaaataa taaatgtgtg ttgtcttaag cttgggttat  71023
   gttttctttt cccatgtgaa ttgagatatt cctggttctt catatgccac ataattttgg  71083
   tgtatttttg atcttttgaa tattatattg tgagactctg gttcttgttt aaattctatg  71143
   ggaaaatgta gatacttttg ttttagcatg caatcggtct aattaggttc aggccacaag  71203
   ttccaacctc atttcttggg ctgtggttcc atttttcaaa gccttttcaa tactcttcag  71263
   atctgtcctg cctgtgtacc tcacaatagg tgatctggta tgtgagctat gtaccattag  71323
   ttcagttctt agaaactttg gtattctgat taggatcgat ccatacattt gcagctcaag  71383
   agtgagccca gaagttcata aacaacttta tagggtccct ttcttgagct cctccctctt  71443
   tgccatctct ctgatacttt gtttccctag ggatttccat ttggggcttt agttacccag  71503
   tgatgccatg tacttcagga attgcacact tctgcagcca agcaagcaag aggagagtag  71563
   aaagaggaag aaaaaaacga cttttacctt accctcttag tatcatagct ctaccaattg  71623
   gagatttccc tcccaaaaaa tattagcttc tgtgagttcc cattgcagcc tctattacca  71683
   ctgctatggg atggcttaag ggttggggca tgaaagaaca gatagaagaa aaaaaaagtg  71743
   aggtgttttc atattgtctc ttgagtgtta aaagattccc tttctcttta ctcgagctag  71803
   aattagaagg tttacctgga gctctctctg tcagtgcaga cacccatctt caggtttcaa  71863
   ataatgttgt cttcagggca ggcagtaaca gaataaaaga aaaggtaaat tcatcacctg  71923
   tttgctgcta ctttaagtcc tggtattcta ttgtaatctg ccttctactc ctttgcaaag  71983
   tcctcaaatg gttgctccat gcatttagga gagagaagat tgaatgtatt tactccattg  72043
   tacctggaac cagatgccct tgccctgcat caccccatgt catttcttag cagagccttt  72103
   gagatttttg tgtgtgtgtg ctttacaatc tctttccaag ttatatcttc tgatacagtc  72163
   atggtcgtga aaagcaaaat aaaatcatgt gttaacattt aaaacttttt aattttattc  72223
   tgacaacagc taaaactatt taatcttctg tttcgctcat ttcttccaag gtaaacttca  72283
   gttggtttta cgtgatttgc tatttcttct tctttgcatt tacaaatgat ctgtgatcat  72343
   attactgatc tttgtaaagg gctaatatct acctgcaaca tttggatatg acagtattta  72403
   ccctttgtaa atacacattt tctatttatc ttcaaaaatt accattcatt agtctgtgtt  72463
   aatgtctgtt tactattgtg tcattatgaa tgtgatgtga acatacgaag ttgaacttat  72523
   ttaaacgaac actctcatga gcttctaatc cacattcctt ccttttcctt ctaagttacc  72583
   atttcttaaa aatcttttag aagtttcctt gatagggaaa acacaaatta ttgaggaatt  72643
   tttctttctc ttgacatctg tttatagtta ctctcttgtt ccagcagtgg atatttcccc  72703
   tccatgtttt tctttgtcta aacatatgtt caaaacaaaa cacttttatt cttctttgca  72763
   ggttttacaa ggatcaactt ttagttttga aacctgctat tacttttaga ggccattttt  72823
   tttttctcta ataatgtgag ttcatgcggg ctgaagtaat tggaatactt tatagaaaag  72883
   attgaatttg tcttctctct gaactctagt ttgaatttct aaattttatg aatcatctag  72943
   atattaaaga ggaggggcat atcaaagagg agaaccctag cagagataag aggcaagagt  73003
   aaatgtttca tgtatgggta agagtggatt tgtatttacc taagtaaagg tagaccctgg  73063
   acaataaggt tggatagatg tggaggtggc aaaccatgga gggtcttgta ggtcaagtgg  73123
   atgtttttag acttgaagtg ttaaattatt atctgaaatc attaagagtc tttttagatc  73183
   cttgagcttc ttgagaagac catggatatt atgcagttat tatataatgt tttaaaatag  73243
   taagtatttt agtttaactg tcttatgtaa ttccatataa atggatgcat gttctttaaa  73303
   aatgttaatg tatttcagta aatcaaaata tactttttga ctcatcattt aaaggaggcc  73363
   ttcagtgaat gctctgtaga ggattatttt ataatactaa ttttgatatc ctaatttatt  73423
   tgttataaag tttagaaggt ttgaagaatt taaaatatag tgttaataaa cacactgaac  73483
   ttttcttttt ttatcttgta tttttatata gtacaacaga aaaaagatga aatgtgaata  73543
   gtaaagagtc tgtgattgtt gttcatag g gcc  cac caa gct ctt cct  cga     73593
                                    Ala  His Gln Ala Leu Pro  Arg
                                    2005                2010
   gat tct gtt gta  gaa gat tta cat tta  caa aat aga tac ctc  caa     73638
   Asp Ser Val Val  Glu Asp Leu His Leu  Gln Asn Arg Tyr Leu  Gln
               2015                 2020                2025
   gaa aaa ctt cat  gct tta gaa aaa cag  ttt tca aag gat aca  tat     73683
   Glu Lys Leu His  Ala Leu Glu Lys Gln  Phe Ser Lys Asp Thr  Tyr
               2030                 2035                2040
   tct aag cct tca  gtaagtgtat atcttttatt atttttttct tttttccatg       73735
   Ser Lys Pro Ser
               2045
   ttaaaatgca tgaaagtgaa atcaacttct ttcttaatct ggccaaaagc attacatctt  73795
   tctcattaat agtaatacag taaattcaac ttttattttt aacaggtagt gatgtgtaat  73855
   aatttattta atccttttta acataataac agtaaactta agattcttaa gcttttcata  73915
   aagctcataa atgatttcta gaaattttaa atatgtagtt atcattatgt attttgctgt  73975
   agcagcagta tacagttaaa taaaatagga aaacatgttc caagactgtt ttcattcaaa  74035
   tatttatgct atatttttag cttataaaaa ctcattaatc attaatgtaa aattatttgt  74095
   tggatttttt aaatatttag tgtattattt ttgtttcttt tttctttcca tgtttcttca  74155
   ttcttccacc ttaagcagaa tcaggtgtgt gacacaacta tgttttctat ccttgttacc  74215
   attattaata aatacaaggg catgatattt ttcacaaaag aaacactttg ttcagaacca  74275
   aaaaagatca tggcaacagt cagaattaaa aatggtaaaa gactaggtgc caaagatgac 74335
   ttacataatt gggtacctag aaatattcta tggtattaca gtaatgatga aaaatacaaa 74395
   ttagaacaca ttttagatcc tattgagtta aataaatcag agtcaagacc aaacaataaa 74455
   taaagtcaat ttacgtcaac aaatggtaag ttggcagatt ttaactccct ttttgaaaat 74515
   gaaccatgat cctaaggttg gtaaaattaa tcaagaatgt tgtcaaaatg ataaagataa 74575
   aaatgaggaa gagaataaga taggcaagag tgagaaagga aagagacaca tagctgaaaa 74635
   tgtgagtcac aacaactaca tagatccgta gaatctgcta tggaggactg tgattatgtg 74695
   acagttgctg atgccgtggc ttagtgagct gagggtgatg cacaggcagg cgatgtaact 74755
   gatgcgtcag tccagccaag aaaggacgcg tccctggttt ggctacgtgg ccgtccttta 74815
   tttctttgtt aactgaattt tcttatagta agtagcttac gtacatatat agtgcaaatg 74875
   ggaaagtgtg taagatttag aaaaagcatt aactattagt aaactttatc ttaagctcta 74935
   acttttgatt agttcctaca aaaattagtg aatatgcatt ttctaattta gtgctttttt 74995
   tttttttaca attggtgttc acttaatgtt atattagata aatgaatagc aaaaataagg 75055
   tactttagag ttgattgttt tgccttacaa acttctaatc catccagctg tatttagaag 75115
   taagatctca ctacagcgaa ttatatcagt aaaattttgt tacagtgttg tgcagtgtcc 75175
   taagatgtat actaagttcc ttcagtggct ttttttgcca tgttttataa cagataattt 75235
   tgttataatg agaaaaggaa acttggatgt gttgctgtct atattgtgtt aggctcaggc 75295
   aggatgctgt ggcttactca tttaatcact ttgggaggca ggggcaggaa gattgcttga 75355
   ggccaagagt ttgagatcag cttgggcagc atagccagac cctgtctcta caaaaaattt 75415
   agacagatgt ggtggaacac atttgtagtc ctagctatta gggaggctgt ggtgggagga 75475
   tcatttgagc ccaggagttt gatgttacat tgccctattg cactccagac tgggcaacag 75535
   agtgagacct gtctctaaaa taataataat gataatgata aatggtgtta ggctctgtgc 75595
   ctaagtatat ttttcacata ggctgggtaa agtggctcat gcctgcaatc ccagcacttt 75655
   gggaggccaa ggcagcagga gcatttgagg ccaggagtca aagaccagcc ttgagagacc 75715
   ccatctctac cagaaaaaaa aaaaaaaaga aacaattagc tgggtgtgat tgtgcacacc 75775
   tgtagtccta gctactcggg aggcagaggt gggcagatca cttgagccca ggagtttgag 75835
   gttatagtga gctaagattg tgccactgca ctccagactg ggcaacagag caagactgtc 75895
   tcaaacaaaa acaaacaaac aaaaagcact ttgcagaata tcagtctaac tctacagttt 75955
   atggactttt tatgtacgta ctacttttgg ctagcttaca ttgagataca gaataaaagt 76015
   ttgttcatag catttatcgt ttttttcttt atactgtcca cctgagatat tccagtcacc 76075
   taagtcatgg aaacatcaac taaaattaaa tatctatgtt aagagaaaat ggctgaaagt 76135
   gatttaattc ataacacttt ttttcacatg ctaataaata agagtttgag acttccacta 76195
   ggcattatct ctaactccta tccactaaga atttgatttt aagtagttga tggcttttaa 76255
   ccggattatt cttctgtaag agtttggaag tctcgtgaag ttcgttatac aagaattctg 76315
   tttacaagag agcattacat tagaatttgt ttttcagaaa tttggactat ctcaacgaat 76375
   acctttagtt ttattatttc aaaatgcaag ggaaaaaatg agccataatc actaatagta 76435
   actgcatcat attttagtga gaaatgtgtt aaaaatatcc tcatgtgaga tcttccttag 76495
   atagaattac cctctactct aatatttaat atattttata tctaccaatc agtgatatta 76555
   ataggtgttt atcatttgct gaatcaaata ggtacaacag aagacaggaa gtttgggaga 76615
   tagaagagct cagggacagg aaatcacaga tgtccatatc tgaaataacc ttaaaagtta 76675
   tcctgtctaa tgccttcact tataaactgt agtggtagaa tttgcctagt attaacctaa 76735
   tagtggtaga tttgaatgta tacttgggct ttcttattaa gtggaaatgt attcctgtga 76795
   tttacatata tcaacaaaaa tgtttgtctt cttttttttg ctacgacata tgtgcatgtg 76855
   cacacacatc tcctcaaaca aaaatcagat ggacacatgc agtcattgga tctaaaagat 76915
   gttataaagt tgtgtataat aggtatttta taataatata ttttaagacc cataatgtcg 76975
   gtggagtaac tgactttaca gcccatcaag ccaatagaga gagaaaggag aaaaaaatga 77035
   aagttgtgct gaataattaa aaaaaattat ttcctatgat gcttataaca gtcctatgag 77095
   gtaggtggta ttctaattta tagaaaaaat gcatagaaaa atataattaa gcacagttaa 77155
   aaaaaataaa gtttagaatg agaagtaaca acataaataa tgacccaatg tagattcagg 77215
   tcaaaagaaa tgaaaatata atattaatgg ttttcaaaga gggaaccatt actttagctc 77275
   aaagaatgaa ggagggcttt ccgaaggagt aaagaattat ggcagttctt ttgtagccta 77335
   gtgtattcat ttgctaaggt ggctgtaaca gactactaca gatttggtgg cttaaacaat 77395
   agaaatttat ggtcttagtt ctggagacct agaagtccaa aatcaagaca tcagcagggt 77455
   tgatttcctc tgcacaatca gagggaaaga tctttcccaa tcctctctcc ttggcttata 77515
   aatgtccatg ttttccctgt ttctttttat catcttcctt ctgtacatgt ctctgtgtct 77575
   aaatccccaa attttctctt ttcataagga taccagtcac agtcgaatag ggtttaccct 77635
   gaaatctcat tttaacttga atacctctgt aaagacccag tctccaaata aagtcacatt 77695
   ctgaggtact ggaaattatg actttaatat ataaatgtgg agggtaaggg gaacacagtt 77755
   caacccataa cggttagata acaatcgtgc tttattttgg actagtaaaa ccaccataga 77815
   tcagtttaac cattatgaaa ttatacatga aggcattata tgtatggaca ttattaagtc 77875
   atacttgctt tgcttccatt gtaattaaaa caaaccatac tacctttgtt ctgcaagttt 77935
   tgtattctaa cttatttatt tttggctttc accagaacac tccgattttc tcatattcct 77995
   ttgaggaaaa aaagttacct tttgacagta ttttcttatc cagtatgtct tttatggctt 78055
   ttatttatta aactttaaaa atattcctaa tttcatttcc ctgaag att tca gga     78110
                                                      Ile Ser Gly
   ata gag  tca gat gat cat tgt  cag aga gaa cag gag  ctt cag aag     78155
   Ile Glu  Ser Asp Asp His Cys  Gln Arg Glu Gln Glu  Leu Gln Lys
       2050                 2055                 2060
   gaa aac  ttg aag ttg tca tct  gaa aat att gaa ctg  aaa ttt cag     78200
   Glu Asn  Leu Lys Leu Ser Ser  Glu Asn Ile Glu Leu  Lys Phe Gln
       2065                 2070                 2075
   ctt gaa  caa gca aat aaa gat  ttg cca aga tta aag  gtgaatttaa      78246
   Leu Glu  Gln Ala Asn Lys Asp  Leu Pro Arg Leu Lys
       2080                 2085                 2090
   tgttttttat taggaaatct aatgcctaaa actccttcct tagttgttat gtttactttt  78306
   attagcttat taagaagtca aaaatgcata ttcctaatat atcatggtga tggtatactt  78366
   tatacatttg ctctttagca tttatttgtt gaaggcctac tttatattaa acactcctcc  78426
   agatgctggg aaacagcagt caaaaaattc cttatactca taggacttac gttctagtgg  78486
   agaagactga caataaacaa gtcactaaat agtatgtcat ctgatgttag tgctaaggag  78546
   agaaataaag catgattggt gtaaagagta tggggagaga gaaggggtgt aactgaaaat  78606
   agagtagtaa gggaggtctt ccttaataag atgatatatg aacagagagc taaggagggg  78666
   taaaggaagt gagtcataca gatactagaa aaataattac agacaacaga aatagcaagt  78726
   tcagatgtcc taaggtggga ggatgcgtgg tatatttcat taaaaattat cacactgtaa  78786
   aatataagaa taatttgttt cttttagaaa ttttacttta ttctgatatt aataatgatt  78846
   ttttaatctt tggttttcca agtcttaccc tatttatggg aatctttttt ttcttttggc  78906
   tagctaattg cttcagtttt gttttctaat ctagaatgtt agcaatctgt taattccact  78966
   ggtaatgata tagttaagct atgtcttgct tctcacactt tatttattta tttactcagg  79026
   gcactaatct gccatttttt cgcacttttt ttcctttttt ttttttttgg tactgcttct  79086
   tattctggtt tttacattga tagaaccaat gttagacgtt catttgcctt ttgctgtgta  79146
   tatttgggta aggatctata tgtgcaatat atgggacagt taaaatcaga attctaaatt  79206
   tgtattattg catcaggcaa taatgtggga aataccttga catttcatat acacaatatt  79266
   cttgtattaa tttaacgtct tagttcaaaa tcttccttgt taatatagag accctattat  79326
   ttggtttggc aatacagttg aagagattga tggttcttat gaattgtttg ccttttcttt  79386
   tcaatggctg tagctatgtt aaattattac atgtttgctt gttatctttc ag aat caa  79444
                                                             Asn Gln
   gtc aga gat  ttg aag gaa atg tgt  gaa ttt ctt aag aaa  gaa aaa     79489
   Val Arg Asp  Leu Lys Glu Met Cys  Glu Phe Leu Lys Lys  Glu Lys
           2095                 2100                 2105
   gca gaa gtt  cag cgg aaa ctt ggc  cat gtt aga ggg gtatgtgaga       79535
   Ala Glu Val  Gln Arg Lys Leu Gly  His Val Arg Gly
           2110                 2115
   atttaccata catttgtttt ggtttcagca gtgataagcc agaaatgaaa agtttagata  79595
   tgttgtaaaa gtactgatat gcctctacaa gtgccctgta gtttcagtgt ttattctgca  79655
   tctgtaatat aaaacagtaa gcatttctat gtgtctcaaa gtattttatc atctgttata  79715
   ccttacatac tttcatctct ctttttattg aatatgcctc cataccttga aaacatttaa  79775
   cttccaggaa tccttttgtt tatggaggta actgctaact ggtccttggt ccaatgctgc  79835
   cattttgtaa ccatttgtta tgatatcttc ccagcttggt ataatgtttt ataattacat  79895
   tgttcctccc cctctttttt tgtgttcttg taattttctc cctatgttat tttgtattca  79955
   ttttatataa tgaataaatg ttgcttatga ggtcaaggcc aaagacttaa gctcctgttg  80015
   atttcatgtt gctgagtgtc ataaatggaa gcaatcataa tgcagagtca ttctggtagt  80075
   aatattaaat atatgatgga ttcagtgaaa atattatgtg ttattagaaa aatattcaga  80135
   acaggccggg ggcagtggct cacacctgta atcccagcaa tttgggaggc cgaggcgggc  80195
   agatcactgg aagtcaggag ttcaagacca gcctggccga catggtgaaa ccccatctct  80255
   actaaaaata tgaaaattag ctgggcatgg tggctcatgc ctgtaatcct agctactcag  80315
   gaggttgagg caggagaatt gcttgaacct ggcaggcgga ggttacagtg agccatggtc  80375
   acacaactgt actccagcct gggcgacaga gcgagactcc atcttttaaa acaaaaaaaa  80435
   aaaaggaaaa atattcagaa cagtatcttg ctggcagcaa catttgtttc atcaatgaaa  80495
   atatgtgtta atttgacctt ttctatctaa gttaattatg aaagtgcata ctaaaatgat  80555
   gtaaaagttt atatttcagg attattctta ttcatggatg attaactaaa atgcaaaaag  80615
   aaattaagca tactgtttgg ctaaactgtt aaaaattatt tttattttaa atgataagca  80675
   gttaaactta ttaagtgatg actcatctct gctgatatat ttatgcaagg ttttttattt  80735
   cagataactc ttctatttat attaaacaga aactgtattt ctaagcaata gcatttctta  80795
   gagaaaattg cctctattat gttgcaatta aaatttaatt actcatgagc tctttaaaga  80855
   cacaatttct cttgtgtggt tttatttcat ataaagaaaa actctgatat actggagaga  80915
   acattagcta aatagactat ttagacttaa tcattttgat cagacatcaa ggctagacta  80975
   tttaagctgt tacttattag ctgcatgatt ttaggaatgt caaatttcct aagtcttggt  81035
   tttcttgtat ttaaaatgga aattataatt cctatctcat agaattgttt taaggatgaa  81095
   ttgaattaat acagttttga cttcaaatat taggaattat tgagtataat aagcctgttg  81155
   tattgttggt acttcgtatt atacttacta aaatatttga ttaaagattt aacatattct  81215
   ttcgtag tct  ggt aga agt gga aag  aca atc cca gaa ctg  gaa aaa     81261
           Ser  Gly Arg Ser Gly Lys  Thr Ile Pro Glu Leu  Glu Lys
           2120                 2125                 2130
   acc att ggt  tta atg aaa aaa gta  gtt gaa aaa gtc cag  aga gaa     81306
   Thr Ile Gly  Leu Met Lys Lys Val  Val Glu Lys Val Gln  Arg Glu
           2135                 2140                 2145
   aat gaa cag  ttg aaa aaa gca tca  gga ata ttg act agt  gaa aaa     81351
   Asn Glu Gln  Leu Lys Lys Ala Ser  Gly Ile Leu Thr Ser  Glu Lys
           2150                 2155                 2160
   atg gct aat  att gag cag gaa aat  gaa aaa ttg aag gtaatttttt       81397
   Met Ala Asn  Ile Glu Gln Glu Asn  Glu Lys Leu Lys
           2165                 2170
   ttaatgtgat catttttagg ggaatatttt acgttttgtt actatttagg aaaatttcaa  81457
   atatgctcat tactatataa aatggcttta atgaatacaa tacatatttt ataaatatag  81517
   aaaaaaactt atgagaggca aggctaaggg ttatagagta ggtctacctg atctttcttg  81577
   ttatttcaag accaatactt ttcacttttc tctctgacag catagattaa ttacctgtgt  81637
   ctctcttttt tttttctttt gagatggagt actgctttgt cacccaggct ggaatgcagt  81697
   ggtgcaatct tgactcactg caagctctgc ctcccgggtt catgccattc tcctgcctca  81757
   gcctccccca gtagctggga ctacaggtgc ccaccaccac gcctggctaa cttttcgtat  81817
   ttttagtaga gatggggttt caccatgtta accaggactg tctcgatctc ctgacctcgt  81877
   gatccgccca ctgcggcctc tgtgtctctt tgtgaaaata cagatgccca agctcccatc  81937
   cctgaaattg atttaattat tttagggtgg gtcctgacac agatatgtat gttgttgtta  81997
   ttttaagtca tcaatttatt ctaatatgta gccaacgttg ggaacttcgt tctcactaat  82057
   attcaaatga agactttaat tctaatcata tcaaatatgg tttctaaaac tactttgaag  82117
   atttatgagt ttataagatt atcttttatt tccttgtttt gataatgtat actttttatt  82177
   ttgtttgttt ttttactag gct  gaa tta gaa aaa ctt  aaa gct cat ctt     82226
                        Ala  Glu Leu Glu Lys Leu  Lys Ala His Leu
                        2175                 2180
   ggg  cat cag ttg agc atg  cac tat gaa tcc aag  acc aaa ggc aca     82271
   Gly  His Gln Leu Ser Met  His Tyr Glu Ser Lys  Thr Lys Gly Thr
   2185                 2190                 2195
   gaa  aaa att att gct gaa  aat gaa agg ctt cgt  aaa gaa ctt aaa     82316
   Glu  Lys Ile Ile Ala Glu  Asn Glu Arg Leu Arg  Lys Glu Leu Lys
   2200                 2205                 2210
   aaa  gtatgacttt tatgactgat tataactttt gatttttatt ttacttaata        82369
   Lys
   2215
   cctcttggaa aaactggaag tagatccttg atgagagtgt ctgtaaaggt agatattaag  82429
   agattgagga attgtgtttc tatgcctgct gtcatcacat tccaccatga aaaacattga  82489
   taataaaagt taatacattt aggctgggca cggtggctca cgcctgtaat cccagcactt  82549
   tgggaggcca aggcgggtgg atcacgaggt caggagatcg agaccatcct ggctaacacg  82609
   gtgaaacccc gtctctacta aaaatacaaa aaattagccg ggcgtggtgg cgggcgcctg  82669
   tagtcccagc tactcgggaa gctgaggcag gagaatcgct tgaacccggg aggcagaggt  82729
   tgcagtgagc cgagatcgca ccactacact ccagcctggg caacagagcg agactccatc  82789
   tcaaacaaac aaaaaaaaga aatgatctac gttgcttaca cataccttat gcttatagct  82849
   aggtctcgta agcattagga agtcaaaaca aagaatcttt tacatgtgta aaggtataaa  82909
   ctatcccatt tttctaaaaa tatagaggaa caaagtgtca aatttaaagt aatcactagt  82969
   aactaaatat attcctctga cctcattttc gtgatctgtt gttctaatta ttattggcca  83029
   tattgctgct ttaaaggaga gatgttgaat ttgttgaaat tttaatcagc atttagagcc  83089
   ccaggttatt tttgttttcc aatttgtaat gataattttg aatacactga atctatgaga  83149
   acagtattat gttttctcat aaaatactaa ttagcattta atgatag gaa act gat   83205
                                                       Glu Thr Asp
   gct gca  gag aaa tta cgg ata  gca aag aat aat tta  gag ata tta     83250
   Ala Ala  Glu Lys Leu Arg Ile  Ala Lys Asn Asn Leu  Glu Ile Leu
       2220                 2225                 2230
   aat gag  aag atg aca gtt caa  cta gaa gag act ggt  aag aga ttg     83295
   Asn Glu  Lys Met Thr Val Gln  Leu Glu Glu Thr Gly  Lys Arg Leu
       2235                 2240                 2245
   cag ttt  gca gaa agc aga ggt  cca cag ctt gaa ggt  gct gac agt     83340
   Gln Phe  Ala Glu Ser Arg Gly  Pro Gln Leu Glu Gly  Ala Asp Ser
       2250                 2255                 2260
   aag agc  tgg aaa tcc att gtg  gtt aca ag  gtaggaacag agttttaaac    83389
   Lys Ser  Trp Lys Ser Ile Val  Val Thr Arg
       2265                 2270
   ttgtacaaag tttaatcatt tcaaattttg gcattgtttt aaaagacaac actattctgg  83449
   ataacctggt ttcttcctga tgaacagttt gtttggttgt tgttttaaca taatactttt  83509
   tttctgttgt agtattgttg gagacttttt cttccttgaa atgtttaact tgtttaacct  83569
   tgtttgggtg gcagggcatg gaacagtgta gagctggggc tgggcgaagg agttggagct  83629
   gtgtgtgcgt catgaagctg tcatcagcta tgagcctggg ctgaggctgc tcagcttctc  83689
   ctgggtgcta tttttctcca actgcagctt cagcttcttg attgtataat ttgcttcctc  83749
   aagtatgagc caggaataat tgagctgtct tgtcacaatg tgtggcatac tggatctagg  83809
   ctgtgctgca atgcttttag agttatatcc tgggcaactt tctcttcaga tagccccaag  83869
   agatgaattc agcaccagct ttgatgtttt actagcttct gctttctggt acttgatttt  83929
   ctcccacccc gaacacatgg gattccaacc tgtgaaacta atttttgtgg ctatgaaaga  83989
   ggtagtggta gtttatgagt aaacattcag tctgttgcca ctatcatcat gtgtggttca  84049
   tcatgactgt gatgagtagg taaaaggctc tttgtgtcat tctcatttcc aattttaagc  84109
   agctgcttca aggagtctgg aagtcattga ccagtgggat cctgcctgtg tcttttccca  84169
   ttaaagccat cctgtatgaa gtggtatcct ttaccatcta gcacatctgc cgcccccatt 84229
   tcaaaaggca tactcatctt tatctcaaca ttctcataca gttccttatg tccatgcacc 84289
   tccaatgtcc cctttgatgt ctttgaggtt ttcatcttcc atgtctgcta tttggaatgg 84349
   tcttgatggg aggcaagata gtgatcacta caactaggat gggagtctta gtaccgtgag 84409
   gctacagcaa gtcccacaga gggcctgctg cactgtactt gcctctgtca accaagtcta 84469
   aggagaaaga ttaagcaggc atattaaagg acagcccaga tggacatgaa gtcctggagg 84529
   aggccttggt tcctgtccta atactaaacc tagagtaccc agaatccaca cttctccact 84589
   ctagctctca cttttcccat ctacacactg ggaaaaatta ttctgtcaga aagccagtgt 84649
   caaggtgaga acaaataaca aatgtgatga tatggagtgg gagaaggggt ctcttctact 84709
   gtcttattgg accctagcag tggctctgag ccagcagtcc tgtcagttga tttcttggtc 84769
   gttcctttgt tttcttctat aatcacatgt ggactcagaa tgaattttga gttactctga 84829
   aatctattta ttcaacagat atttacttag tacctcctat tgccagactc tgctttatgt 84889
   tggatattat tttttaaaag cccaccttgc ctagatttcc tcaaaggacc aggtggcttc 84949
   cctggttttg aaagacccta attcttacta tgatcttaag taaattatat cctttctgtg 85009
   ggctcaagtt ctttctaaga gggctctttg gggctacaaa agaaattgtt agtgcaaaaa 85069
   gagtttataa ggtttataaa tggttagtag aggtgatgat gatatttaac cataattgaa 85129
   gatgactttg cattttagat catatacgtg tttttcgtct gagaacgata caggtcactg 85189
   agcataccat aagccttcag taaatcattt gcagaagaca ttgcagaaga cataagtcta 85249
   agtagaaatc tcttgacaga gagaaggctc gttttgatcc ttgacctcaa atttaggttc 85309
   cctaaatcca ttaaaaaaga gaaagaaaaa gaaaaaaagt tactaaagtt taaatctggg 85369
   aggattatat acccttctca ataaagcagt ttagagagat ctcttttggg acccatgaca 85429
   caggtcttgc tcatgctgac atctttatag ttgctttatt atttattcaa caaacttagt 85489
   aacacgtatt ctatgtcagg ccttttcctg actactggga caaaccaggg tgatgtgggg 85549
   gctgttttag atagggtgat cagaggaggc ctctctgttt gggtggcttt tgaatagaaa 85609
   attagatgaa gtgaaggagt aagcttctga tatttcactg tttacttgtg gtagatctgt 85669
   gataatctct gtcaggttaa aaacattccc ttctaatcta agtttctaag atctatcaaa 85729
   agctgtttga atatatttag acaatcataa ttttcctttc ttgtattatc ctagcagatt 85789
   ttgttgccaa agctatactg gccattttaa cttagaatgc agtctttcta ttcatttctc 85849
   tggaaaagtt tggatattgt aagcattatt tttctttagg tatgatgaac ctgcagaact 85909
   gtttggttca attatgaatt ttttttttct ggagtctgta tttttttgaa ctattaatca 85969
   tttctttaat gattataaat ctattcagat ttttacaagc tttatccctc tcccatcata 86029
   cactattttt cttacccatg cttttgcaca attttttcct ctcccttagt gttttcctac 86089
   ctagatacct cctatgtgtg tctgtgtatg tgagaaaagc tttttatttg ccatctttat  86149
   atttctaaga atatctagta atacagaatt ttatattctg aagaatttta ctttgcattt  86209
   tcttattttg tgattgaaaa aaggtattaa ttttaaaatg gtcaaatcag gctccatcct  86269
   tggaaaatac ccaaatcctt tattttgatt gggccatctg ttaattaggg ataccttatc  86329
   tcttgccacc actttttaat gctaaataaa tatgtagcta aaactttgac tagaagaaac  86389
   agtaaaataa gatattcttg cttattttta gtacagttat ttgaactgac ttttaaatca  86449
   gtgacataaa ttatttgcca tgtctatact ttttttcctt atacttttag a atg tat   86506
                                                            Met Tyr
                                                                2275
   gaa acc aag tta aaa  gaa ttg gaa act gat  att gcc aaa aaa aat      86551
   Glu Thr Lys Leu Lys  Glu Leu Glu Thr Asp  Ile Ala Lys Lys Asn
                   2280                 2285                 2290
   caa agc att act gac  ctt aaa cag ctt gta  aaa gaa gca aca gag      86596
   Gln Ser Ile Thr Asp  Leu Lys Gln Leu Val  Lys Glu Ala Thr Glu
                   2295                 2300                 2305
   aga gaa caa aaa gtt  aac aaa tac aat gaa  gac ctt gaa caa cag      86641
   Arg Glu Gln Lys Val  Asn Lys Tyr Asn Glu  Asp Leu Glu Gln Gln
                   2310                 2315                 2320
   gtaagtaacg taatttttct ttacatgata aaataatgca taatatcgca agatgttcct  86701
   tgcattgtct tatatagata aaaatggact ctattaagaa gacccatcta actgaagggc  86761
   accccattca cccatttgct taagccagaa actttggatc atcaacgact tcattctttt  86821
   cattctccac attttctatc attaaatcat gtcagctcta ttttcaaact atatcctaaa  86881
   tatgaccact tcttggtatc ttgagacatc actaccagtc ttgtccaagc tattgtttta  86941
   tacctgaata actgcaataa tttccaagct ggtatctcag cttccactct tggattattt  87001
   caccctattt ctatttctgg gctgtctcca cacagttgcc aggtaaccct tttaaaacat  87061
   aaagcacatc acaaagcaca aagtcctatc ctcagaatct tccagtggtt ctccatcacc  87121
   ctaaaataaa acttaaaagt tcttttcata tcccaaaaca acatatgagg tctggcaccc  87181
   agttttcttc ccaatctcat cttctactac ttttcccttc atttcattca caatgtttta  87241
   accacagtaa ccttctttca gtactttaaa caatccaaac tcgtttaagc gtcaagtcct  87301
   tatacttgtt tcctttgttt agaatactgt tcacccaaat attctcatag cttgctccca  87361
   gacttcatgt ctctgctgaa atagaggctc cttagagaga ccttccctaa ccctaaccct  87421
   aaccctatac tacttgccat cactctttat cctcttaccc tggattattt tttcttgata  87481
   gctcttccta ccatctggca ctatattaca tcatatcata ttaaacacac attctttgtg  87541
   cttccccact aaacaaggac catgcaagat ggaacattgc cattttgttc actgctgtta  87601
   gcctctgtgc ctaggacaat gccagttatg cagtagttac tcaatacttg ttgaatgaat  87661
   ggtgaataga acatagaaat ttgcctatgc gtgcttttga aaaccatatt ttaatattac  87721
   gctttgttaa aaatgtgtat ctttataaat cctcatattt ccatggcaaa ccttatcttc  87781
   taacttttca ttgtcctcaa ag att aag att ctt aaa  cat gtt cct gaa      87830
                            Ile Lys Ile Leu Lys  His Val Pro Glu
                                            2325
   ggt  gct gag aca gag caa  ggc ctt aaa cgg gag  ctt caa gtt ctt     87875
   Gly  Ala Glu Thr Glu Gln  Gly Leu Lys Arg Glu  Leu Gln Val Leu
   2330                 2335                 2340
   ag  gtacatcatg tattcatatg actactttgt ttttttcttt aaaaaaaaaa         87927
   Arg
   ttattagttt ttatatactc cgaattgcta caactagaga caagcatttt tcgactttac  87987
   tgcctaacag gcttattagg tccttatttc ttccctctaa tgctaatcac tctttttcat  88047
   aatacacact agaaaaaaag gataaaccca actctaagtt tccagtttgt aatttagttt  88107
   aaacttttct aagagcatag aatgagttaa accttagctt cccagaggaa aatactaatg  88167
   aaagagaaca agtaattttt ttactttcag gggtctctgt agcctgcttt cattaagctc  88227
   ctcttataac gaaaccacac ttgcaaatgc catcaggtca gatattaaga aaaacgtgaa  88287
   ggcttttgta ttccaggctt tttgtttgag aatggtgaca ttgtagcatt gagagtaaat  88347
   gtttacttcg ataaaggcta gcttgttctg attactgtac atcactagtt cataagaaat  88407
   gcccatatat tttatgaagc aatatctgct ttattttttt aacacattat cattgtgttc  88467
   tag a tta gct aat cat cag  ctg gat aaa gag aaa  gca gaa tta atc    88513
         Leu Ala Asn His Gln  Leu Asp Lys Glu Lys  Ala Glu Leu Ile
                         2350                 2355
   cat  cag ata gaa gct aac  aag gac caa agt gga  gct gaa agc acc     88558
   His  Gln Ile Glu Ala Asn  Lys Asp Gln Ser Gly  Ala Glu Ser Thr
   2360                 2365                 2370
   ata  cct g gtaatgtatt ttaaaaaaca tgttagctac ccccaagttt ttgaatttgg  88615
   Ile  Pro
   2375
   gtttgccttt tttttttttt tttggctcag atttctgatc attgtctccc tgtaaaatcg  88675
   aattcctgat aagctttggg tcttttgtct ctctgtgcta ttaatataaa aatattccca  88735
   tttttctctt tgtgttgttt atactataga gtagcaagta cccaagtgtt cttctctttg  88795
   ttctccatct gggtgttaca gatttaatca caatacagtg ctaagcaatg aatactaaat  88855
   ctgttgcttc cagtttctaa gtataggctc tttcaagtcc tctgaacatt tttaaaaact  88915
   gcaaataagt aaatactgcc tatatttttt tccgtttaca aagtaaaaag aaaatctttc  88975
   tgctcccttc cattcccatt caaaagtgat tactaatcat tcctcattcc tgcatataca  89035
   tacacacata ttttgtatac atatatatca cacatatgca tacatgtgtt tgtatgttca  89095
   tatgtacaat gtacatatcc tcattatttg tggattctgt attttctaaa tcacctcctc  89155
   actaaagtgt gtatgtaatc ccaaatcaac actcgcagca catttgcaaa catccacaga 89215
   gccttggaaa gtttgaataa tccaacctac atgtccccag cagaagtcca acaaggcagt 89275
   gctcagtatc ctcatttcag ttttcataga gaaatgagca gaggatggag acagtagagg 89335
   gcagcacagc atagtgcaag aagctgtggc tctggggcct ggtggaaggg atttgaatcc 89395
   caattctgag gcttgttact gctctagcct taggagagtc atgtaacact tctgaatctt 89455
   gttttcttat gtaaataaat agaatttacc aggatgagtt atctttagga tttaagatta 89515
   tcatctgtgt gagatatgta ggtgtatgta tatatatgcg tgtatgtata tatatgcgtg 89575
   tatgtatata tatgcatgtc tgtacatatt tcccgtagca gcagtggttt gatattcact 89635
   aattgggcta actttataga ccaaaactac tatggataga gaatactttg tttgcattta 89695
   cgtatatata ttttcttggc aagtaacata aaattgaact aatactatac acatttctag 89755
   catatttgcc tttaacagtt tatcatggac atcttttgag gtctgttcat aaattatctc 89815
   atccatttaa taattccata gtgtattatt gcatgtataa gcacatcgaa ccatttatgt 89875
   tttgatggat atttagtttg cttccaagtt tctgcttcta taaaatatga ttaatctatt 89935
   gacctaatta tgccattgtg ataggatgat agagatgcca ttctctccaa aggattatac 89995
   caatttatat ctgaactatc tttgactatc tcttgtagct ttttcagtat gctatgtagt 90055
   cctattacta atttgtaata aaagccatca tgtgtgagtt gtactagaca ctatgctaat 90115
   tgccttacaa gcattctata tttacaacca tatatgatag gtattactgt ctccatttta 90175
   tgtgataaac aaattcaaag tggttaagta accattccct aagccagcta ggaaatagag 90235
   gcaggattaa aatctaaatg tatgaaactc cacagctcct tggcattcct agtccttaac 90295
   ccgctatgct atgctacgtc ttggtaacta aaagtacata ttaaatactc tcaaaatatg 90355
   tctcatagca gccagcttgg tatgtacact agacacagta ttaatgctgt tgatgtgagg 90415
   aaaattttat aattttcctt ccatccatat actaaccagg cccaacagtg cttagcttct 90475
   gagatcagag atcaggtgca tgtgcattaa gggtcatatg gccatagata gttctctaat 90535
   ctttccattc ctcagtttct taagggaatt tctgaaccct caaaattcct tatttcctaa 90595
   gtagacagat tacctgtcat ttttcaaaga ttaaggctta agatcaaacc agaactgttt 90655
   tggaaattct aaatcactgt ctatataaat ggcaagataa cttttaagat atttatacca 90715
   agcccagtac agtagcacac cacacctgta atcccagcac tttgggaggc tgaagtgggt 90775
   ggatcacatg aggtcaggag ttcgagacca ctctggccaa catggtgaaa ccctgtctct 90835
   actaaaaata taaaaattag ccaggcatgg tggcacttgc ctgttatccc agctacaagg 90895
   gaggctaagg caggagaatc gctttaacct gggaggcagt ggttgttgca gtgagccaag 90955
   attgcaccac tgcactctag cctgggcgac agagtgagac tgtctcaaaa aaaaaaaaaa 91015
   aaaaaaagat acttgtccca gccatgaaaa tgtttgctgc cccttacttt cgcaaacttt  91075
   tagtatttta ttatttttca atggctgtaa aatatgactt attaaatgta gtataatata  91135
   aagaaaagag atatctagca aagatagcat taaagcaaaa atcctatttg cctgctgata  91195
   aagttagagg tgttaacttg gagggtgaat ccaataaatt agaacttttg tgctatattt  91255
   ggagactttt gttttcctac caaagtatca gggctatgtc ttacttatct ttgtattaca  91315
   cagcctgcat gacacgtttt gcacatagta attgcacagt aaatgtgtaa taacctacat  91375
   ggaatagcca gtgttgtgtt ggatagcggg agcatttggc tagcttatgg ttatagtccc  91435
   ttacccaaca gtctgctttt cttctgttgt acttttagta cctaacaagt ttccctggct  91495
   ttaggatttt ttccatgtaa aatttctatc atgtgaagaa aaaataactt ggcctacact  91555
   tctaatacct agcacatacc tctttctgcc tgctatgaaa ttataatact tgatggaggg  91615
   aggcagcatt aagtgtttac atcctgaagt atttcagcca taacatccag tgttttccag  91675
   gttctaggtt tcataaaatg tatctctgtt ctctagaaca aatccattac cttgaactca  91735
   ttcgtagtgg gaaaaagctg agtctaattt gtatgacttt ttcaacag at  gct gat   91791
                                                        Asp Ala Asp
   caa  cta aag gaa aaa ata  aaa gat cta gag aca  cag ctc aaa atg     91836
   Gln  Leu Lys Glu Lys Ile  Lys Asp Leu Glu Thr  Gln Leu Lys Met
   2380                 2385                 2390
   tca  gat cta gaa aag cag  cat ttg aag gtaatattta attatatttt        91883
   Ser  Asp Leu Glu Lys Gln  His Leu Lys
   2395                 2400
   agtatcgttt tgtgaaaaca gctgttgaaa actattttca ttaccatctt taactacgta  91943
   tcctaaaaaa ttcagtaata acatcttata tttgaccttt atattgcaaa gttaattatg  92003
   ttcatctgac tattcctaac atattagagt taacaaaaaa ttcagactca acataggatt  92063
   aagtagtaaa tttatttttt aattgtaaca aatatatgcc attagtatgt tcttaagttt  92123
   tgggtcacat tggcaacagt gtctttattt tttttttgaa attcttttca ggaatcctaa  92183
   ggttatagtt cccttaaaaa aatatttgct gttttacctc ttttaagact gtaaacagga  92243
   caaaaaggca tggatatgag aattagctag tgatcactgg ctattctaaa tagtcactaa  92303
   ggcttgaatt gtctcttcac cagatgcctg tcagaagtcc caaaggtttc cctgatcata  92363
   ttaataactt tataaaaaat tgatcattat tcattaaata ttagatatta gtaaggaaaa  92423
   tataaatgaa gtctaaacca aaactcttaa ccagactaac ttcaatgtta tgaatcacaa  92483
   aatctttttg attgattgct ctattgacaa gctcttatat gcttttagag aaagattaag  92543
   tcccattata agagatgata aattttagtc aaagactaga acacaactta cagaatacat  92603
   aactggactt gacagttaac aacttagtta tttacactgt acaatggaac aaagaaaaat  92663
   cttaattctt ctgcctttat tgctgtattt gaccattcag gaatactttg gctttcatat  92723
   ttacaattaa atctccttgt tcaaacgtaa aatatgtata tttcctatat gcaactttta  92783
   aagataatgt ttccattag gag gaa  ata aag aag ctg aaa  aaa gaa ctg     92832
                        Glu Glu  Ile Lys Lys Leu Lys  Lys Glu Leu
                            2405                 2410
   gaa aat  ttt gat cct tca ttt  ttt gaa gaa att gaa  gat ctt aag     92877
   Glu Asn  Phe Asp Pro Ser Phe  Phe Glu Glu Ile Glu  Asp Leu Lys
       2415                 2420                 2425
   tat aat  tac aag gaa gaa gtg  aag aag aat att ctc  tta gaa gag     92922
   Tyr Asn  Tyr Lys Glu Glu Val  Lys Lys Asn Ile Leu  Leu Glu Glu
       2430                 2435                 2440
   aag gta  aaa aaa ctt tca gaa  caa ttg gga gtt gaa  tta act agc     92967
   Lys Val  Lys Lys Leu Ser Glu  Gln Leu Gly Val Glu  Leu Thr Ser
       2445                 2450                 2455
   cct gtt  gct gct tct gaa gag  ttt gaa gat gaa gaa  gaa agt cct     93012
   Pro Val  Ala Ala Ser Glu Glu  Phe Glu Asp Glu Glu  Glu Ser Pro
       2460                 2465                 2470
   gtt aat  ttc ccc att tac taa aggtcaccta taaactttgt ttcatttaac      93063
   Val Asn  Phe Pro Ile Tyr
       2475
   tatttattaa ctttataagt taaatatact tggaaataag cagttctccg aactgtagta  93123
   tttccttctc actaccttgt acctttatac ttagattgga attcttaata aataaaatta  93183
   tatgaaattt tcaacttatt                                              93203
<210> 2
   <211> 7972
   <212> DNA
   <213> Homo sapiens
<220>
   <221> CDS
   <222> (345)..(7784)
<400> 2
   atttgaagtc ctcgttccac gccttctcat catcctgaac accgagctct gggactccgg     60
   cggagaatct aaacgtaaag catcacccac ggtcgtgaac tgtaggctct cctggcatcc    120
   gggatcttat tctggccttg gcggagttgg ggatggtgtc gcctagcagc cgctgccgct    180
   ttggcttgct cgggaccatt tggctggacc cagagtccgc gtggaaccgc gatagggatc    240
   tgtcagggcc cgcggccggg tccagcttgg tggttgcggt agtgagaggc ctccgctggt    300
   tgccaggctt ggtctagagg tggagcacag tgaaagaatt caag atg cca cct aat     356
                                                   Met Pro Pro Asn
                                                   1
   ata aac tgg aaa gaa ata atg aaa gtt gac cca gat gac ctg ccc cgt      404
   Ile Asn Trp Lys Glu Ile Met Lys Val Asp Pro Asp Asp Leu Pro Arg
   5                   10                  15                  20
   caa gaa gaa ctg gca gat aat tta ttg att tcc tta tcc aag gtg gaa      452
   Gln Glu Glu Leu Ala Asp Asn Leu Leu Ile Ser Leu Ser Lys Val Glu
                   25                  30                  35
   gta aat gag cta aaa agt gaa aag caa gaa aat gtg ata cac ctt ttc      500
   Val Asn Glu Leu Lys Ser Glu Lys Gln Glu Asn Val Ile His Leu Phe
               40                  45                  50
   aga att act cag tca cta atg aag atg aaa gct caa gaa gtg gag ctg      548
   Arg Ile Thr Gln Ser Leu Met Lys Met Lys Ala Gln Glu Val Glu Leu
           55                  60                  65
   gct ttg gaa gaa gta gaa aaa gct gga gaa gaa caa gca aaa ttt gaa      596
   Ala Leu Glu Glu Val Glu Lys Ala Gly Glu Glu Gln Ala Lys Phe Glu
       70                  75                  80
   aat caa tta aaa act aaa gta atg aaa ctg gaa aat gaa ctg gag atg      644
   Asn Gln Leu Lys Thr Lys Val Met Lys Leu Glu Asn Glu Leu Glu Met
   85                  90                  95                  100
   gct cag cag tct gca ggt gga cga gat act cgg ttt tta cgt aat gaa      692
   Ala Gln Gln Ser Ala Gly Gly Arg Asp Thr Arg Phe Leu Arg Asn Glu
                   105                 110                 115
   att tgc caa ctt gaa aaa caa tta gaa caa aaa gat aga gaa ttg gag      740
   Ile Cys Gln Leu Glu Lys Gln Leu Glu Gln Lys Asp Arg Glu Leu Glu
               120                 125                 130
   gac atg gaa aag gag ttg gag aaa gag aag aaa gtt aat gag caa ttg      788
   Asp Met Glu Lys Glu Leu Glu Lys Glu Lys Lys Val Asn Glu Gln Leu
           135                 140                 145
   gct ctt cga aat gag gag gca gaa aat gaa aac agc aaa tta aga aga      836
   Ala Leu Arg Asn Glu Glu Ala Glu Asn Glu Asn Ser Lys Leu Arg Arg
       150                 155                 160
   gag aac aaa cgt cta aag aaa aag aat gaa caa ctt tgt cag gat att      884
   Glu Asn Lys Arg Leu Lys Lys Lys Asn Glu Gln Leu Cys Gln Asp Ile
   165                 170                 175                 180
   att gac tac cag aaa caa ata gat tca cag aaa gaa aca ctt tta tca      932
   Ile Asp Tyr Gln Lys Gln Ile Asp Ser Gln Lys Glu Thr Leu Leu Ser
                   185                 190                 195
   aga aga ggg gaa gac agt gac tac cga tca cag ttg tct aaa aaa aac      980
   Arg Arg Gly Glu Asp Ser Asp Tyr Arg Ser Gln Leu Ser Lys Lys Asn
               200                 205                 210
   tat gag ctt atc caa tat ctt gat gaa att cag act tta aca gaa gct     1028
   Tyr Glu Leu Ile Gln Tyr Leu Asp Glu Ile Gln Thr Leu Thr Glu Ala
           215                 220                 225
   aat gag aaa att gaa gtt cag aat caa gaa atg aga aaa aat tta gaa     1076
   Asn Glu Lys Ile Glu Val Gln Asn Gln Glu Met Arg Lys Asn Leu Glu
       230                 235                 240
   gag tct gta cag gaa atg gag aag atg act gat gaa tat aat aga atg     1124
   Glu Ser Val Gln Glu Met Glu Lys Met Thr Asp Glu Tyr Asn Arg Met
   245                 250                 255                 260
   aaa gct att gtg cat cag aca gat aat gta ata gat cag tta aaa aaa     1172
   Lys Ala Ile Val His Gln Thr Asp Asn Val Ile Asp Gln Leu Lys Lys
                   265                 270                 275
   gaa aac gat cat tat caa ctt caa gtg cag gag ctt aca gat ctt ctg      1220
   Glu Asn Asp His Tyr Gln Leu Gln Val Gln Glu Leu Thr Asp Leu Leu
               280                 285                 290
   aaa tca aaa aat gaa gaa gat gat cca att atg gta gct gtc aat gca      1268
   Lys Ser Lys Asn Glu Glu Asp Asp Pro Ile Met Val Ala Val Asn Ala
           295                 300                 305
   aaa gta gaa gaa tgg aag cta att ttg tct tct aaa gat gat gaa att      1316
   Lys Val Glu Glu Trp Lys Leu Ile Leu Ser Ser Lys Asp Asp Glu Ile
       310                 315                 320
   att gag tat cag caa atg tta cat aac cta agg gag aaa ctt aag aat      1364
   Ile Glu Tyr Gln Gln Met Leu His Asn Leu Arg Glu Lys Leu Lys Asn
   325                 330                 335                 340
   gct cag ctt gat gct gat aaa agt aat gtt atg gct cta cag cag ggt      1412
   Ala Gln Leu Asp Ala Asp Lys Ser Asn Val Met Ala Leu Gln Gln Gly
                   345                 350                 355
   ata cag gaa cga gac agt caa att aag atg ctc acc gaa caa gta gaa      1460
   Ile Gln Glu Arg Asp Ser Gln Ile Lys Met Leu Thr Glu Gln Val Glu
               360                 365                 370
   caa tat aca aaa gaa atg gaa aag aat act tgt att att gaa gat ttg      1508
   Gln Tyr Thr Lys Glu Met Glu Lys Asn Thr Cys Ile Ile Glu Asp Leu
           375                 380                 385
   aaa aat gag ctc caa aga aac aaa ggt gct tca acc ctt tct caa cag      1556
   Lys Asn Glu Leu Gln Arg Asn Lys Gly Ala Ser Thr Leu Ser Gln Gln
       390                 395                 400
   act cat atg aaa att cag tca acg tta gac att tta aaa gag aaa act      1604
   Thr His Met Lys Ile Gln Ser Thr Leu Asp Ile Leu Lys Glu Lys Thr
   405                 410                 415                 420
   aaa gag gct gag aga aca gct gaa ctg gct gag gct gat gct agg gaa      1652
   Lys Glu Ala Glu Arg Thr Ala Glu Leu Ala Glu Ala Asp Ala Arg Glu
                   425                 430                 435
   aag gat aaa gaa tta gtt gag gct ctg aag agg tta aaa gat tat gaa      1700
   Lys Asp Lys Glu Leu Val Glu Ala Leu Lys Arg Leu Lys Asp Tyr Glu
               440                 445                 450
   tcg gga gta tat ggt tta gaa gat gct gtc gtt gaa ata aag aat tgt      1748
   Ser Gly Val Tyr Gly Leu Glu Asp Ala Val Val Glu Ile Lys Asn Cys
           455                 460                 465
   aaa aac caa att aaa ata aga gat cga gag att gaa ata tta aca aag      1796
   Lys Asn Gln Ile Lys Ile Arg Asp Arg Glu Ile Glu Ile Leu Thr Lys
       470                 475                 480
   gaa atc aat aaa ctt gaa ttg aag atc agt gat ttc ctt gat gaa aat      1844
   Glu Ile Asn Lys Leu Glu Leu Lys Ile Ser Asp Phe Leu Asp Glu Asn
   485                 490                 495                 500
   gag gca ctt aga gag cgt gtg ggc ctt gaa cca aag aca atg att gat      1892
   Glu Ala Leu Arg Glu Arg Val Gly Leu Glu Pro Lys Thr Met Ile Asp
                   505                 510                 515
   tta act gaa ttt aga aat agc aaa cac tta aaa cag cag cag tac aga      1940
   Leu Thr Glu Phe Arg Asn Ser Lys His Leu Lys Gln Gln Gln Tyr Arg
               520                 525                 530
   gct gaa aac cag att ctt ttg aaa gag att gaa agt cta gag gaa gaa      1988
   Ala Glu Asn Gln Ile Leu Leu Lys Glu Ile Glu Ser Leu Glu Glu Glu
           535                 540                 545
   cga ctt gat ctg aaa aaa aaa att cgt caa atg gct caa gaa aga gga      2036
   Arg Leu Asp Leu Lys Lys Lys Ile Arg Gln Met Ala Gln Glu Arg Gly
       550                 555                 560
   aaa aga agt gca act tca gga tta acc act gag gac ctg aac cta act      2084
   Lys Arg Ser Ala Thr Ser Gly Leu Thr Thr Glu Asp Leu Asn Leu Thr
   565                 570                 575                 580
   gaa aac att tct caa gga gat aga ata agt gaa aga aaa ttg gat tta      2132
   Glu Asn Ile Ser Gln Gly Asp Arg Ile Ser Glu Arg Lys Leu Asp Leu
                   585                 590                 595
   ttg agc ctc aaa aat atg agt gaa gca caa tca aag aat gaa ttt ctt      2180
   Leu Ser Leu Lys Asn Met Ser Glu Ala Gln Ser Lys Asn Glu Phe Leu
               600                 605                 610
   tca aga gaa cta att gaa aaa gaa aga gat tta gaa agg agt agg aca      2228
   Ser Arg Glu Leu Ile Glu Lys Glu Arg Asp Leu Glu Arg Ser Arg Thr
           615                 620                 625
   gtg ata gcc aaa ttt cag aat aaa tta aaa gaa tta gtt gaa gaa aat      2276
   Val Ile Ala Lys Phe Gln Asn Lys Leu Lys Glu Leu Val Glu Glu Asn
       630                 635                 640
   aag caa ctt gaa gaa ggt atg aaa gaa ata ttg caa gca att aag gaa      2324
   Lys Gln Leu Glu Glu Gly Met Lys Glu Ile Leu Gln Ala Ile Lys Glu
   645                 650                 655                 660
   atg cag aaa gat cct gat gtt aaa gga gga gaa aca tct cta att atc      2372
   Met Gln Lys Asp Pro Asp Val Lys Gly Gly Glu Thr Ser Leu Ile Ile
                   665                 670                 675
   cct agc ctt gaa aga cta gtt aat gct ata gaa tca aag aat gca gaa      2420
   Pro Ser Leu Glu Arg Leu Val Asn Ala Ile Glu Ser Lys Asn Ala Glu
               680                 685                 690
   gga atc ttt gat gcg agt ctg cat ttg aaa gcc caa gtt gat cag ctt      2468
   Gly Ile Phe Asp Ala Ser Leu His Leu Lys Ala Gln Val Asp Gln Leu
           695                 700                 705
   acc gga aga aat gaa gaa tta aga cag gag ctc agg gaa tct cgg aaa      2516
   Thr Gly Arg Asn Glu Glu Leu Arg Gln Glu Leu Arg Glu Ser Arg Lys
       710                 715                 720
   gag gct ata aat tat tca cag cag ttg gca aaa gct aat tta aag ata      2564
   Glu Ala Ile Asn Tyr Ser Gln Gln Leu Ala Lys Ala Asn Leu Lys Ile
   725                 730                 735                 740
   gac cat ctt gaa aaa gaa act agt ctt tta cga caa tca gaa gga tcg      2612
   Asp His Leu Glu Lys Glu Thr Ser Leu Leu Arg Gln Ser Glu Gly Ser
                   745                 750                 755
   aat gtt gtt ttt aaa gga att gac tta cct gat ggg ata gca cca tct      2660
   Asn Val Val Phe Lys Gly Ile Asp Leu Pro Asp Gly Ile Ala Pro Ser
               760                 765                 770
   agt gcc agt atc att aat tct cag aat gaa tat tta ata cat ttg tta      2708
   Ser Ala Ser Ile Ile Asn Ser Gln Asn Glu Tyr Leu Ile His Leu Leu
           775                 780                 785
   cag gaa cta gaa aat aaa gaa aaa aag tta aag aat tta gaa gat tct      2756
   Gln Glu Leu Glu Asn Lys Glu Lys Lys Leu Lys Asn Leu Glu Asp Ser
       790                 795                 800
   ctt gaa gat tac aac aga aaa ttt gct gta att cgt cat caa caa agt      2804
   Leu Glu Asp Tyr Asn Arg Lys Phe Ala Val Ile Arg His Gln Gln Ser
   805                 810                 815                 820
   ttg ttg tat aaa gaa tac cta agt gaa aag gag acc tgg aaa aca gaa      2852
   Leu Leu Tyr Lys Glu Tyr Leu Ser Glu Lys Glu Thr Trp Lys Thr Glu
                   825                 830                 835
   tct aaa aca ata aaa gag gaa aag aga aaa ctt gag gat caa gtc caa      2900
   Ser Lys Thr Ile Lys Glu Glu Lys Arg Lys Leu Glu Asp Gln Val Gln
               840                 845                 850
   caa gat gct ata aaa gta aaa gaa tat aat aat ttg ctc aat gct ctt      2948
   Gln Asp Ala Ile Lys Val Lys Glu Tyr Asn Asn Leu Leu Asn Ala Leu
           855                 860                 865
   cag atg gat tcg gat gaa atg aaa aaa ata ctt gca gaa aat agt agg      2996
   Gln Met Asp Ser Asp Glu Met Lys Lys Ile Leu Ala Glu Asn Ser Arg
       870                 875                 880
   aaa att act gtt ttg caa gtg aat gaa aaa tca ctt ata agg caa tat      3044
   Lys Ile Thr Val Leu Gln Val Asn Glu Lys Ser Leu Ile Arg Gln Tyr
   885                 890                 895                 900
   aca acc tta gta gaa ttg gag cga caa ctt aga aaa gaa aat gag aag      3092
   Thr Thr Leu Val Glu Leu Glu Arg Gln Leu Arg Lys Glu Asn Glu Lys
                   905                 910                 915
   caa aag aat gaa ttg ttg tca atg gag gct gaa gtt tgt gaa aaa att      3140
   Gln Lys Asn Glu Leu Leu Ser Met Glu Ala Glu Val Cys Glu Lys Ile
               920                 925                 930
   ggg tgt ttg caa aga ttt aag gaa atg gcc att ttc aag att gca gct      3188
   Gly Cys Leu Gln Arg Phe Lys Glu Met Ala Ile Phe Lys Ile Ala Ala
           935                 940                 945
   ctc caa aaa gtt gta gat aat agt gtt tct ttg tct gaa cta gaa ctg      3236
   Leu Gln Lys Val Val Asp Asn Ser Val Ser Leu Ser Glu Leu Glu Leu
       950                 955                 960
   gct aat aaa cag tac aat gaa ctg act gct aag tac agg gac atc ttg      3284
   Ala Asn Lys Gln Tyr Asn Glu Leu Thr Ala Lys Tyr Arg Asp Ile Leu
   965                 970                 975                 980
   caa aaa gat aat atg ctt gtt caa aga aca agt aac ttg gaa cac ctg      3332
   Gln Lys Asp Asn Met Leu Val Gln Arg Thr Ser Asn Leu Glu His Leu
                   985                 990                 995
   gag tgt gaa aac  atc tcc tta aaa gaa  caa gtg gag tct ata  aat      3377
   Glu Cys Glu Asn  Ile Ser Leu Lys Glu  Gln Val Glu Ser Ile  Asn
               1000                 1005                 1010
   aaa gaa ctg gag  att acc aag gaa aaa  ctt cac act att gaa  caa      3422
   Lys Glu Leu Glu  Ile Thr Lys Glu Lys  Leu His Thr Ile Glu  Gln
               1015                 1020                 1025
   gcc tgg gaa cag  gaa act aaa tta ggt  aat gaa tct agc atg  gat      3467
   Ala Trp Glu Gln  Glu Thr Lys Leu Gly  Asn Glu Ser Ser Met  Asp
               1030                 1035                 1040
   aag gca aag aaa  tca ata acc aac agt  gac att gtt tcc att  tca      3512
   Lys Ala Lys Lys  Ser Ile Thr Asn Ser  Asp Ile Val Ser Ile  Ser
               1045                 1050                 1055
   aaa aaa ata act  atg ctg gaa atg aag  gaa tta aat gaa agg  cag      3557
   Lys Lys Ile Thr  Met Leu Glu Met Lys  Glu Leu Asn Glu Arg  Gln
               1060                 1065                 1070
   cgg gct gaa cat  tgt caa aaa atg tat  gaa cac tta cgg act  tcg      3602
   Arg Ala Glu His  Cys Gln Lys Met Tyr  Glu His Leu Arg Thr  Ser
               1075                 1080                 1085
   tta aag caa atg  gag gaa cgt aat ttt  gaa ttg gaa acc aaa  ttt      3647
   Leu Lys Gln Met  Glu Glu Arg Asn Phe  Glu Leu Glu Thr Lys  Phe
               1090                 1095                 1100
   gct gag ctt acc  aaa atc aat ttg gat  gca cag aag gtg gaa  cag      3692
   Ala Glu Leu Thr  Lys Ile Asn Leu Asp  Ala Gln Lys Val Glu  Gln
               1105                 1110                 1115
   atg tta aga gat  gaa tta gct gat agt  gtg agc aag gca gta  agt      3737
   Met Leu Arg Asp  Glu Leu Ala Asp Ser  Val Ser Lys Ala Val  Ser
               1120                 1125                 1130
   gat gct gat agg  caa cgg att cta gaa  tta gag aag aat gaa  atg      3782
   Asp Ala Asp Arg  Gln Arg Ile Leu Glu  Leu Glu Lys Asn Glu  Met
               1135                 1140                 1145
   gaa cta aaa gtt  gaa gtg tca aaa ctg  aga gag att tct gat  att      3827
   Glu Leu Lys Val  Glu Val Ser Lys Leu  Arg Glu Ile Ser Asp  Ile
               1150                 1155                 1160
   gcc aga aga caa  gtt gaa att ttg aat  gca caa caa caa tct  agg      3872
   Ala Arg Arg Gln  Val Glu Ile Leu Asn  Ala Gln Gln Gln Ser  Arg
               1165                 1170                 1175
   gac aag gaa gta  gag tcc ctc aga atg  caa ctg cta gac tat  cag      3917
   Asp Lys Glu Val  Glu Ser Leu Arg Met  Gln Leu Leu Asp Tyr  Gln
               1180                 1185                 1190
   gca cag tct gat  gaa aag tcg ctc att  gcc aag ttg cac caa  cat      3962
   Ala Gln Ser Asp  Glu Lys Ser Leu Ile  Ala Lys Leu His Gln  His
               1195                 1200                 1205
   aat gtc tct ctt  caa ctg agt gag gct  act gct ctt ggt aag  ttg      4007
   Asn Val Ser Leu  Gln Leu Ser Glu Ala  Thr Ala Leu Gly Lys  Leu
               1210                 1215                 1220
   gag tca att aca  tct aaa ctg cag aag  atg gag gcc tac aac  ttg      4052
   Glu Ser Ile Thr  Ser Lys Leu Gln Lys  Met Glu Ala Tyr Asn  Leu
               1225                 1230                 1235
   cgc tta gag cag  aaa ctt gat gaa aaa  gaa cag gct ctc tat  tat      4097
   Arg Leu Glu Gln  Lys Leu Asp Glu Lys  Glu Gln Ala Leu Tyr  Tyr
               1240                 1245                 1250
   gct cgt ttg gag  gga aga aac aga gca  aaa cat ctg cgc caa  aca      4142
   Ala Arg Leu Glu  Gly Arg Asn Arg Ala  Lys His Leu Arg Gln  Thr
               1255                 1260                 1265
   att cag tct cta  cga cga cag ttt agt  gga gct tta ccc ttg  gca       4187
   Ile Gln Ser Leu  Arg Arg Gln Phe Ser  Gly Ala Leu Pro Leu  Ala
               1270                 1275                 1280
   caa cag gaa aag  ttc tcc aaa aca atg  att caa cta caa aat  gac       4232
   Gln Gln Glu Lys  Phe Ser Lys Thr Met  Ile Gln Leu Gln Asn  Asp
               1285                 1290                 1295
   aaa ctt aag ata  atg caa gaa atg aaa  aat tct caa caa gaa  cat       4277
   Lys Leu Lys Ile  Met Gln Glu Met Lys  Asn Ser Gln Gln Glu  His
               1300                 1305                 1310
   aga aat atg gag  aac aaa aca ttg gag  atg gaa tta aaa tta  aag       4322
   Arg Asn Met Glu  Asn Lys Thr Leu Glu  Met Glu Leu Lys Leu  Lys
               1315                 1320                 1325
   ggc ctg gaa gag  tta ata agc act tta  aag gat acc aaa gga  gcc       4367
   Gly Leu Glu Glu  Leu Ile Ser Thr Leu  Lys Asp Thr Lys Gly  Ala
               1330                 1335                 1340
   caa aag gta atc  aac tgg cat atg aaa  ata gaa gaa ctt cgt  ctt       4412
   Gln Lys Val Ile  Asn Trp His Met Lys  Ile Glu Glu Leu Arg  Leu
               1345                 1350                 1355
   caa gaa ctt aaa  cta aat cgg gaa tta  gtc aag gat aaa gaa  gaa       4457
   Gln Glu Leu Lys  Leu Asn Arg Glu Leu  Val Lys Asp Lys Glu  Glu
               1360                 1365                 1370
   ata aaa tat ttg  aat aac ata att tct  gaa tat gaa cgt aca  atc       4502
   Ile Lys Tyr Leu  Asn Asn Ile Ile Ser  Glu Tyr Glu Arg Thr  Ile
               1375                 1380                 1385
   agc agt ctt gaa  gaa gaa att gtg caa  cag aac aag ttt cat  gaa       4547
   Ser Ser Leu Glu  Glu Glu Ile Val Gln  Gln Asn Lys Phe His  Glu
               1390                 1395                 1400
   gaa aga caa atg  gcc tgg gat caa aga  gaa gtt gac ctg gaa  cgc       4592
   Glu Arg Gln Met  Ala Trp Asp Gln Arg  Glu Val Asp Leu Glu  Arg
               1405                 1410                 1415
   caa cta gac att  ttt gac cgt cag caa  aat gaa ata cta aat  gcg       4637
   Gln Leu Asp Ile  Phe Asp Arg Gln Gln  Asn Glu Ile Leu Asn  Ala
               1420                 1425                 1430
   gca caa aag ttt  gaa gaa gct aca gga  tca atc cct gac cct  agt       4682
   Ala Gln Lys Phe  Glu Glu Ala Thr Gly  Ser Ile Pro Asp Pro  Ser
               1435                 1440                 1445
   ttg ccc ctt cca  aat caa ctt gag atc  gct cta agg aaa att  aag       4727
   Leu Pro Leu Pro  Asn Gln Leu Glu Ile  Ala Leu Arg Lys Ile  Lys
               1450                 1455                 1460
   gag aac att cga  ata att cta gaa aca  cgg gca act tgc aaa  tca       4772
   Glu Asn Ile Arg  Ile Ile Leu Glu Thr  Arg Ala Thr Cys Lys  Ser
               1465                 1470                 1475
   cta gaa gag aaa  cta aaa gag aaa gaa  tct gct tta agg tta  gca       4817
   Leu Glu Glu Lys  Leu Lys Glu Lys Glu  Ser Ala Leu Arg Leu  Ala
               1480                 1485                 1490
   gaa caa aat ata  ctg tca aga gac aaa  gta atc aat gaa ctg  agg       4862
   Glu Gln Asn Ile  Leu Ser Arg Asp Lys  Val Ile Asn Glu Leu  Arg
                   1495                1500                1505
   ctt cga ttg cct  gcc act gca gaa aga  gaa aag ctc ata gct  gag       4907
   Leu Arg Leu Pro  Ala Thr Ala Glu Arg  Glu Lys Leu Ile Ala  Glu
                   1510                1515                1520
   cta ggc aga aaa  gag atg gaa cca aaa  tct cac cac aca ttg  aaa       4952
   Leu Gly Arg Lys  Glu Met Glu Pro Lys  Ser His His Thr Leu  Lys
                   1525                1530                1535
   att gct cat caa  acc att gca aac atg  caa gca agg tta aat  caa       4997
   Ile Ala His Gln  Thr Ile Ala Asn Met  Gln Ala Arg Leu Asn  Gln
                   1540                1545                1550
   aaa gaa gaa gta  tta aag aag tat caa  cgt ctt cta gaa aaa  gcc       5042
   Lys Glu Glu Val  Leu Lys Lys Tyr Gln  Arg Leu Leu Glu Lys  Ala
                   1555                1560                1565
   aga gag gag caa  aga gaa att gtg aag  aaa cat gag gaa gac  ctt       5087
   Arg Glu Glu Gln  Arg Glu Ile Val Lys  Lys His Glu Glu Asp  Leu
                   1570                1575                1580
   cat att ctt cat  cac aga tta gaa cta  cag gct gat agt tca  cta       5132
   His Ile Leu His  His Arg Leu Glu Leu  Gln Ala Asp Ser Ser  Leu
                   1585                1590                1595
   aat aaa ttc aaa  caa acg gct tgg gat  tta atg aaa cag tct  ccc       5177
   Asn Lys Phe Lys  Gln Thr Ala Trp Asp  Leu Met Lys Gln Ser  Pro
                   1600                1605                1610
   act cca gtt cct  acc aac aag cat ttt  att cgt ctg gct gag  atg       5222
   Thr Pro Val Pro  Thr Asn Lys His Phe  Ile Arg Leu Ala Glu  Met
                   1615                1620                1625
   gaa cag aca gta  gca gaa caa gat gac  tct ctt tcc tca ctc  ttg       5267
   Glu Gln Thr Val  Ala Glu Gln Asp Asp  Ser Leu Ser Ser Leu  Leu
                   1630                1635                1640
   gtc aaa cta aag  aaa gta tca caa gat  ttg gag aga caa aga  gaa       5312
   Val Lys Leu Lys  Lys Val Ser Gln Asp  Leu Glu Arg Gln Arg  Glu
                   1645                1650                1655
   atc act gaa tta  aaa gta aaa gaa ttt  gaa aat atc aaa tta  cag       5357
   Ile Thr Glu Leu  Lys Val Lys Glu Phe  Glu Asn Ile Lys Leu  Gln
                   1660                1665                1670
   ctt caa gaa aac  cat gaa gat gaa gtg  aaa aaa gta aaa gcg  gaa       5402
   Leu Gln Glu Asn  His Glu Asp Glu Val  Lys Lys Val Lys Ala  Glu
                   1675                1680                1685
   gta gag gat tta  aag tat ctt ctg gac  cag tca caa aag gag  tca       5447
   Val Glu Asp Leu  Lys Tyr Leu Leu Asp  Gln Ser Gln Lys Glu  Ser
                   1690                1695                1700
   cag tgt tta aaa  tct gaa ctt cag gct  caa aaa gaa gca aat  tca       5492
   Gln Cys Leu Lys  Ser Glu Leu Gln Ala  Gln Lys Glu Ala Asn  Ser
                   1705                1710                1715
   aga gct cca aca  act aca atg aga aat  cta gta gaa cgg cta  aag       5537
   Arg Ala Pro Thr  Thr Thr Met Arg Asn  Leu Val Glu Arg Leu  Lys
                   1720                1725                1730
   agc caa tta gcc  ttg aag gag aaa caa  cag aaa gca ctt agt  cgg       5582
   Ser Gln Leu Ala  Leu Lys Glu Lys Gln  Gln Lys Ala Leu Ser  Arg
               1735                 1740                 1745
   gca ctt tta gaa  ctc cgg gca gaa atg  aca gca gct gct gaa  gaa      5627
   Ala Leu Leu Glu  Leu Arg Ala Glu Met  Thr Ala Ala Ala Glu  Glu
               1750                 1755                 1760
   cgt att att tct  gca act tct caa aaa  gag gcc cat ctc aat  gtt      5672
   Arg Ile Ile Ser  Ala Thr Ser Gln Lys  Glu Ala His Leu Asn  Val
               1765                 1770                 1775
   caa caa atc gtt  gat cga cat act aga  gag cta aag aca caa  gtt      5717
   Gln Gln Ile Val  Asp Arg His Thr Arg  Glu Leu Lys Thr Gln  Val
               1780                 1785                 1790
   gaa gat tta aat  gaa aat ctt tta aaa  ttg aaa gaa gca ctt  aaa      5762
   Glu Asp Leu Asn  Glu Asn Leu Leu Lys  Leu Lys Glu Ala Leu  Lys
               1795                 1800                 1805
   aca agt aaa aac  aga gaa aac tca cta  act gat aat ttg aat  gac      5807
   Thr Ser Lys Asn  Arg Glu Asn Ser Leu  Thr Asp Asn Leu Asn  Asp
               1810                 1815                 1820
   tta aat aat gaa  ctg caa aag aaa caa  aaa gcc tat aat aaa  ata      5852
   Leu Asn Asn Glu  Leu Gln Lys Lys Gln  Lys Ala Tyr Asn Lys  Ile
               1825                 1830                 1835
   ctt aga gag aaa  gag gaa att gat caa  gag aat gat gaa ctg  aaa      5897
   Leu Arg Glu Lys  Glu Glu Ile Asp Gln  Glu Asn Asp Glu Leu  Lys
               1840                 1845                 1850
   agg caa att aaa  aga cta acc agt gga  tta cag ggc aaa ccc  ctg      5942
   Arg Gln Ile Lys  Arg Leu Thr Ser Gly  Leu Gln Gly Lys Pro  Leu
               1855                 1860                 1865
   aca gat aat aaa  caa agt cta att gaa  gaa ctc caa agg aaa  gtt      5987
   Thr Asp Asn Lys  Gln Ser Leu Ile Glu  Glu Leu Gln Arg Lys  Val
               1870                 1875                 1880
   aaa aaa cta gag  aac caa tta gag gga  aag gtg gag gaa gta  gac      6032
   Lys Lys Leu Glu  Asn Gln Leu Glu Gly  Lys Val Glu Glu Val  Asp
               1885                 1890                 1895
   cta aaa cct atg  aaa gaa aag aat gct  aaa gaa gaa tta att  agg      6077
   Leu Lys Pro Met  Lys Glu Lys Asn Ala  Lys Glu Glu Leu Ile  Arg
               1900                 1905                 1910
   tgg gaa gaa ggt  aaa aag tgg caa gcc  aaa ata gaa gga att  cga      6122
   Trp Glu Glu Gly  Lys Lys Trp Gln Ala  Lys Ile Glu Gly Ile  Arg
               1915                 1920                 1925
   aac aag tta aaa  gag aaa gag ggg gaa  gtc ttt act tta aca  aag      6167
   Asn Lys Leu Lys  Glu Lys Glu Gly Glu  Val Phe Thr Leu Thr  Lys
               1930                 1935                 1940
   cag ttg aat act  ttg aag gat ctt ttt  gcc aaa gcc gat aaa  gag      6212
   Gln Leu Asn Thr  Leu Lys Asp Leu Phe  Ala Lys Ala Asp Lys  Glu
               1945                 1950                 1955
   aaa ctt act ttg  cag agg aaa cta aaa  aca act ggc atg act  gtt      6257
   Lys Leu Thr Leu  Gln Arg Lys Leu Lys  Thr Thr Gly Met Thr  Val
               1960                 1965                 1970
   gat cag gtt ttg  gga ata cga gct ttg  gag tca gaa aaa gaa  ttg      6302
   Asp Gln Val Leu  Gly Ile Arg Ala Leu  Glu Ser Glu Lys Glu  Leu
               1975                 1980                 1985
   gaa gaa tta aaa  aag aga aat ctt gac  tta gaa aat gat ata  ttg      6347
   Glu Glu Leu Lys  Lys Arg Asn Leu Asp  Leu Glu Asn Asp Ile  Leu
               1990                 1995                 2000
   tat atg agg gcc  cac caa gct ctt cct  cga gat tct gtt gta  gaa      6392
   Tyr Met Arg Ala  His Gln Ala Leu Pro  Arg Asp Ser Val Val  Glu
               2005                 2010                 2015
   gat tta cat tta  caa aat aga tac ctc  caa gaa aaa ctt cat  gct      6437
   Asp Leu His Leu  Gln Asn Arg Tyr Leu  Gln Glu Lys Leu His  Ala
               2020                 2025                 2030
   tta gaa aaa cag  ttt tca aag gat aca  tat tct aag cct tca  att      6482
   Leu Glu Lys Gln  Phe Ser Lys Asp Thr  Tyr Ser Lys Pro Ser  Ile
               2035                 2040                 2045
   tca gga ata gag  tca gat gat cat tgt  cag aga gaa cag gag  ctt      6527
   Ser Gly Ile Glu  Ser Asp Asp His Cys  Gln Arg Glu Gln Glu  Leu
               2050                 2055                 2060
   cag aag gaa aac  ttg aag ttg tca tct  gaa aat att gaa ctg  aaa      6572
   Gln Lys Glu Asn  Leu Lys Leu Ser Ser  Glu Asn Ile Glu Leu  Lys
               2065                 2070                 2075
   ttt cag ctt gaa  caa gca aat aaa gat  ttg cca aga tta aag  aat      6617
   Phe Gln Leu Glu  Gln Ala Asn Lys Asp  Leu Pro Arg Leu Lys  Asn
               2080                 2085                 2090
   caa gtc aga gat  ttg aag gaa atg tgt  gaa ttt ctt aag aaa  gaa      6662
   Gln Val Arg Asp  Leu Lys Glu Met Cys  Glu Phe Leu Lys Lys  Glu
               2095                 2100                 2105
   aaa gca gaa gtt  cag cgg aaa ctt ggc  cat gtt aga ggg tct  ggt      6707
   Lys Ala Glu Val  Gln Arg Lys Leu Gly  His Val Arg Gly Ser  Gly
               2110                 2115                 2120
   aga agt gga aag  aca atc cca gaa ctg  gaa aaa acc att ggt  tta      6752
   Arg Ser Gly Lys  Thr Ile Pro Glu Leu  Glu Lys Thr Ile Gly  Leu
               2125                 2130                 2135
   atg aaa aaa gta  gtt gaa aaa gtc cag  aga gaa aat gaa cag  ttg      6797
   Met Lys Lys Val  Val Glu Lys Val Gln  Arg Glu Asn Glu Gln  Leu
               2140                 2145                 2150
   aaa aaa gca tca  gga ata ttg act agt  gaa aaa atg gct aat  att      6842
   Lys Lys Ala Ser  Gly Ile Leu Thr Ser  Glu Lys Met Ala Asn  Ile
               2155                 2160                 2165
   gag cag gaa aat  gaa aaa ttg aag gct  gaa tta gaa aaa ctt  aaa      6887
   Glu Gln Glu Asn  Glu Lys Leu Lys Ala  Glu Leu Glu Lys Leu  Lys
               2170                 2175                 2180
   gct cat ctt ggg  cat cag ttg agc atg  cac tat gaa tcc aag  acc      6932
   Ala His Leu Gly  His Gln Leu Ser Met  His Tyr Glu Ser Lys  Thr
               2185                 2190                 2195
   aaa ggc aca gaa  aaa att att gct gaa  aat gaa agg ctt cgt  aaa      6977
   Lys Gly Thr Glu  Lys Ile Ile Ala Glu  Asn Glu Arg Leu Arg  Lys
               2200                 2205                 2210
   gaa ctt aaa aaa  gaa act gat gct gca  gag aaa tta cgg ata  gca       7022
   Glu Leu Lys Lys  Glu Thr Asp Ala Ala  Glu Lys Leu Arg Ile  Ala
               2215                 2220                 2225
   aag aat aat tta  gag ata tta aat gag  aag atg aca gtt caa  cta       7067
   Lys Asn Asn Leu  Glu Ile Leu Asn Glu  Lys Met Thr Val Gln  Leu
               2230                 2235                 2240
   gaa gag act ggt  aag aga ttg cag ttt  gca gaa agc aga ggt  cca       7112
   Glu Glu Thr Gly  Lys Arg Leu Gln Phe  Ala Glu Ser Arg Gly  Pro
               2245                 2250                 2255
   cag ctt gaa ggt  gct gac agt aag agc  tgg aaa tcc att gtg  gtt       7157
   Gln Leu Glu Gly  Ala Asp Ser Lys Ser  Trp Lys Ser Ile Val  Val
               2260                 2265                 2270
   aca aga atg tat  gaa acc aag tta aaa  gaa ttg gaa act gat  att       7202
   Thr Arg Met Tyr  Glu Thr Lys Leu Lys  Glu Leu Glu Thr Asp  Ile
               2275                 2280                 2285
   gcc aaa aaa aat  caa agc att act gac  ctt aaa cag ctt gta  aaa       7247
   Ala Lys Lys Asn  Gln Ser Ile Thr Asp  Leu Lys Gln Leu Val  Lys
               2290                 2295                 2300
   gaa gca aca gag  aga gaa caa aaa gtt  aac aaa tac aat gaa  gac       7292
   Glu Ala Thr Glu  Arg Glu Gln Lys Val  Asn Lys Tyr Asn Glu  Asp
               2305                 2310                 2315
   ctt gaa caa cag  att aag att ctt aaa  cat gtt cct gaa ggt  gct       7337
   Leu Glu Gln Gln  Ile Lys Ile Leu Lys  His Val Pro Glu Gly  Ala
               2320                 2325                 2330
   gag aca gag caa  ggc ctt aaa cgg gag  ctt caa gtt ctt aga  tta       7382
   Glu Thr Glu Gln  Gly Leu Lys Arg Glu  Leu Gln Val Leu Arg  Leu
               2335                 2340                 2345
   gct aat cat cag  ctg gat aaa gag aaa  gca gaa tta atc cat  cag       7427
   Ala Asn His Gln  Leu Asp Lys Glu Lys  Ala Glu Leu Ile His  Gln
               2350                 2355                 2360
   ata gaa gct aac  aag gac caa agt gga  gct gaa agc acc ata  cct       7472
   Ile Glu Ala Asn  Lys Asp Gln Ser Gly  Ala Glu Ser Thr Ile  Pro
               2365                 2370                 2375
   gat gct gat caa  cta aag gaa aaa ata  aaa gat cta gag aca  cag       7517
   Asp Ala Asp Gln  Leu Lys Glu Lys Ile  Lys Asp Leu Glu Thr  Gln
               2380                 2385                 2390
   ctc aaa atg tca  gat cta gaa aag cag  cat ttg aag gag gaa  ata       7562
   Leu Lys Met Ser  Asp Leu Glu Lys Gln  His Leu Lys Glu Glu  Ile
               2395                 2400                 2405
   aag aag ctg aaa  aaa gaa ctg gaa aat  ttt gat cct tca ttt  ttt       7607
   Lys Lys Leu Lys  Lys Glu Leu Glu Asn  Phe Asp Pro Ser Phe  Phe
               2410                 2415                 2420
   gaa gaa att gaa  gat ctt aag tat aat  tac aag gaa gaa gtg  aag       7652
   Glu Glu Ile Glu  Asp Leu Lys Tyr Asn  Tyr Lys Glu Glu Val  Lys
               2425                 2430                 2435
   aag aat att ctc  tta gaa gag aag gta  aaa aaa ctt tca gaa  caa       7697
   Lys Asn Ile Leu  Leu Glu Glu Lys Val  Lys Lys Leu Ser Glu  Gln
                2440                2445                2450
   ttg gga gtt gaa  tta act agc cct gtt  gct gct tct gaa gag  ttt      7742
   Leu Gly Val Glu  Leu Thr Ser Pro Val  Ala Ala Ser Glu Glu  Phe
                2455                2460                2465
   gaa gat gaa gaa  gaa agt cct gtt aat  ttc ccc att tac taa           7784
   Glu Asp Glu Glu  Glu Ser Pro Val Asn  Phe Pro Ile Tyr
                2470                2475
   aggtcaccta taaactttgt ttcatttaac tatttattaa ctttataagt taaatatact   7844
   tggaaataag cagttctccg aactgtagta tttccttctc actaccttgt acctttatac   7904
   ttagattgga attcttaata aataaaatta tatgaaattt tcaacttatt aaaaaaaaaa   7964
   aaaaaaaa                                                            7972
<210> 3
   <211> 2479
   <212> PRT
   <213> Homo sapiens
<400> 3
   Met Pro Pro Asn Ile Asn Trp Lys Glu Ile Met Lys Val Asp Pro Asp
   1               5                   10                  15
   Asp Leu Pro Arg Gln Glu Glu Leu Ala Asp Asn Leu Leu Ile Ser Leu
               20                  25                  30
   Ser Lys Val Glu Val Asn Glu Leu Lys Ser Glu Lys Gln Glu Asn Val
           35                  40                  45
   Ile His Leu Phe Arg Ile Thr Gln Ser Leu Met Lys Met Lys Ala Gln
       50                  55                  60
   Glu Val Glu Leu Ala Leu Glu Glu Val Glu Lys Ala Gly Glu Glu Gln
   65                  70                  75                  80
   Ala Lys Phe Glu Asn Gln Leu Lys Thr Lys Val Met Lys Leu Glu Asn
                   85                  90                  95
   Glu Leu Glu Met Ala Gln Gln Ser Ala Gly Gly Arg Asp Thr Arg Phe
               100                 105                 110
   Leu Arg Asn Glu Ile Cys Gln Leu Glu Lys Gln Leu Glu Gln Lys Asp
           115                 120                 125
   Arg Glu Leu Glu Asp Met Glu Lys Glu Leu Glu Lys Glu Lys Lys Val
       130                 135                 140
   Asn Glu Gln Leu Ala Leu Arg Asn Glu Glu Ala Glu Asn Glu Asn Ser
   145 150 155 160
   Lys Leu Arg Arg Glu Asn Lys Arg Leu Lys Lys Lys Asn Glu Gln Leu
   165 170 175
   Cys Gln Asp Ile Ile Asp Tyr Gln Lys Gln Ile Asp Ser Gln Lys Glu
   180 185 190
   Thr Leu Leu Ser Arg Arg Gly Glu Asp Ser Asp Tyr Arg Ser Gln Leu
   195 200 205
   Ser Lys Lys Asn Tyr Glu Leu Ile Gln Tyr Leu Asp Glu Ile Gln Thr
   210 215 220
   Leu Thr Glu Ala Asn Glu Lys Ile Glu Val Gln Asn Gln Glu Met Arg
   225 230 235 240
   Lys Asn Leu Glu Glu Ser Val Gln Glu Met Glu Lys Met Thr Asp Glu
   245 250 255
   Tyr Asn Arg Met Lys Ala Ile Val His Gln Thr Asp Asn Val Ile Asp
   260 265 270
   Gln Leu Lys Lys Glu Asn Asp His Tyr Gln Leu Gln Val Gln Glu Leu
   275 280 285
   Thr Asp Leu Leu Lys Ser Lys Asn Glu Glu Asp Asp Pro Ile Met Val
   290 295 300
   Ala Val Asn Ala Lys Val Glu Glu Trp Lys Leu Ile Leu Ser Ser Lys
   305 310 315 320
   Asp Asp Glu Ile Ile Glu Tyr Gln Gln Met Leu His Asn Leu Arg Glu
   325 330 335
   Lys Leu Lys Asn Ala Gln Leu Asp Ala Asp Lys Ser Asn Val Met Ala
   340 345 350
   Leu Gln Gln Gly Ile Gln Glu Arg Asp Ser Gln Ile Lys Met Leu Thr
   355 360 365
   Glu Gln Val Glu Gln Tyr Thr Lys Glu Met Glu Lys Asn Thr Cys Ile
   370 375 380
   Ile Glu Asp Leu Lys Asn Glu Leu Gln Arg Asn Lys Gly Ala Ser Thr
   385 390 395 400
   Leu Ser Gln Gln Thr His Met Lys Ile Gln Ser Thr Leu Asp Ile Leu
   405 410 415
   Lys Glu Lys Thr Lys Glu Ala Glu Arg Thr Ala Glu Leu Ala Glu Ala
   420 425 430
   Asp Ala Arg Glu Lys Asp Lys Glu Leu Val Glu Ala Leu Lys Arg Leu
   435 440 445
   Lys Asp Tyr Glu Ser Gly Val Tyr Gly Leu Glu Asp Ala Val Val Glu
   450 455 460
   Ile Lys Asn Cys Lys Asn Gln Ile Lys Ile Arg Asp Arg Glu Ile Glu
   465 470 475 480
   Ile Leu Thr Lys Glu Ile Asn Lys Leu Glu Leu Lys Ile Ser Asp Phe
   485 490 495
   Leu Asp Glu Asn Glu Ala Leu Arg Glu Arg Val Gly Leu Glu Pro Lys
   500 505 510
   Thr Met Ile Asp Leu Thr Glu Phe Arg Asn Ser Lys His Leu Lys Gln
   515 520 525
   Gln Gln Tyr Arg Ala Glu Asn Gln Ile Leu Leu Lys Glu Ile Glu Ser
   530 535 540
   Leu Glu Glu Glu Arg Leu Asp Leu Lys Lys Lys Ile Arg Gln Met Ala
   545 550 555 560
   Gln Glu Arg Gly Lys Arg Ser Ala Thr Ser Gly Leu Thr Thr Glu Asp
   565 570 575
   Leu Asn Leu Thr Glu Asn Ile Ser Gln Gly Asp Arg Ile Ser Glu Arg
   580 585 590
   Lys Leu Asp Leu Leu Ser Leu Lys Asn Met Ser Glu Ala Gln Ser Lys
   595 600 605
   Asn Glu Phe Leu Ser Arg Glu Leu Ile Glu Lys Glu Arg Asp Leu Glu
   610 615 620
   Arg Ser Arg Thr Val Ile Ala Lys Phe Gln Asn Lys Leu Lys Glu Leu
   625 630 635 640
   Val Glu Glu Asn Lys Gln Leu Glu Glu Gly Met Lys Glu Ile Leu Gln
   645 650 655
   Ala Ile Lys Glu Met Gln Lys Asp Pro Asp Val Lys Gly Gly Glu Thr
   660 665 670
   Ser Leu Ile Ile Pro Ser Leu Glu Arg Leu Val Asn Ala Ile Glu Ser
   675 680 685
   Lys Asn Ala Glu Gly Ile Phe Asp Ala Ser Leu His Leu Lys Ala Gln
   690 695 700
   Val Asp Gln Leu Thr Gly Arg Asn Glu Glu Leu Arg Gln Glu Leu Arg
   705 710 715 720
   Glu Ser Arg Lys Glu Ala Ile Asn Tyr Ser Gln Gln Leu Ala Lys Ala
   725 730 735
   Asn Leu Lys Ile Asp His Leu Glu Lys Glu Thr Ser Leu Leu Arg Gln
   740 745 750
   Ser Glu Gly Ser Asn Val Val Phe Lys Gly Ile Asp Leu Pro Asp Gly
   755 760 765
   Ile Ala Pro Ser Ser Ala Ser Ile Ile Asn Ser Gln Asn Glu Tyr Leu
   770 775 780
   Ile His Leu Leu Gln Glu Leu Glu Asn Lys Glu Lys Lys Leu Lys Asn
   785 790 795 800
   Leu Glu Asp Ser Leu Glu Asp Tyr Asn Arg Lys Phe Ala Val Ile Arg
   805 810 815
   His Gln Gln Ser Leu Leu Tyr Lys Glu Tyr Leu Ser Glu Lys Glu Thr
   820 825 830
   Trp Lys Thr Glu Ser Lys Thr Ile Lys Glu Glu Lys Arg Lys Leu Glu
   835 840 845
   Asp Gln Val Gln Gln Asp Ala Ile Lys Val Lys Glu Tyr Asn Asn Leu
   850 855 860
   Leu Asn Ala Leu Gln Met Asp Ser Asp Glu Met Lys Lys Ile Leu Ala
   865 870 875 880
   Glu Asn Ser Arg Lys Ile Thr Val Leu Gln Val Asn Glu Lys Ser Leu
   885 890 895
   Ile Arg Gln Tyr Thr Thr Leu Val Glu Leu Glu Arg Gln Leu Arg Lys
   900 905 910
   Glu Asn Glu Lys Gln Lys Asn Glu Leu Leu Ser Met Glu Ala Glu Val
   915 920 925
   Cys Glu Lys Ile Gly Cys Leu Gln Arg Phe Lys Glu Met Ala Ile Phe
   930 935 940
   Lys Ile Ala Ala Leu Gln Lys Val Val Asp Asn Ser Val Ser Leu Ser
   945 950 955 960
   Glu Leu Glu Leu Ala Asn Lys Gln Tyr Asn Glu Leu Thr Ala Lys Tyr
   965 970 975
   Arg Asp Ile Leu Gln Lys Asp Asn Met Leu Val Gln Arg Thr Ser Asn
   980 985 990
   Leu Glu His Leu Glu Cys Glu Asn Ile Ser Leu Lys Glu Gln Val Glu
   995 1000 1005
   Ser Ile Asn Lys Glu Leu Glu Ile Thr Lys Glu Lys Leu His Thr
   1010 1015 1020
   Ile Glu Gln Ala Trp Glu Gln Glu Thr Lys Leu Gly Asn Glu Ser
   1025 1030 1035
   Ser Met Asp Lys Ala Lys Lys Ser Ile Thr Asn Ser Asp Ile Val
   1040 1045 1050
   Ser Ile Ser Lys Lys Ile Thr Met Leu Glu Met Lys Glu Leu Asn
   1055 1060 1065
   Glu Arg Gln Arg Ala Glu His Cys Gln Lys Met Tyr Glu His Leu
   1070 1075 1080
   Arg Thr Ser Leu Lys Gln Met Glu Glu Arg Asn Phe Glu Leu Glu
   1085 1090 1095
   Thr Lys Phe Ala Glu Leu Thr Lys Ile Asn Leu Asp Ala Gln Lys
   1100 1105 1110
   Val Glu Gln Met Leu Arg Asp Glu Leu Ala Asp Ser Val Ser Lys
   1115 1120 1125
   Ala Val Ser Asp Ala Asp Arg Gln Arg Ile Leu Glu Leu Glu Lys
   1130 1135 1140
   Asn Glu Met Glu Leu Lys Val Glu Val Ser Lys Leu Arg Glu Ile
   1145 1150 1155
   Ser Asp Ile Ala Arg Arg Gln Val Glu Ile Leu Asn Ala Gln Gln
   1160 1165 1170
   Gln Ser Arg Asp Lys Glu Val Glu Ser Leu Arg Met Gln Leu Leu
   1175 1180 1185
   Asp Tyr Gln Ala Gln Ser Asp Glu Lys Ser Leu Ile Ala Lys Leu
   1190 1195 1200
   His Gln His Asn Val Ser Leu Gln Leu Ser Glu Ala Thr Ala Leu
   1205 1210 1215
   Gly Lys Leu Glu Ser Ile Thr Ser Lys Leu Gln Lys Met Glu Ala
   1220 1225 1230
   Tyr Asn Leu Arg Leu Glu Gln Lys Leu Asp Glu Lys Glu Gln Ala
   1235 1240 1245
   Leu Tyr Tyr Ala Arg Leu Glu Gly Arg Asn Arg Ala Lys His Leu
   1250 1255 1260
   Arg Gln Thr Ile Gln Ser Leu Arg Arg Gln Phe Ser Gly Ala Leu
   1265 1270 1275
   Pro Leu Ala Gln Gln Glu Lys Phe Ser Lys Thr Met Ile Gln Leu
   1280 1285 1290
   Gln Asn Asp Lys Leu Lys Ile Met Gln Glu Met Lys Asn Ser Gln
   1295 1300 1305
   Gln Glu His Arg Asn Met Glu Asn Lys Thr Leu Glu Met Glu Leu
   1310 1315 1320
   Lys Leu Lys Gly Leu Glu Glu Leu Ile Ser Thr Leu Lys Asp Thr
   1325 1330 1335
   Lys Gly Ala Gln Lys Val Ile Asn Trp His Met Lys Ile Glu Glu
   1340 1345 1350
   Leu Arg Leu Gln Glu Leu Lys Leu Asn Arg Glu Leu Val Lys Asp
   1355 1360 1365
   Lys Glu Glu Ile Lys Tyr Leu Asn Asn Ile Ile Ser Glu Tyr Glu
   1370 1375 1380
   Arg Thr Ile Ser Ser Leu Glu Glu Glu Ile Val Gln Gln Asn Lys
   1385 1390 1395
   Phe His Glu Glu Arg Gln Met Ala Trp Asp Gln Arg Glu Val Asp
   1400 1405 1410
   Leu Glu Arg Gln Leu Asp Ile Phe Asp Arg Gln Gln Asn Glu Ile
   1415 1420 1425
   Leu Asn Ala Ala Gln Lys Phe Glu Glu Ala Thr Gly Ser Ile Pro
   1430 1435 1440
   Asp Pro Ser Leu Pro Leu Pro Asn Gln Leu Glu Ile Ala Leu Arg
   1445 1450 1455
   Lys Ile Lys Glu Asn Ile Arg Ile Ile Leu Glu Thr Arg Ala Thr
   1460 1465 1470
   Cys Lys Ser Leu Glu Glu Lys Leu Lys Glu Lys Glu Ser Ala Leu
   1475 1480 1485
   Arg Leu Ala Glu Gln Asn Ile Leu Ser Arg Asp Lys Val Ile Asn
   1490 1495 1500
   Glu Leu Arg Leu Arg Leu Pro Ala Thr Ala Glu Arg Glu Lys Leu
   1505 1510 1515
   Ile Ala Glu Leu Gly Arg Lys Glu Met Glu Pro Lys Ser His His
   1520 1525 1530
   Thr Leu Lys Ile Ala His Gln Thr Ile Ala Asn Met Gln Ala Arg
   1535 1540 1545
   Leu Asn Gln Lys Glu Glu Val Leu Lys Lys Tyr Gln Arg Leu Leu
   1550 1555 1560
   Glu Lys Ala Arg Glu Glu Gln Arg Glu Ile Val Lys Lys His Glu
   1565 1570 1575
   Glu Asp Leu His Ile Leu His His Arg Leu Glu Leu Gln Ala Asp
   1580 1585 1590
   Ser Ser Leu Asn Lys Phe Lys Gln Thr Ala Trp Asp Leu Met Lys
   1595 1600 1605
   Gln Ser Pro Thr Pro Val Pro Thr Asn Lys His Phe Ile Arg Leu
   1610 1615 1620
   Ala Glu Met Glu Gln Thr Val Ala Glu Gln Asp Asp Ser Leu Ser
   1625 1630 1635
   Ser Leu Leu Val Lys Leu Lys Lys Val Ser Gln Asp Leu Glu Arg
   1640 1645 1650
   Gln Arg Glu Ile Thr Glu Leu Lys Val Lys Glu Phe Glu Asn Ile
   1655 1660 1665
   Lys Leu Gln Leu Gln Glu Asn His Glu Asp Glu Val Lys Lys Val
   1670 1675 1680
   Lys Ala Glu Val Glu Asp Leu Lys Tyr Leu Leu Asp Gln Ser Gln
   1685 1690 1695
   Lys Glu Ser Gln Cys Leu Lys Ser Glu Leu Gln Ala Gln Lys Glu
   1700 1705 1710
   Ala Asn Ser Arg Ala Pro Thr Thr Thr Met Arg Asn Leu Val Glu
   1715 1720 1725
   Arg Leu Lys Ser Gln Leu Ala Leu Lys Glu Lys Gln Gln Lys Ala
   1730 1735 1740
   Leu Ser Arg Ala Leu Leu Glu Leu Arg Ala Glu Met Thr Ala Ala
   1745 1750 1755
   Ala Glu Glu Arg Ile Ile Ser Ala Thr Ser Gln Lys Glu Ala His
   1760 1765 1770
   Leu Asn Val Gln Gln Ile Val Asp Arg His Thr Arg Glu Leu Lys
   1775 1780 1785
   Thr Gln Val Glu Asp Leu Asn Glu Asn Leu Leu Lys Leu Lys Glu
   1790 1795 1800
   Ala Leu Lys Thr Ser Lys Asn Arg Glu Asn Ser Leu Thr Asp Asn
   1805 1810 1815
   Leu Asn Asp Leu Asn Asn Glu Leu Gln Lys Lys Gln Lys Ala Tyr
   1820 1825 1830
   Asn Lys Ile Leu Arg Glu Lys Glu Glu Ile Asp Gln Glu Asn Asp
   1835 1840 1845
   Glu Leu Lys Arg Gln Ile Lys Arg Leu Thr Ser Gly Leu Gln Gly
   1850 1855 1860
   Lys Pro Leu Thr Asp Asn Lys Gln Ser Leu Ile Glu Glu Leu Gln
   1865 1870 1875
   Arg Lys Val Lys Lys Leu Glu Asn Gln Leu Glu Gly Lys Val Glu
   1880 1885 1890
   Glu Val Asp Leu Lys Pro Met Lys Glu Lys Asn Ala Lys Glu Glu
   1895 1900 1905
   Leu Ile Arg Trp Glu Glu Gly Lys Lys Trp Gln Ala Lys Ile Glu
   1910 1915 1920
   Gly Ile Arg Asn Lys Leu Lys Glu Lys Glu Gly Glu Val Phe Thr
   1925 1930 1935
   Leu Thr Lys Gln Leu Asn Thr Leu Lys Asp Leu Phe Ala Lys Ala
   1940 1945 1950
   Asp Lys Glu Lys Leu Thr Leu Gln Arg Lys Leu Lys Thr Thr Gly
   1955 1960 1965
   Met Thr Val Asp Gln Val Leu Gly Ile Arg Ala Leu Glu Ser Glu
   1970 1975 1980
   Lys Glu Leu Glu Glu Leu Lys Lys Arg Asn Leu Asp Leu Glu Asn
   1985 1990 1995
   Asp Ile Leu Tyr Met Arg Ala His Gln Ala Leu Pro Arg Asp Ser
   2000 2005 2010
   Val Val Glu Asp Leu His Leu Gln Asn Arg Tyr Leu Gln Glu Lys
   2015 2020 2025
   Leu His Ala Leu Glu Lys Gln Phe Ser Lys Asp Thr Tyr Ser Lys
   2030 2035 2040
   Pro Ser Ile Ser Gly Ile Glu Ser Asp Asp His Cys Gln Arg Glu
   2045 2050 2055
   Gln Glu Leu Gln Lys Glu Asn Leu Lys Leu Ser Ser Glu Asn Ile
   2060 2065 2070
   Glu Leu Lys Phe Gln Leu Glu Gln Ala Asn Lys Asp Leu Pro Arg
   2075 2080 2085
   Leu Lys Asn Gln Val Arg Asp Leu Lys Glu Met Cys Glu Phe Leu
   2090 2095 2100
   Lys Lys Glu Lys Ala Glu Val Gln Arg Lys Leu Gly His Val Arg
   2105 2110 2115
   Gly Ser Gly Arg Ser Gly Lys Thr Ile Pro Glu Leu Glu Lys Thr
   2120 2125 2130
   Ile Gly Leu Met Lys Lys Val Val Glu Lys Val Gln Arg Glu Asn
   2135 2140 2145
   Glu Gln Leu Lys Lys Ala Ser Gly Ile Leu Thr Ser Glu Lys Met
   2150 2155 2160
   Ala Asn Ile Glu Gln Glu Asn Glu Lys Leu Lys Ala Glu Leu Glu
   2165 2170 2175
   Lys Leu Lys Ala His Leu Gly His Gln Leu Ser Met His Tyr Glu
   2180 2185 2190
   Ser Lys Thr Lys Gly Thr Glu Lys Ile Ile Ala Glu Asn Glu Arg
   2195 2200 2205
   Leu Arg Lys Glu Leu Lys Lys Glu Thr Asp Ala Ala Glu Lys Leu
   2210 2215 2220
   Arg Ile Ala Lys Asn Asn Leu Glu Ile Leu Asn Glu Lys Met Thr
   2225 2230 2235
   Val Gln Leu Glu Glu Thr Gly Lys Arg Leu Gln Phe Ala Glu Ser
   2240 2245 2250
   Arg Gly Pro Gln Leu Glu Gly Ala Asp Ser Lys Ser Trp Lys Ser
   2255 2260 2265
   Ile Val Val Thr Arg Met Tyr Glu Thr Lys Leu Lys Glu Leu Glu
   2270 2275 2280
   Thr Asp Ile Ala Lys Lys Asn Gln Ser Ile Thr Asp Leu Lys Gln
   2285 2290 2295
   Leu Val Lys Glu Ala Thr Glu Arg Glu Gln Lys Val Asn Lys Tyr
   2300 2305 2310
   Asn Glu Asp Leu Glu Gln Gln Ile Lys Ile Leu Lys His Val Pro
   2315 2320 2325
   Glu Gly  Ala Glu Thr Glu Gln  Gly Leu Lys Arg Glu  Leu Gln Val
       2330                 2335                 2340
   Leu Arg  Leu Ala Asn His Gln  Leu Asp Lys Glu Lys  Ala Glu Leu
       2345                 2350                 2355
   Ile His  Gln Ile Glu Ala Asn  Lys Asp Gln Ser Gly  Ala Glu Ser
       2360                 2365                 2370
   Thr Ile  Pro Asp Ala Asp Gln  Leu Lys Glu Lys Ile  Lys Asp Leu
       2375                 2380                 2385
   Glu Thr  Gln Leu Lys Met Ser  Asp Leu Glu Lys Gln  His Leu Lys
       2390                 2395                 2400
   Glu Glu  Ile Lys Lys Leu Lys  Lys Glu Leu Glu Asn  Phe Asp Pro
       2405                 2410                 2415
   Ser Phe  Phe Glu Glu Ile Glu  Asp Leu Lys Tyr Asn  Tyr Lys Glu
       2420                 2425                 2430
   Glu Val  Lys Lys Asn Ile Leu  Leu Glu Glu Lys Val  Lys Lys Leu
       2435                 2440                 2445
   Ser Glu  Gln Leu Gly Val Glu  Leu Thr Ser Pro Val  Ala Ala Ser
       2450                 2455                 2460
   Glu Glu  Phe Glu Asp Glu Glu  Glu Ser Pro Val Asn  Phe Pro Ile
       2465                 2470                 2475
   Tyr
<210> 4
   <211> 128
   <212> DNA
   <213> Homo sapiens
<220>
   <221> misc_feature
   <223> 128 nucleotide aberrant CEO290 exon
<220>
   <221> misc_feature
   <223> 128 nucleotide aberrant CEP290 exon
<400> 4
   tagagatggg gtttcacctt gttagccagg atggtgtcga tctcctgaac tcgtgatcca 60
   cccgcctcgg cctcctaaag tgctgggatt acagatgtga gccaccgcac ctggccccag 120
   ttgtaatt 128
<210> 5
   <211> 997
   <212> PRT
   <213> Homo sapiens
<220>
   <221> misc-feature
   <222> (1)..(997)
   <223> Aberrant CEP290 polypeptide
<400> 5
   Met Pro Pro Asn Ile Asn Trp Lys Glu Ile Met Lys Val Asp Pro Asp
   1               5                   10                  15
   Asp Leu Pro Arg Gln Glu Glu Leu Ala Asp Asn Leu Leu Ile Ser Leu
               20                  25                  30
   Ser Lys Val Glu Val Asn Glu Leu Lys Ser Glu Lys Gln Glu Asn Val
           35                  40                  45
   Ile His Leu Phe Arg Ile Thr Gln Ser Leu Met Lys Met Lys Ala Gln
       50                  55                  60
   Glu Val Glu Leu Ala Leu Glu Glu Val Glu Lys Ala Gly Glu Glu Gln
   65                  70                  75                  80
   Ala Lys Phe Glu Asn Gln Leu Lys Thr Lys Val Met Lys Leu Glu Asn
                   85                  90                  95
   Glu Leu Glu Met Ala Gln Gln Ser Ala Gly Gly Arg Asp Thr Arg Phe
               100                 105                 110
   Leu Arg Asn Glu Ile Cys Gln Leu Glu Lys Gln Leu Glu Gln Lys Asp
           115                 120                 125
   Arg Glu Leu Glu Asp Met Glu Lys Glu Leu Glu Lys Glu Lys Lys Val
       130                 135                 140
   Asn Glu Gln Leu Ala Leu Arg Asn Glu Glu Ala Glu Asn Glu Asn Ser
   145                 150                 155                 160
   Lys Leu Arg Arg Glu Asn Lys Arg Leu Lys Lys Lys Asn Glu Gln Leu
                   165                 170                 175
   Cys Gln Asp Ile Ile Asp Tyr Gln Lys Gln Ile Asp Ser Gln Lys Glu
               180                 185                 190
   Thr Leu Leu Ser Arg Arg Gly Glu Asp Ser Asp Tyr Arg Ser Gln Leu
           195                 200                 205
   Ser Lys Lys Asn Tyr Glu Leu Ile Gln Tyr Leu Asp Glu Ile Gln Thr
       210                 215                 220
   Leu Thr Glu Ala Asn Glu Lys Ile Glu Val Gln Asn Gln Glu Met Arg
   225                 230                 235                 240
   Lys Asn Leu Glu Glu Ser Val Gln Glu Met Glu Lys Met Thr Asp Glu
                   245                 250                 255
   Tyr Asn Arg Met Lys Ala Ile Val His Gln Thr Asp Asn Val Ile Asp
               260                 265                 270
   Gln Leu Lys Lys Glu Asn Asp His Tyr Gln Leu Gln Val Gln Glu Leu
           275                 280                 285
   Thr Asp Leu Leu Lys Ser Lys Asn Glu Glu Asp Asp Pro Ile Met Val
       290                 295                 300
   Ala Val Asn Ala Lys Val Glu Glu Trp Lys Leu Ile Leu Ser Ser Lys
   305                 310                 315                 320
   Asp Asp Glu Ile Ile Glu Tyr Gln Gln Met Leu His Asn Leu Arg Glu
                   325                 330                 335
   Lys Leu Lys Asn Ala Gln Leu Asp Ala Asp Lys Ser Asn Val Met Ala
               340                 345                 350
   Leu Gln Gln Gly Ile Gln Glu Arg Asp Ser Gln Ile Lys Met Leu Thr
           355                 360                 365
   Glu Gln Val Glu Gln Tyr Thr Lys Glu Met Glu Lys Asn Thr Cys Ile
       370                 375                 380
   Ile Glu Asp Leu Lys Asn Glu Leu Gln Arg Asn Lys Gly Ala Ser Thr
   385                 390                 395                 400
   Leu Ser Gln Gln Thr His Met Lys Ile Gln Ser Thr Leu Asp Ile Leu
                   405                 410                 415
   Lys Glu Lys Thr Lys Glu Ala Glu Arg Thr Ala Glu Leu Ala Glu Ala
               420                 425                 430
   Asp Ala Arg Glu Lys Asp Lys Glu Leu Val Glu Ala Leu Lys Arg Leu
           435                 440                 445
   Lys Asp Tyr Glu Ser Gly Val Tyr Gly Leu Glu Asp Ala Val Val Glu
       450                 455                 460
   Ile Lys Asn Cys Lys Asn Gln Ile Lys Ile Arg Asp Arg Glu Ile Glu
   465                 470                 475                 480
   Ile Leu Thr Lys Glu Ile Asn Lys Leu Glu Leu Lys Ile Ser Asp Phe
                   485                 490                 495
   Leu Asp Glu Asn Glu Ala Leu Arg Glu Arg Val Gly Leu Glu Pro Lys
               500                 505                 510
   Thr Met Ile Asp Leu Thr Glu Phe Arg Asn Ser Lys His Leu Lys Gln
           515                 520                 525
   Gln Gln Tyr Arg Ala Glu Asn Gln Ile Leu Leu Lys Glu Ile Glu Ser
       530                 535                 540
   Leu Glu Glu Glu Arg Leu Asp Leu Lys Lys Lys Ile Arg Gln Met Ala
   545                 550                 555                 560
   Gln Glu Arg Gly Lys Arg Ser Ala Thr Ser Gly Leu Thr Thr Glu Asp
                   565                 570                 575
   Leu Asn Leu Thr Glu Asn Ile Ser Gln Gly Asp Arg Ile Ser Glu Arg
               580                 585                 590
   Lys Leu Asp Leu Leu Ser Leu Lys Asn Met Ser Glu Ala Gln Ser Lys
           595                 600                 605
   Asn Glu Phe Leu Ser Arg Glu Leu Ile Glu Lys Glu Arg Asp Leu Glu
       610                 615                 620
   Arg Ser Arg Thr Val Ile Ala Lys Phe Gln Asn Lys Leu Lys Glu Leu
   625                 630                 635                 640
   Val Glu Glu Asn Lys Gln Leu Glu Glu Gly Met Lys Glu Ile Leu Gln
                   645                 650                 655
   Ala Ile Lys Glu Met Gln Lys Asp Pro Asp Val Lys Gly Gly Glu Thr
               660                 665                 670
   Ser Leu Ile Ile Pro Ser Leu Glu Arg Leu Val Asn Ala Ile Glu Ser
           675                 680                 685
   Lys Asn Ala Glu Gly Ile Phe Asp Ala Ser Leu His Leu Lys Ala Gln
   690 695 700
   Val Asp Gln Leu Thr Gly Arg Asn Glu Glu Leu Arg Gln Glu Leu Arg
   705 710 715 720
   Glu Ser Arg Lys Glu Ala Ile Asn Tyr Ser Gln Gln Leu Ala Lys Ala
   725 730 735
   Asn Leu Lys Ile Asp His Leu Glu Lys Glu Thr Ser Leu Leu Arg Gln
   740 745 750
   Ser Glu Gly Ser Asn Val Val Phe Lys Gly Ile Asp Leu Pro Asp Gly
   755 760 765
   Ile Ala Pro Ser Ser Ala Ser Ile Ile Asn Ser Gln Asn Glu Tyr Leu
   770 775 780
   Ile His Leu Leu Gln Glu Leu Glu Asn Lys Glu Lys Lys Leu Lys Asn
   785 790 795 800
   Leu Glu Asp Ser Leu Glu Asp Tyr Asn Arg Lys Phe Ala Val Ile Arg
   805 810 815
   His Gln Gln Ser Leu Leu Tyr Lys Glu Tyr Leu Ser Glu Lys Glu Thr
   820 825 830
   Trp Lys Thr Glu Ser Lys Thr Ile Lys Glu Glu Lys Arg Lys Leu Glu
   835 840 845
   Asp Gln Val Gln Gln Asp Ala Ile Lys Val Lys Glu Tyr Asn Asn Leu
   850 855 860
   Leu Asn Ala Leu Gln Met Asp Ser Asp Glu Met Lys Lys Ile Leu Ala
   865 870 875 880
   Glu Asn Ser Arg Lys Ile Thr Val Leu Gln Val Asn Glu Lys Ser Leu
   885 890 895
   Ile Arg Gln Tyr Thr Thr Leu Val Glu Leu Glu Arg Gln Leu Arg Lys
   900 905 910
   Glu Asn Glu Lys Gln Lys Asn Glu Leu Leu Ser Met Glu Ala Glu Val
   915 920 925
   Cys Glu Lys Ile Gly Cys Leu Gln Arg Phe Lys Glu Met Ala Ile Phe
   930 935 940
   Lys Ile Ala Ala Leu Gln Lys Val Val Asp Asn Ser Val Ser Leu Ser
   945                 950                 955                 960
   Glu Leu Glu Leu Ala Asn Lys Gln Tyr Asn Glu Leu Thr Ala Lys Tyr
                   965                 970                 975
   Arg Asp Ile Leu Gln Lys Asp Asn Met Leu Val Gln Arg Thr Ser Asn
               980                 985                 990
   Leu Glu His Leu Glu
           995
<210> 6
   <211> 143
   <212> DNA
   <213> Homo sapiens
<220>
   <221> misc_feature
   <222> (1)..(143)
   <223> 143 nucleotide motif
<400> 6
   tagagatggg gtttcacctt gttagccagg atggtgtcga tctcctgaac tcgtgatcca      60
   cccgcctcgg cctcctaaag tgctgggatt acagatgtga gccaccgcac ctggccccag     120
   ttgtaattgt gaatatctca tac                                             143
<210> 7
   <211> 42
   <212> DNA
   <213> Homo sapiens
<220>
   <221> misc_feature
   <222> (1)..(42)
   <223> 42 nucleotide motif
<400> 7
   acagatgtga gccaccgcac ctggccccag ttgtaattgt ga 42
<210> 8
   <211> 24
   <212> DNA
   <213> Homo sapiens
<220>
   <221> misc_feature
   <222> (1)..(24)
   <223> 24 nucleotide motif
<400> 8
   ccaccgcacc tggccccagt tgta 24
<210> 9
   <211> 20
   <212> DNA
   <213> Artificial
<220>
   <223> AON-1
<400> 9
   taatcccagc actttaggag 20
<210> 10
   <211> 16
   <212> DNA
   <213> Artificial
<220>
   <223> AON-2
<400> 10
   gggccaggtg cggtgg 16
<210> 11
   <211> 17
   <212> DNA
   <213> Artificial
<220>
   <223> AON-3
<400> 11
   aactggggcc aggtgcg 17
<210> 12
   <211> 18
   <212> DNA
   <213> Artificial
<220>
   <223> AON-4
<400> 12
   tacaactggg gccaggtg 18
<210> 13
   <211> 20
   <212> DNA
   <213> Artificial
<220>
   <223> AON-5
<400> 13
   actcacaatt acaactgggg 20
<210> 14
   <211> 17
   <212> DNA
   <213> Artificial
<220>
   <223> SON-3
<400> 14
   cgcacctggc cccagtt 17
<210> 15
   <211> 23
   <212> DNA
   <213> Artificial
<220>
   <223> PCR primer
<400> 15
   tgctaagtac agggacatct tgc 23
<210> 16
   <211> 25
   <212> DNA
   <213> Artificial
<220>
   <223> PCR primer
<400> 16
   agactccact tgttctttta aggag 25
<210> 17
   <211> 69
   <212> DNA
   <213> Homo sapiens
<220>
   <221> misc_feature
   <222> (1)..(69)
   <223> 69 nucleotide motif
<400> 17
   gcctcctaaa gtgctgggat tacagatgtg agccaccgca cctggcccca gttgtaattg 60
   tgaatatct 69
<210> 18
   <211> 17
   <212> DNA
   <213> Artificial
<220>
   <223> Nkd AON
<400> 18
   aactggggcc aggtgcg 17
<210> 19
   <211> 17
   <212> DNA
   <213> Artificial
<220>
   <223> (AAV) AON1
<400> 19
   aactggggcc aggtgcg 17
<210> 20
   <211> 44
   <212> DNA
   <213> Artificial
<220>
   <223> (AAV) AON2
<400> 20
   ccatggtgcg gtggctcaca tcgtaatccc agcactttag gagg 44
<210> 21
   <211> 45
   <212> DNA
   <213> Artificial
<220>
   <223> (AAV) AON3
<400> 21
   gatactcaca attacaactg ggggtaatcc cagcacttta ggagg 45
<210> 22
   <211> 21
   <212> DNA
   <213> Artificial
<220>
   <223> (AAV) AON4
<400> 22
   ccaggtgcgg tggctcacat c 21
<210> 23
   <211> 44
   <212> DNA
   <213> Artificial
<220>
   <223> (AAV) AON5
<400> 23
   gatactcaca attacaactg gggccaggtg cggtggctca catc 44
<210> 24
   <211> 23
   <212> DNA
   <213> Artificial
<220>
   <223> (AAV) AON6
<400> 24
   gatactcaca attacaactg ggg 23
<210> 25
   <211> 17
   <212> DNA
   <213> Artificial
<220>
   <223> (pAAV) AON1
<400> 25
   cgcacctggc cccagtt 17
<210> 26
   <211> 44
   <212> DNA
   <213> Artificial
<220>
   <223> (pAAV) AON2
<400> 26
   gcctcctaaa gtgctgggat tacgatgtga gccaccgcac ctgg 44
<210> 27
   <211> 45
   <212> DNA
   <213> Artificial
<220>
   <223> (pAAV) AON3
<400> 27
   cctcctaaag tgctgggatt acccccagtt gtaattgtga atatc 45
<210> 28
   <211> 21
   <212> DNA
   <213> Artificial
<220>
   <223> (pAAV) AON4
<400> 28
   gatgtgagcc accgcacctg g 21
<210> 29
   <211> 44
   <212> DNA
   <213> Artificial
<220>
   <223> (pAAV) AON5
<400> 29
   gatgtgagcc accgcacctg gccccagttg taattgtgaa tatc 44
<210> 30
   <211> 23
   <212> DNA
   <213> Artificial
<220>
   <223> (pAAV) AON6
<400> 30
   ccccagttgt aattgtgaat atc 23
<210> 31
   <211> 23
   <212> DNA
   <213> Artificial
<220>
   <223> PCR primer
<400> 31
   tgctaagtac agggacatct tgc 23
<210> 32
   <211> 25
   <212> DNA
   <213> Artificial
<220>
   <223> PCR primer
<400> 32
   agactccact tgttctttta aggag 25
<210> 33
   <211> 29
   <212> DNA
   <213> Artificial
<220>
   <223> PCR primer
<400> 33
   gggtctagat aacaacatag gagctgtga 29
<210> 34
   <211> 28
   <212> DNA
   <213> Artificial
<220>
   <223> PCR primer
<400> 34
   aaagctagcc acaacgcgtt tcctagga 28
<210> 35
   <211> 18
   <212> DNA
   <213> Artificial
<220>
   <223> PCR primer
<400> 35
   atctgctgcg gcaagaac 18
<210> 36
   <211> 20
   <212> DNA
   <213> Artificial
<220>
   <223> PCR primer
<400> 36
   aggtgtaggg gatgggagac 20
<210> 37
   <211> 20
   <212> DNA
   <213> Artificial
<220>
   <223> PCR primer
<400> 37
   actgggacga catggagaag 20
<210> 38
   <211> 20
   <212> DNA
   <213> Artificial
<220>
   <223> PCR primer
<400> 38
   tctcagctgt ggtggtgaag 20
<210> 39
   <211> 42
   <212> DNA
   <213> Artificial
<220>
   <223> PCR primer
<400> 39
   aactggggcc aggtgcgaat ttttggagca ggttttctsg ac 42
<210> 40
   <211> 37
   <212> DNA
   <213> Artificial
<220>
   <223> PCR primer
<400> 40
   cgcacctggc cccagttttg cggaagtgcg tctgtag 37
<210> 41
   <211> 47
   <212> DNA
   <213> Artificial
<220>
   <223> PCR primer
<400> 41
   gattacgatg tgagccaccg cacctggttg cggaagtgcg tctgtag 47
<210> 42
   <211> 52
   <212> DNA
   <213> Artificial
<220>
   <223> PCR primer
<400> 42
   cacatcgtaa tcccagcact ttaggaggaa tttttggagc aggttttctg ac 52
<210> 43
   <211> 49
   <212> DNA
   <213> Artificial
<220>
   <223> PCR primer
<400> 43
   gattaccccc agttgtaatt gtgagtatct tgcggaagtg cgtctgtag 49
<210> 44
   <211> 51
   <212> DNA
   <213> Artificial
<220>
   <223> PCR primer
<400> 44
   tgggggtaat cccagcactt taggaggaat ttttggagca ggttttctga c 51
<210> 45
   <211> 41
   <212> DNA
   <213> Artificial
<220>
   <223> PCR primer
<400> 45
   gtgcggtggc tcacatcaat ttttggagca ggttttctga c 41
<210> 46
   <211> 37
   <212> DNA
   <213> Artificial
<220>
   <223> PCR primer
<400> 46
   tgagccaccg cacctggttg cggaagtgcg tctgtag 37
<210> 47
   <211> 48
   <212> DNA
   <213> Artificial
<220>
   <223> PCR primer
<400> 47
   cctggcccca gttgtaattg tgagtatctt gcggaagtgc gtctgtag 48
<210> 48
   <211> 50
   <212> DNA
   <213> Artificial
<220>
   <223> PCR primer
<400> 48
   tggggccagg tgcggtggct cacatcaatt tttggagcag gttttctgac 50
<210> 49
   <211> 41
   <212> DNA
   <213> Artificial
<220>
   <223> PCR primer
<400> 49
   ggggccaggt gcggtggaat ttttggagca ggttttctga c 41
<210> 50
   <211> 38
   <212> DNA
   <213> Artificial
<220>
   <223> PCR primer
<400> 50
   cacctggccc cagttgtatt gcggaagtgc gtctgtag 38